# EUROPEAN PATENT APPLICATION

(11) **EP 4 772 520 A1**
(43) Date of publication of application: **08.07.2026**
(21) Application number: 24860008.2
(22) Date of filing: 30.08.2024
(51) Int. Cl.: C07H 17/00, A61K 31/198, A61K 31/337, A61K 31/365, A61K 31/513, A61K 31/704, A61K 31/706, A61K 31/4745, A61K 31/7048, A61K 47/54, A61K 49/00, A61P 35/00, C07H 17/08, C07K 5/068

(54) **NOVEL PRODRUG-TYPE ANTICANCER AGENT TARGETING CANCER-SPECIFIC ENZYME ACTIVITY**

(30) Priority: 31.08.2023 JP 2023141180; 16.02.2024 JP 2024022432
(71) Applicant: The University of Tokyo, Bunkyo-ku, Tokyo 113-8654 (JP)
(72) Inventor: URANO, Yasuteru, Tokyo 113-8654 (JP); FUJITA, Kyohhei, Tokyo 113-8654 (JP); UTSUMI, Takenori, Tokyo 113-8654 (JP)
(74) Representative: Gille Hrabal Partnerschaftsgesellschaft mbB Patentanwälte
(86) International application number: PCT/JP2024/031416
(87) International publication number: WO 2025/047983

(57) **Abstract**

[Problem]

To provide a novel compound promising as a prodrug-type anticancer agent that exhibits a sufficient anticancer effect and is capable of reducing adverse effects.

[Solution]

A compound represented by general formula (I) or a pharmaceutically acceptable salt of the compound. Formula (I) : Y-T-(X₁)ₙ

## Description

### Technical Field

The present invention relates to a novel compound that is promising as a prodrug-type anticancer agent, and a prodrug-type anticancer agent using said compound.

### Background Art

SN38 is a compound that exhibits an antitumor effect by inhibiting topoisomerase I. SN38 is difficult to administer by itself due to its high hydrophobicity, and is currently mainly used as irinotecan (CPT-11), which is a compound having a hydrolysis site of carboxylesterase (CES) that adds water solubility to a molecular structure, in the field of cancer chemotherapy (Non Patent Literatures 1 and 2).

CPT-11 is a prodrug designed to release SN38 in response to degradation of a side chain by CES. However, CES is not an enzyme specific to a cancer site, and has high expression in various organs (particularly large intestine, liver, or the like). Therefore, SN38 cannot be released specifically to a cancer site, and organ damage is a problem. It has also been reported that CPT-11 itself pharmacologically inhibits acetylcholinesterase.

For this reason, in clinical practice, severe side effects such as cholinergic syndrome in the acute phase and severe diarrhea and neutropenia in the delayed phase have been reported.

Therefore, if a novel small molecule prodrug-type anticancer agent that releases SN38 targeting a cancerspecific enzyme instead of CES can be developed, it is considered that side effects can be further reduced and a treatment specific to a cancer site can be realized, but this has not yet been realized.

### Citation List

### Non Patent Literature

Non Patent Literature 1: Annals of Oncology 13: 1841-1851, 2002
Non Patent Literature 2: Int. J. Mol. Sci. 2020, 21, 4919

### Summary of Invention

### Technical Problem

An object of the present invention is to provide a novel compound which exhibits a sufficient anticancer effect and is promising as a prodrug-type anticancer agent capable of reducing side effects.

### Solution to Problem

In the laboratories of the present inventors, a group of fluorescent probes capable of detecting various glycosidase activities has been developed so far, and screening has been performed on cancer sites and normal sites of human clinical specimens using the group of fluorescent probes, thereby finding various cancer cell-specific glycosidase activities. In particular, the present inventors have found that a fluorescent probe targeting β-galactosidase or β-hexosaminidases is effective for specifically illuminating lung cancer, and have newly found that these target enzymes β-Galactosidase 1 (GLB1), β-Hexosaminidase A, and β-Hexosaminidase B are highly expressed at lung cancer sites.

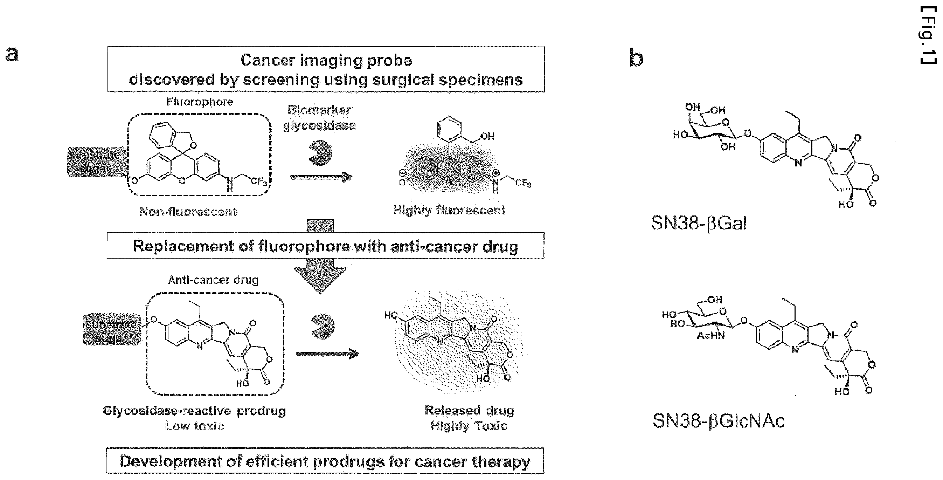

Based on these research results in this laboratory, the present inventors have conceived that it is possible to develop a novel prodrug-type anticancer agent based on an anticancer agent such as SN38 which selectively exhibits drug efficacy at a cancer site and has reduced side effects, by introducing a substrate site targeting cancer cell-specific enzyme activity which was found by screening of a fluorescent probe into a molecule of an anticancer agent such as SN38 (refer to Fig. 1), and as a result of intensive studies, have completed the present invention.

That is, the present invention relates to:
[1] A compound represented by the following General Formula (I) or a pharmaceutically acceptable salt thereof:

   Y-T-(X₁)ₙ (I)

   (wherein,
   Y is selected from

      -OL₁ (1)

      or

      -NH-L₂ (2),
   L₁ is a partial structure of saccharides,
   the partial structure of the saccharides is a structure obtained by removing one hydroxyl group from the saccharides, and
   L₂ is a monovalent substituent containing an amino acid residue or an oligopeptide residue;
   T is a linker or a bond;
   X₁ is at least one selected from the group consisting of
      (1) a binding residue of bioactive compound having one or more functional groups selected from the group consisting of an aliphatic hydroxyl group, an aromatic hydroxyl group, an amino group, and a carboxy group,
      (2) an antibody, and
      (3) a ligand selected from a small molecule, a peptide, an aptamer, or a hormone-drug conjugate,
   here, at least one of X₁ is a binding residue of the bioactive compound, and
   the bioactive compound is an anticancer agent; and
   n is an integer of 1 or more.)
[2] The compound or a pharmaceutically acceptable salt thereof according to [1], wherein Y is selected from any of the following groups. 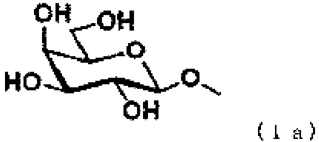 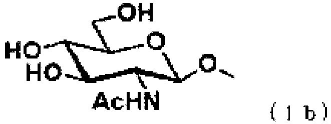 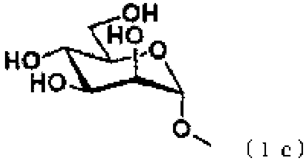 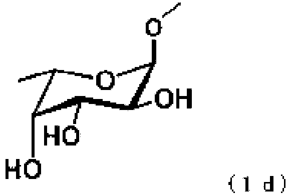 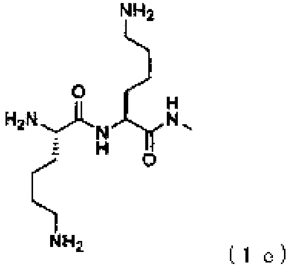 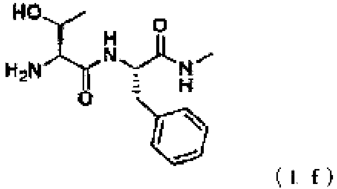 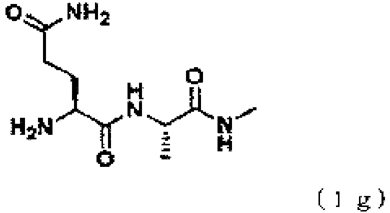
[3] The compound or a pharmaceutically acceptable salt thereof according to [1] or [2], wherein the linker is selected from the group consisting of the following structures (a) to (f), and a combination of two or more thereof. 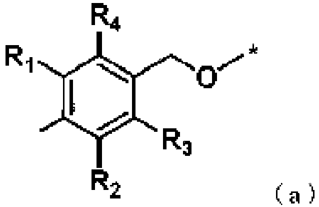 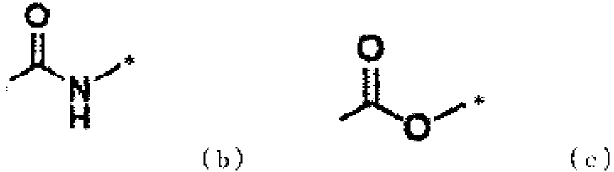 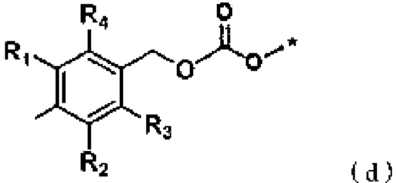 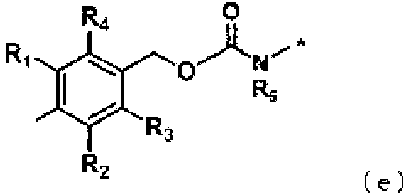 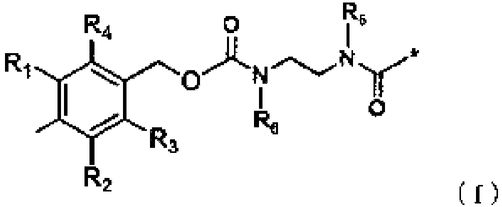
   (wherein, R₁ to R₄ may be the same or different and are each independently selected from the group consisting of hydrogen, a methyl group, a methoxy group, -COOMe, - NHMe, F, Cl, Br, and NO₂ (Me represents a methyl group); R₅ is hydrogen or an alkyl group having 1 to 3 carbon atoms;
   R₆, in each occurrence, is independently hydrogen or an alkyl group having 1 to 3 carbon atoms,
      (i) at least one of R₁ to R₄ may be a linking group selected from the following: 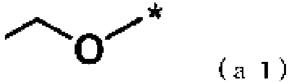 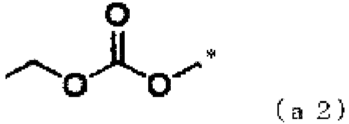 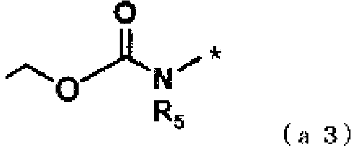 where, when at least one of R₁ to R₄ is a linking group, a linker is also bound to X₁ via the linking group, and
      (ii) at least one of R₁ to R₄ may be a linker having a binding site that is usable as a point of attachment to an antibody or ligand;
   * represents a direction of binding with X₁,
   here, when T has a structure in which T is a combination of two or more of Formulae (a) to (f), * for one or more linkers other than the linker that binds to X₁ may represent the direction of binding to the linker; and
   when at least one X₁ is an antibody or a ligand, the linker of Formulae (a) to (f), the linking group of a1 to a3 is capable of binding to a linker having a binding site that is usable as a binding point to the antibody or the ligand in the direction indicated by *.)
[4] The compound or the pharmaceutically acceptable salt thereof according to any one of [1] to [3], wherein the bioactive compound is selected from any one of the following. 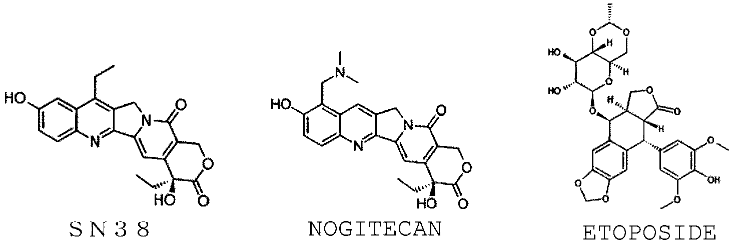 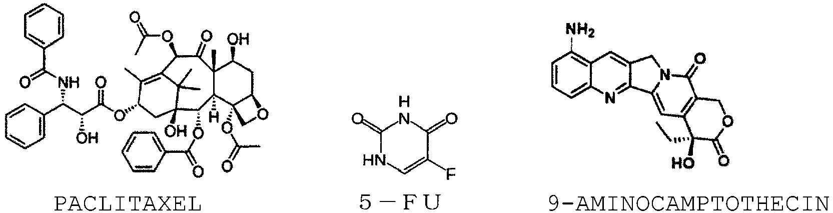 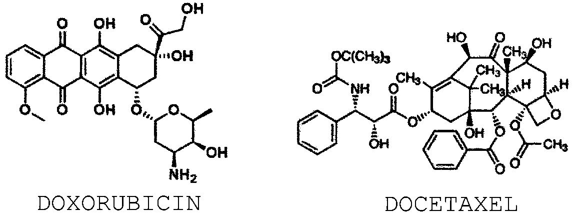 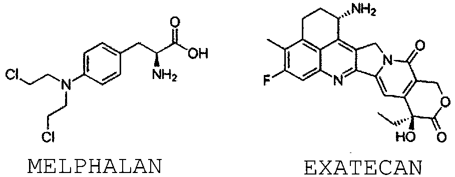 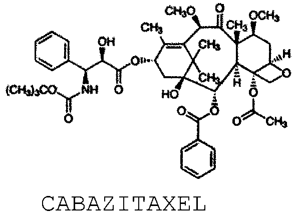 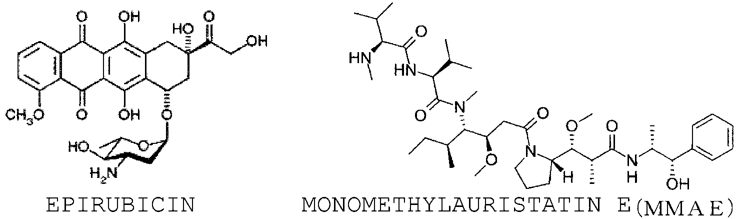 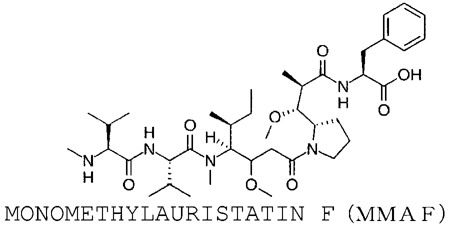
[5] The compound or the pharmaceutically acceptable salt thereof according to any one of [1] to [4], wherein the bioactive compound is SN38, nogitecan, exatecan, etoposide, doxolibicin, epirubicin, melphalan, MMAE, MMAF, paclitaxel, docetaxel, or cabazitaxel.
[6] The compound or the pharmaceutically acceptable salt thereof according to [4], wherein the bioactive compound is SN38, nogitecan, exatecan, etoposide, doxolibicin, epirubicin, melphalan, MMAE, MMAF, paclitaxel, docetaxel, or cabazitaxel.
[7] A prodrug-type anticancer agent comprising the compound or the pharmaceutically acceptable salt thereof according to any one of [1] to [6].
[8] A prodrug-type anticancer agent comprising the compound or the pharmaceutically acceptable salt thereof according to any one of [1] to [6], wherein the prodrug-type anticancer agent selectively acts on cells by cancer cell-specific enzyme activity.
[9] The prodrug-type anticancer agent according to [7] or [8], wherein the enzyme is peptidase or glycosidase.
[10] A fluorescent probe for detecting lung cancer, comprising a compound represented by the following General Formula (II) or a salt thereof: 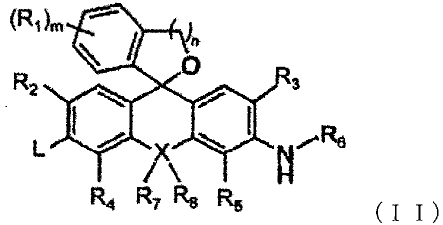 (wherein,
   R₁, if present, represents the same or different monovalent substituents present on a benzene ring;
   R₂ and R₃ each independently represent a hydrogen atom, an alkyl group having 1 to 6 carbon atoms, or a halogen atom;
   R₄ and R₅ each independently represent a hydrogen atom, an alkyl group having 1 to 6 carbon atoms, or a halogen atom;
   R₆ represents a hydrogen atom, an alkyl group having 1 to 5 carbon atoms, or a fluoroalkyl group having 1 to 5 carbon atoms;
   R₇ and R₈, if present, each independently represent an alkyl group or an aryl group having 1 to 6 carbon atoms,
   here, when X is an oxygen atom, R₇ and R₈ are not present;
   X represents an oxygen atom, a silicon atom, or a carbon atom;
   n is an integer of 1 to 3; and
   L is a group of the following Formula (1b).) 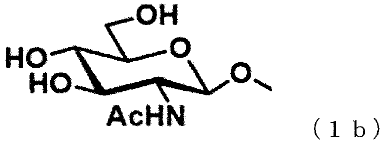
[11] The fluorescent probe according to [10], wherein X is an oxygen atom.
[12] The fluorescent probe according to [10] or [11], wherein R₆ is a fluoroalkyl group having 1 to 5 carbon atoms.
[13] A companion diagnostic agent for diagnosing a need for cancer treatment with a prodrug-type anticancer agent, comprising a compound represented by the following General Formula (I) or a pharmaceutically acceptable salt thereof, the companion diagnostic agent comprising: a compound represented by the following General Formula (II) or a salt thereof.

   Y-T-(X₁)ₙ (I)

   (wherein,
   Y is selected from any of the following groups; 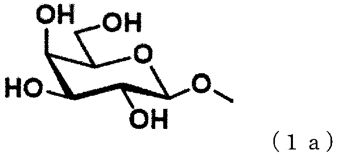 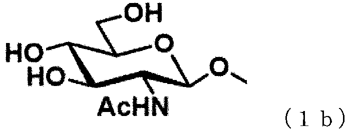 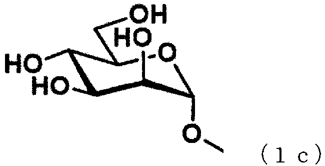 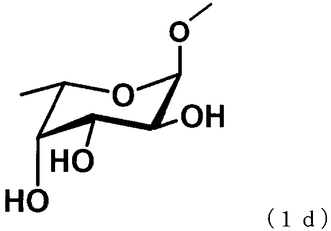 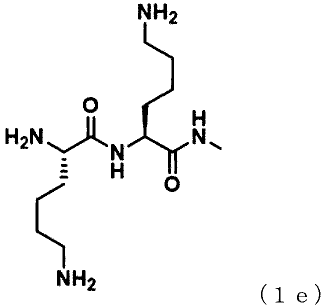 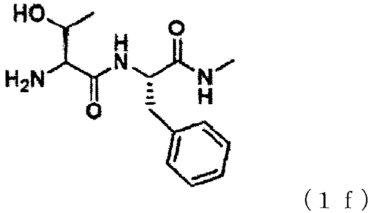 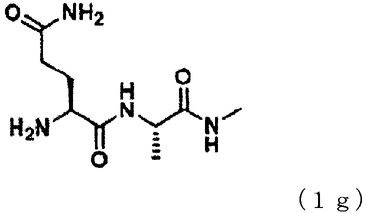
   T is a linker or a bond;
   X₁ is at least one selected from the group consisting of
      (1) a binding residue of bioactive compound having one or more functional groups selected from the group consisting of an aliphatic hydroxyl group, an aromatic hydroxyl group, an amino group, and a carboxy group,
      (2) an antibody, and
      (3) a ligand selected from a small molecule, a peptide, an aptamer, or a hormone-drug conjugate,
   here, at least one of X₁ is a binding residue of the bioactive compound, and
   the bioactive compound is an anticancer agent; and
   n is an integer of 1 or more.) 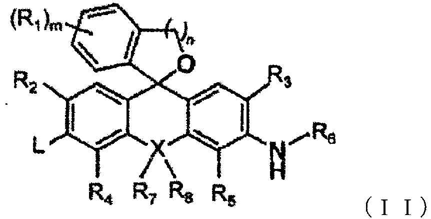
   (wherein,
   R₁, if present, represents the same or different monovalent substituents present on a benzene ring;
   R₂ and R₃ each independently represent a hydrogen atom, an alkyl group having 1 to 6 carbon atoms, or a halogen atom;
   R₄ and R₅ each independently represent a hydrogen atom, an alkyl group having 1 to 6 carbon atoms, or a halogen atom;
   R₆ represents a hydrogen atom, an alkyl group having 1 to 5 carbon atoms, or a fluoroalkyl group having 1 to 5 carbon atoms;
   R₇ and R₈, if present, each independently represent an alkyl group or an aryl group having 1 to 6 carbon atoms,
   here, when X is an oxygen atom, R₇ and R₈ are not present;
   X represents an oxygen atom, a silicon atom, or a carbon atom;
   n is an integer of 1 to 3; and
   L is selected from any of the following groups.) 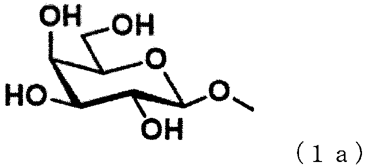 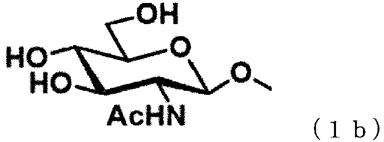 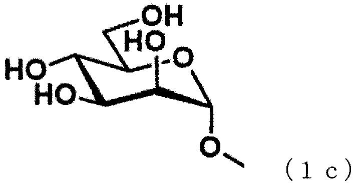 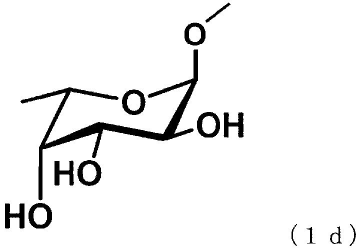 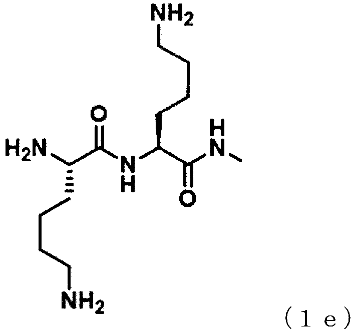 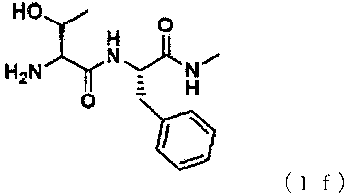 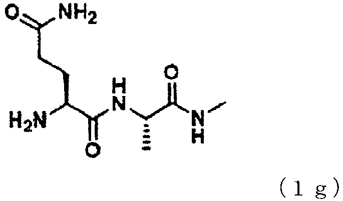
[14] A pharmaceutical composition for treating or preventing cancer, comprising: the compound or the pharmaceutically acceptable salt thereof according to any one of [1] to [6].
[15] A pharmaceutical composition for treating or preventing cancer, further comprising: another anticancer agent, in addition to the compound represented by General Formula (I) or the pharmaceutically acceptable salt thereof, a hydrate, or a solvate.
[16] The pharmaceutical composition according to [15], wherein the another anticancer agent is selected from methotrexate, doxorubicin, cisplatin, or an anti-PD-1 antibody.
[17] A method for treating a patient with cancer, the method comprising:
   a step of administering a therapeutically effective amount of the prodrug-type anticancer agent according to any one of [7] to [9] to the patient.
[18] The treatment method according to [17], wherein the prodrug-type anticancer agent acts cell selectively with an enzyme activity specific to cancer cells, and the cancer is characterized by a higher expression level of the enzyme.
[19] The treatment method according to [17] or [18], wherein the prodrug-type anticancer agent acts cell selectively with an enzyme activity specific to cancer tissue, and the cancer is characterized by a higher expression level of the enzyme.
[20] The treatment method according to [18], wherein the prodrug-type anticancer agent releases a molecule of the anticancer agent by cleaving an enzyme recognition site and/or a linker site due to an enzyme activity specific to cancer cells.
[21] The treatment method according to [19], wherein the prodrug-type anticancer agent releases a molecule of the anticancer agent by cleaving an enzyme recognition site and/or a linker site due to an enzyme activity specific to cancer tissue.
[22] The treatment method according to any one of [18] to [21], further comprising: identifying the level of enzyme activity of the enzyme in a patient's biological sample using a visualization agent, or one or more enzyme diagnostic agents selected from an enzyme activity reporter, and defining groups that may respond to treatment as a function of the enzyme activity.
[23] The treatment method of any one of [18] to [22], further comprising: stratifying a set of patients and defining a subset of patients that may respond to the prodrug-type anticancer agent by using the enzyme diagnostic agent.
[24] Use of an enzyme recognition site-linker binding site represented by General Formula (III),
   wherein the enzyme recognition site has an enzyme activity specific to a cancer cell, and
   the enzyme recognition site-linker binding site is used for activating an antibody or a ligand (a small molecule, a peptide, an aptamer, a hormone-drug conjugate, or the like) having a payload released by cleavage of the binding site by the enzyme activity specific to the enzyme recognition site-linker binding site.

      Y-T1 (III)

      (wherein,
      Y is selected from

         -OL₁ (1)

         or

         -NH-L₂ (2),
      L₁ is a partial structure of saccharides,
      here, the partial structure of the saccharides is a structure obtained by removing one hydroxyl group from the saccharide, and
      L₂ is a monovalent substituent containing an amino acid residue or an oligopeptide residue; and
      T1 is a linker.)
[25] Use of an enzyme recognition site-linker binding site represented by General Formula (III),
   wherein the enzyme recognition site has an enzyme activity specific to a cancer cell, and
   the enzyme recognition site-linker binding site is used for preventing prodrug activation or antibody or ligand binding until the enzyme recognition site-linker binding site is cleaved by enzyme activity specific to the binding site.
[26] Use of an enzyme recognition site-linker binding site represented by General Formula (III),
   wherein the enzyme recognition site has an enzyme activity specific to a cancer cell, and
   the enzyme recognition site-linker binding site is used for activating an antibody or a ligand having a payload that is released or activated by cleaving the enzyme recognition site-linker binding site by the enzyme activity specific to the binding site in a target cell or tissue overexpressing an oncogene.
[27] Use of an enzyme recognition site-linker binding site represented by General Formula (III):
   wherein the enzyme recognition site has an enzyme activity specific to a cancer cell, and
   the enzyme recognition site-linker binding site is used for targeting cancer.

### Advantageous Effects of Invention

According to the present invention, it is possible to provide a novel compound which exhibits a sufficient anticancer effect and is promising as a prodrug-type anticancer agent capable of reducing side effects.

The present invention can also provide a novel lung cancer detection probe. The lung cancer detection probe of the present invention is excellent in both sensitivity and specificity, and the present invention can provide a very effective lung cancer detection probe.

### Brief Description of Drawings

Fig. 1 illustrates a concept (a) of an SN38 type prodrug exhibiting drug efficacy by cancer cell-specific enzyme activity developed in the present invention, and a specific molecular structure (b) thereof.
Fig. 2 illustrates structures of 12 glycosidasereactive fluorescent probes subjected to screening for glycosidase activity detection fluorescent probes in Example 1.
Fig. 3 illustrates results of screening for glycosidase activity detection fluorescent probes in human lung cancer specimens.
Fig. 4 illustrates results of an identification test of a target enzyme of HMRef-βGlcNAc.
Fig. 5 illustrates results of fluorescence imaging of human lung cancer by HMRef-βGlcNAc.
Fig. 6 illustrates results of imaging of lung cancer (mouse model) by intraperitoneal administration and systemic administration of HMRef-βGlcNAc.
Fig. 7 illustrates results of the cytotoxicity test by MTT assay.
Fig. 8 illustrates suppression of cell proliferation of each cancer cell by SN38-βGal.
Fig. 9 illustrates suppression of cell proliferation of each cancer cell by SN38-βGlcNAc.
Fig. 10 illustrates suppression of cell proliferation of each cancer cell by SN38.
Fig. 11 illustrates suppression of cell proliferation of each cancer cell by CPT-11.
Fig. 12 illustrates results of confirming the reactivity of SN38-βGal with GLB1 by LC-MS analysis.
Fig. 13 illustrates results of pharmacokinetics in 30 mg/kg intraperitoneal administration (i.p.) of SN38-βGal.
Fig. 14 illustrates transition of body weight in repeated administration of a novel prodrug and CPT-11.
Fig. 15 illustrates a comparison of weights of liver, kidney, and spleen after the test.
Fig. 16 illustrates a tissue image of the small intestine after repeated drug administration (Scale bar=100 µm).
Fig. 17 illustrates a tissue image of the large intestine after repeated drug administration (Scale bar=100 µm).
Fig. 18 illustrates a tissue image of the kidney after repeated drug administration (Scale bar=100 µm).
Fig. 19 illustrates a tissue image of the liver after repeated drug administration (Scale bar=100 µm).
Fig. 20 illustrates a tissue image of the spleen after repeated drug administration (Scale bar=100 µm).
Fig. 21 illustrates the evaluation results of the AChE inhibitory activity of SN38-βGal, SN38-βGlcNAc, and SN38-αMan.
Fig. 22 illustrates results of momentum evaluation after drug administration.
Fig. 23 illustrates the results of therapeutic evaluation of A549 peritoneal dissemination model mice using SN38-βGal.
Fig. 24 illustrates the results of therapeutic evaluation (therapeutic experiment 1) of the A549 lung cancer orthotopic transplantation model by SN38-βGal, SN38-βGlcNAc, and CPT-11.
Fig. 25 illustrates the results of therapeutic evaluation (therapeutic experiment 2) of the A549 lung cancer orthotopic transplantation model by SN38-βGal and SN38-βGlcNAc.
Fig. 26 illustrates analysis results of pharmacokinetic parameters in a common marmoset.
Fig. 27 illustrates results of the cytotoxicity test by MTT assay.
Fig. 28 illustrates a tumor proliferation suppression effect of SN38-βGlcNAc in H226 subcutaneous tumor model mice.
Fig. 29 illustrates the results of a proliferation suppression test in lung squamous cell carcinoma organoids.
Fig. 30 illustrates a tumor proliferation suppression effect of SN38-βGlcNAc in PDX model mice.
Fig. 31 illustrates the results of a therapeutic experiment in a small cell lung cancer orthotopic transplantation model.
Fig. 32 illustrates an example of fluorescence detection of human small cell lung cancer tissue by HMRef-βGlcNAc.
Fig. 33 illustrates the results of a therapeutic experiment (first round) of SKOV3 ovarian cancer peritoneal dissemination model by SN38-βGal.
Fig. 34 illustrates the results of a therapeutic experiment (second round) of SKOV3 ovarian cancer peritoneal dissemination model by SN38-βGal.
Fig. 35 illustrates the results of a combined experiment of an anti-PD-1 antibody and SN38-βGlcNAc.
Fig. 36 illustrates the results of a therapeutic experiment (first round) of SKOV3 ovarian cancer peritoneal dissemination model by KK-SN38.
Fig. 37 illustrates the results of a therapeutic experiment (second round) of SKOV3 ovarian cancer peritoneal dissemination model by KK-SN38.
Fig. 38 illustrates cytotoxicity test results using JFCR39 cell panel: GI₅₀ values in each cancer cell.
Fig. 39 illustrates cytotoxicity test results using JFCR39 cell panel: suppression of cell proliferation of each cancer cell in SN38-αMan.
Fig. 40 illustrates cytotoxicity test results using JFCR39 cell panel: suppression of cell proliferation of each cancer cell in KK-SN38.
Fig. 41 illustrates cytotoxicity test results using JFCR39 cell panel: suppression of cell proliferation of each cancer cell in QA-SN38.
Fig. 42 illustrates cytotoxicity test results using JFCR39 cell panel: suppression of cell proliferation of each cancer cell in TF-SN38.
Fig. 43 illustrates cytotoxicity test results using JFCR39 cell panel: suppression of cell proliferation of each cancer cell in NGT-βGlcNAc.
Fig. 44 illustrates cytotoxicity test results using JFCR39 cell panel: suppression of cell proliferation of each cancer cell in EXT-βGlcNAc.
Fig. 45 illustrates cytotoxicity test results using JFCR39 cell panel: suppression of cell proliferation of each cancer cell in ETP-βGlcNAc.
Fig. 46 illustrates cytotoxicity test results using JFCR39 cell panel: suppression of cell proliferation of each cancer cell in DOX-βGlcNAc.
Fig. 47 illustrates cytotoxicity test results using JFCR39 cell panel: suppression of cell proliferation of each cancer cell in EPI-βGlcNAc.
Fig. 48 illustrates cytotoxicity test results using JFCR39 cell panel: suppression of cell proliferation of each cancer cell in Melphalan-βGlcNAc.
Fig. 49 illustrates cytotoxicity test results using JFCR39 cell panel: suppression of cell proliferation of each cancer cell in MMAE-βGlcNAc.
Fig. 50 illustrates cytotoxicity test results using JFCR39 cell panel: suppression of cell proliferation of each cancer cell in MMAF-βGlcNAc.
Fig. 51 illustrates cytotoxicity test results using JFCR39 cell panel: suppression of cell proliferation of each cancer cell in PTX-βGlcNAc.
Fig. 52 illustrates cytotoxicity test results using JFCR39 cell panel: suppression of cell proliferation of each cancer cell in DTX-βGlcNAc.
Fig. 53 illustrates cytotoxicity test results using JFCR39 cell panel: suppression of cell proliferation of each cancer cell in CB2-βGlcNAc.
Fig. 54-a illustrates a verification result of a therapeutic effect using a peritoneal dissemination model using DOX-βGlcNAc.

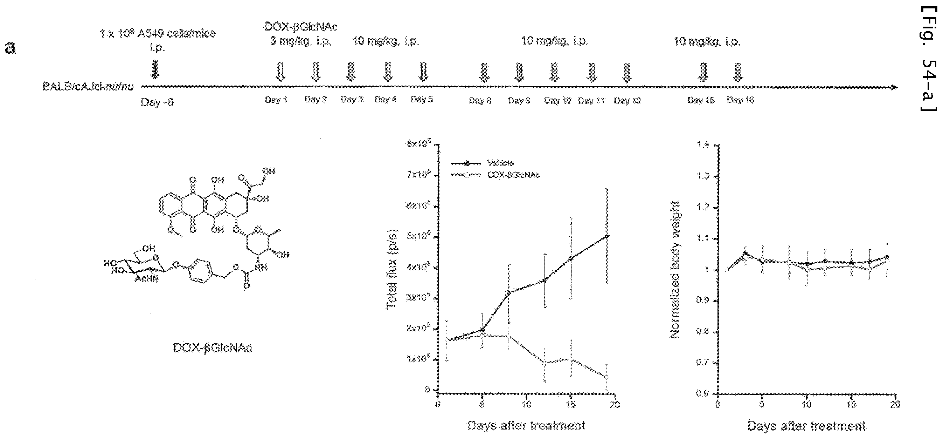

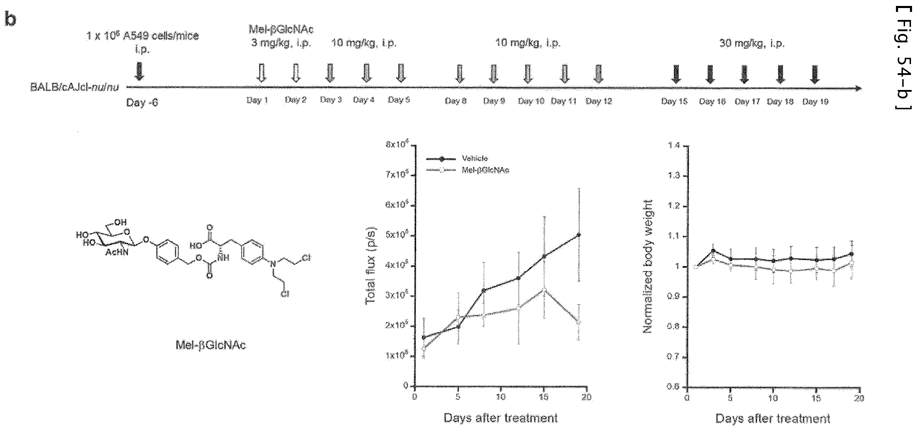

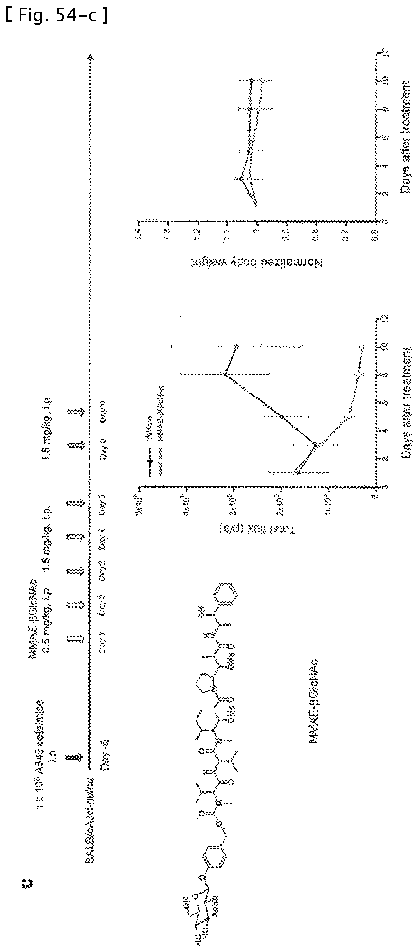

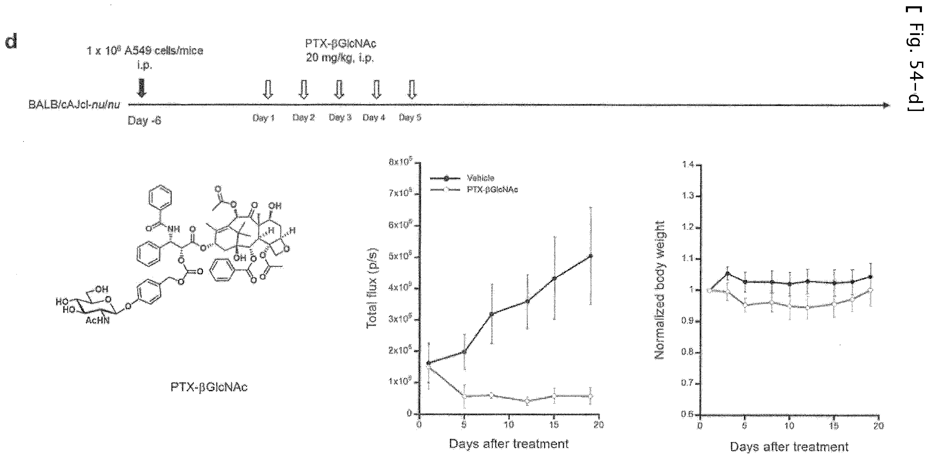

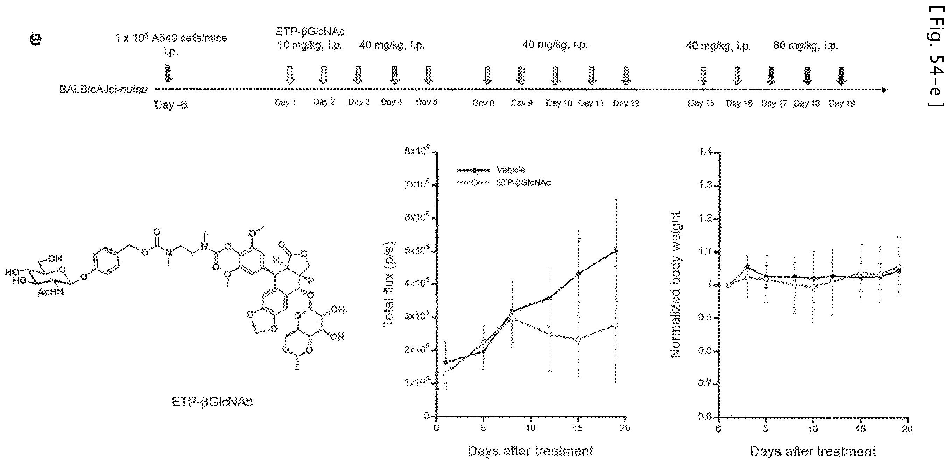

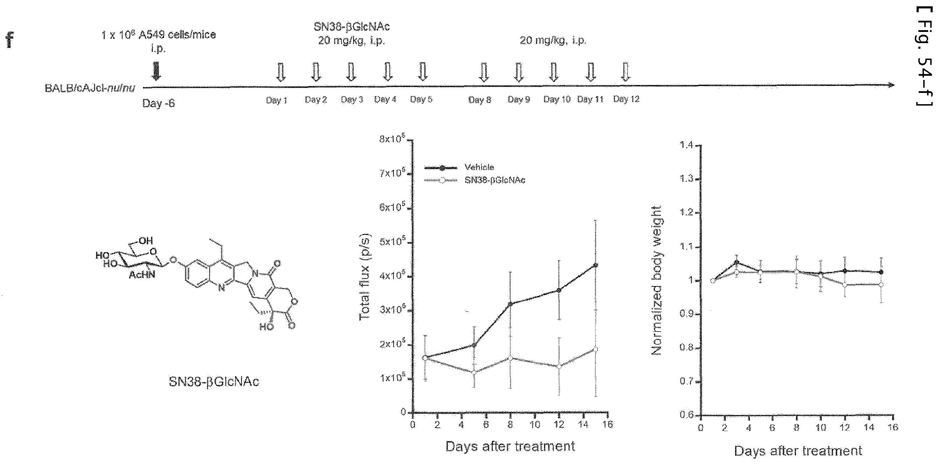

Fig. 54-b illustrates a verification result of a therapeutic effect using a peritoneal dissemination model using Mel-βGlcNAc.
Fig. 54-c illustrates a verification result of a therapeutic effect using a peritoneal dissemination model using MMAE-βGlcNAc.
Fig. 54-d illustrates a verification result of a therapeutic effect using a peritoneal dissemination model using PTX-βGlcNAc.
Fig. 54-e illustrates a verification result of a therapeutic effect using a peritoneal dissemination model using ETP-βGlcNAc.
Fig. 54-f illustrates a verification result of a therapeutic effect using a peritoneal dissemination model using SN38-βGlcNAc.

### Description of Embodiments

In the present specification, an "alkyl group" or an alkyl moiety of a substituent (for example, an alkoxy group) including an alkyl moiety means, unless otherwise specified, an alkyl group having, for example, 1 to 6 carbon atoms, preferably 1 to 4 carbon atoms, more preferably about 1 to 3 carbon atoms and consisting of a straight chain, branched chain, cyclic, or a combination thereof. More specific examples of the alkyl group include a methyl group, an ethyl group, a n-propyl group, an isopropyl group, a cyclopropyl group, a n-butyl group, a sec-butyl group, an isobutyl group, a tert-butyl group, a cyclopropylmethyl group, a n-pentyl group, and a n-hexyl group.

In the present specification, the "halogen atom" may be any of a fluorine atom, a chlorine atom, a bromine atom, and an iodine atom, and is preferably a fluorine atom, a chlorine atom, or a bromine atom.

### 1. Compound represented by General Formula (I) or salt thereof:

One embodiment of the present invention is a compound represented by the following General Formula (I) or a pharmaceutically acceptable salt thereof (hereinafter, also referred to as "compound of the present invention").

Y-T-X₁ (I)

(wherein,
Y is selected from

   -OL₁ (1)

   or

   -NH-L₂ (2),
L₁ is a partial structure of saccharides,
the partial structure of the saccharides is a structure obtained by removing one hydroxyl group from the saccharides, and
L₂ is a monovalent substituent containing an amino acid residue or an oligopeptide residue;
T is a linker or a bond;
X₁ is at least one selected from the group consisting of
   (1) a binding residue of bioactive compound having one or more functional groups selected from the group consisting of an aliphatic hydroxyl group, an aromatic hydroxyl group, an amino group, and a carboxy group,
   (2) an antibody, and
   (3) a ligand selected from a small molecule, a peptide, an aptamer, or a hormone-drug conjugate,
here, at least one of X₁ is a binding residue of the bioactive compound, and
the bioactive compound is an anticancer agent; and
n is an integer of 1 or more.)

That is, the present inventors have conceived that it is possible to develop a novel prodrug-type anticancer agent based on an anticancer agent such as SN38 which selectively exhibits drug efficacy at a cancer site and has reduced side effects by introducing a substrate site targeting cancer cell-specific enzyme activity which was found by screening of a fluorescent probe into molecules of various anticancer agents including SN38 (refer to Fig. 1), and incentive studies about it was performed. As a result, it has been found that the compound represented by General Formula (I) above is cleaved at an enzyme recognition site and/or a linker site by an enzyme activity specific to a cancer cell, thereby releasing a molecule of an anticancer agent and selectively exhibiting a drug efficacy at a cancer site, and is useful for a novel prodrug-type anticancer agent.

Here, in General Formula (I), Y represents an enzyme recognition site, and a part thereof is cleaved by a cancer cell-specific enzyme activity to release a molecule of an anticancer agent.

As described above, SN38 is mainly used as irinotecan (CPT-11), which is a compound to which a hydrolysis site of carboxylesterase (CES) is imparted, and CPT-11 is a prodrug designed to release SN38 in response to degradation of a side chain by CES. However, CES is not an enzyme specific to a cancer site, and has high expression in various organs (particularly large intestine, liver, or the like). Therefore, SN38 cannot be released specifically to a cancer site, and organ damage is a problem. In order to solve such a problem, it is general to deal with the problem by diverting a known prodrug substrate used for other anticancer agents.

On the other hand, in the present invention, a substrate site targeting a cancer cell-specific enzyme activity found by screening of a fluorescent probe is used as a substrate of a prodrug-type anticancer agent, and according to the understanding of the present inventors, the present invention is the first one to develop a prodrug-type anticancer agent by such a technique.

Y can be selected according to the type of the target enzyme. When a cancer biomarker enzyme that is the target enzyme of the prodrug-type anticancer agent is glycosidase, Y is selected from a group derived from saccharide, and when the target enzyme is peptidase, Y is selected from a group derived from an amino acid and a group containing an amino acid.

In General Formula (I), Y is selected from

-OL₁ (1)

or

-NH-L₂ (2).

In Formula (1), L₁ represents a partial structure of saccharides. The partial structure of the saccharides of L₁ constitutes saccharides, a part of the saccharides, together with O to which L₁ is bound.

The partial structure of the saccharides is a structure in which one hydroxyl group is removed from the saccharides.

Examples of the saccharides include β-D-glucose, β-D-galactose, β-L-galactose, β-D-xylose, α-D-mannose, β-D-fucose, α-L-fucose, β-L-fucose, β-L-fucose, β-D-arabinose, β-L-arabinose, β-D-N-acetylglucosamine, and β-D-N-acetylgalactosamine, and β-D-galactose, β-D-N-acetylgalactosamine, α-D-mannose, and α-L-fucose are preferable.

That is, the present inventors have performed screening on glycosidase activity detection fluorescent probes in human lung cancer specimens in conducting the study of the present invention. As a result, the present inventors have found that when specific saccharides selected from β-D-galactose (β-D-Gal), β-D-N-acetylgalactosamine (β-D-GlcNAc), α-D-mannose (α-D-Man), and α-L-fucose (α-L-Fuc) are introduced into a molecule of an anticancer agent as a substrate site that targets the cancer cell-specific enzyme activity confirmed to be effective as a lung cancer detection probe, an excellent prodrug anticancer agent can be provided.

In Formula (2): -NH-L₂, L₂ is a monovalent substituent containing an amino acid residue or an oligopeptide residue.

In the present specification, as the "amino acid", any compound can be used as long as it is a compound having both an amino group and a carboxyl group, and includes natural and non-natural compounds. The amino acid may be any of a neutral amino acid, a basic amino acid, and an acidic amino acid. In addition to an amino acid that itself functions as a transmitter such as a neurotransmitter, an amino acid that is a constituent of a polypeptide compound such as a physiologically active peptide (contains a dipeptide, a tripeptide, a tetrapeptide, and an oligopeptide) or a protein can be used. For example, the amino acid may be an α-amino acid, a β-amino acid, a γ-amino acid, or the like. As the amino acid, it is preferable to use an optically active amino acid. For example, as the α-amino acid, either D- or L-amino acid may be used, but it may be preferable to select an optically active amino acid that functions in a living body.

In the present specification, the "amino acid residue" refers to a structure corresponding to the remaining partial structure obtained by removing a hydroxyl group from a carboxyl group of an amino acid.

The amino acid residue includes an α-amino acid residue, a β-amino acid residue, and a γ-amino acid residue. Preferable examples of the amino acid residue include a γ-glutamyl group of a GGT substrate, a dipeptide consisting of a dipeptide (amino acid-proline) of a DPP4 substrate, and a dipeptide such as KK, QA or TF.

As used herein, the term "peptide" refers to a structure in which two or more amino acids are linked by a peptide bond.

The "peptide residue" refers to a structure corresponding to the remaining partial structure obtained by removing a hydroxyl group from the carboxyl group of the C-terminal amino acid constituting the peptide.

Preferable examples of the peptide include a dipeptide of the DPP4 substrate (dipeptide consisting of amino acid-proline; here, the amino acid may be, for example, glycine, glutamic acid, or proline), KK which is a target sequence of PSA, and the like.

N-terminus of the monovalent substituent including an amino acid residue or an oligopeptide residue may be protected, and examples of a protecting group include an acetyl group, a glutaryl group, a succinyl group, a tertbutoxycarbonyl group, a benzyloxycarbonyl group, and the like, but substituents other than these may be used.

In the present invention, Y has a structure selected from the following.

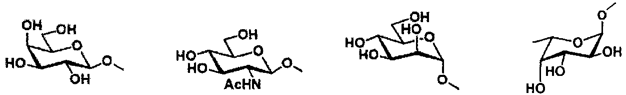

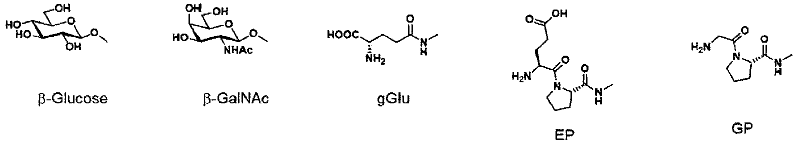

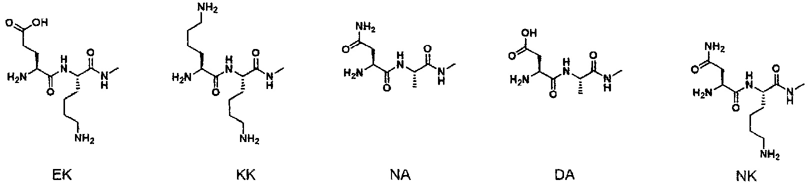

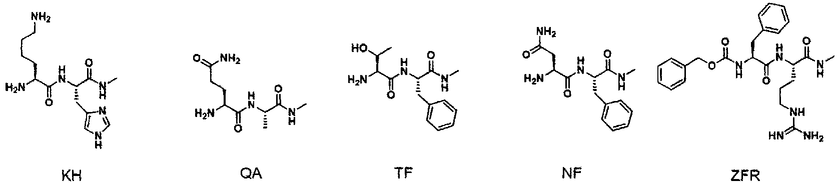

In addition, in one preferred aspect of the present invention, Y has a structure selected from the following.

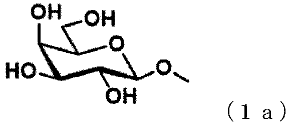

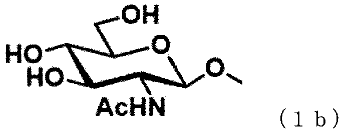

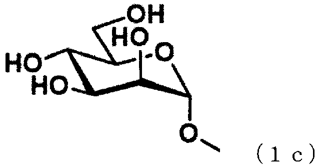

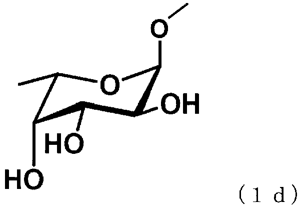

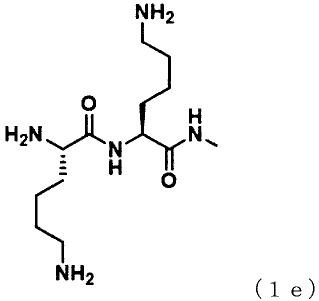

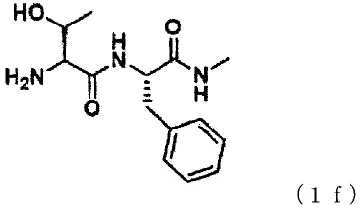

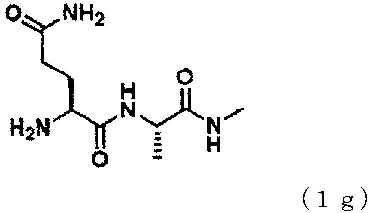

In General Formula (1), T represents a linker or a bond. Here, the case where T is a bond refers to a case where Y is bound to X₁ without interposing a linker.

The linker is a site in which a divalent group linking X₁ and Y described later or two or more divalent groups are linked, or a combination thereof.

As the linker, various linkers can be used, but in the present invention, a linker that is detached after the enzymatic reaction (so-called self-cleaving linker) is preferable, and as such a linker, a benzyl group-type linker, a carbamate group-type linker, a carbonate group-type linker, and a combination of two or more thereof are preferable.

As the benzyl group-type linker, in a case where Y is -OL₁, together with an oxygen atom thereof the benzyl group-type linker can constitute -O-Ph-CH₂-O-*, and in a case where Y is -NH-L₂, together with a nitrogen atom thereof the benzyl group-type linker can constitute -NH-Ph-CH₂-O-*. Here, Ph represents a substituted or unsubstituted benzene ring, and * represents a direction of binding to X₁.

As the carbamate group-type linker, in the case where Y is -OL₁, together with an oxygen atom thereof the carbamate group-type linker can constitute -O-CO-NH-* together with its oxygen atom.

As the carbonate group-type linker, in the case where Y is -OL₁, together with an oxygen atom thereof the carbonate group-type linker can constitute -O-CO-O-* together with its oxygen atom.

In addition, a linker in which a benzyl group-type and a carbamate group-type are combined or a linker in which a benzyl group-type and a carbonate group-type are combined can also be used.

For example, in the case where Y is -OL₁, together with an oxygen atom thereof it can constitute -O-Ph-CH₂-O-CO-NH-*, and in the case where Y is -NH-L₂, together with a nitrogen atom thereof it can constitute -NH-Ph-CH₂-O-CO-NH-^{*}.

In addition, for example, in the case where Y is - OL₁, together with an oxygen atom thereof it can constitute -O-Ph-CH₂-O-CO-O-^{*}, and in the case where Y is -NH-L₂, together with a nitrogen atom thereof it can constitute - NH-Ph-CH₂-O-CO-O-^{*}.

Here, Ph represents a substituted or unsubstituted benzene ring, and * represents a direction of binding to X₁.

The linker that can be used in the present invention also includes a linker that can be bound to two or more X₁.

In one preferred aspect of the present invention, the linker represented by T in General Formula (1) is selected from the group consisting of the following structures (a) to (f) and combinations of two or more thereof.

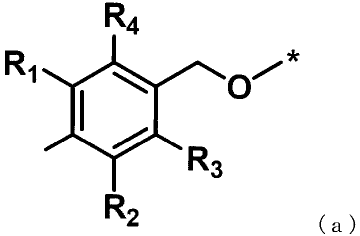

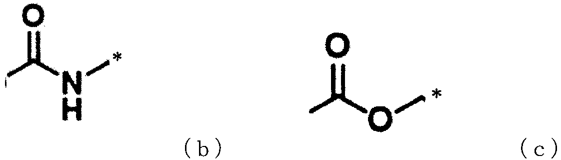

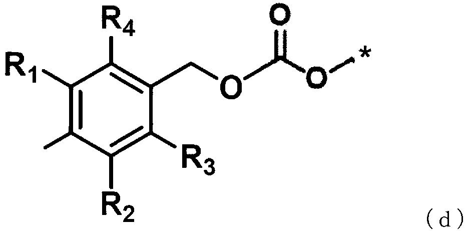

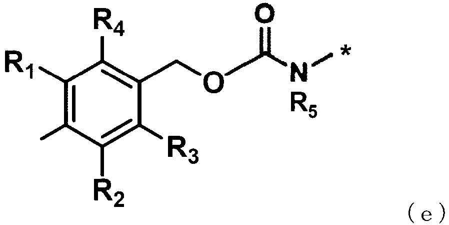

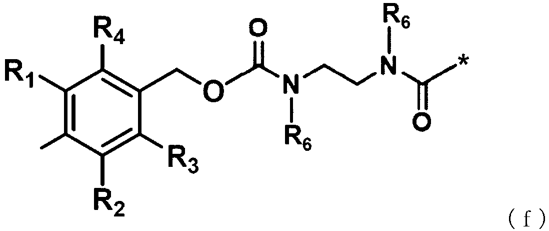

In the above Formulae (a) and (d) to (f), R₁ to R₄ may be the same or different, and are each independently selected from the group consisting of hydrogen, a methyl group, a methoxy group, -COOMe (Me represents a methyl group), -NHMe, F, Cl, Br, and NO₂.

R₅ in Formula (e) represents hydrogen or an alkyl group having 1 to 3 carbon atoms.

R₆ in Formula (f) in each occurrence is independently hydrogen or an alkyl group having 1 to 3 carbon atoms.

For example, non-limiting examples of linkers having the structure (a) include the following.

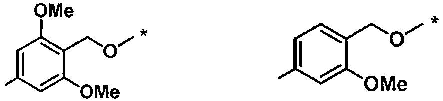

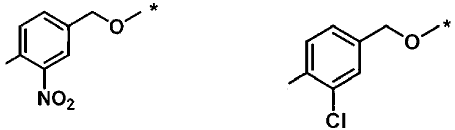

In addition, at least one of R₁ to R₄ in Formulae (a) and (d) to (f) may be a linking group selected from the following.

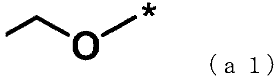

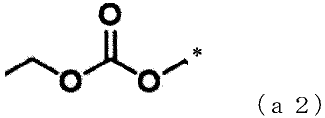

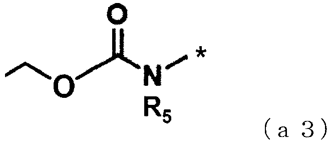

Here, in a case where at least one of R₁ to R₄ is a linking group in the linker represented by Formulae (a) and (d) to (f), the linker can be also bound to X₁ with the linking group therebetween.

In Formulae (a) to (f), * represents a direction of binding to X.

When T has a structure in which T is a combination of two or more of Formulae (a) to (f), * for one or more linkers other than the linker that binds to X₁ (also referred to as "linker A") may represent the direction of binding to the linker A.

In addition, in a case where at least one of X₁ is an antibody or a ligand, the linkers of Formulae (a) to (f), and the linking groups of a1 to a3 can be bound to a linker having a binding site that can be used as a binding point to an antibody or a ligand described later in the direction indicated by *.

Non-limiting examples of the case where T has a structure in which T is a combination of two or more of Formulae (a) to (f) include, but are not limited to, for example, as a case where T has a structure in which T is a combination of two formulae selected from Formulae of (a) to (f), (1) a case where the linker A is represented by Formula (a) and another linker is represented by any of Formulae (a) to (f), and a case where the linker A is a linker represented by Formula (d) and another linker is represented by any of Formulae (a) to (f).

Non-limiting specific structures in the case where T has a structure in which T is a combination of two formulae selected from Formulae of (a) to (f) include the following.

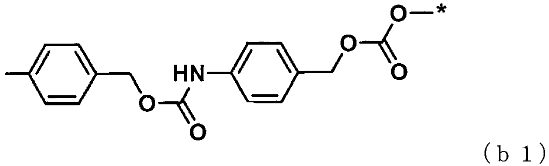

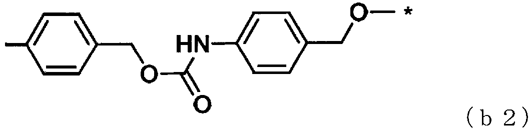

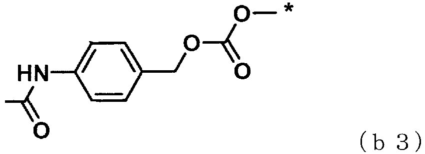

In the linkers having the structures (b1) to (b3), any benzene ring is unsubstituted, but any one or more benzene rings may have substituents defined as R₁ to R₄ above.

A structure in which T is a combination of three or more formulae selected from Formulae (a) to (f) can also be configured in the same manner as the structures (b1) to (b3) described above.

The linker represented by T also includes a linker having a linking group selected from (a1) to (a3) as at least one of R₁ to R₄ in Formulae (a) and (d) to (f) as described above. Also, in the case of a linker having a structure that is a combination of two or more of Formulae (a) to (f), the linker moieties of Formulae (a) and (d) to (f) may have a linking group selected from (a1) to (a3) as at least one of R₁ to R₄.

These linkers can also be bound to X₁ via the linking group. That is, the compound of the present invention having such a linker structure can be bound to two or more X₁. Among R₁ to R₄ in Formulae (a) and (d) to (f), the number of linking groups capable of binding to X₁ may be one or more, two or more, or three or more.

As the introduction position of the linking group, R₁ and R₂ are preferably in ortho positions with respect to the direction in which the linking group is bound to the enzyme recognition site in the linkers of Formulae (a) and (d) to (f) (bond of benzene ring).

In one aspect of the present invention, in Formulae (a) and (d) to (f), a linking group selected from (a1) to (a3) is introduced into any one or both of R₁ and R₂.

Non-limiting structures of the linker having a linking group selected from (a1) to (a3) described above include the following.

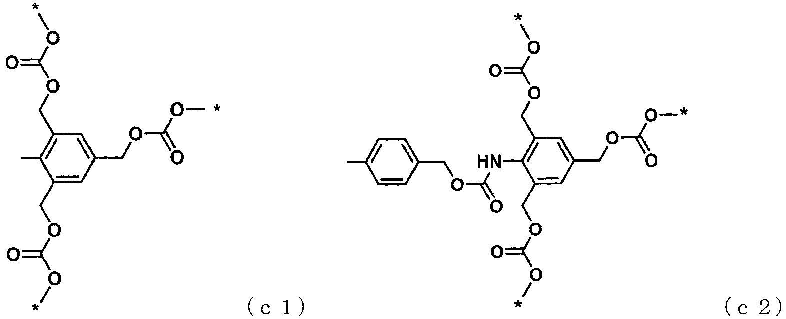

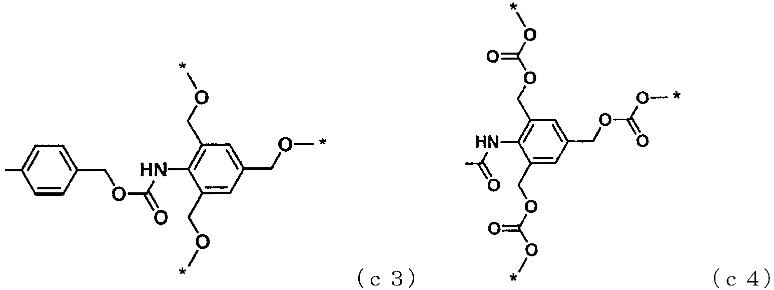

Here, in the linker having a structure (c1), another linker may be bound to the bond side of the benzene ring.

In one preferred aspect of the present invention, when Y is -NH-L₂, the linker represented by T in General Formula (1) is preferably any one of Formulae (a), (d), (e), and (f), or a combination of one of them and any one or more of Formulae (a) to (f).

In the compound of the present invention, further, at least one of R₁ to R₄ in Formulae (a) and (d) to (f) may be a linker having a binding site that can be used as a binding point to an antibody or a ligand.

The compound of the present invention may contain, as X₁, (2) an antibody, (3) a ligand selected from a small molecule, a peptide, an aptamer, or a hormone-drug conjugate, and both of them, but when such a molecule is contained as X₁, at least one of R₁ to R₄ is preferably a linker having an appropriate binding site capable of binding to an antibody or a ligand.

In addition, when X₁ includes (2) the antibody, (3) the ligand selected from a small molecule, a peptide, an aptamer, or a hormone-drug conjugate, and both of them, the linkers of Formula (a) to (f) may bind to the antibody or the ligand, but the linkers of Formula (a) to (f) and the linking groups of (a1) to (a3) may further have a linker having an appropriate binding site capable of binding to the antibody or the ligand. (That is, the linkers of Formulae (a) to (f) and the linking groups of (a1) to (a3) may be bound to a linker having an appropriate binding site capable of binding to an antibody or a ligand in the direction indicated by *.)

As such a linker, polyethylene glycol, alkylene, a peptide, a combination thereof, or the like can be used. In addition, a known linker used for a conjugate of a cell binding molecule (cell binding ligand or receptor analog) and a cytotoxic agent, an antibody-drug conjugate, or the like can be used.

In General Formula (I), X₁ represents at least one selected from the group consisting of (1) a binding residue of bioactive compound having one or more functional groups selected from the group consisting of an aliphatic hydroxyl group, an aromatic hydroxyl group, an amino group, and a carboxy group; (2) an antibody; (3) a ligand selected from the group consisting of a small molecule, a peptide, an aptamer, or a hormone-drug conjugate.

In the present specification, the binding residue of the bioactive compound means a moiety obtained by removing a functional group involved in the binding from the molecular structure of the bioactive compound when the bioactive compound is directly bound to -OL₁ or -NH-L₂ which is Y (bound without a linker) or bound to a linker.

Specifically, when Y is bound to X₁ without a linker interposed therebetween, it means a residue in which a terminal binding group of Y and a bioactive compound are bound to each other, that is, when Y is Formula (1): -OL₁, it means a residue in which an oxygen atom and a bioactive compound are bound to each other, and when Y is Formula (2): -NH-L₂, it means a residue in which a nitrogen atom and a bioactive compound are bound to each other. That is, the binding residue of the bioactive compound is a residue obtained by removing an aliphatic hydroxyl group or an aromatic hydroxyl group from the bioactive compound (when Y is -OL₁), a residue obtained by removing a hydroxyl group from a carboxy group of the bioactive compound, or a residue obtained by removing an amino group therefrom (when Y is -NH-L₂).

When Y is bound to X₁ via a linker, the binding residue of the bioactive compound is a residue obtained by removing an aliphatic hydroxyl group or an aromatic hydroxyl group from the bioactive compound, a residue obtained by removing a hydroxyl group from a carboxy group of the bioactive compound, or a residue obtained by removing an amino group from the bioactive compound, depending on the binding group at the terminal of the linker (preferably, an oxygen atom or NH).

The bioactive compound in the binding residue of the bioactive compound represented by X₁ is preferably an antitumor active substance, and is preferably an anticancer agent.

The bioactive compound that can be used in the present invention is preferably selected from the following anticancer agents.

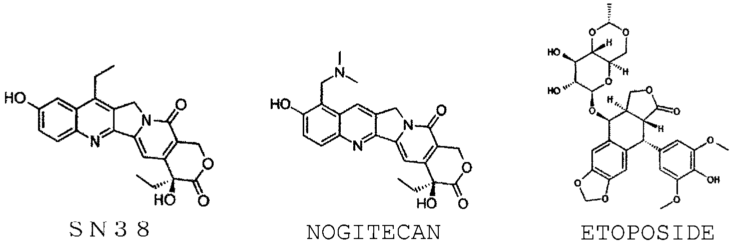

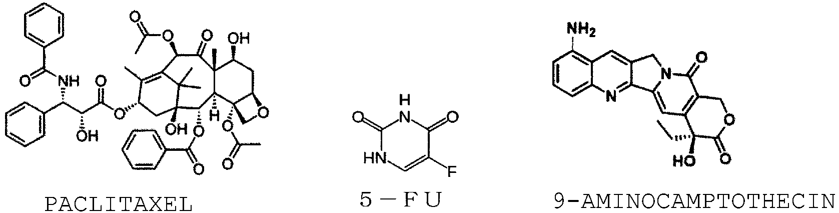

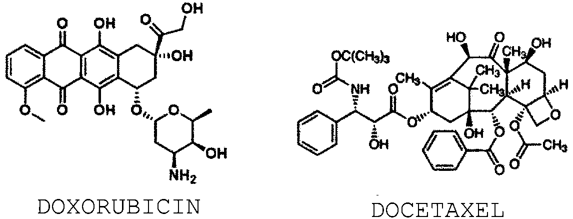

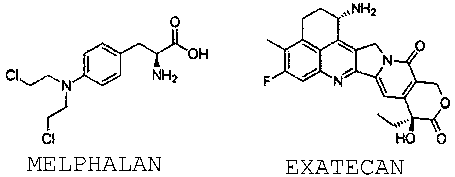

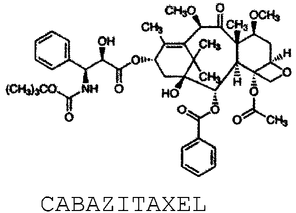

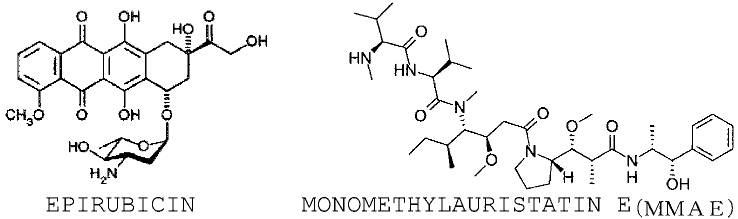

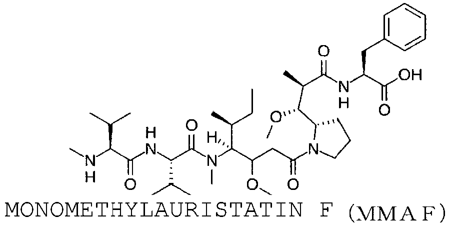

Among the above anticancer agents, in the present invention, it is more preferable to use SN38, nogitecan, exatecan, etoposide, doxolivicin, epirubicin, melphalan, MMAE, MMAF, paclitaxel, docetaxel, or cabazitaxel as the bioactive compound, and it is particularly preferable to use SN38, etoposide, doxolivicin, melphalan, MMAE, or paclitaxel.

When the bioactive compound has two or more functional groups selected from the group consisting of an aliphatic hydroxyl group, an aromatic hydroxyl group, an amino group, and a carboxy group, it is preferable to introduce Y as one of substituents capable of sufficiently inhibiting drug activity when a substrate site is introduced.

For example, when SN38 is used as the bioactive compound, it is preferable to introduce a partial structure of the L saccharide at the position of the hydroxyl group bound to the terminal benzene ring and bind the partial structure.

In addition, when paclitaxel is used as the bioactive compound, it is preferable to introduce a substrate site to the hydroxyl group of the terminal side chain structure or the hydroxyl group at the 9-position to bind the hydroxyl group.

In the compound represented by General Formula (I), at least one of X₁ is a binding residue of a bioactive compound.

The compound of the present invention includes, as X₁, a compound containing a binding residue of a bioactive compound that is one anticancer agent, and a compound containing two or more (preferably two or three) binding residues of a bioactive compound that is an anticancer agent. When two or more (preferably two or three) binding residues of a bioactive compound which is an anticancer agent are contained, the anticancer agents may be the same as or different from each other (in whole or in part).

In addition, the compound of the present invention may contain, as X₁, (2) an antibody, (3) a ligand selected from the group consisting of a small molecule, a peptide, an aptamer, or a hormone-drug conjugate, and both of them.

Here, an antibody and a ligand selected from a small molecule, a peptide, an aptamer, or a hormone-drug conjugate that can be used in the compound of the present invention are a tumor-associated antibody or ligand, or a diseased tissue-associated antibody or ligand.

Examples of such a ligand include the following cell-binding ligands or receptor analogs.

Structures of LB01 (PMSA ligand conjugate), LB02 (folate receptor conjugate), LB03 (somatostatin receptor conjugate), LB04 (octreotide, somatostatin analog receptor conjugate), LB05 (ranreotide, somatostatin analog receptor conjugate), LB06 (CAIX receptor conjugate), LB07 (CAIX receptor conjugate), LB08 (luteinizing hormone-releasing hormone (LH-RH) ligand and GnRH conjugate), LB09 (luteinizing hormone-releasing hormone (LH-RH) and GnRH ligand conjugate), LB10 (GnRH antagonist, avalerics conjugate), LB11 (cobalamin, VB12 analogue conjugate), LB12 (gastrin-releasing peptide receptor (GRPr), MBA conjugate), LB13 (αvβ3 integrin receptor, cyclic RGD pentapeptide conjugate), LB14 (heterobivalent peptide ligand of VEGF receptor conjugate), LB15 (neuromedin B conjugate), LB16 (G protein coupled receptor, bombesin conjugate), and LB17 (Toll-like receptor, TLR2 conjugate).

Examples of the tumor-associated antibody include an anti-HER2 antibody, an anti-TROP2 antibody, an anti-claudin antibody, an anti-PD-L1 antibody, and the like.

Specific anti-human PD-L1 monoclonal antibodies useful as PD-L1 antibodies include, for example, but are not limited to, avelumab (MSB0010718C), nivolumab (BMS-936558), MPDL3280A (IgG1 engineered anti-PD-L1 antibody), BMS-936559 (fully human anti-PD-L1, IgG4 monoclonal antibody), MEDI4736 (engineered IgG1k monoclonal antibody with triple mutations in Fc domain to remove antibody-dependent cell-mediated cytotoxic activity), and an antibody containing heavy and light chain variable regions of SEQ ID NO: 24 and SEQ ID NO: 21, respectively, of WO 2013/019906.

The "anti-HER2 antibody" refers to an antibody that specifically binds to HER2 (Human Epidermal Growth Factor Receptor Type 2; ErbB-2) and preferably has an activity of internalizing HER2-expressing cells by binding to HER2.

Examples of the anti-HER2 antibody include trastuzumab (US Patent No. 5821337) and pertuzumab (WO 01/00245), and preferably include trastuzumab.

The "anti-TROP2 antibody" refers to an antibody that specifically binds to TROP2 (TACSTD2: Tumor-associated calcium signal transducer 2; EGP-1) and preferably has an activity of internalizing in TROP2 expressing cells by binding to TROP2.

Examples of the anti-TROP2 antibody include hTINA1-H1L1 (WO 2015/098099 A).

The claudins are integral membrane proteins located at an epithelial and endothelial tight junction. The claudins are predicted to have four transmembrane regions with two extracellular loops and N and C termini located in the cytoplasm. The claudin (CLDN) family of transmembrane proteins plays an important role in the maintenance of the epithelial and endothelial tight junction, and may also be involved in cytoskeletal maintenance and cell signaling.

A claudin 18 (CLDN18) molecule contains four hydrophobic transmembrane regions and two extracellular loops (loop 1 surrounded by hydrophobic region 1 and hydrophobic region 2, and loop 2 surrounded by hydrophobic region 3 and hydrophobic region 4).

In the compound of the present invention, a known anti-claudin antibody can be used.

When a compound of the present invention contains, as X₁, a tumor-associated antibody as described above, it can also be used as a targeted drug delivery, so-called antibody-drug conjugate (ADC).

When the compound of the invention contains an antibody against a tumor-associated antigen, the antibody can serve as a highly potent targeting medium for cytotoxic agents (anticancer agents in the compound of the invention), that is, "payloads".

Generally, the ADC, after administration, is distributed in the patient's body and binds to its antigen on the surface of tumor cells. The antibody-antigen complex is then internalized by the cell and directed to the lysosome by an endogenous intracellular transport pathway. After reaching the lysosome, the ADC is degraded, thereby releasing its toxic cargo. The free toxins can then bind to their intracellular targets and thus induce apoptosis and cancer cell killing. Healthy cells are largely conserved because antibodies should only bind to cancer cells expressing antigens and deliver toxins.

By utilizing a prodrug-type anticancer agent containing the compound of the present invention as an ADC, when the prodrug-type anticancer agent is selectively delivered to a cancer cell and taken into the cancer cell, an enzyme recognition site and/or a linker site is cleaved by an enzyme activity specific to the cancer cell, whereby the anticancer agent can be released with higher selectivity in the cancer cell.

When the compound of the present invention has, as X₁, an antibody; a ligand selected from the group consisting of a small molecule, a peptide, an aptamer, or a hormone-drug conjugate; or both of them, the linker represented by T in General Formula (I) preferably includes a binding site that can be used as a binding point to the antibody or the ligand. Thus, at least one of R₁ to R₄ in Formulae (d) to (f) may be a linker having a binding site that can be used as a binding point to an antibody or a ligand.

In addition, when X₁ includes (2) the antibody, (3) the ligand selected from a small molecule, a peptide, an aptamer, or a hormone-drug conjugate, and both of them, X₁ may be bound to a linker having an appropriate binding site capable of binding to the antibody or the ligand in the direction indicated by the linker * of Formulae (a) to (f).

As such a linker, polyethylene glycol, alkylene, a peptide, a combination thereof, or the like can be used. In addition, a known linker used for a conjugate of a cell binding molecule (cell binding ligand or receptor analog) and a cytotoxic agent, an antibody-drug conjugate, or the like can be used.

The ligand can bind to cysteine, lysine, glutamine, or other reactive complex sites on the ligand via a binding site.

In General Formula (I), n is an integer of 1 or more, and may be an integer of 2 or more (preferably 2 or 3).

In one preferred aspect of the present invention, n is 1.

Non-limiting examples of the compound of the invention are described below, but the compound of the invention is not limited thereto.

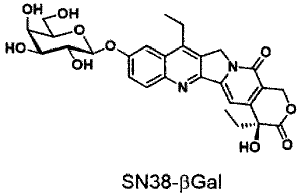

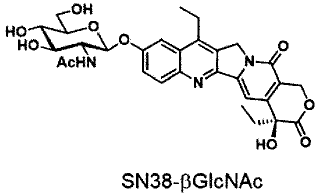

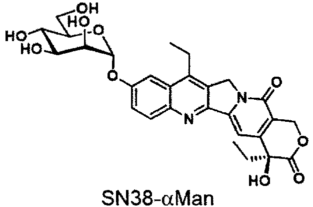

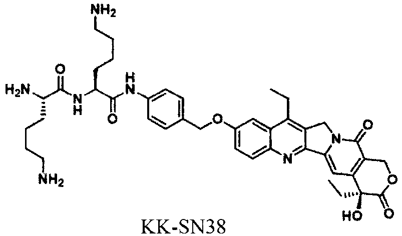

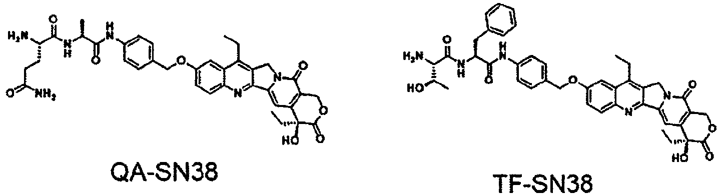

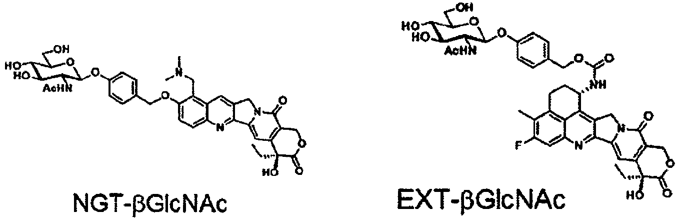

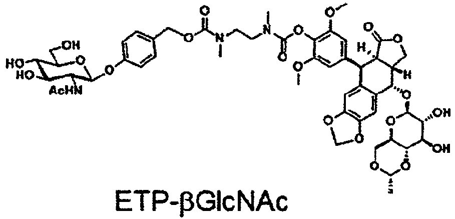

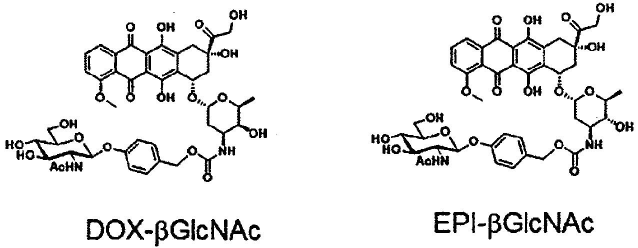

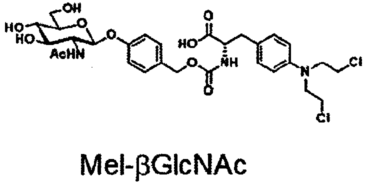

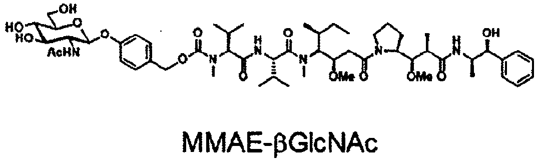

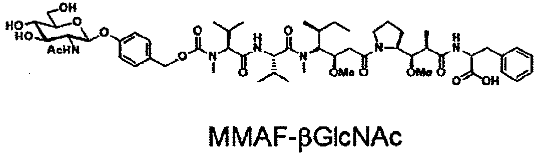

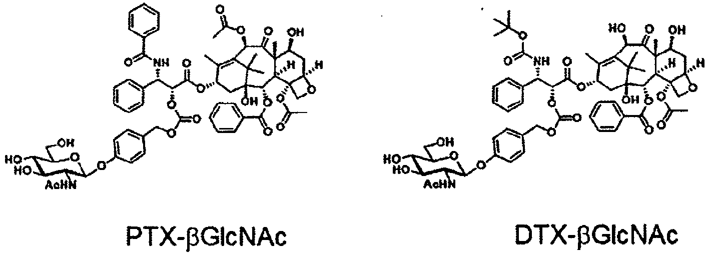

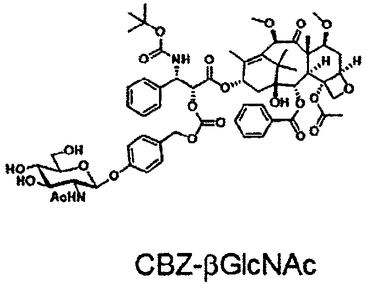

Unless otherwise specified, the compound represented by General Formula (I) also includes stereoisomers such as tautomers, geometric isomers (for example, E-form and Z-form), and enantiomers thereof. That is, when one or two or more asymmetric carbons are contained in the compound represented by General Formula (I), either an (R) form or an (S) form can be independently taken as stereochemistry of the asymmetric carbon, and the compound may exist as a stereoisomer such as an enantiomer or a diastereoisomer of the derivative. Therefore, as the active ingredient of the prodrug-type anticancer agent of the present invention described later, any stereoisomer in pure form, any mixture of stereoisomers, racemate, and the like can be used, and all of these are included in the scope of the compound of the present invention.

The method for producing the compound represented by General Formula (I) is not particularly limited, but the synthesis method for a representative compound among the compounds included in General Formula (I) was specifically described in Examples of the present specification. A person skilled in the art can produce a compound included in Formula (I) by appropriately changing or modifying a starting material, a reaction reagent, reaction conditions, and the like as necessary while referring to Examples of the present specification.

### 2. Prodrug-type anticancer agent

Another embodiment of the present invention is a prodrug-type anticancer agent comprising a compound of General Formula (I) or a pharmaceutically acceptable salt thereof (hereinafter, also referred to as "prodrug-type anticancer agent of the present invention").

In addition, another embodiment of the present invention is a prodrug-type anticancer agent comprising a compound of General Formula (I) or a pharmaceutically acceptable salt thereof and acts cell selectively by cancer cell-specific enzyme activity.

The cancer cell specific enzyme is glycosidase or peptidase.

Examples of the glycosidase include β-galactosidase, β-glucosidase, α-mannosidase, α-fucosidase, β-N-acetylgalactosaminidase, and β-hexosaminidase (a dimer of β-hexosaminidase subunit A (HEXA; hereafter, abbreviated as "A") and β-hexosaminidase subunit B (HEXB; hereafter, abbreviated as "B"), consisting of AA, AB, BB, or the like.)

Examples of the peptidase include γ-glutamyl transpeptidase (GGT), dipeptidyl peptidase IV (DPP-IV), calpain, puromycin sensitive aminopeptidase (PSA), aminopeptidase N (APN), cathepsin, and the like.

Another embodiment of the present invention is a pharmaceutical composition for treating or preventing cancer such as breast cancer, esophageal cancer, lung cancer, stomach cancer, large bowel cancer, small intestine cancer, pancreatic cancer, liver cancer, head and neck cancer, oral cancer, ovarian cancer, brain tumor, kidney cancer, prostate cancer, or skin cancer, comprising a compound of General Formula (I) or a pharmaceutically acceptable salt thereof (hereinafter, also referred to as "pharmaceutical composition of the present invention").

Another embodiment of the present invention is a method for treating cancer such as breast cancer, esophageal cancer, lung cancer, stomach cancer, large bowel cancer, small intestine cancer, pancreatic cancer, liver cancer, head and neck cancer, oral cancer, ovarian cancer, brain tumor, renal cancer, prostate cancer, skin cancer and the like in mammals, particularly humans, wherein a pharmaceutical composition comprising an effective amount of a compound of the present invention represented by General Formula (I) or a pharmaceutically acceptable salt thereof, or a compound represented by General Formula (I) or a pharmaceutically acceptable salt thereof is administered to a mammal in need of such treatment.

The prodrug-type anticancer agent or the pharmaceutical composition of the present invention may contain not only the compound represented by General Formula (I) but also a salt thereof or a solvate or a hydrate of the compound or the salt. The salt is not particularly limited as long as it is a pharmaceutically acceptable salt, and examples thereof include a base addition salt, an acid addition salt, and an amino acid salt. Examples of the base addition salt include alkaline earth metal salts such as a sodium salt, a potassium salt, a calcium salt, and a magnesium salt, ammonium salts, or organic amine salts such as a triethylamine salt, a piperidine salt, and a morpholine salt, and examples of the acid addition salts include mineral acid salts such as a hydrochloride, a hydrobromide salt, a sulfate salt, a nitrate salt, and a phosphate salt; and organic acid salts such as methanesulfonic acid, benzenesulfonic acid, p-toluenesulfonic acid, acetic acid, propionate, tartaric acid, fumaric acid, maleic acid, malic acid, oxalic acid, succinic acid, citric acid, benzoic acid, mandelic acid, cinnamic acid, lactic acid, glycolic acid, glucuronic acid, ascorbic acid, nicotinic acid, and salicylic acid. Examples of the amino acid salt include a glycine salt, an aspartic acid salt, and a glutamic acid salt. In addition, it may be a metal salt such as an aluminum salt.

The type of solvent forming the solvate is not particularly limited, and examples thereof include solvents such as ethanol, acetone, and isopropanol.

As a disease that can be treated or prevented by the prodrug-type anticancer agent of the present invention, a large effect can be expected for a wide range of cancer types, particularly breast cancer, esophageal cancer, lung cancer, stomach cancer, large bowel cancer, small intestine cancer, pancreatic cancer, liver cancer, head and neck cancer, oral cancer, ovarian cancer, brain tumor, kidney cancer, prostate cancer, skin cancer, and the like.

In the prodrug-type anticancer agent or the pharmaceutical composition of the present invention, the compound represented by General Formula (I), which is an active ingredient, or a pharmaceutically acceptable salt, a hydrate, or a solvate thereof itself may be administered, but in general, it is desirable to administer the prodrug-type anticancer agent or the pharmaceutical composition in the form of a pharmaceutical composition containing the above-described substance, which is an active ingredient, and one or more additives for formulation. The term "composition" as in the pharmaceutical composition encompasses not only products containing an active ingredient and an inert ingredient (pharmaceutically acceptable excipient) that constitutes a carrier, but also any product that occurs directly or indirectly as a result of association, complexation or aggregation of any two or more ingredients, or as a result of dissociation of one or more ingredients, or as a result of another type of reaction or interaction of one or more ingredients.

As the active ingredient of the prodrug-type anticancer agent or the pharmaceutical composition of the present invention, two or more of the above compounds can be used in combination.

In addition, the prodrug-type anticancer agent or the pharmaceutical composition of the present invention can also be used as a combination drug in which a compound represented by General Formula (I) or a pharmaceutically acceptable salt, a hydrate or a solvate thereof, which is an active ingredient, is used in combination with an existing anticancer agent. As the existing anticancer agent, those known in the art can be used, and examples thereof include methotrexate, doxorubicin, cisplatin, and an anti-PD-1 antibody.

Anti-PD-1 antibodies include, for example, but are not limited to, avelumab (MSB0010718C), nivolumab (BMS-936558), MPDL3280A (IgG1 engineered anti-PD-L1 antibody), BMS-936559 (fully human anti-PD-L1, IgG4 monoclonal antibody), MEDI4736 (engineered IgG1k monoclonal antibody with triple mutations in Fc domain to remove antibody-dependent cell-mediated cytotoxic activity), and an antibody containing heavy and light chain variable regions of SEQ ID NO: 24 and SEQ ID NO: 21, respectively, of WO 2013/019906.

The type of the prodrug-type anticancer agent or the pharmaceutical composition of the present invention is not particularly limited, and examples of the dosage form include tablets, capsules, granules, powders, syrups, suspensions, suppositories, ointments, creams, gels, patches, inhalants, and injections. These formulations are prepared according to a conventional method. The liquid formulation may be dissolved or suspended in water or another appropriate solvent at the time of use. The tablets and granules may be coated by a well-known method. In the case of the injections, it is prepared by dissolving the compound of the present invention in water, but may be dissolved in physiological saline or a glucose solution as necessary, or a buffer or a preservative may be added. It is provided in any formulation form for oral or parenteral administration. For example, it can be prepared as a pharmaceutical composition for oral administration in the form of granules, fine granules, powders, hard capsules, soft capsules, syrups, emulsions, suspensions, solutions, or the like, an injection for intravenous administration, intramuscular administration, subcutaneous administration, or the like, a pharmaceutical composition for parenteral administration in the form of drops, transdermal absorption, transmucosal absorption, nasal drops, inhalants, suppositories, or the like. The injections, drops, and the like can be prepared as a powdered dosage form such as a freeze-dried form, and can be used by being dissolved in an appropriate aqueous medium such as physiological saline at the time of use. It is also possible to directly administer a sustained release formulation coated with a polymer or the like into the brain.

A person skilled in the art can appropriately select the type of the additive for formulation used in the production of the prodrug-type anticancer agent or the pharmaceutical composition of the present invention, the ratio of the additive for formulation to the active ingredient, or the method for producing the pharmaceutical composition according to the form of the composition. As the additive for formulation, an inorganic or organic substance or a solid or liquid substance can be used, and generally, the additive for formulation can be blended in an amount of 1% by weight to 90% by weight with respect to the weight of the active ingredient. Specifically, examples of such a substance include lactose, glucose, mannitol, dextrin, cyclodextrin, starch, sucrose, magnesium aluminometasilicate, synthetic aluminum silicate, sodium carboxymethyl cellulose, hydroxypropyl starch, carboxymethyl cellulose calcium, an ion-exchanged resin, methyl cellulose, gelatin, gum arabic, hydroxypropyl cellulose, hydroxypropyl methyl cellulose, polyvinyl pyrrolidone, polyvinyl alcohol, light anhydrous silicic acid, magnesium stearate, talc, tragacanth, bentonite, veegum, titanium oxide, sorbitan fatty acid ester, sodium lauryl sulfate, glycerin, fatty acid glycerin ester, purified lanolin, glycerogelatin, polysorbate, macrogol, vegetable oil, wax, liquid paraffin, white petrolatum, fluorocarbon, a nonionic surfactant, propylene glycol, and water.

In order to produce a solid formulation for oral administration, an active ingredient and an excipient component such as lactose, starch, crystalline cellulose, calcium lactate, and silicic anhydride are mixed to form powders, or if necessary, a binder such as sucrose, hydroxypropyl cellulose, and polyvinylpyrrolidone, a disintegrant such as carboxymethyl cellulose and carboxymethyl cellulose calcium, and the like are added to form granules by wet or dry granulation. In order to produce tablets, these powders and granules may be compressed as they are or after adding a lubricant such as magnesium stearate or talc. These granules or tablets can also be coated with an enteric solvent base such as hydroxypropyl methylcellulose phthalate or a methacrylic acid-methyl methacrylate polymer and coated with an enteric solvent formulation or ethyl cellulose, carnauba wax, hardened oil or the like to form a long-acting formulation. In order to produce capsules, powders or granules can be filled in a hard capsule, or an active ingredient can be used as it is or dissolved in glycerin, polyethylene glycol, sesame oil, olive oil or the like and then coated with a gelatin film to form a soft capsule.

In order to produce injections, the active ingredient may be dissolved in distilled water for injection together with a pH adjusting agent such as hydrochloric acid, sodium hydroxide, lactose, lactic acid, sodium, sodium monohydrogen phosphate, or sodium dihydrogen phosphate, or an isotonizing agent such as sodium chloride or glucose, if necessary, and the solution may be filled into an ampoule by aseptic filtration, or further vacuum freeze-dried by adding mannitol, dextrin, cyclodextrin, gelatin, or the like, and may be used as a ready-to-use solution type injections. In addition, reticin, polysorbate 80, polyoxyethylene-hydrogenated castor oil or the like is added to the active ingredient and emulsified in water to form an emulsion for injection.

The dose and the number of administrations of the prodrug-type anticancer agent or the pharmaceutical composition of the present invention are not particularly limited, and can be appropriately selected by the judgment of a doctor according to conditions such as the purpose of prevention and/or treatment of exacerbation/progression of the disease to be treated, the type of disease, the body weight and age of the patient, and the severity of the disease. In general, an adult daily dose in oral administration is about 0.01 to 1000 mg (active ingredient weight), and can be administered once or several times a day, or every several days. When used as an injection, it is desirable to continuously or intermittently administer a daily dose of 0.001 to 100 mg (active ingredient weight) to an adult.

### 3. Novel fluorescent probe for detection of lung cancer

The present inventors have screened glycosidase activity detection fluorescent probes in human lung cancer specimens in conducting development studies of the prodrug-type anticancer agent of the present invention, and found that fluorescent probes that have not been known so far are very effective as probes for detecting lung cancer.

That is, another embodiment of the present invention is a fluorescent probe for detecting lung cancer, comprising a compound represented by the following General Formula (II) or a salt thereof (hereinafter, also referred to as "fluorescent probe of the present invention").

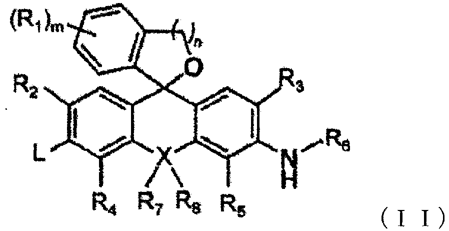

In General Formula (I), R₁, if present, represents the same or different monovalent substituents present on a benzene ring. Examples of the monovalent substituent include halogen and an optionally substituted alkyl group.

m is an integer of 0 to 4.

In one preferred aspect of the invention, m is 0 and R₁ is absent. That is, the benzene ring bound to the xanthene skeleton is an unsubstituted benzene ring.

In General Formula (I), R₂ and R₃ each independently represent a hydrogen atom, an alkyl group having 1 to 6 carbon atoms, or a halogen atom.

When R₂ and R₃ represent an alkyl group, one or two or more halogen atoms, carboxy groups, sulfonyl groups, hydroxyl groups, amino groups, alkoxy groups, and the like may be present in the alkyl group, and for example, the alkyl group represented by R₂ and R₃ may be a halogenated alkyl group, a hydroxyalkyl group, a carboxyalkyl group, or the like.

It is preferable that R₂ and R₃ are each independently a hydrogen atom or a halogen atom. When R₂ and R₃ are halogen atoms, it is preferable that R₂ and R₃ are both fluorine atoms or chlorine atoms.

In one preferred aspect of the present invention, both R₂ and R₃ are hydrogen atoms.

R₄ and R₅ each independently represent a hydrogen atom, an alkyl group having 1 to 6 carbon atoms, or a halogen atom, and are the same as those described for R₂ and R₃. It is preferable that both R₄ and R₅ are hydrogen atoms.

R₆ represents a hydrogen atom, an alkyl group having 1 to 5 carbon atoms, or a fluorinated alkyl group having 1 to 5 carbon atoms (fluoroalkyl group). The alkyl group of R₆ is preferably a methyl group or an ethyl group. The fluoroalkyl group of R₆ is preferably -CH₂-CF₃ or -CH₂-CH₂-CF₃.

In one preferred aspect of the present invention, R₆ is -CH₂-CF₃.

In General Formula (I), R₇ and R₈, if present, each independently represent an alkyl group or aryl group having 1 to 6 carbon atoms, but R₇ and R₈ are each independently preferably an alkyl group having 1 to 3 carbon atoms, and more preferably both R₇ and R₈ are methyl groups. One or two or more halogen atoms, carboxy groups, sulfonyl groups, hydroxyl groups, amino groups, alkoxy groups, and the like may be present in the alkyl group represented by R₇ and R₈, and for example, the alkyl group represented by R₇ and R₈ may be a halogenated alkyl group, a hydroxyalkyl group, a carboxyalkyl group, or the like.

When R₇ and R₈ represents an aryl group, the aryl group may be either a monocyclic aromatic group or a fused aromatic group, and the aryl ring may contain one or two or more ring-constituting heteroatoms (for example, a nitrogen atom, an oxygen atom, a sulfur atom, or the like). The aryl group is preferably a phenyl group. One or more substituents may be present on the aryl ring. As the substituent, for example, one or two or more halogen atoms, carboxy groups, sulfonyl groups, hydroxyl groups, amino groups, alkoxy groups, and the like may be present.

When X described later is an oxygen atom, R₇ and R₈ are not present.

X represents an oxygen atom, a silicon atom, or a carbon atom.

In one preferred aspect of the present invention, X is an oxygen atom.

n is an integer of 1 to 3, preferably n is 1.

L is a group of the following Formula (1b).

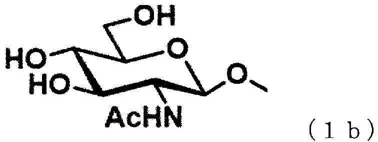

The compound represented by General Formula (II) can exist as an acid addition salt or a base addition salt. Examples of the acid addition salt include mineral acid salts such as hydrochloride, sulfate, and nitrate, or organic acid salts such as methanesulfonate, p-toluenesulfonate, oxalate, citrate, and tartrate, and examples of the base addition salt include metal salts such as a sodium salt, a potassium salt, a calcium salt, and a magnesium salt, and organic amine salts such as an ammonium salt and a triethylamine salt. In addition to these, a salt with an amino acid such as glycine may be formed. The compound represented by General Formula (II) or a salt thereof may exist as a hydrate or a solvate, but these substances can also be used in the present invention.

The compound represented by General Formula (II) may have one or two or more asymmetric carbons depending on the type of the substituent, but in the present invention, in addition to stereoisomers such as optically active forms based on one or two or more asymmetric carbons and diastereoisomers based on two or more asymmetric carbons, optional mixtures of stereoisomers, racemates, and the like can also be used.

The fluorescent probe of the present invention can detect lung adenocarcinoma and lung squamous cell carcinoma, and can also detect thymoma as small cell lung cancer, large cell lung cancer, and a mediastinum tumor.

The method for using the fluorescent probe of the present invention is not particularly limited, and the fluorescent probe can be used in the same manner as a conventionally known fluorescent probe. Usually, the compound of General Formula (II) or a salt thereof may be dissolved in an aqueous medium such as physiological saline or a buffer solution, or a mixture of an aqueous mixed organic solvent such as ethanol, acetone, ethylene glycol, dimethyl sulfoxide, or dimethylformamide and an aqueous medium, and this solution may be added to an appropriate buffer solution containing cells and tissues to measure a fluorescence spectrum. The fluorescent probe of the present invention may be used in the form of a composition in combination with appropriate additives. For example, it can be combined with additives such as a buffering agent, a solubilizing agent, a pH adjusting agent, and the like.

The concentration of the compound of General Formula (II) in the fluorescent probe of the present invention can be appropriately determined according to the type of cells or the like to be measured, measurement conditions, and the like.

Another embodiment of the present invention is a method for detecting lung cancer, the method comprising: (a) applying the fluorescent probe of the present invention to a clinical specimen of the lung cancer; and (b) measuring a fluorescence image of the clinical specimen to which the fluorescent probe is applied.

The applying of the fluorescent probe to a clinical specimen of lung cancer in the step (a) can be performed, for example, by locally spraying a solution of the fluorescent probe to the clinical specimen of lung cancer.

The method for detecting lung cancer of the present invention can detect lung adenocarcinoma and lung squamous cell carcinoma, and can also detect thymoma as small cell lung cancer, large cell lung cancer, and a mediastinum tumor.

Another embodiment of the present invention is a kit for detecting lung cancer, comprising the fluorescent probe of the present invention.

In the kit, the fluorescent probe of the present invention is usually prepared as a solution, but for example, the fluorescent probe of the present invention is provided as a composition in an appropriate form such as a mixture in a powder form, a lyophilizate, a granule, a tablet, or a liquid, and can also be dissolved in distilled water for injection or an appropriate buffer solution at the time of use and applied.

In addition, the kit may appropriately contain other reagents and the like as necessary. For example, additives such as a solubilizing agent, a pH adjusting agent, a buffering agent, and an isotonizing agent can be used as the additive, and the blending amount thereof can be appropriately selected by those skilled in the art.

### 4. Companion diagnostic agent

The present inventors have screened glycosidase activity detection fluorescent probes in human lung cancer specimens in conducting development studies of the prodrug-type anticancer agent of the present invention, and found that the above-described fluorescent probe of the present invention (fluorescent probe containing a compound represented by Formula (1b) as L in General Formula (II)) is very effective as a novel probe for detecting lung cancer.

Furthermore, as described in Example 1, a fluorescent probe in which β-D-Glc, β-D-Gal, or α-D-Man is introduced as L is also effective as a probe for detecting lung cancer.

In addition, depending on the substrate sequence of L, it can also be used as a probe for detecting cancer other than lung cancer.

Here, the prodrug-type anticancer agent of the present invention has a common substrate site targeting cancer cell-specific enzyme activity as those of these probes for detection of lung cancer. Then, in a patient with lung cancer, by knowing in advance the level of the target cancer cell-specific enzyme activity using these probes for detecting cancer such as lung cancer, it becomes possible to grasp whether the prodrug-type anticancer agent of the present invention is effective in the cancer patient, and thus these probes for detecting cancer such as lung cancer can also be used as a companion diagnostic agent.

That is, another embodiment of the present invention is a companion diagnostic agent for diagnosing a need for cancer treatment with a prodrug-type anticancer agent, comprising a compound represented by the following General Formula (I) or a pharmaceutically acceptable salt thereof, the companion diagnostic agent containing: a compound represented by the following General Formula (II) or a salt thereof (hereinafter, also referred to as "companion diagnostic agent of the present invention").

Y-T-(X₁)ₙ (I)

In Formula (I),
Y is selected from any of the following groups. 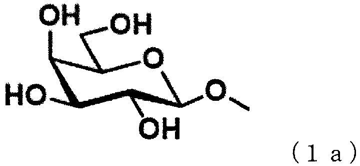 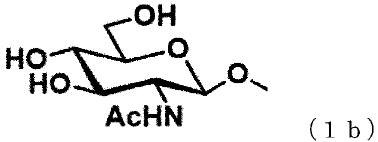 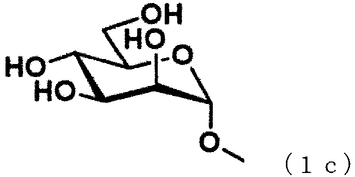 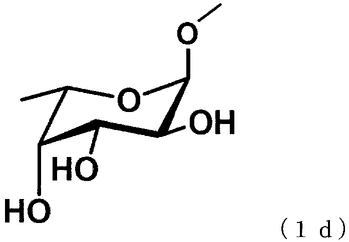 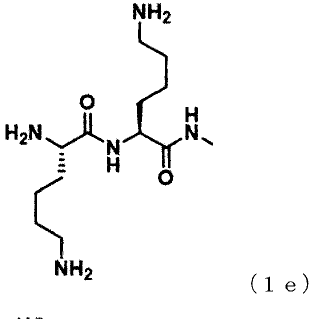 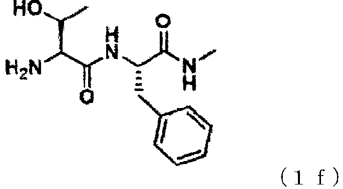 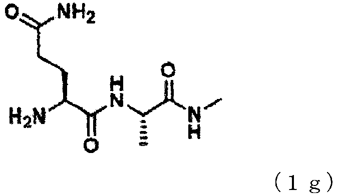
T represents a linker or a bond.
X₁ selected from the group consisting of (1) a binding residue of bioactive compound having one or more functional groups selected from the group consisting of an aliphatic hydroxyl group, an aromatic hydroxyl group, an amino group, and a carboxy group; (2) an antibody; (3) a ligand selected from the group consisting of a small molecule, a peptide, an aptamer, or a hormone-drug conjugate.

Here, at least one of X₁ is a binding residue of the bioactive compound.

The bioactive compound is an anticancer agent.

n is an integer of 1 or more.

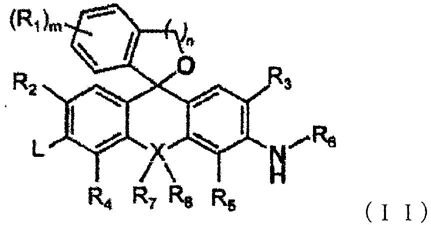

In Formula (II),
R₁, if present, represents the same or different monovalent substituents present on a benzene ring.

R₂ and R₃ each independently represent a hydrogen atom, an alkyl group having 1 to 6 carbon atoms, or a halogen atom.

R₄ and R₅ each independently represent a hydrogen atom, an alkyl group having 1 to 6 carbon atoms, or a halogen atom.

R₆ represents a hydrogen atom, an alkyl group having 1 to 5 carbon atoms, or a fluoroalkyl group having 1 to 5 carbon atoms.

R₇ and R₈, if present, each independently represent an alkyl group or an aryl group having 1 to 6 carbon atoms. Here, when X is an oxygen atom, R₇ and R₈ are not present.

X represents an oxygen atom, a silicon atom, or a carbon atom.

n is an integer of 1 to 3.

L is selected from any of the following groups.

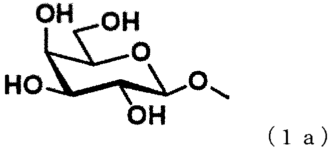

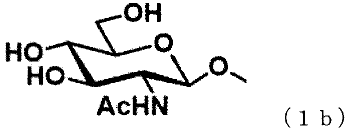

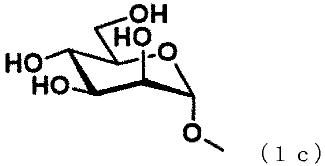

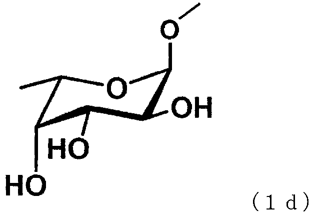

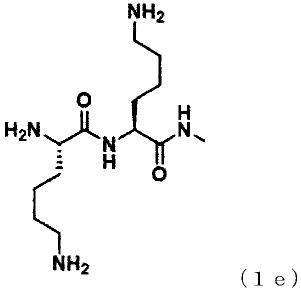

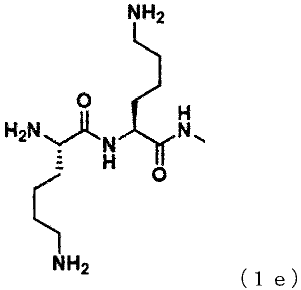

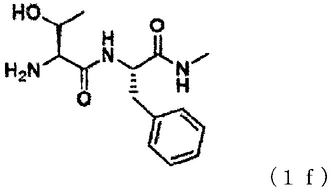

In the companion diagnostic agent of the present invention, details of T, X₁, and n in General Formula (I) are the same as those described in detail for the compound of the present invention, and details of R₁ to R₈, X, and n in General Formula (II) are the same as those described in detail for the fluorescent probe of the present invention.

As used herein, the term "treatment" refers to preventing, preferably maintaining a current state, more preferably reducing, even more preferably causing regression of a disease or disorder (for example, cancer) when the disease or disorder is in such a state, and includes exhibiting a symptom improving effect or a prophylactic effect on the disease of a patient or one or more symptoms associated with the disease. Performing diagnosis in advance and performing appropriate treatment is referred to as "companion treatment", and a diagnostic agent therefor is referred to as "companion diagnostic agent".

Here, in the "companion diagnosis", it is usually determined whether treatment is indicated (whether efficacy can be expected) by measuring a specific biomarker (specific glycosidases, peptidases, or the like) using a collected specimen (blood, clinical specimen, or the like).

The companion diagnostic agent of the present invention is used to diagnose the need for the treatment of cancer, preferably the treatment of lung cancer.

### 5. Method for treating cancer

Another aspect of the present invention is a method for treating a patient with cancer, the method comprising:
a step of administering a therapeutically effective amount of the prodrug-type anticancer agent of the present invention or the pharmaceutical composition of the present invention to a patient (hereinafter, also referred to as "treatment method of the present invention").

The prodrug-type anticancer agent of the present invention can act cell selectively by an enzyme activity specific to cancer cells. Therefore, the cancer to which the prodrug-type anticancer agent is applied is characterized by a higher expression level of the enzyme.

In addition, in the treatment method of the present invention, preferably, the prodrug-type anticancer agent releases a molecule of the anticancer agent by cleaving an enzyme recognition site and/or a linker site due to an enzyme activity specific to cancer cells.

That is, one aspect of the treatment method of the present invention is a method for treating cancer in which the prodrug-type anticancer agent of the present invention acts cell selectively with an enzyme activity specific to cancer cells, and the cancer is characterized by a higher expression level of the enzyme.

In addition, another aspect of the treatment method of the present invention is a method for treating cancer in which the prodrug-type anticancer agent of the present invention acts cell selectively with an enzyme activity specific to cancer tissues, and the cancer is characterized by a higher expression level of the enzyme.

Another aspect of the treatment method of the present invention is a method for treating cancer in which the prodrug-type anticancer agent of the present invention releases a molecule of the anticancer agent by cleaving an enzyme recognition site and/or a linker site due to an enzyme activity specific to cancer cells.

Still another aspect of the treatment method of the present invention is a method for treating cancer in which the prodrug-type anticancer agent of the present invention releases a molecule of the anticancer agent by cleaving an enzyme recognition site and/or a linker site due to an enzyme activity specific to cancer tissues.

Details of the prodrug-type anticancer agent or the pharmaceutical composition of the present invention are as described in detail in "2. Prodrug-type anticancer agent". In addition, the prodrug-type anticancer agent or the pharmaceutical composition of the present invention that can be used in the method for treating cancer of the present invention can also be used as a combination drug in which a compound represented by General Formula (I) or a pharmaceutically acceptable salt, a hydrate or a solvate thereof, which is an active ingredient, is used in combination with an existing anticancer agent.

Since the prodrug-type anticancer agent used in the treatment method of the present invention acts cell selectively by an enzyme activity specific to cancer cells, a patient (subject) to which the prodrug-type anticancer agent is applied preferably has an enzyme activity specific to cancer cells.

It is preferable to identify whether the patient (subject) has such enzyme activity and the level of the enzyme activity before the start of treatment and preferably also during the duration of treatment.

The level of enzyme activity is preferably identified using one or more enzyme diagnostic agents selected from a visualization agent or an enzyme activity reporter.

As the visualization agent and the enzyme activity reporter, enzyme activity detection fluorescent probes as listed in the present application and other known enzyme activity diagnostic probes can be used.

Thus, one preferred aspect of the treatment method of the present invention includes identifying the level of enzyme activity of the enzyme in a patient's biological sample using a visualization agent, or one or more enzyme diagnostic agents selected from an enzyme activity reporter, and defining groups that may respond to treatment as a function of the enzyme activity.

Defining groups that may respond to therapy as a function of enzyme activity can be done by measuring the enzyme activity of a patient or a sample specimen from a patient using an enzyme activity detection fluorescent probe or other diagnostic probes.

Also, the treatment method of the present invention preferably includes stratifying a set of patients and defining a subset of patients who may respond to the prodrug-type anticancer agent of the present invention or the pharmaceutical composition of the present invention by using an enzyme diagnostic agent.

Stratifying a set of patients and defining a subset of patients who may respond to the prodrug-type anticancer agent by using enzyme diagnostics can be performed by measuring the enzyme activity of a patient or a sample specimen from a patient using an enzyme activity detection fluorescent probe or other diagnostic probes.

In order to identify the level of enzyme activity, it is also possible to use a companion diagnostic agent for diagnosing the necessity of treatment of cancer with the prodrug-type anticancer agent of the present invention described above.

### 6. Enzyme recognition site-linker binding site of the present invention

As described above in detail, the prodrug-type anticancer agent of the present invention can act cell selectively by enzyme activity specific to cancer cells.

In addition, the prodrug-type anticancer agent of the present invention can be bound to the anticancer agent via a linker, but in this case, a part of the enzyme recognition site is cleaved by the enzyme activity specific to the cancer cell, and the linker (preferably, a self-cleaving linker) is detached after the enzyme reaction to release the molecule of the anticancer agent.

As described above, the compound of the present invention can bind to a ligand selected from an antibody, a ligand selected from the group consisting of a small molecule, a peptide, an aptamer, or a hormone-drug conjugate, or both via a linker. However, when a part of the enzyme recognition site is cleaved by the enzyme activity specific to cancer cells, and the linker (preferably, a self-cleaving linker) is detached after the enzyme reaction, these antibodies and ligands are also liberated from the compound or the prodrug-type anticancer agent of the present invention. Here, in a case where the antibody or the ligand has a payload (cytotoxic agent, enzyme inhibitor/modulator, or any molecule having biological, pharmaceutical, or diagnostic utility) different from the anticancer agent possessed by the compound of the present invention, the payload is inactive (activity is masked) until a part of the enzyme recognition site is cleaved by the enzyme activity, but cleavage of the linker moiety by the enzyme serves as a trigger, and the payload can be activated.

Therefore, the moiety of Y-T- of General Formula (I) of the present invention, that is, the moiety in which the enzyme recognition site and the linker are bound (hereinafter, also referred to as "enzyme recognition site-linker binding site" or "cleavable linker by enzyme activity") can also be used to activate an antibody or a ligand having a payload as described above.

Here, the enzyme recognition site in the "enzyme recognition site-linker binding site" is a substrate site targeting cancer cell-specific enzyme activity. The term "cancer cell-specific" means that a diseased tissue containing cancer, preferably a cancer cell, has higher enzyme activity than that of a normal tissue. As a method for confirming the cancer cell-specific enzyme activity, a method using a fluorescent probe can be used, but the method is not limited thereto.

In order to select an enzyme recognition site having an enzyme activity specific to a cancer cell, for example, it can be found by screening a fluorescent probe. In this case, in designing the enzyme recognition site, first, in screening of fluorescent probes, a fluorescent probe in which a significant increase in fluorescence is observed from a tumor tissue as compared with a normal tissue can be selected with reference to a T/N ratio (Tumor to Normal ratio).

That is, another embodiment of the present invention is "an enzyme recognition site-linker binding site, represented by General Formula (III), wherein the enzyme recognition site has an enzyme activity specific to a cancer cell".

Y-T1 (III)

(wherein,
Y is selected from

   -OL₁ (1)

   or

   -NH-L₂ (2),
L₁ is a partial structure of saccharides,
here, the partial structure of the saccharides is a structure obtained by removing one hydroxyl group from the saccharide, and
L₂ is a monovalent substituent containing an amino acid residue or an oligopeptide residue; and
T1 is a linker (hereinafter also referred to as "enzyme recognition site-linker binding site of the present invention").)

In the enzyme recognition site-linker binding site of the present invention, the enzyme recognition site preferably has a structure found by the screening described above and selected from the following.

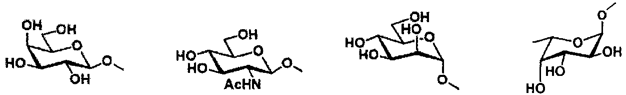

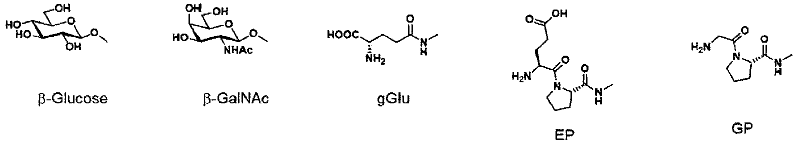

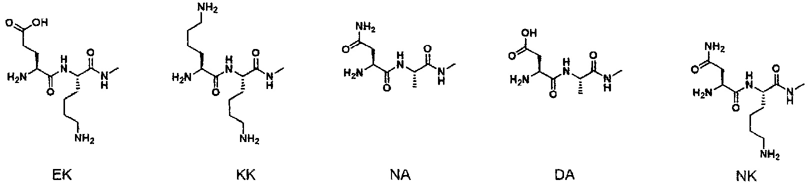

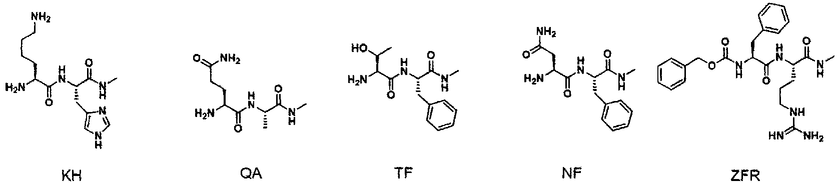

In addition, a more preferred enzyme recognition site in the present invention has a structure selected from the following.

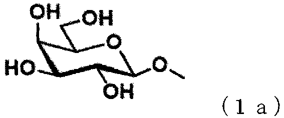

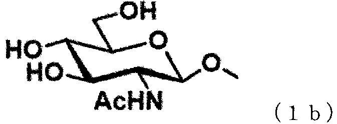

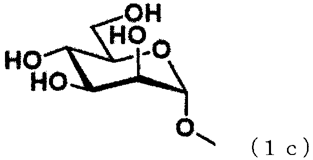

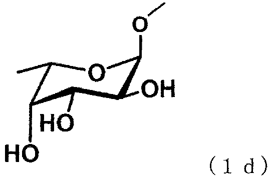

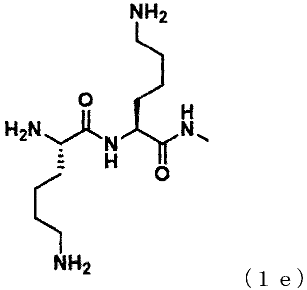

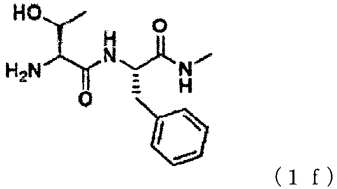

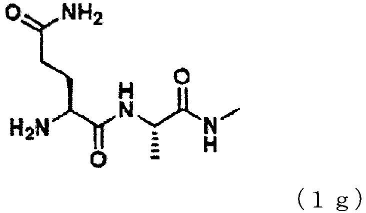

In addition, the linker moiety in the "enzyme recognition site-linker binding site" of the present invention is selected from the group consisting of the following structures (a1) to (f1) and combinations of two or more thereof.

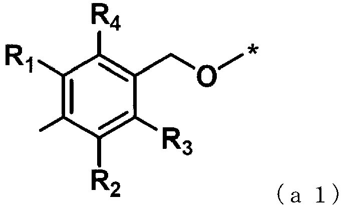

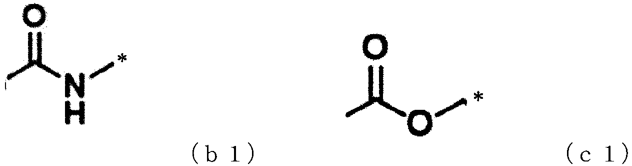

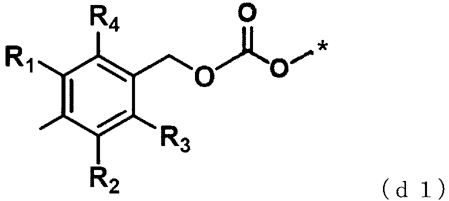

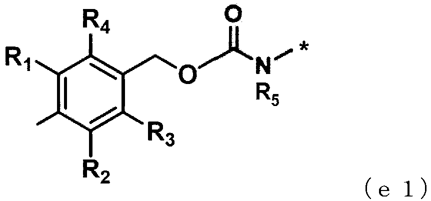

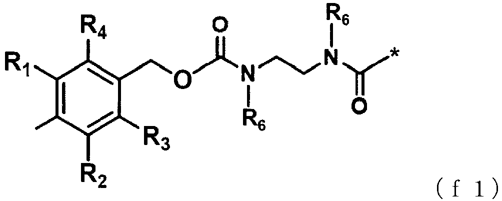

In the above Formulae (a1) and (d1) to (f1), R₁ to R₄ may be the same or different, and are each independently selected from the group consisting of hydrogen, a methyl group, a methoxy group, -COOMe (Me represents a methyl group), -NHMe, F, Cl, Br, and NO₂.

R₅ in Formula (e1) represents hydrogen or an alkyl group having 1 to 3 carbon atoms.

R₆ in Formula (f1) in each occurrence is independently hydrogen or an alkyl group having 1 to 3 carbon atoms.

For example, non-limiting examples of linkers having the structure (a1) include the following.

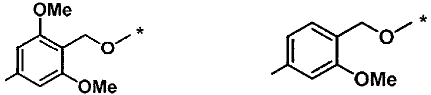

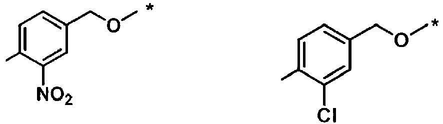

In addition, at least one of R₁ to R₄ in Formulae (d1) to (f1) may be a linking group selected from the following.

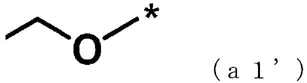

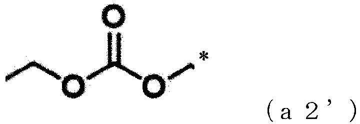

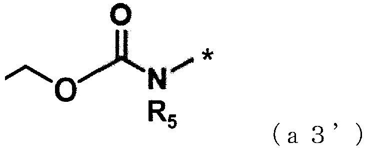

Here, in a case where at least one of R₁ to R₄ is a linking group in the linker represented by Formulae (d1) to (f1), the linker can be also bound to X₁ with the linking group therebetween.

In Formulae (a1) to (f1), * indicates a direction of binding to an anticancer agent, an antibody, or a ligand selected from the group consisting of a small molecule, a peptide, an aptamer, or a hormone-drug conjugate. Here, the linker of Formulae (a1) to (f1), the linking group of (a1') to (a3') may bind in the direction indicated by * with a linker having a binding site which can be used as a binding point to an antibody or a ligand.

In addition, at least one of R₁ to R₄ in Formulae (d1) to (f1) may be a linker having a binding site that can be used as a binding point to an antibody or a ligand.

In addition, in a case where T1 has a structure in which T1 is a combination of two or more of Formulae of (a1) to (f1), * for one or more linkers other than the linker that binds to an anticancer agent, an antibody, or a ligand can represent a direction of binding to the linker.

The anticancer agent includes the same anticancer agent as described in detail in the compound of the present invention.

As the linker having a binding site capable of binding to an antibody or a ligand, polyethylene glycol, alkylene, a peptide, a combination thereof, or the like can be used. In addition, a known linker used for a conjugate of a cell binding molecule (cell binding ligand or receptor analog) and a cytotoxic agent, an antibody-drug conjugate, or the like can be used.

Non-limiting specific structures of the linker moiety in the "enzyme recognition site-linker binding site" of the present invention include the following. 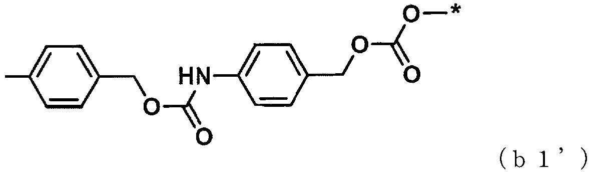 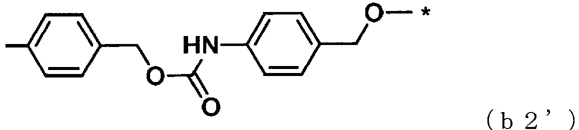 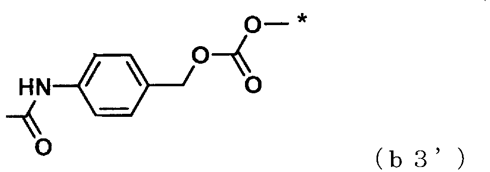
*indicates a direction of binding to an anticancer agent, an antibody or a ligand, and the linker of the above formula may bind to a linker having a binding site that can be used as a binding point to an anticancer agent, an antibody or a ligand toward the direction indicated by *.

In the linkers having the structures, any benzene ring is unsubstituted, but any one or more benzene rings may have substituents defined as R₁ to R₄ above.

A structure in which T is a combination of three or more formulae selected from Formulae (a1) to (f1) can also be configured in the same manner as the structures (b1') to (b3') described above.

The linker represented by T also includes a linker having a linking group selected from (a1') to (a3') as at least one of R₁ to R₄ in Formulae (d1) to (f1) as described above. Also, in the case of a linker having a structure that is a combination of two or more of Formulae (a1) to (f1), the linkers of Formulae (d1) to (f1) may have a linking group selected from (a1') to (a3') as at least one of R₁ to R₄.

These linkers can also bind to an anticancer agent, an antibody or a ligand via the linking group, that is, can bind to an anticancer agent, an antibody or a ligand. Among R₁ to R₄ in Formulae (d1) to (f1), the number of linking groups capable of binding to an anticancer agent, an antibody or a ligand may be one or more, two or more, or three or more.

As the introduction position of the linking group, R₁ and R₂ are preferably in ortho positions with respect to the direction in which the linking group is bound to the enzyme recognition site.

In one aspect of the present invention, in Formulae (d1) to (f1), a linking group selected from (a1') to (a3') is introduced into any one or both of R₁ and R₂.

Further non-limiting structures of the linker moiety in the "enzyme recognition site-linker binding site" of the present invention include the following. 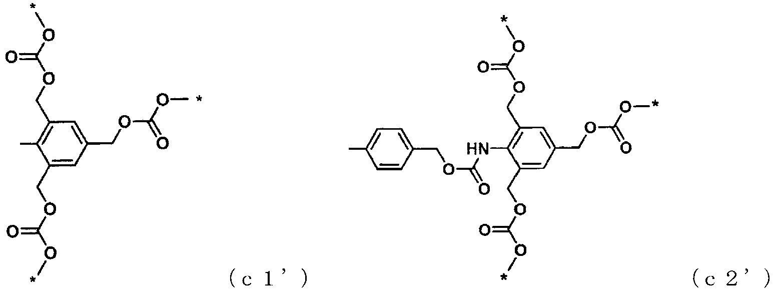 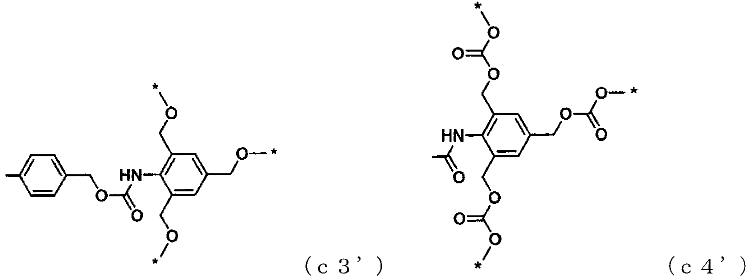
*indicates a direction of binding to an anticancer agent, an antibody or a ligand, and the linker of the above formula may bind to a linker having a binding site that can be used as a binding point to an anticancer agent, an antibody or a ligand toward the direction indicated by *.

Here, in the linker having the structure of (c1'), another linker may be bound to the bond of the benzene ring.

The "enzyme recognition site-linker binding site" of the present invention can be used, for example, in the following embodiments.
(1) Use of an "enzyme recognition site-linker binding site" of the present invention is used for activating an antibody or a ligand (a small molecule, a peptide, an aptamer, a hormone-drug conjugate, or the like) having a payload released by cleavage of the binding site by the enzyme activity specific to the enzyme recognition site-linker binding site.
(2) Use of an "enzyme recognition site-linker binding site" of the present invention is used for preventing prodrug activation or antibody or ligand binding until the enzyme recognition site-linker binding site is cleaved by enzyme activity specific to the binding site.
(3) Use of an "enzyme recognition site-linker binding site" of the present invention is used for activating an antibody or a ligand having a payload that is released or activated by cleaving the enzyme recognition site-linker binding site by the enzyme activity specific to the binding site in a target cell or tissue overexpressing an oncogene.
   Here, the oncogene may be any mutated or activated gene known to drive a cancer, such as a RAS or MYC oncogene.
(4) Use of the "enzyme recognition site-linker binding site" of the present invention for targeting cancer.

Here, the cancer is cancer such as breast cancer, esophageal cancer, lung cancer, stomach cancer, large bowel cancer, small intestine cancer, pancreatic cancer, liver cancer, head and neck cancer, oral cancer, ovarian cancer, brain tumor, kidney cancer, prostate cancer, or skin cancer.

### Examples

Hereinafter, the present invention will be described with reference to examples, but the present invention is not limited thereto.

### I. Screening of glycosidase activity detection fluorescent probes in human lung cancer specimens and development of novel lung cancer detection probes

In development and study of the prodrug-type anticancer agent of the present invention, first, glycosidase activity detection fluorescent probes in human lung cancer specimens was screened. Furthermore, a novel lung cancer detection fluorescent probe found from the results of this screening was evaluated.

### [Example 1]

### Screening for glycosidase activity detection fluorescent probes in human lung cancer specimens

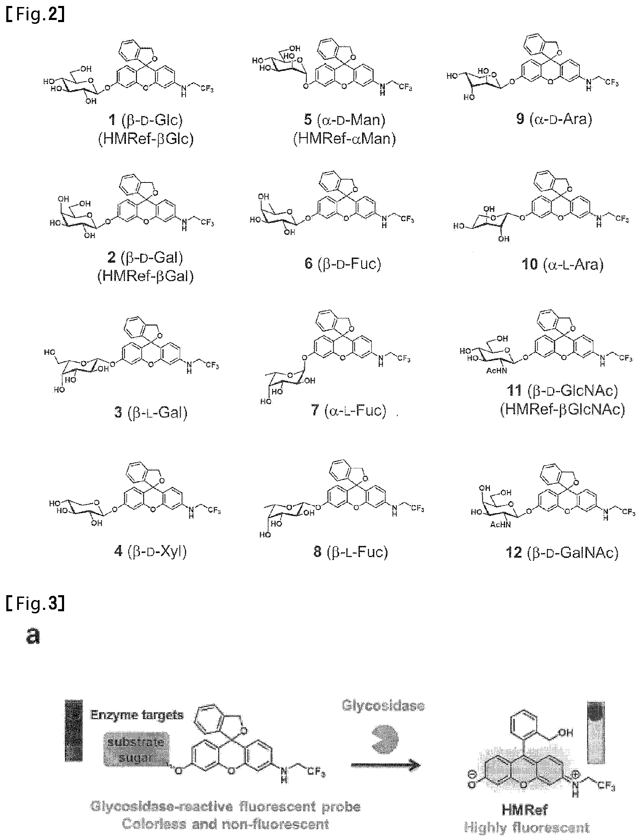

Using 12 glycosidase-reactive fluorescent probes (refer to Fig. 2), screening for the detection of lung cancer was performed in the following procedure.

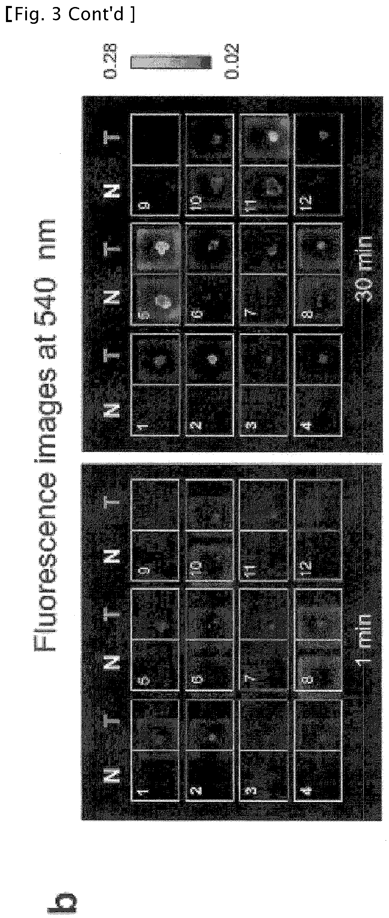

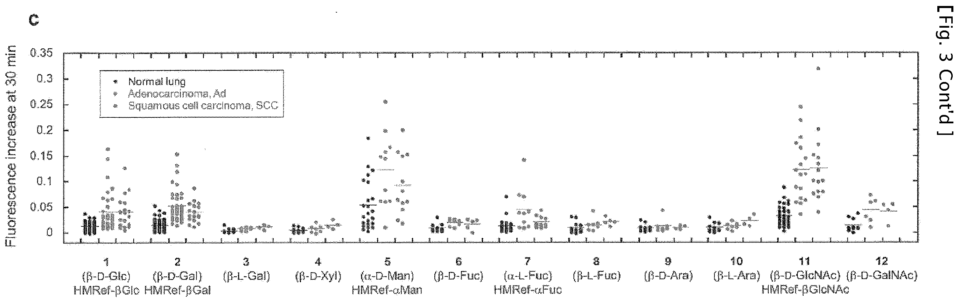

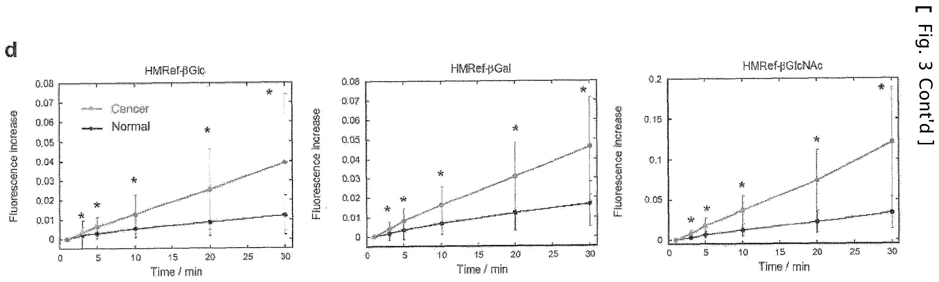

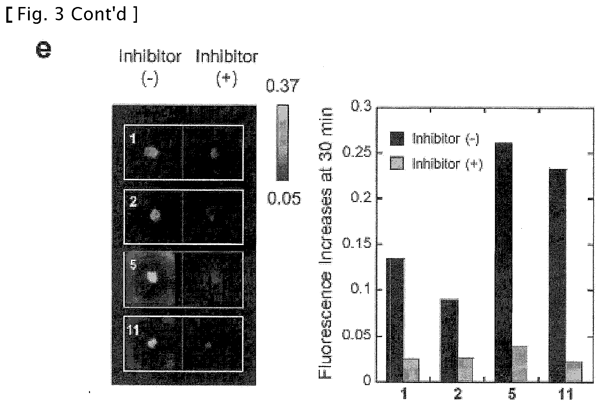

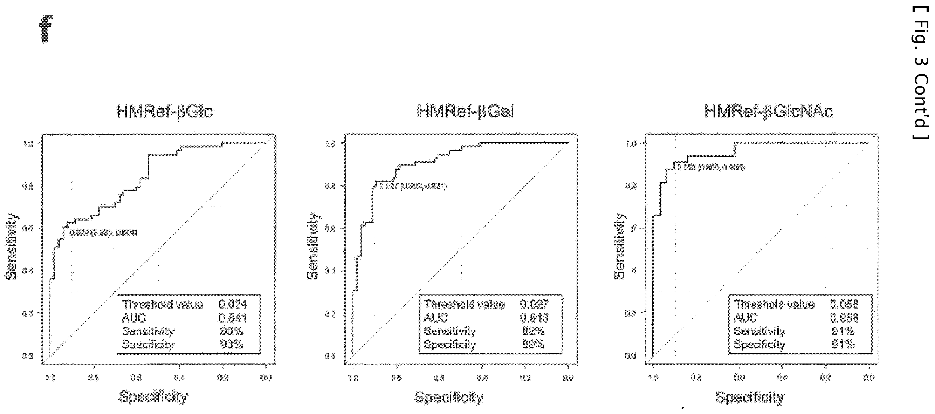

The results are illustrated in Fig. 3.

a of Fig. 3 illustrates a reaction scheme of a glycosidase-reactive probe.

b of Fig. 3 is the result of an example of screening using surgically resected human lung cancer (SCC) and normal tissue. The compound number of the applied probe is displayed in each well. [Fluorescent probe] = 50 µM. A scale bar is 2 cm.

c of Fig. 3 illustrates the results of a comprehensive analysis based on fluorescence imaging of glycosidase activity in normal lung, Ad and SCC tissues using 12 fluorescent probes. Fluorescence increases represent increases from 1 minute to 30 minutes after addition of the fluorescent probe. Each dot represents the fluorescence increases in normal lung, Ad, SCC tissues, respectively, from left to right.

From c of Fig. 3, glycosidase-reactive fluorescent probes 1, 2, 5, and 11 represent the fluorescence increases for both Ad (lung adenocarcinoma) and SCC (lung squamous cell carcinoma) tissues.

d of Fig. 3 illustrates the time-dependent fluorescence increases of probes 1 (also abbreviated as HMRef-βGlc; β-D-Glc), 2 (also abbreviated as HMRef-βGal; β-D-Gal), and 11 (also abbreviated as HMRef-βGlcNAc; β-D-GlcNAc) in normal and cancer (Ad and SCC) lung tissues. Dotted lines represent the fluorescence increases in normal lung tissue. Black lines represent the fluorescence increases in lung cancer (Ad and SCC) tissues. Error bars represent standard deviations.

e of Fig. 3 represents the fluorescence increases after 30 minutes in lung cancer (SCC) tissue in the presence and absence of each inhibitor. The black bar on the right side of the drawing represents the fluorescence increases in the absence of inhibitor and the gray bar represents the fluorescence increases in the presence of inhibitor. The concentrations of probe and inhibitor are as follows.
[Fluorescent probe] = 50 µM, [isofagomin] = 500 µM (for probe 1 (β-D-Glc)), [N-(n-nonyl) deoxygalactonyrimycin] = 500 µM (for probe 2 (β-D-Gal)), [swainsonine] = 500 µM (for probe 5 (α-D-Man)), and [PUGNAc] = 500 µM (for probe 11 (β-D-GlcNAc)).

f of Fig. 3 is an ROC curve of a hit probe in detection of lung cancer.

Table 1 illustrates the lung cancer detection capability of each probe. The threshold, sensitivity, and specificity of each probe were evaluated from a receiver operating characteristic curve. AUC is an area under the curve.

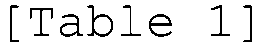

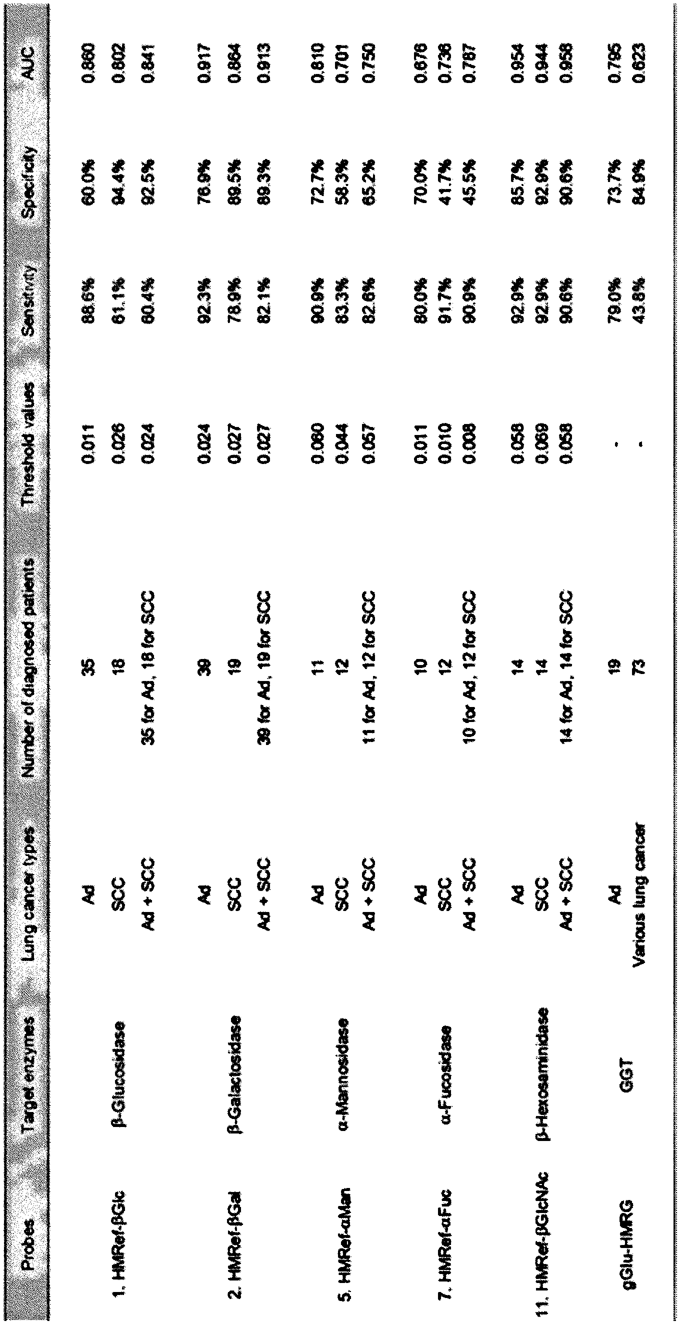

Table 1 illustrates that the probe 11 (HMRef-βGlcNAc) is excellent in both sensitivity and specificity. Therefore, the probe 11 is very effective as a lung cancer detection probe, which is a new discovery even in light of our previous work.

### [Example 2]

### Identification of target enzyme of HMRef-βGlcNAc

Next, the target glycosidase of probe 11 (HMRef-βGlcNAc) found useful as a lung cancer detection probe in Example 1 was identified by the following procedure.

The fluorescence of HMRef-βGal was increased by the addition of β-Galactosidase 1 (GLB1), and the fluorescence was suppressed by the addition of the inhibitor N-(n-nonyl) deoxygalactonoirimycin. Since only GLB1 of human Galactosidase was reported, it was confirmed that the target enzyme of HMRef-βGal was GLB1. The fluorescence of HMRef-βGlcNAc was increased by the addition of β-Hexosaminidase A (HEXA) and β-Hexosaminidase B (HEXB), and the fluorescence was suppressed by the addition of the inhibitor PUGNAc. From this, it was estimated that the target enzymes of HMRef-βGlcNAc were HEXA and HEXB.

In the DEG assay, lysates of lung cancer clinical specimens were separated by two-dimensional electrophoresis based on molecular weight and isoelectric point, and HMRef-βGlcNAc was added to the gel, and a single fluorescence spot was detected. This spot was subjected to peptide mass fingerprinting analysis to identify HEXA and HEXB, which were found to be target enzymes of HMRef-βGlcNAc in lung cancer.

A specific procedure will be described below.

### (1) Confirming reactivity with target purified protein

(1) Purified protein was added at a concentration of 10 ng/µL to a PBS solution of 1 µM for each fluorescent probe, and the fluorescence increase for 60 minutes was evaluated. Each inhibitor was added at a concentration of 10 µM.

### (2) DEG assay

(1) The lysates of lung cancer clinical specimens were separated by two-dimensional electrophoresis based on molecular weight and isoelectric point, and a PBS solution in which 1 µM of HMRef-βGlcNAc was dissolved was added to a gel.
(2) A fluorescence value was measured 24 hours after the addition, and spots where fluorescence was confirmed were resected, washed with PBS, and then frozen at -70°C until evaluation.
(3) The cut-out gel piece was analyzed by peptide mass fingerprinting, and the target enzyme was confirmed from the identified protein list.

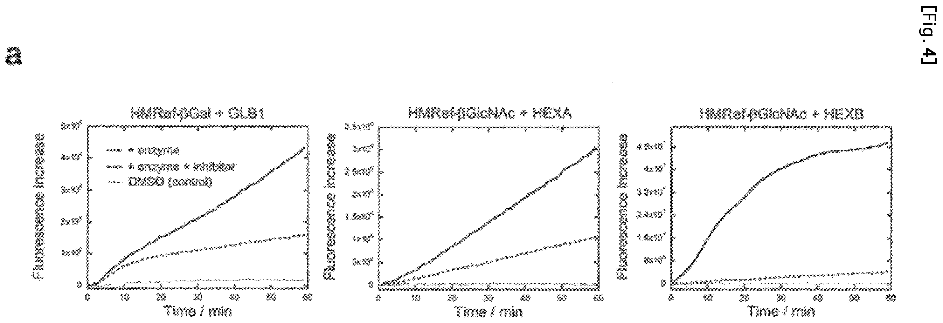

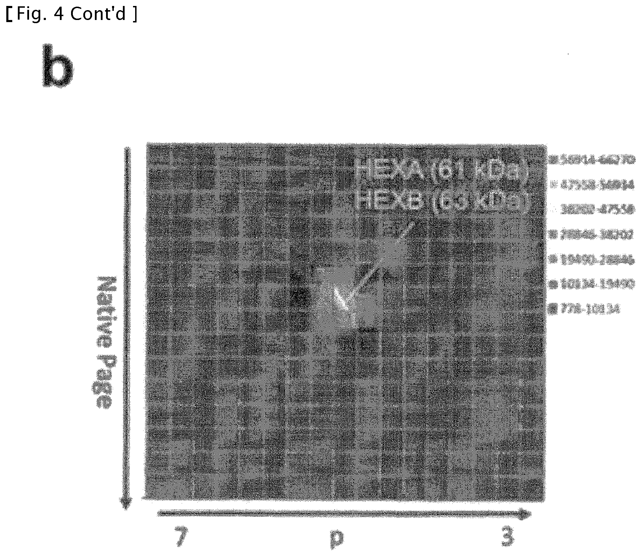

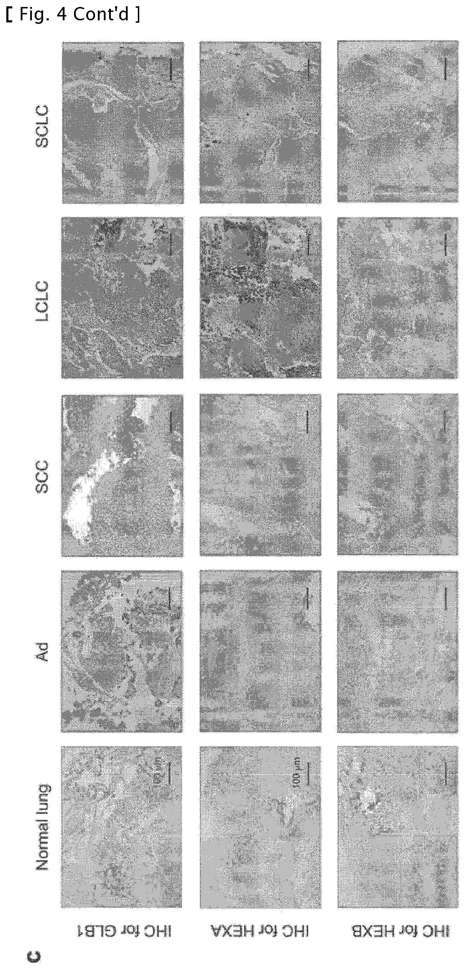

The results are illustrated in Fig. 4.

a of Fig. 4 illustrates the results of a fluorescence analysis assay of a hit fluorescent probe using the corresponding target purified protein in the presence and absence of each inhibitor. [Fluorescent probe] = 1 µM, [Inhibitor] = 10 µM, [Purified protein] = 10 ng/µL.

b of Fig. 4 illustrates the results of a DEG assay of surgically resected lung Ad tissue using HMRef-βGlcNAc. A single fluorescence spot was detected.

c of Fig. 4 is an example of IHC staining of GLB1, HEXA or HEXB in normal lung, Ad, SCC, LCLC, and SCLC tissues. Malignant tissues (lung adenocarcinoma, lung squamous cell carcinoma, small cell lung cancer, large cell lung cancer) tend to have high expression of GLB1, HEXA, and HEXB. The scale bar is 100 µm.

### [Example 3]

### Human lung cancer imaging of HMRef-βGlcNAc

Next, fluorescence imaging of human lung cancer was performed by the following procedure using the probe 11 (HMRef-βGlcNAc).

As a result, it was possible to visualize adenocarcinoma and squamous cell carcinoma of the lung up to minute cancer tissue having a size of 0.2 mm. In addition, it was also possible to visualize specimens of breast cancer lung metastasis in the same manner.

A specific procedure will be described below.
(1) 50 µM of HMRef-βGlcNAc dissolved in PBS (1-2 mL) was sprayed on the specimen to be evaluated, and the fluorescence imaging image was evaluated using a fluorescence imaging device.
(2) After formalin fixation of the specimen after fluorescence imaging, HE and IHC analysis and pathological diagnosis were performed.

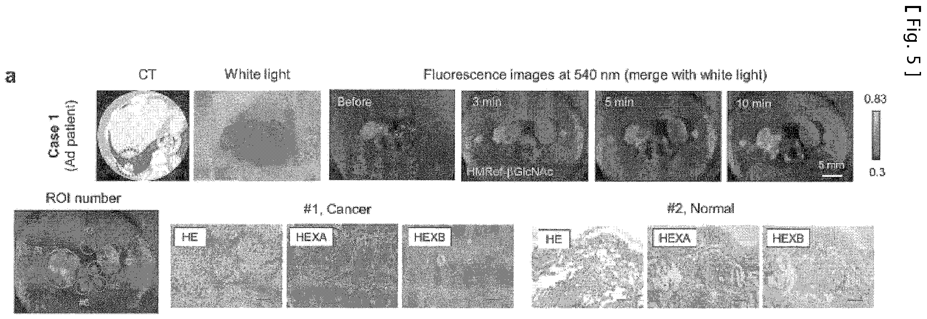

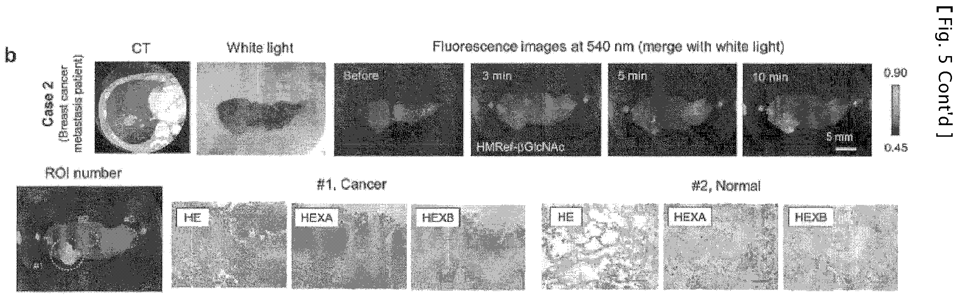

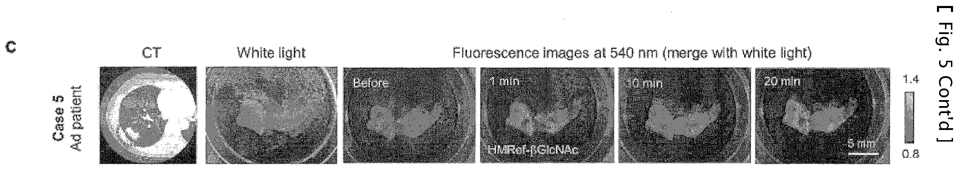

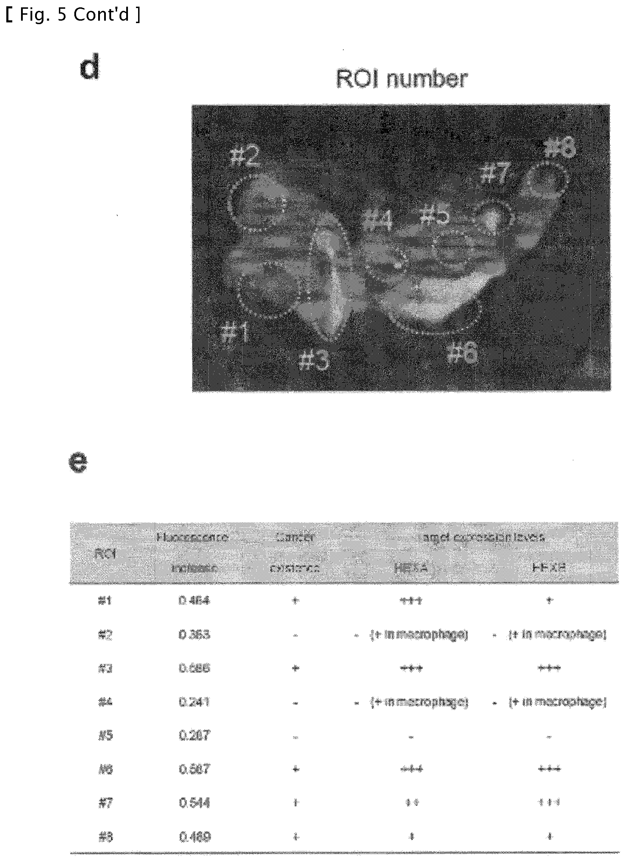

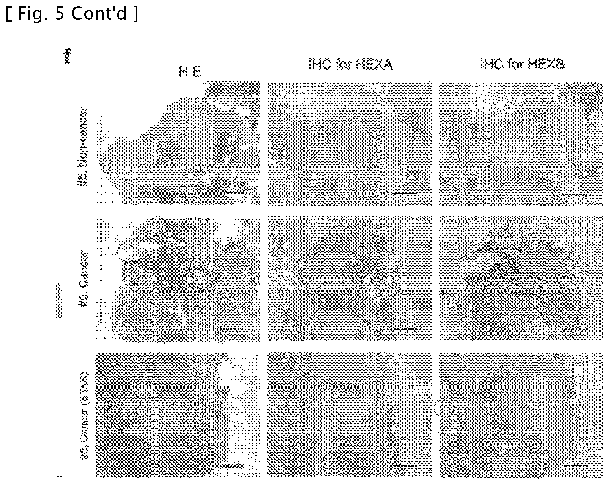

The results are illustrated in Fig. 5. Application of HEXA and HEXB reactive fluorescent probes to lung cancer-specific imaging

a of Fig. 5 illustrates time dependent fluorescence images of surgically resected fresh lung specimens including both normal tissues and cancer (Ad) tissues after HMRef-βGlcNAc administration. [Fluorescent probe] = 50 µM

b of Fig. 5 illustrates time dependent fluorescence images of surgically resected fresh lung specimens including both normal tissues and cancer (metastatic breast cancer to the lung) tissues after HMRef-βGlcNAc administration. [Fluorescent probe] = 50 µM

c of Fig. 5 illustrates time dependent fluorescence images of surgically resected fresh lung specimens including both normal tissues and cancer (Ad) tissues after HMRef-βGlcNAc administration. [Fluorescent probe] = 50 µM

d of Fig. 5 is an enlarged fluorescence image at 30 minutes.

e of Fig. 5 illustrates fluorescence increase and pathology of each ROI shown in d.

f of Fig. 5 illustrates the results of histological analysis and IHC analysis of each target glycosidase (HEXA or HEXB). In case 1-3, the pathological cancer lesion is surrounded by a dotted line.

### [Example 4]

### Visualization example of cancer in intraperitoneal administration and systemic administration of HMRef-βGlcNAc

Using the probe 11 (HMRef-βGlcNAc), visualization of cancer in intraperitoneal administration and systemic administration was performed by the following procedure.

HMRef-βGlcNAc was capable of clearly imaging a minute cancer lesion of 1 mm or less in peritoneal dissemination. Also in the intravenous administration, the lung cancer site in the lung cancer orthotopic transplantation model was clearly visualized, and the result that the background fluorescence was significantly suppressed in organs other than the cancer site was obtained. From these results, it was shown that HMRef-βGlcNAc is useful for visualization of cancer even in the intraperitoneal administration and the systemic administration.

A specific procedure will be described below.
(1) Study on intraperitoneal administration
(1) Luciferase expressing A549 cells 5 × 10⁶ cells/mL (200 µL) was intraperitoneally administered to BALB/cAJcl-nu/nu (7 weeks old). The tumor was grown for one month to prepare peritoneal dissemination model mice.
(2) 50 µM of HMRef-βGlcNAc dissolved in PBS (300 µL) was administered to peritoneal dissemination model mice by intraperitoneal administration.
(3) After 1 hour, the mice were sacrificed by cervical dislocation, and then the abdomen was opened to take out the intestinal tract and the mesentery, and fluorescence imaging was performed with an in vivo fluorescence imaging device.

### (2) Study on intravenous administration

(1) Model mice were created by the following operation using BALB/cAJcl-nu/nu (7 weeks old). The skin of the chest was incised, and the skin was subcutaneously detached, then luciferase-expressing A549 cells 2 × 10⁷ cells/mL (50 µL, 5% Matrigel) was injected from the intercostal pleura to the left lung. After injection, the skin was sutured. A tumor was grown for 1 month to prepare lung cancer orthotopic transplantation model mice.
(2) 10 mg/kg of HMRef-βGlcNAc dissolved in PBS (100 µL) was administered to lung cancer orthotopically transplanted mice by intravenous administration.
(3) 30 minutes later, the mice were sacrificed by cervical dislocation, and the abdomen and thorax were opened to expose the lungs, followed by ex vivo fluorescence imaging using a fluorescence imaging device.

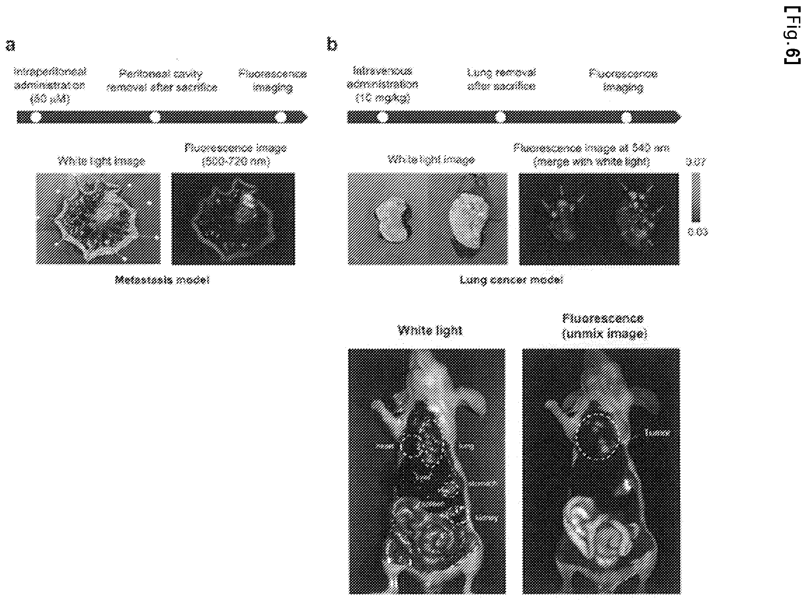

The results are illustrated in Fig. 6.

a of Fig. 6 illustrates the results of ex vivo fluorescence imaging of freshly resected abdominal cavity after intraperitoneal administration of 50 µM of HMRef-βGlcNAc to A549 peritoneal metastasis mice.

b of Fig. 6 illustrates the results of ex vivo fluorescence imaging of freshly resected lungs after intravenous administration of 10 mg/kg of HMRef-βGlcNAc to A549 lung cancer orthotopically transplanted mice.

### II. Development of prodrug-type anticancer agent of present invention

Next, development and study of a novel prodrug-type anticancer agent were conducted by introducing a substrate site targeting cancer-specific enzyme activity found by screening for fluorescent probes in human lung cancer specimens in Example 1 into a molecule of SN38.

### (A) Development of novel prodrug-type anticancer agent targeting glycosidase activity

### 1. Synthesis of prodrug compounds

Prodrug compounds based on three kinds of SN38 were synthesized by the following procedure, in which -βGal, - βGlcNAc, and -αMan were introduced into a molecule of SN38 among substrate sites confirmed to be promising as substrate sites for targeting cancer-specific enzyme activity in Example 1.

### [Synthesis Example 1]

### Synthesis of SN38-βGal

SN38-βGal, which is a compound of the present invention, was synthesized according to the following Scheme 1.

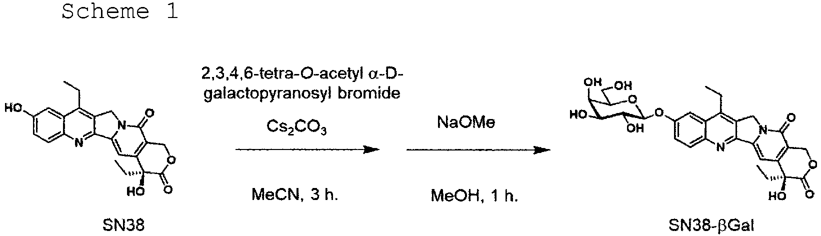

7-ethyl-10 hydroxycaptothecin (SN38) (190 mg, 0.484 mmol), 2,3,4,6-tetra-O-acetyl-α-D-galactopyranosyl bromide (1.50 g, 3.65 mmol), and Cs₂CO₃ (1.00 g, 3.07 mmol) were dissolved in 10.0 mL of acetonitrile, and the mixture was stirred at room temperature for 3 hours. The solvent was distilled off under reduced pressure, and silica gel column chromatography (CH₂Cl₂: MeOH = 95:5) was performed to obtain an acetylated sugar adduct of SN38. This was dissolved in 5.0 mL of MeOH, and NaOMe (130 mg, 2.41 mmol) dissolved in 5.0 mL of MeOH was added dropwise thereto, then the mixture was stirred at room temperature for 1 hour. The solvent was distilled off under reduced pressure and purified by HPLC, followed by lyophilization to obtain the target product SN38-βGal (188 mg, 0.339 mmol) as a yellow solid at a yield of 70.1%. The obtained target product was identified by ¹HNMR, ¹³CNMR, and ESI-HRMS.

¹HNMR (400 MHz, DMSO-d6): δ8.10 (d, J = 9.6 Hz, 1H), 7.74 (s, 1H), 7.58 (dd, J = 1.2 Hz, 9.2 Hz, 1H), 7.28 (s, 1H), 5.43 (s, 2H), 5.30 (s, 2H), 5.10 (d, J = 7.6 Hz, 1H, overlaps with HOD), 3.75-3.68 (m, 3H), 3.60-3.20 (m, 2H), 3.50 (dd, J = 2.4 Hz, 9.2 Hz, 1H), 3.21-3.20 (m, 2H), 1.95-1.80 (m, 2H), 1.31 (t, J = 7.2 Hz, 3H), 0.88 (t, J = 7.2 Hz, 3H), ¹³CNMR (101 MHz, DMSO-d6): 5172.5, 156.8, 156.1, 150.1, 150.0, 146.2, 144.7, 144.2, 131.2, 128.3, 127.6, 122.5, 118.4, 106.6, 101.1, 96.1, 75.9, 73.3, 72.4, 70.2, 68.2, 65.2, 60.5, 49.5, 30.2, 22.2, 13.5, 7.7. ESI-HRMS (ESI +) m/z calcd. for [M+Na]+, 577.17981; found, 577.18002.

### [Synthesis Example 2]

### Synthesis of SN38-βGlcNAc

SN38-βGlcNAc which is a compound of the present invention was synthesized according to the following Scheme 2.

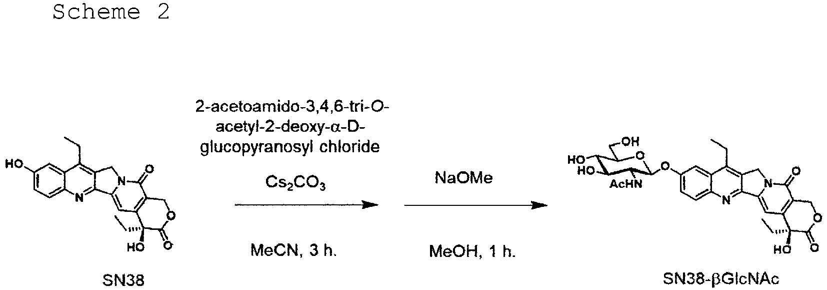

7-ethyl-l-10 hydroxycamptothecin (SN38) (1.12 g, 2.85 mmol), 2-acetamide-3,4,6-tri-O-acetyl-2 deoxy-α-D-glucopyranosyl chloride (3.2 g, 8.75 mmol), and Cs₂CO₃ (3.1 g, 9.51 mmol) were dissolved in 100 mL of acetonitrile, and the mixture was stirred at normal temperature for 3 hours. The solvent was distilled off under reduced pressure, and silica gel column chromatography (CH₂Cl₂: MeOH = 95:5) was performed to obtain an acetylated sugar adduct of SN38. This was dissolved in 30 mL of MeOH, and NaOMe (700 mg, 13.0 mmol) dissolved in 5.0 mL of MeOH was added dropwise thereto, then the mixture was stirred at room temperature for 1 hour. The solvent was distilled off under reduced pressure and purified by HPLC, followed by lyophilization to obtain the target product SN38-βGlcNAc (456 mg, 0.766 mmol) as a yellow solid at a yield of 26.8%. The obtained target product was identified by ¹HNMR, ¹³CNMR, and ESI-HRMS.

¹HNMR (400 MHz, CD3OD): 57.57 (d, J = 9.2 Hz, 1H), 7.38 (s, 1H), 7.24 (s, 1H), 7.20 (d, J = 2.4 Hz, 1H), 5.39 (d, J = 16 Hz, 1H), 5.17 (d, J = 16 Hz, 1H), 5.13 (d, J = 8.4 Hz, 1H), 3.99-3.93 (m, 2H), 3.75 (dd, J = 6.8 Hz, 12 Hz, 1H), 3.62 (t, J = 8.8 Hz, 1H), 3.55-3.51 (m, 1H), 3.39 (t, J = 9.2 Hz, 1H), 2.98-2.94 (m, 2H), 1.98 (s, 3H), 1.86-1.78 (m, 2H), 1.26 (t, J = 7.6 Hz, 3H), 0.90 (t, J = 7.6 Hz, 3H), ¹³CNMR (101 MHz, CD3OD): δ174.8, 174.0, 158.8, 157.7, 152.6, 160.6, 147.4, 146.5, 145.8, 131.8, 129.0, 128.7, 123.6, 119.8, 108.1, 100.8, 99.1, 79.0, 75.8, 74.1, 72.2, 66.6, 62.8, 57.4, 40.4, 32.2, 23.9, 23.2, 14.1, 8.2. ESI-HRMS (ESI +) m/z calcd. for [M+Na]+, 618.20582; found, 618.20633.

### [Synthesis Example 3]

### Synthesis of SN38-αMan

SN38-αMan, which is a compound of the present invention, was synthesized according to the following Scheme 3.

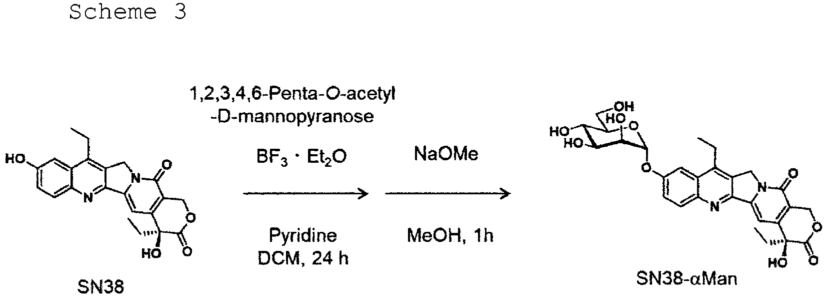

7-ethyl-10-hydroxycamptothecin (SN38) (100 mg, 0.255 mmol), 1,2,3,4,6-penta-O-acetyl-D-mannopyranose (1.00 g, 2.56 mmol) were dissolved in 8.0 mL of dichloromethane, and BF₃ · Et₂O (8.0 mL) and pyridine (2.5 mL) were added, then the mixture was stirred at room temperature under an argon atmosphere for 48 hours. After neutralization with a saturated aqueous sodium bicarbonate solution, an organic phase was washed with water and saturated saline, and released with sodium sulfate. After sodium sulfate was removed, the solvent was distilled off under reduced pressure, and silica gel column chromatography (CH₂Cl₂: MeOH = 95:5) was performed to obtain an acetylated sugar adduct of SN38. This was dissolved in 4.0 mL of MeOH, and NaOMe (150 mg, 2.78 mmol) dissolved in 4.0 mL of MeOH was added dropwise thereto, then the mixture was stirred at room temperature for 1 hour. The solvent was distilled off under reduced pressure and purified by HPLC, followed by lyophilization to obtain the target product SN38-αMan (49.0 mg, 0.0884 mmol) as a yellow solid at a yield of 34.7%. The obtained target product was identified by ¹HNMR, ¹³CNMR, and ESI-HRMS.

¹HNMR (400 MHz, DMSO-d6): δ8.03 (dd, J = 1.2 Hz, 9.2 Hz, 1H), 7.76 (s, 1H), 7.54 (d, J = 9.2 Hz, 1H), 7.21 (d, J = 1.2 Hz, 1H), 5.58 (s, 1H), 5.36 (s, 2H), 5.23 (s, 2H), 3.87 (d, J = 1.6 Hz, 1H), 3.70 (dd, J = 2.4 Hz, 9.2 Hz, 1H), 3.57-3.38 (m, 4H), 3.09 (d, J = 7.6 Hz, 2H), 1.90-1.70 (m, 2H), 1.25 (t, J = 7.6 Hz, 3H), 0.81 (t, J = 5.2 Hz, 3H) .¹³CNMR (101 MHz, DMSO-d6): δ172.5, 156.8, 155.1, 150.1, 150.0, 146.2, 144.8, 144.2, 131.3, 128.3, 127.6, 122.9, 118.4, 107.2, 99.1, 96.1, 75.1, 72.4, 70.6, 70.0, 66.7, 65.2, 61.0, 49.5, 30.2, 22.3, 13.5, 7.7. ESI-HRMS (ESI +) m/z calcd. for [M+Na]+, 577.17927; found, 577.17869.

### [Example 5]

### Cytotoxicity test of developed prodrugs by MTT assay method

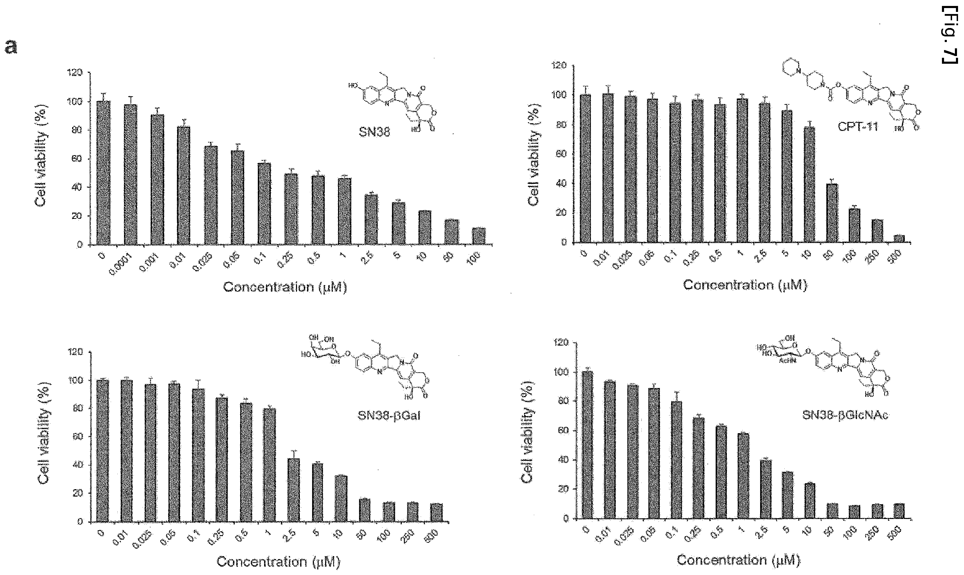

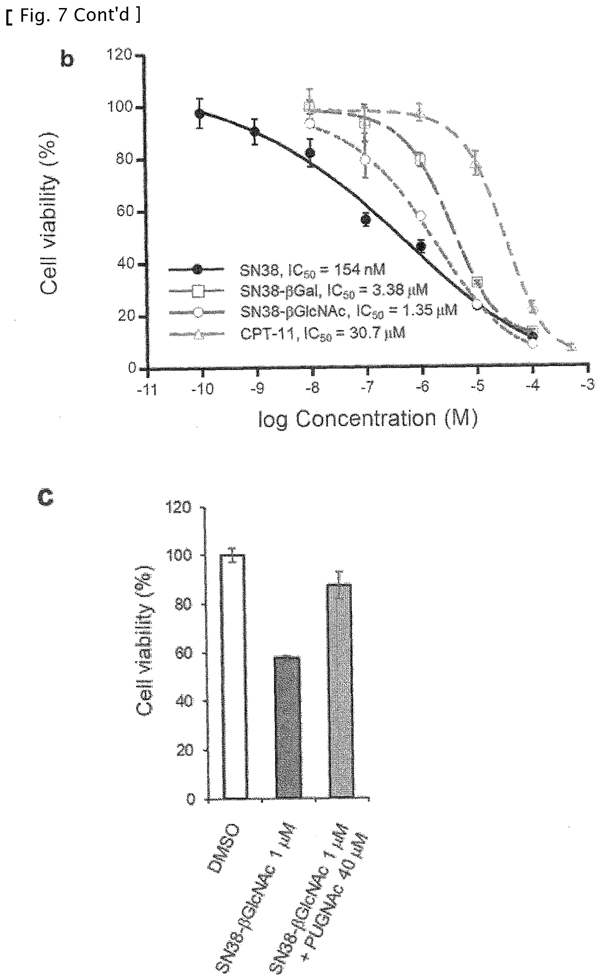

A cytotoxicity test of SN38-βGal and SN38-βGlcNAc in cells of lung adenocarcinoma cell A549 was performed by an MTT assay method. As a result, cell proliferation was suppressed as the concentrations of SN38-βGal and SN38-βGlcNAc increased (Fig. 7). Each IC50 value under the following measurement conditions were calculated as SN38 (154 nM), SN38-βGal (3.38 µM), SN38-βGlcNAc (1.35 µM), and CPT-11 (30.7 µM). SN38-βGal and SN38-βGlcNAc showed lower IC50 values than CPT-11. In addition, with regard to SN38-βGlcNAc, it was confirmed that the cytotoxicity was significantly reduced by an inhibitor PUGNAc of β-hexosaminidase (β-hexosaminidase) as a target enzyme. In this test, Cell Proliferation Kit I (MTT) and SIGMA-ALDRICH were used.

The procedure of the specific cytotoxicity test is shown below.
(1) 100 µL of DMEM containing 1.0 × 10⁴ cells of A549 cells, 10%FBS, and 1%PS was added to a 96 well plate, and the plate was incubated in an environment of 37°C and 5% CO₂ for 24 hours.
(2) 10 µL of a medium containing each concentration of the drug was added, and the mixture was incubated in a 37°C, 5% CO₂ environment for 72 hours.
(3) 10 µL of MTT labeling reagent was added and incubated in a 37°C, 5% CO₂ environment for 4 hours.
(4) 100 uL solubilization buffer was added and incubated in 5% CO₂ environment for 24 hours.
(5) The absorbance at 560 nm was measured, cell viability was evaluated, and the IC50 value was calculated.

Fig. 7 illustrates results of the cytotoxicity test by MTT assay. a of Fig. 7 illustrates the result of suppression of cell proliferation at each drug concentration of lung adenocarcinoma A549 cells, and b of Fig. 7 illustrates the cell proliferation suppression curve and IC50 value of each drug. c of Fig. 7 illustrates suppression of drug efficacy in the presence of an inhibitor.

### [Example 6]

### Cytotoxicity test using JFCR39 cell panel

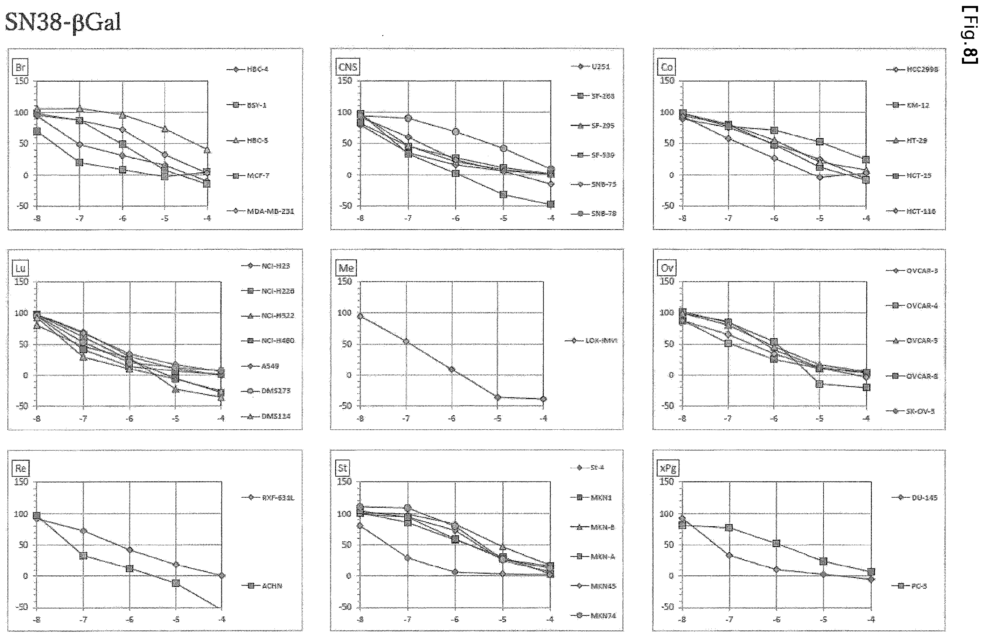

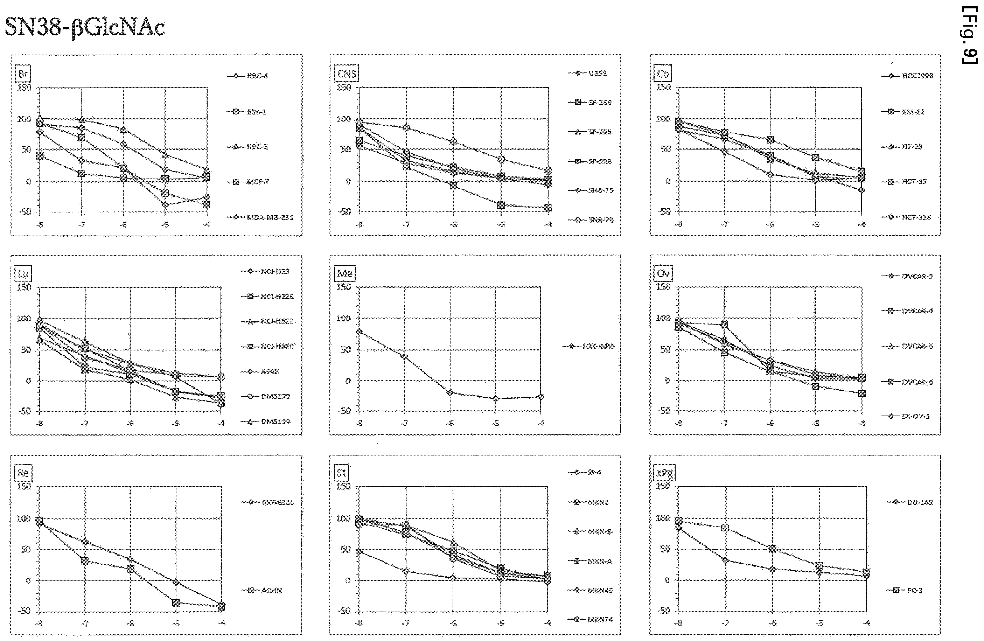

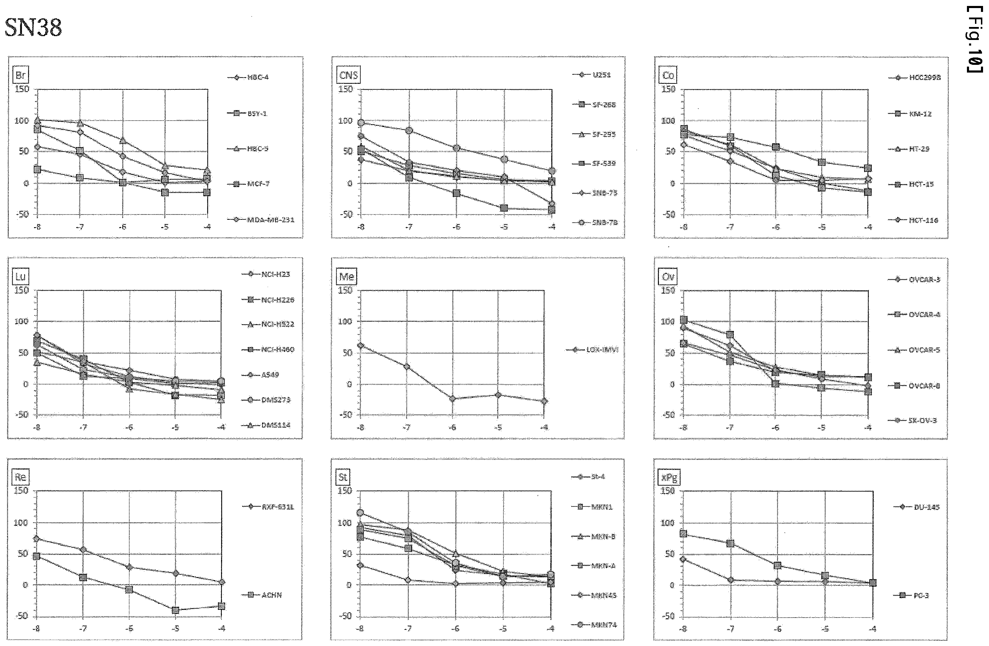

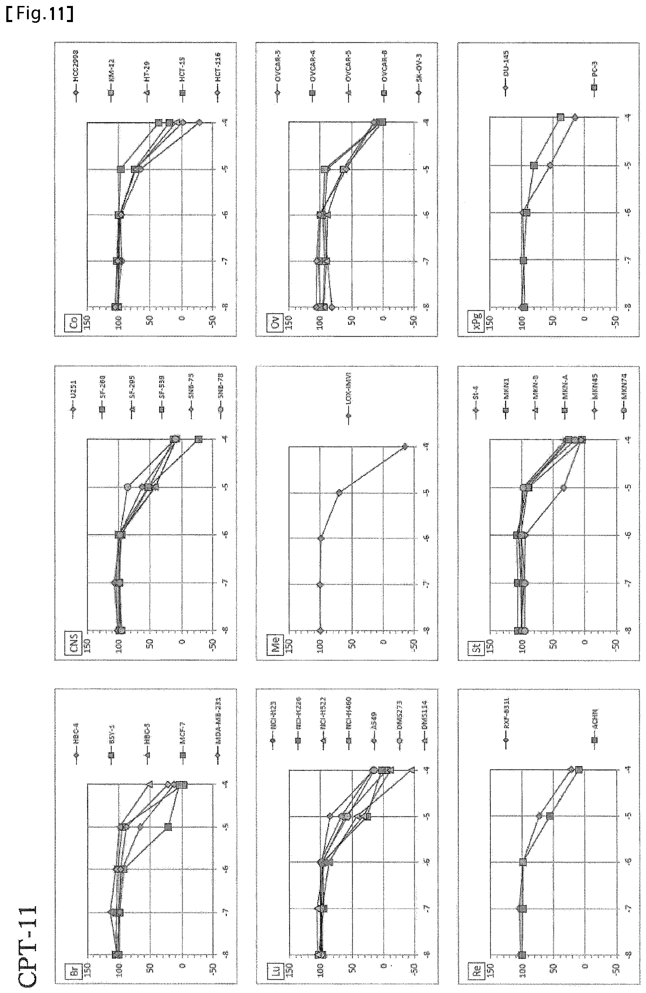

Cell proliferation of SN38-βGal, SN38-βGlcNAc, SN38, and CPT-11 was evaluated for a total of 39 cell lines of JFCR39 cell panel (lung cancer type 7, gastric cancer type 6, large bowel cancer type 5, ovarian cancer type 5, brain tumor type 6, breast cancer type 5, renal cancer type 2, prostate cancer type 2, and melanoma type 1). As illustrated in Figs. 8 to 11, SN38-βGal and SN38-βGlcNAc suppressed proliferation of almost all cancer cells, and showed a GI50 value close to SN38 and lower than CPT-11 (Table 2).

A specific test procedure is shown below.

The cancer cells were seeded in a 96 well plate, and on the next day, the drug solution was added according to the dilution series, and after culturing for 2 days (48 hours), cell proliferation was determined by colorimetric assay using sulforhodamine B.

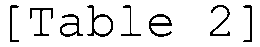

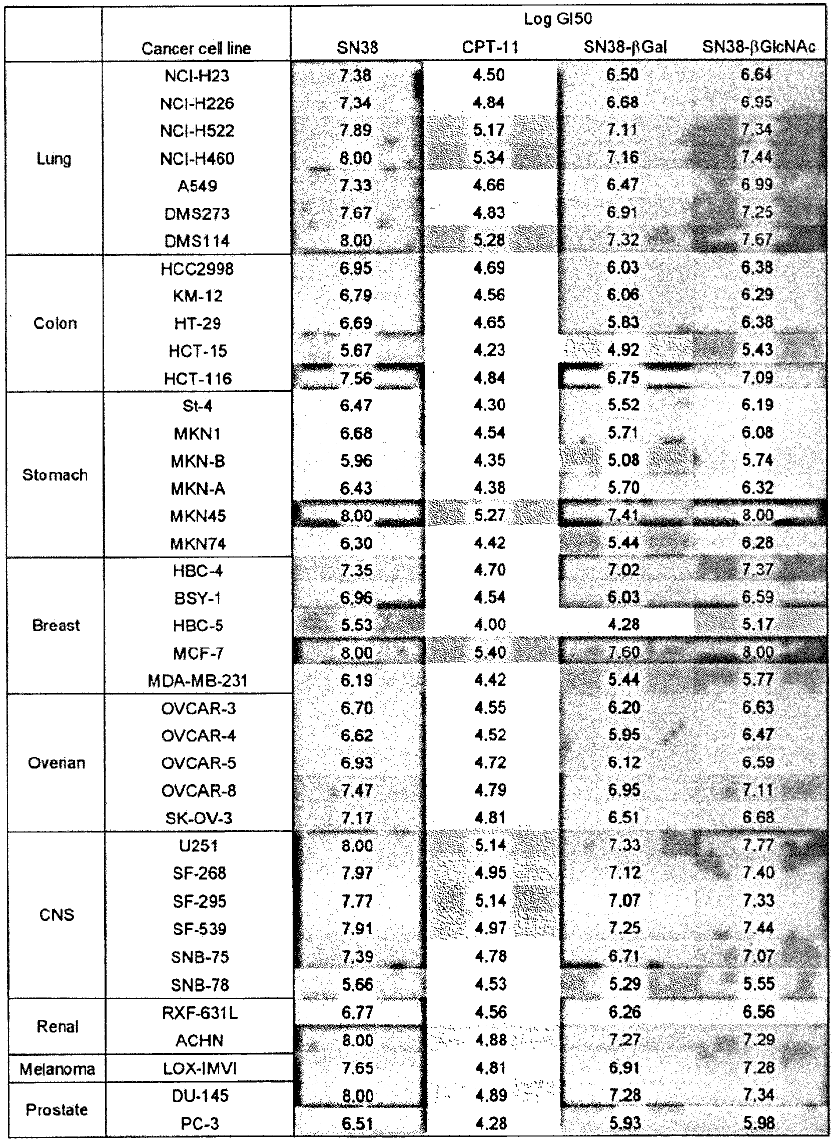

### [Example 7]

### Evaluation of drug release using LC-MS

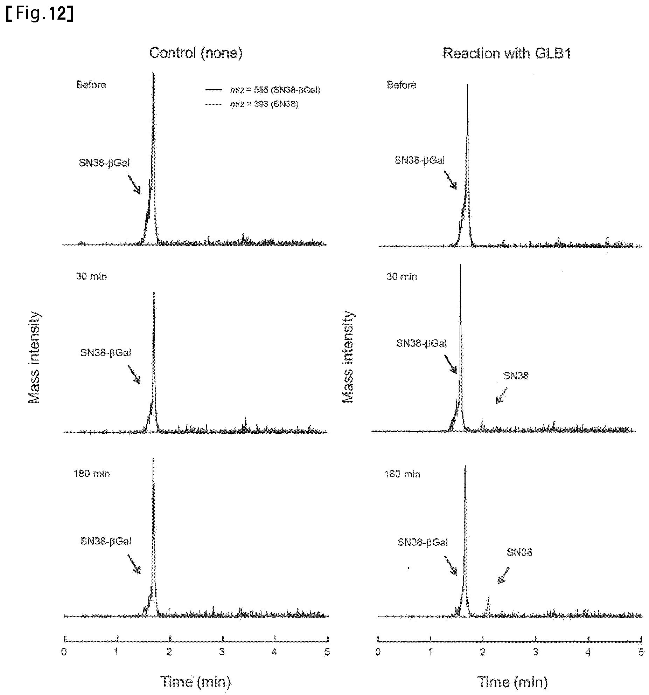

Reactivity with Human β-Galactosidase 1 (GLB1), which is an assumed target enzyme of SN38-βGal, was confirmed using LC-MS. As a result, when the GLB1 purified enzyme was added, the release of SN38 showing an anticancer effect was confirmed (Fig. 12).

Fig. 12 illustrates results of confirming the reactivity of SN38-βGal with GLB1 by LC-MS analysis. SN38-βGal releases SN38 with anticancer activity in the presence of GLB1.

### [Example 8]

### Kinetic test in intraperitoneal administration (i.p.) of SN38-βGal

### Mice (intraperitoneal)

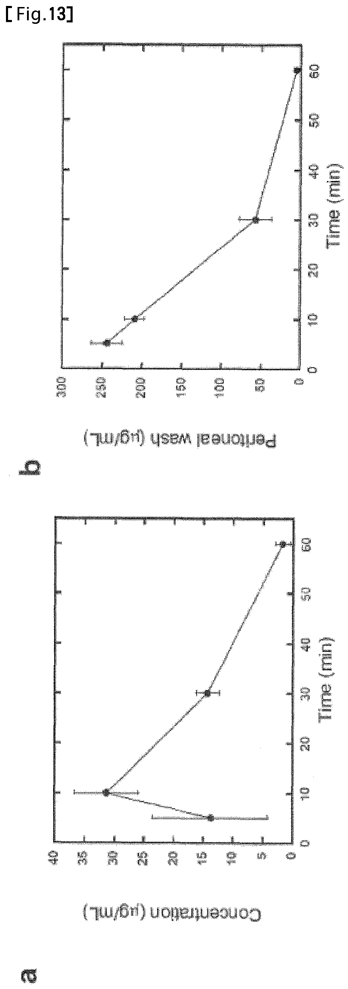

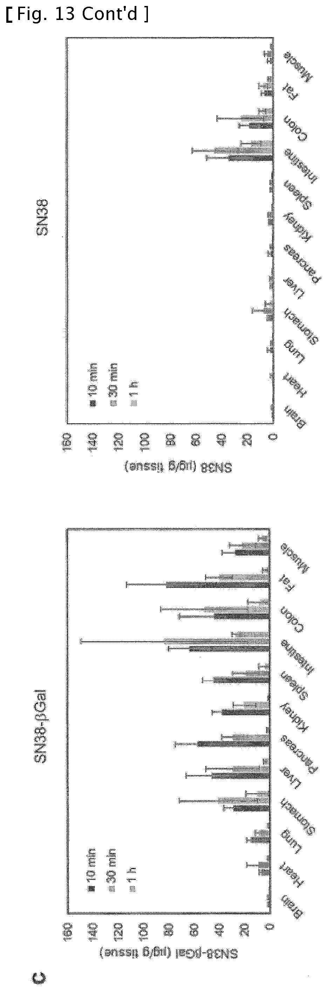

The evaluation of pharmacokinetics in 30 mg/kg intraperitoneal administration (i.p.) of SN38-βGal was performed. The blood concentration of SN38-βGal was the highest around 10 minutes after administration, and almost disappeared at 60 minutes. The blood half-life was about 30 minutes. In the evaluation of the change in intraperitoneal concentration, the intraperitoneal concentration decreased to about 17% at 30 minutes, and almost disappeared from the abdominal cavity at 60 minutes. When organ distribution of SN38-βGal was evaluated, distribution in each organ was confirmed at 10 minutes to 60 minutes after administration. In particular, it was suggested that the absorption from the small intestine was large, and only a slight distribution was confirmed in the brain. The distribution of SN38 of the active form was also observed in a small amount in each organ, and particularly in the small intestine and the large intestine (Fig. 13). Pharmacokinetic parameters in the intraperitoneal administration of SN38-βGal are shown in Table 3.

A specific procedure of the pharmacokinetics test is shown below.
(1) SN38-βGal (30 mg/kg) was dissolved in 100 µL of PBS and administered by i.p.
(2) 150 to 200 µL of anesthesia (in the case of a mixture of three types of Domitor + Betorphal + Mitazolam) was intraperitoneally administered 5 minutes before dissection at each time.
(3) The inside of a syringe was coated with sodium heparin, and at each evaluation time, the mouse was quickly stuck to the dissecting pad, and the abdomen was opened without bleeding, and whole blood was collected from the right ventricle with a 23 G needle.
   100 µM β-Galactosidase inhibitor was added to whole blood.
(4) After whole blood collection, brain, heart, lung, liver, pancreas, spleen, kidney, stomach, small intestine, large intestine, muscle, and fat were taken out. As the muscle, a muscle near the thigh was resected. The weight of each organ was measured.
(5) Whole blood was centrifuged at 1200 G for 15 min at 4°C, and 200 µL of supernatant was collected. 800 µL of a solution of MeOH/MeCN = 1:1 (containing Rhodamine 123 = 25 µM as an internal standard) was added thereto, and the mixture was allowed to stand for 10 minutes.
(6) After standing, centrifugation was performed at 1700 G for 15 min at 4°C, and the supernatant was collected. About 80 µL of the mixture was fractionated, loaded on a Cosmo Spin Filter G (0.2 um), centrifuged at 12000 G for 5 min at 4°C, and filtered to collect a sample.
(7) Each resected organ was finely cut and put into a tube of Lysing Matrix D, 1000 µL of Lysis Buffer (added with 100 µM β-Galactosidase inhibitor) was added thereto, and the mixture was crushed with a shaker.
(8) The supernatant was collected from the tube of Lysing Matrix, and then centrifuged at 10,000 G for 5 min at 4°C to collect the supernatant to obtain a lysate solution.
(9) To 100 µL of the organ lysate solution, 200 µL of MeOH/MeCN = 1:1 (containing 30 µM of Rhodamine 123 as an internal standard) was added, and the mixture was allowed to stand for 10 minutes.
(10) Centrifugation was performed at 1700 G for 15min at 4°C to collect 200 µL of the supernatant. The supernatant was loaded on a Cosmo Spin Filter G (0.2 µm) and centrifuged at 12000 G for 5 min at 4°C to collect a sample.
(11) 50 µL of purified water and 50 µL of a filtered plasma sample or organ lysate sample were mixed, 10 µL of the mixture was injected into HPLC, and data of the area under the curve of the PDA spectrum at 380 nm (derived from SN 38) and 490 nm (derived from Rhodamine 123) were acquired, and then the respective numerical values were calculated from the volume for blood and the weight for organs by one inspection amount of Rhodamine 123.

A specific procedure of the intraperitoneal concentration evaluation test is shown below.
(1) Intraperitoneal administration (i.p.) was performed on an evaluation amount of the drug.
(2) 2.0 mL of PBS was administered by i.p. at each evaluation time, and the abdomen was immediately massaged and sacrificed so that the drug could be collected as much as possible on the PBS side after administration. A small hole was made in the abdomen, and PBS in the abdominal cavity was quickly collected with a 1.0 mL syringe in a pushing manner, and this solution was used as an peritoneal washing solution.
(3) To 200 µL of the collected peritoneal washing solution, 800 µL (containing Rhodamine 123 = 25 µM) of 800 µL MeCN/MeOH = 1:1 was added to precipitate proteins. The supernatant was collected by centrifugation at 1700 G for 5 min at 4°C.
(4) 80 µL of the recovered supernatant was loaded on a cosmo spin filter G and centrifuged at 12000 G for 5 min at 4°C, and 50 µL of purified water was added to 50 µL of the recovered material to adjust the total amount to 100 µL to prepare a sample.
(5) The concentration of SN38-βGal was measured by HPLC, and the concentration of the recovered peritoneal washing solution was calculated.

**[Table 3]**

| PHARMACOKINETIC PARAMETERS IN INTRAPERITONEAL ADMINISTRATION OF SN38-βGal | | | | | | |
|---|---|---|---|---|---|---|
| Drugs (30 mg/kg) | | t_{1/2} (h) | AUC₀₋₁ₕ (µg · h/mL) | AUC_{0-∞} (µg · h/mL) | CLₜₒₜ (mL/min/kg) | V_{d} (L/kg) |
| SN38-βGal | i.p. | 0.197 | 15.39 | 15.88 | 31.5 | 0.630 |

Fig. 13 illustrates results of pharmacokinetics in 30 mg/kg intraperitoneal administration (i.p.) of SN38-βGal. a of Fig. 13 illustrates a change in blood concentration. b of Fig. 13 illustrates an intraperitoneal concentration change, and c of Fig. 13 illustrates organ distributions of SN38-βGal and SN38.

### [Example 9]

### Toxicity tests

### (1) Single dose toxicity test 1

### Mice (intravenous)

Single dose toxicity was evaluated by intravenously administering 200 mg/kg each of the novel prodrug-type anticancer agents SN38-βGal and SN38-βGlcNAc to Jcl: ICR mice (female). Immediately after administration, reduced locomotor activity, tremor, and prone states were found in more than half of the mice in both groups, but these symptoms were improved in several hours after administration. Since no death was observed immediately after and 10 days after administration in either group, the LD50 value was estimated to be 200 mg/kg or more (Table 4). In CPT-11, which is an existing drug, the LD50 value is reported to be 132.4 mg/kg in mice (female), and thus it was confirmed that the LD50 value was significantly larger than that in CPT-11.

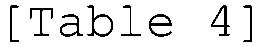

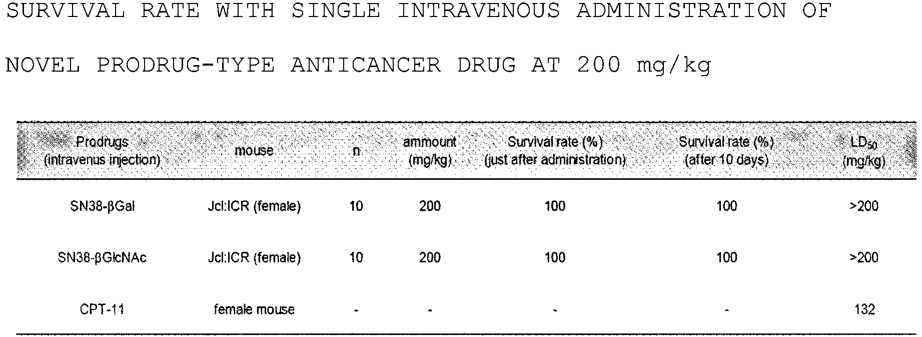

### (2) Single dose toxicity test 2

### Mice (intravenous)

Single dose toxicity was evaluated by intravenously administering the novel prodrug-type anticancer agents SN38-βGal and SN38-βGlcNAc to Jcl: ICR mice (female). SN38-βGal was intravenously administered at 300 mg/kg from the upper limit of solubility. 500 mg/kg of SN38-βGlcNAc was intravenously administered. Immediately after administration, reduced locomotor activity and prone states were found in more than half of the mice in both groups, but these symptoms were improved in several hours after administration. Since no death was observed immediately after and 14 days after administration in either group, the LD50 value was estimated to be 300 mg/kg or more in SN38-βGal and 500 mg/kg or more in SN38-βGlcNAc (Table 5). In CPT-11, which is an existing drug, the LD50 value is reported to be 132.4 mg/kg in mice (female), and thus it was confirmed that the LD50 value was significantly larger than that in CPT-11.

The specific toxicity test procedure is shown below.
(1) Jcl: ICR mice (7 weeks old, female) were intravenously administered with 100 µL of a drug dissolved in PBS through the tail vein.
(2) The state immediately after administration was observed for about 30 minutes, and the survival rate 14 days after administration was further evaluated.

**[Table 5]**

| EVALUATION OF LD50 VALUE BY INTRAVENOUS ADMINISTRATION | | | | | | |
|---|---|---|---|---|---|---|
| Prodrugs (intravenus injection) | mouse | n | ammount (mg/kg) | Survival rate (%) (just after administration) | Survival rate (%) (after 14 days) | LD₅₀ (mg/kg) |
| SN38-βGal | Jcl:ICR (female) | 10 | 200 | 100 | 100 | |
| SN38-βGlcNAc | Jcl:ICR (female) | 10 | 300 | 100 | 100 | >300 |
| | Jcl:ICR (female) | 10 | 200 | 100 | 100 | |
| | Jcl:ICR (female) | 10 | 500 | 100 | 100 | >500 |
| CPT-11 | female mouse | - | - | - | - | 132 |

### (3) Repeated dose toxicity test

### Mice (intravenous)

Toxicity evaluation in repeated administration of SN38-βGal, SN38-βGlcNAc, and CPT-11 at 40 mg/kg to Jcl: ICR mice (female) was performed. Each drug was intravenously administered three times a week (i.v.) for three weeks, and the body weight change was observed. For the Vehicle group, a corresponding amount of DMSO was administered.

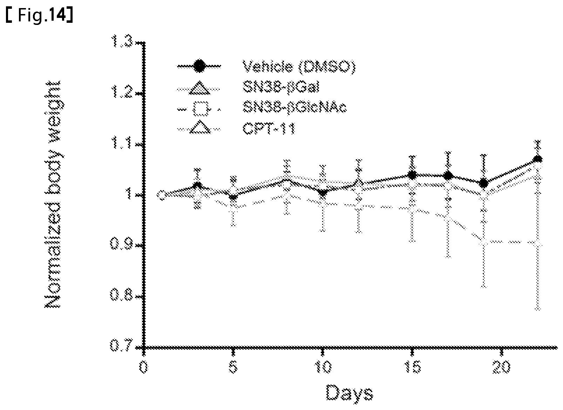

As a result, in the CPT-11 administration group, a body weight loss of about 20% at maximum was observed by the final day in a plurality of mice. On the other hand, in the SN38-βGal and SN38-βGlcNAc administration groups, the above-described significant body weight loss was not observed, and almost no difference in body weight change was found as compared with the Vehicle group (Fig. 14).

Fig. 14 illustrates transition of the body weight in repeated administration of a novel prodrug and CPT-11. From Fig. 14, a tendency of body weight loss was found in CPT-11, which is an existing drug, as compared with SN38-βGal and SN38-βGlcNAc. The black arrow indicates the day on which 40 mg/kg i.v. administration was performed. n = 8.

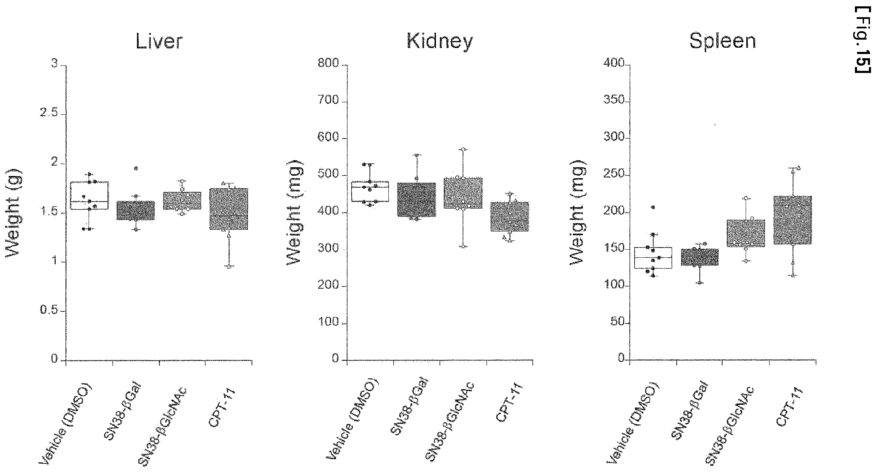

In addition, in CPT-11, reduced locomotor activity, tremor, prone states, protrusion of the eye, and the like were found immediately after administration. On the other hand, symptoms immediately after administration of SN38-βGal and SN38-βGlcNAc were not found. After blood was collected on day 22 and then euthanized, the liver, kidney, and spleen were taken out and their weights were compared. In the kidney, a slight decrease in weight was observed in the CPT-11 administration group, but this tendency was not observed in SN38-βGal and SN38-βGlcNAc. In the spleen, the weight tended to increase in the SN38-βGlcNAc and CPT-11 administration groups, and this tendency was particularly remarkable in the CPT-11 administration group (Fig. 15).

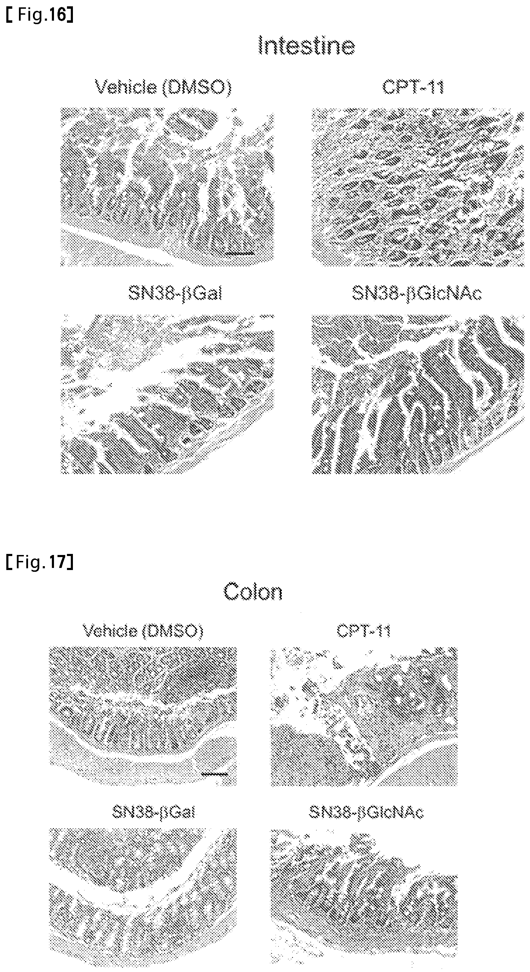

Histological analysis (Figs. 16 to 20) was performed on the collected organ, and blood tests were performed on whole blood and serum, and main findings obtained in comparison with the Vehicle group are shown below (Tables 6 to 8).

### SN38-βGal

In the biochemical test, a slight decrease in AST and ALT values was observed. In the blood cell test, a slight increase in eosinophils and a decrease in basophils were estimated. In the histological findings, no organ damage was observed in the small intestine, large intestine, kidney, liver, and spleen.

### SN38-βGlcNAc

An increase in spleen weight was observed. In the biochemical test, slight increases in urea nitrogen and creatinine were observed. In the blood cell test, an increase in eosinophils and a decrease in basophils were estimated. In the histological findings, no organ damage was observed in the small intestine, large intestine, kidney, liver, and spleen.

### CPT-11

A body weight loss of about 20% at maximum was observed. A decrease in liver and kidney weight and a clear increase in spleen weight were observed. In the biochemical test, a decrease in albumin, A/G ratio, AST, ALT, and ALP, and an increase in urea nitrogen and creatinine were observed. In addition, an increase in the proportion of neutrophils and monocytes and a decrease in the proportion of lymphocytes and basophils were observed. In a hematological test value, side effects to the kidney, liver, digestive tract, and the like were mainly estimated, and these side effects were considered to be more remarkable than SN38-βGal and SN38-βGlcNAc. In the histological findings, significant organ damage was observed in the small intestine and the large intestine. Organ damage in the kidneys, liver, and spleen was very mild or absent.

From the above results, it was estimated that the toxicity of the novel prodrugs SN38-βGal and SN38-βGlcNAc was very mild as compared with CPT-11 from the comprehensive viewpoint such as body weight change, blood examination, change in organ weight, histological analysis, and form after administration, and no serious organ disorder occurred in the administration period and condition of this test.

The histological findings are shown below.

### Histological findings in small intestine

In the small intestine, in the CPT-11 administration group, (1) erosion, (2) reduction in embryonic cells, (3) atrophy, disappearance, and degeneration of gland ducts, (4) fibrosis of the stroma, (5) infiltration of inflammatory cells in the entire lymphocytes, (6) degeneration and extensive necrosis of the lamina propria, and the like were found, and side effects of large organ damage were observed. On the other hand, in the SN38-βGal and SN38-βGlcNAc administration groups, (1) slight erosion, (2) slight reduction in embryonic cells, (3) slight degeneration of gland ducts, and (5) slight lymphocyte infiltration were observed, and fibrosis of the stroma and the like were hardly observed, and the side effects were very mild as compared with CPT-11. (Fig. 16)

### Histological findings in large intestine

In the large intestine, in the CPT-11 administration group, (1) erosion, (2) reduction in embryonic cells, (3) atrophy, disappearance, and degeneration of gland ducts, (4) fibrosis of stroma, (5) infiltration of inflammatory cells in the entire lymphocytes, and the like were found, and side effects of large organ damage were observed. On the other hand, in the SN38-βGal and SN38-βGlcNAc administration groups, (1) slight erosion, (2) slight reduction in embryonic cells, (3) slight degeneration of gland ducts, and (5) slight lymphocyte infiltration were observed, and fibrosis of the stroma and the like were hardly observed, and the side effects were very mild as compared with CPT-11 (Fig. 17).

### Histological findings in kidney

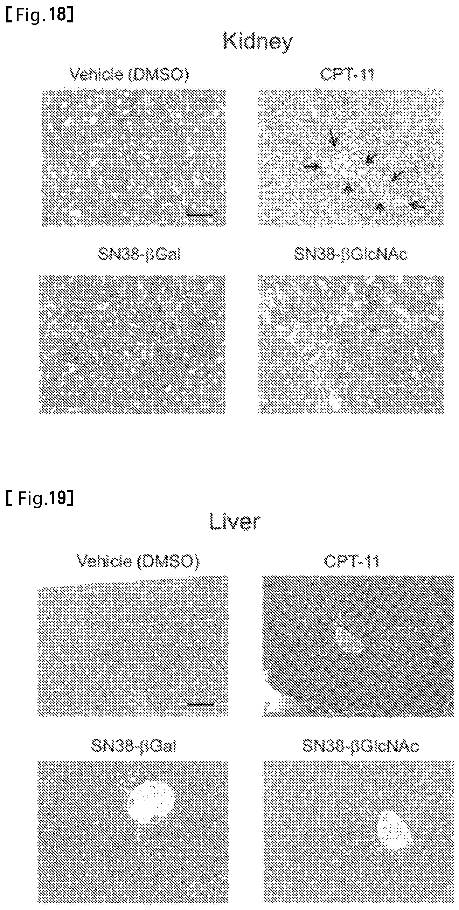

In the kidney, in the CPT-11 administration group, (1) slight lymphocyte infiltration and (2) loss of renal tubules (indicated by the black arrow) were observed, but no significant changes were observed in the organ as a whole, and organ damage to the kidney was very mild. Slight lymphocyte infiltration was observed also in the SN38-βGal and SN38-βGlcNAc administration groups, but no significant changes were observed in the organ as a whole, and organ damage to the kidney was not observed even in the novel prodrug group (Fig. 18).

### Histological findings in liver

In the liver, in the CPT-11 administration group, (1) hypertrophy of nuclei of hepatocytes (increase in chromatin) was frequently observed, and (2) lymphocyte infiltration was slightly observed, but organ damage to the liver was mild as a whole. Also, in the SN38-βGal and SN38-βGlcNAc administration groups, (1) hypertrophy of the nucleus of hepatocytes (chromatin was normal) was slightly observed, but no significant changes were observed in the organ as a whole, and organ damage to the liver was not observed in the novel prodrug group (Fig. 19).

### Histological findings in spleen

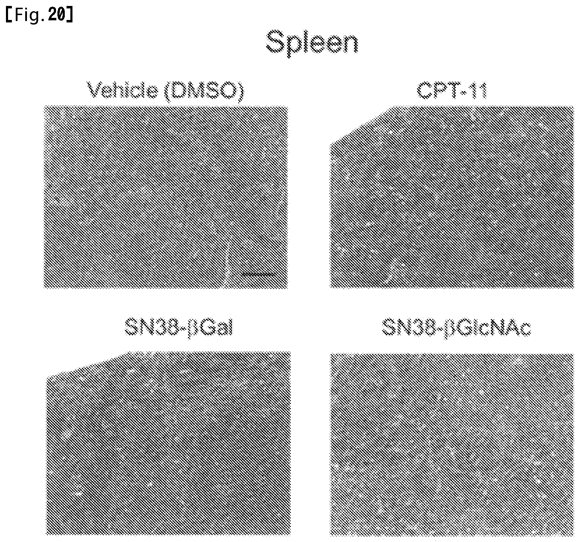

In the spleen, in the CPT-11, SN38-βGal, and SN38-βGlcNAc administration groups, slightly multinucleated cells and splenomegaly tended to be observed, but no significant changes were not observed in the organ as a whole, and organ damage to the spleen was very mild (Fig. 20).

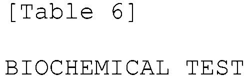

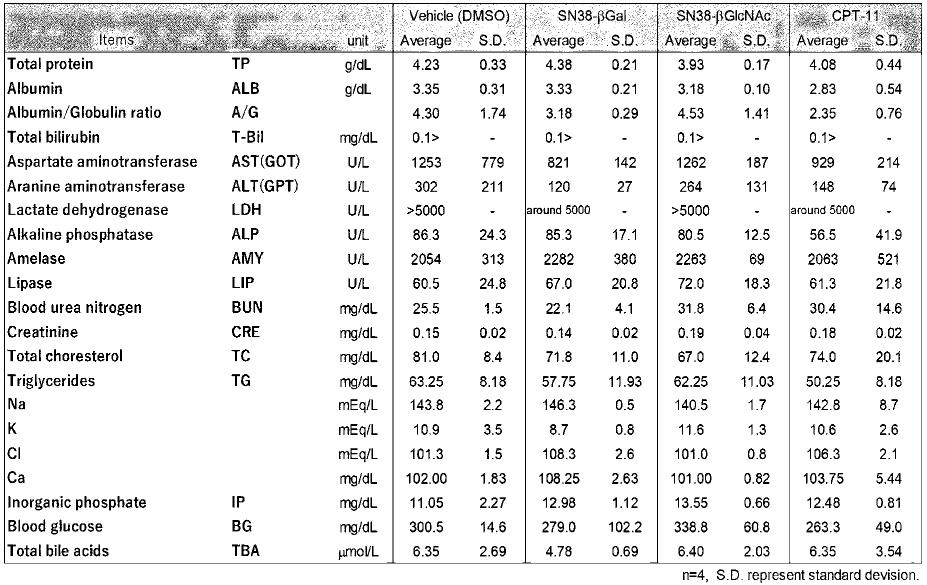

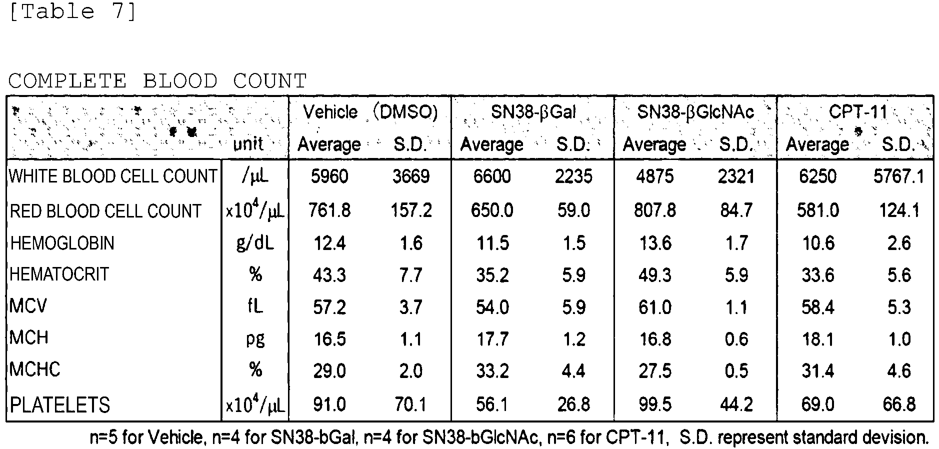

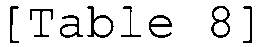

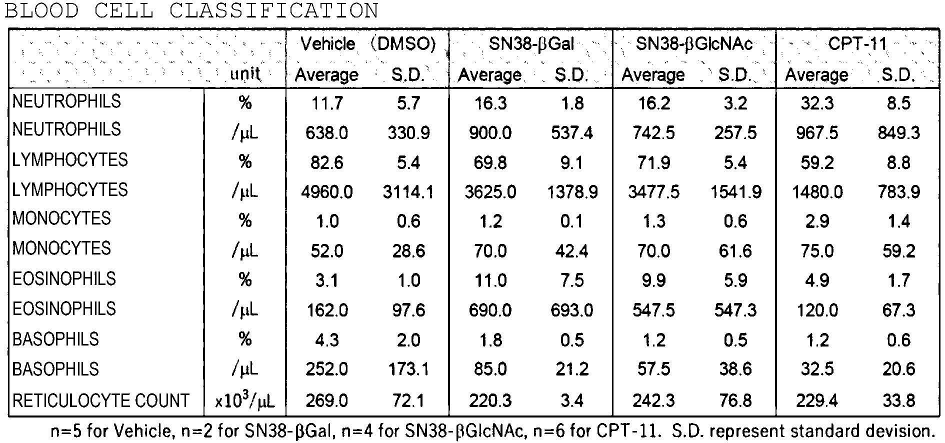

### (4) Evaluation of inhibitory activity on acetylcholinesterase (AChE)

From the results of the toxicity test, it was suggested that in SN38-βGal and SN38-βGlcNAc, the side effect (reduced locomotor activity, tremor, prone) of cholinergic syndrome, which is acute toxicity occurring in the administrable concentration range of Irinotecan (CPT-11), was hardly observed. Since it is considered that cholinergic syndrome is caused by inhibition of AChE by CPT-11, AChE inhibitory activity of newly developed prodrugs SN38-βGal, SN38-βGlcNAc, and SN38-αMan was evaluated in comparison with CPT-11.

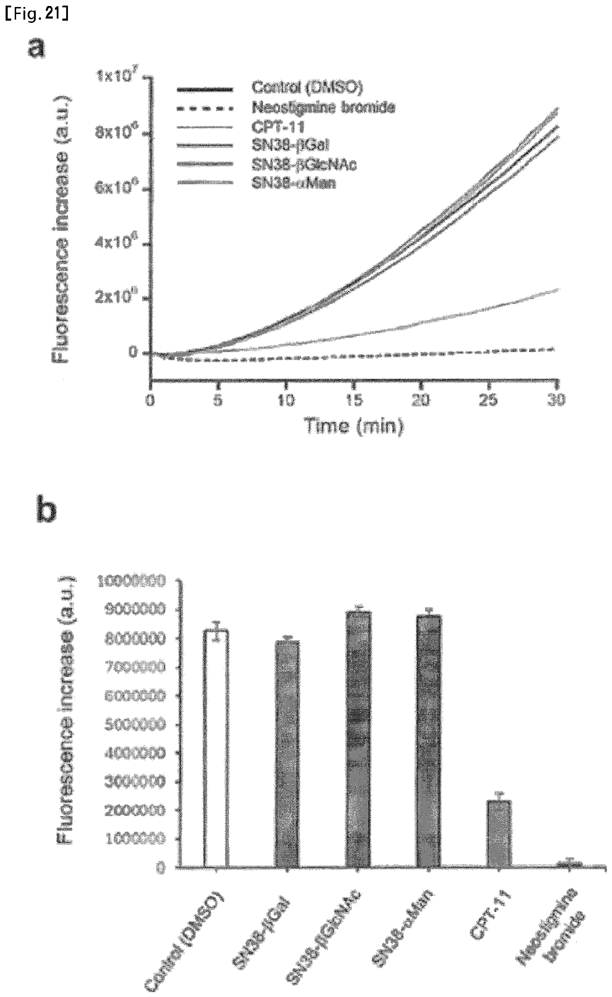

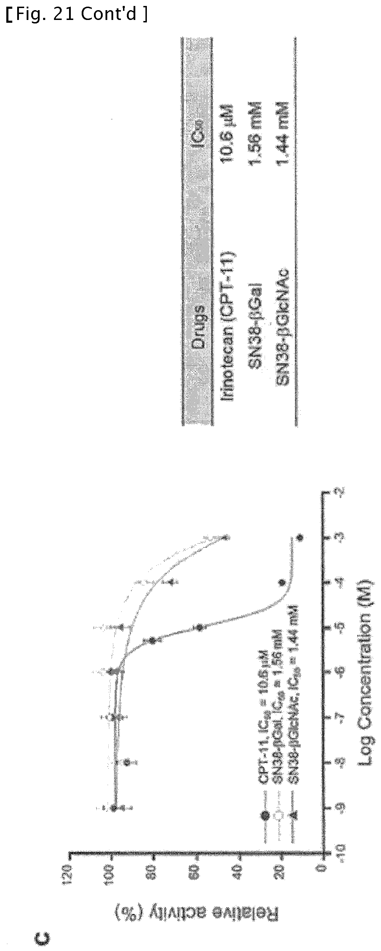

As a result, it was confirmed that CPT-11 remarkably inhibited human AChE activity in the presence of 10 µM of each drug, whereas SN38-βGal, SN38-βGlcNAc, and SN38-αMan did not inhibit AChE activity in this concentration range (a and b in Fig. 21).

When the AChE inhibitory activity of CPT-11, SN38-βGal, and SN38-βGlcNAc was confirmed in a concentration-dependent manner, the IC50 value for AChE inhibition of each drug was calculated to be 10.6 µM for CPT-11, 1.56 mM for SN38-βGal, and 1.44 mM for SN38-βGlcNAc, and the IC50 values of SN38-βGal and SN38-βGlcNAc were larger than CPT-11 by 2 or more orders of magnitude (c of Fig. 21).

From these results, the newly developed prodrugs SN38-βGal, SN38-βGlcNAc, and SN38-αMan had very low AChE inhibitory activity as compared with CPT-11, and this result confirmed that cholinergic syndrome was not found.

The procedure of the specific inhibition test is shown below.
(1) A test drug at each concentration was added to a 96 well plate, and acetylcholine, an AChE substrate, and an AChE purified enzyme were added according to a protocol, using SensoLyte (registered trademark) 520 Acetylcholine esterase Activity Assay Kit Fluorometric (AnaSpec, Inc.). Neostigmine bromide known as an AChE inhibitor was used as negative control.
(2) The fluorescence value at 520 nm was measured, and the IC50 value was calculated.

a of Fig. 21 illustrates the evaluation of AChE inhibitory activity when 10 µM of each drug is added, and b illustrates the comparison of the inhibitory activity at the 30 minute value. c of Fig. 21 illustrates the concentration-dependent AChE inhibitory activity and IC50 value of each drug at the 60 min value. Error bars = S.D.

### [Example 10]

### Evaluation of locomotion after administration of drug

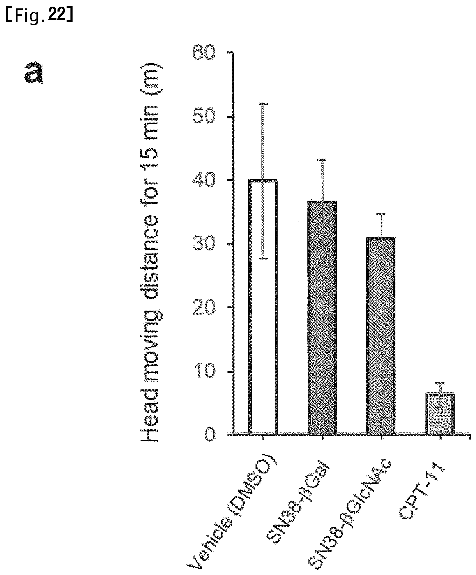

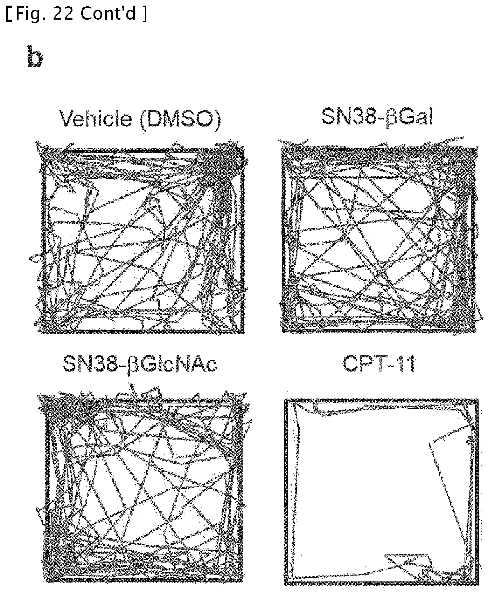

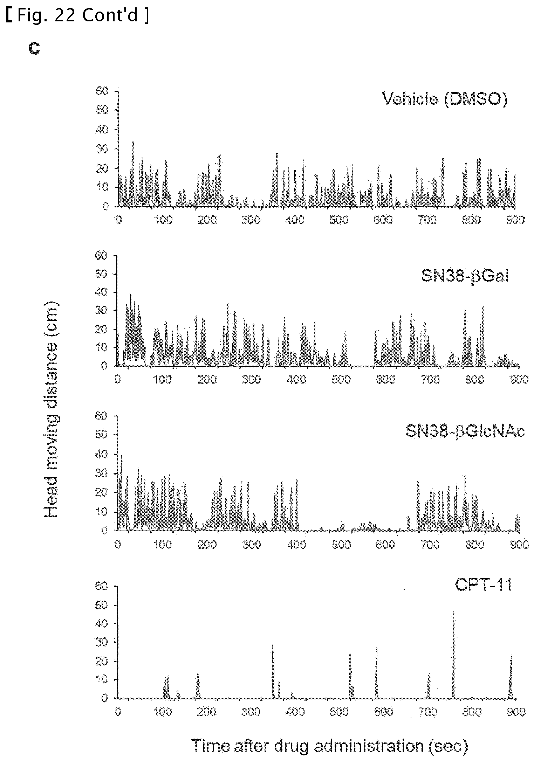

The momentum after drug administration was compared (n = 4). In CPT-11, a significant decrease in the locomotion was observed immediately after administration by intravenous administration of 40 mg/kg. On the other hand, in SN38-βGal and SN38-βGlcNAc, these decreases in the locomotion were hardly observed (Fig. 22). The cholinergic syndrome has been reported as acute toxicity after administration in CPT-11, and it is considered that the decrease in the locomotion is based on this. SN38-βGal and SN38-βGlcNAc did not cause the cholinergic syndrome, suggesting that acute toxicity is significantly lower than existing clinically used CPT-11.

Fig. 22 illustrates results of locomotion evaluation after drug administration. a of Fig. 22 illustrates the head movement distance for 15 minutes from 1 minute after administration in each group. The drug is administered intravenously at 40 mg/kg (n = 4). b of Fig. 22 illustrates an example of a movement trajectory for 15 minutes from 1 minute after administration in each group. c of Fig. 22 illustrates an example of a movement distance for each time from 1 minute after administration in each group.

### [Example 11]

### Therapeutic experiments in peritoneal dissemination model

The therapeutic effect was verified using a peritoneal dissemination model using SN38-βGal. Whether the tumor suppression effect was confirmed was evaluated as compared with the Vehicle group (DMSO administration) by separating the SN38-βGal from the 10 mg/kg and 20 mg/kg administration groups.

n = 3 for each group, and evaluation was performed on luciferase-expressing A549 cell peritoneal dissemination model mice using BALB/cAJcl-nu/nu. The administration form was intraperitoneal administration (i.p.), and continuous administration was performed for 40 days. Tumor burden was determined by evaluating luciferin/luciferase chemiluminescence. An endpoint was a time point at which significant body weight loss (20% loss) or intraperitoneal hemorrhage was found.

As a result, the result that the tumor growth was significantly suppressed in the administration group as compared with the Vehicle group was obtained. In addition, among the doses of 10 mg/kg and 20 mg/kg, the 20 mg/kg group slightly exhibited a high tumor suppression effect, but there was no significant difference in the effect. On Day 29 of administration, one of the mice in the Vehicle group reached the endpoint and died, but no death was found in the administration group.

When the mice after completion of the evaluation were subjected to laparotomy to confirm the presence or absence of a tumor, a significant decrease in peritoneal disseminated lesions was confirmed in macroscopic findings in the administration group as compared with the Vehicle. When the fluorescent probe gGlu-HMRG, which can visualize A549 tumor lesions, was sprayed and the tumor lesions were fluorescently visualized and photographed, regions that emitted significant fluorescence (tumor sites) were confirmed in the Vehicle, while the number of such regions was significantly reduced in the administration group.

In addition, as a result of evaluating the transition of the body weight through this study, a slight body weight loss was found in the 20 mg/kg group by Day 10 of administration, but thereafter, no significant loss was observed as compared with the Vehicle group. In the 10 mg/kg administration group, there was almost no difference from the Vehicle. In addition, no significant side effects in macroscopic findings were observed immediately after administration of SN38-βGal or the like.

From the above results, the therapeutic efficacy of SN38-βGal in peritoneal dissemination model mice was confirmed.

A specific procedure of the therapeutic experiment is shown below.
(1) Luciferase expressing A549 cells 5 × 10⁶ cells/mL (200 µL) was intraperitoneally administered to BALB/cAJcl-nu/nu (7 weeks old).
(2) After one week, the formation of peritoneal dissemination was confirmed by luciferin/luciferase chemiluminescence, and drug administration was started. 3 mg (200 µL) of luciferin was administered by intraperitoneal administration, and anesthetized with isoflurane. Thereafter, the chemiluminescent value at 20 minutes after administration was measured by an in vivo imaging device. The drug was prepared by dissolving a 100 mM of DMSO stock in PBS, and administered in a total amount of 100 µL.
(3) On the day of evaluation, the tumor burden was evaluated by luciferin/luciferase chemiluminescence.
(4) After completion of the treatment, gGlu-HMRG (10 µM, 500 µL PBS) was intraperitoneally administered, and 10 minutes later, the abdomen was opened, and the intestinal tract and the peritoneum were taken out.
(5) The seeded lesion was fluorescently imaged by an in vivo fluorescence imaging device, and comparison between the Vehicle and the administration group was performed.

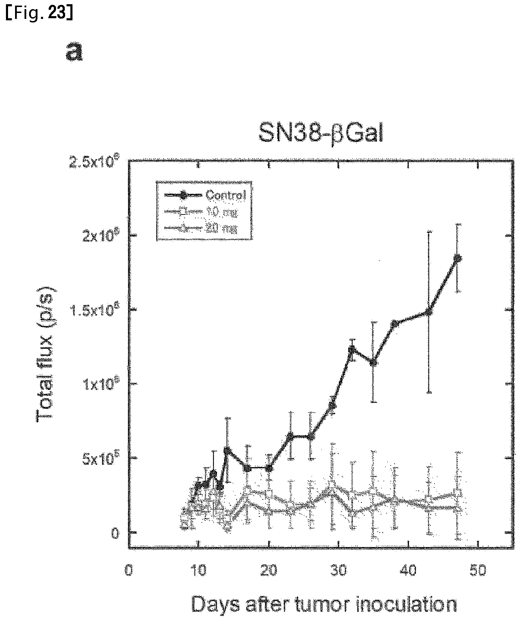

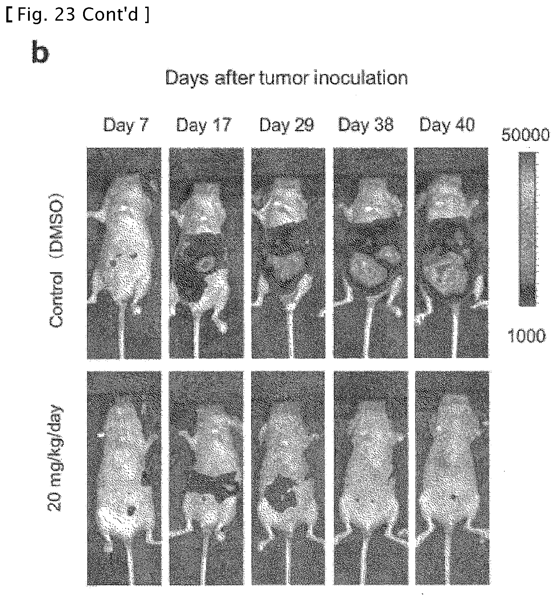

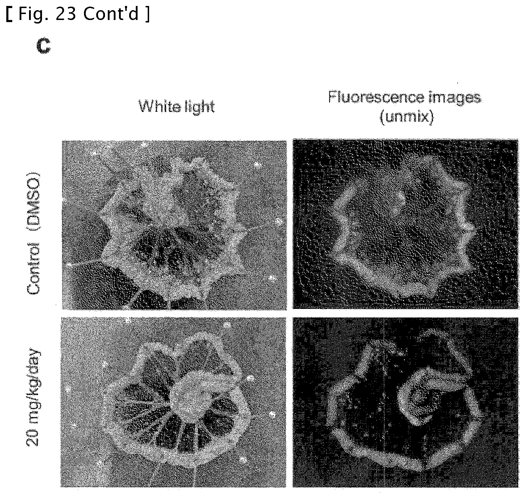

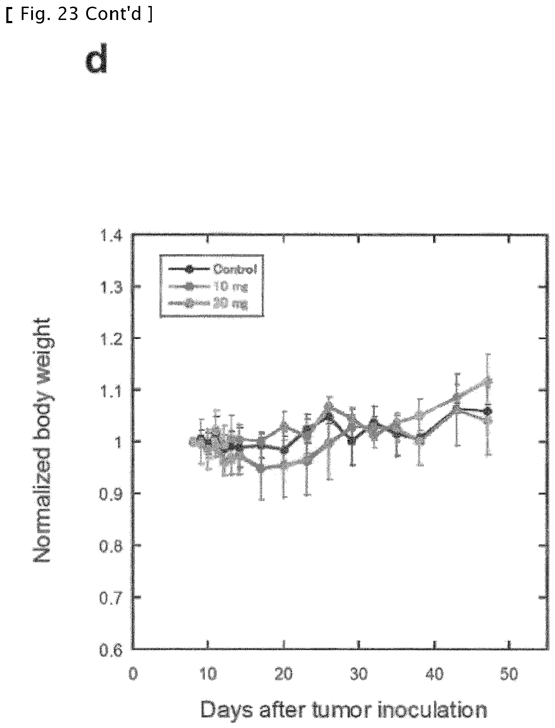

Fig. 23 illustrates the results of therapeutic evaluation of A549 peritoneal dissemination model mice using SN38-βGal. a of Fig. 23 illustrates a change in luminescence intensity in each group. b of Fig. 23 illustrates a temporal change of a luminescence image in the Vehicle and the 20 mg/kg administration group. c of Fig. 23 illustrates a result of confirming the peritoneal tumor lesion after evaluation by a white light image (left) and a fluorescence image (right) using gGlu-HMRG. d of Fig. 23 illustrates the transition of the body weight change in each group.

### [Example 12]

### Therapeutic experiment 1 in lung cancer orthotopic transplantation model

The therapeutic effect in the lung cancer orthotopic transplantation model was verified using SN38-βGal, SN38-βGlcNAc, and CPT-11. Tumor suppression effect was confirmed by the evaluation of Vehicle (DMSO administration), SN38-βGal, SN38-βGlcNAc, and CPT-11 administration groups. The evaluation was performed on luciferase-expressing A549 cell peritoneal dissemination model mice using BALB/cAJcl-nu/nu with n = 6 for the Vehicle group, n = 7 for the SN38-βGal group, n = 8 for the SN38-βGlcNAc group, and n = 7 for the CPT-11 group. The administration form was intravenous administration (i.v.). The dose was 40 mg/kg, and administration was performed 3 times a week for 4 times. The tumor burden was determined by evaluating the luciferin/luciferase chemiluminescence value. The endpoint was the time point of significant body weight loss (20% loss).

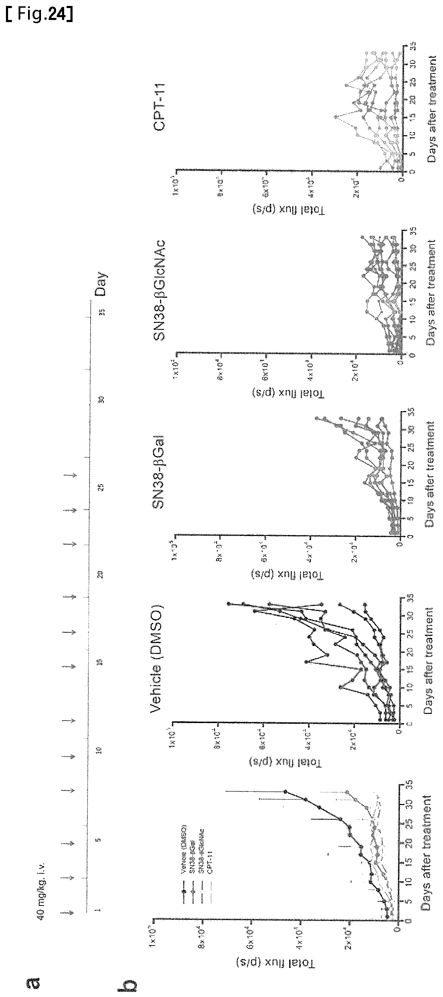

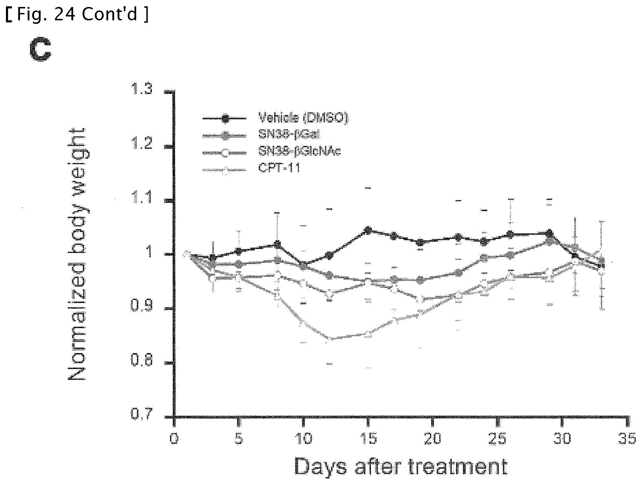

As a result, it was confirmed that compared with the Vehicle group, SN38-βGal and SN38-βGlcNAc tended to suppress tumor growth as in the CPT-11 administration group. After Day 26 when the administration was terminated, tumor regrowth was observed in the SN38-βGal group (Fig. 24).

In addition, as a result of evaluating the transition of the body weight through this study, no significant loss was observed in both groups as compared with the Vehicle group. In addition, no significant side effects in macroscopic findings were observed immediately after administration of SN38-βGal and SN38-βGlcNAc. On the other hand, in the CPT-11 administration group, a significant body weight loss was found in the first 2 weeks, but recovery of the body weight was confirmed after 2 weeks. In the SN38-βGal and SN38-βGlcNAc administration groups, the slight body weight loss was found, but it was not remarkable as in CPT-11.

From the above results, it was shown that SN38-βGal and SN38-βGlcNAc can be treated to the same extent or more without losing body weight as compared with CPT-11.

A specific procedure of the therapeutic experiment is shown below.
(1) Model mice were created by the following operation using BALB/cAJcl-nu/nu (7 weeks old). The skin of the chest was incised, and the skin was subcutaneously detached, then luciferase-expressing A549 cells 2 × 10⁷ cells/mL (50 µL, 5% Matrigel) was injected from the intercostal pleura to the left lung. After injection, the skin was sutured.
(2) After 17 days, the formation of cancer was confirmed by luciferin/luciferase chemiluminescence, and the intravenous administration of the drug was started. The drug was prepared by dissolving a 200 mM of DMSO stock in PBS, and administered in a total amount of 100 µL. 3 mg (200 µL) of luciferin was administered by intraperitoneal administration, and anesthetized with isoflurane. Thereafter, the chemiluminescent value at 20 minutes after administration was measured by an in vivo imaging device.
(3) On the day of evaluation, the tumor burden was evaluated by the body weight and the luciferin/luciferase chemiluminescence.

Fig. 24 illustrates the results of therapeutic evaluation of the A549 lung cancer orthotopic transplantation model by SN38-βGal, SN38-βGlcNAc, and CPT-11. a of Fig. 24 illustrates the dosage schedule, and b of Fig. 24 illustrates the tumor suppression effect in each administration group. c of Fig. 24 illustrates the transition of the body weight during the treatment period in each group.

### [Example 13]

### Therapeutic experiment 2 in lung cancer orthotopic transplantation model

Using SN38-βGal and SN38-βGlcNAc, the dose was increased to verify the therapeutic effect in the lung cancer orthotopic transplantation model. Whether the tumor suppression effect was confirmed was evaluated as compared with the Vehicle group (DMSO administration) by separating the SN38-βGal and SN38-βGlcNAc administration groups. The evaluation was performed on luciferase-expressing A549 cell peritoneal dissemination model mice using BALB/cAJcl-nu/nu with n = 3 for the Vehicle group, n = 4 for the SN38-βGal group, and n = 5 for the SN38-βGlcNAc group. The administration form was intravenous administration (i.v.).

For administration, 40 mg/kg was administered 5 times in total every other day, and then 80 mg/kg was administered 7 times in total every other day. The tumor burden was determined by evaluating the luciferin/luciferase chemiluminescence value. The endpoint was a significant body weight loss (20% loss).

As a result, as compared with the Vehicle group, in the SN38-βGal and SN38-βGlcNAc administration groups, the result was obtained that the tumor growth was significantly suppressed in all mice. In particular, an increase in the tumor burden after 2 weeks from the start of treatment was remarkable in the Vehicle group, whereas this increase was remarkably suppressed in the SN38-βGal and SN38-βGlcNAc administration groups. In addition, the SN38-βGlcNAc group showed a slightly higher tumor suppression effect than the SN38-βGal group, but there was no significant difference in the effect. When the lungs were taken out from the mice after completion of the evaluation, and the tumor burden was checked, a significant decrease in tumor lesions was confirmed in macroscopic findings in the administration group as compared with the Vehicle. When the fluorescent reagent HMRef-βGlcNAc, which can visualize A549 tumor lesions, was sprayed and the tumor lesions were fluorescently visualized and photographed, areas that emitted significant fluorescence (tumor sites) were confirmed in the Vehicle, while a site showing fluorescence was significantly reduced in the administration group.

In addition, as a result of evaluating the transition of the body weight through this study, no significant loss was observed in both groups as compared with the Vehicle group. In addition, no significant side effects in macroscopic findings were observed immediately after administration of SN38-βGal and SN38-βGlcNAc. On the other hand, in the CPT-11 administration group (n = 3, 40 mg/kg, q2d x 12), the body weight loss was found in the first 2 weeks, but the body weight gain was found after 2 weeks. In SN38-βGal and SN38-βGlcNAc, the body weight loss was not found in the first 2 weeks although the same drug amount was administered.

From the above results, the therapeutic efficacy of SN38-βGal and SN38-βGlcNAc in a mouse lung cancer model was confirmed.

A specific procedure of the therapeutic experiment is shown below.
(1) Model mice were created by the following operation using BALB/cAJcl-nu/nu (7 weeks old). The skin of the chest was incised, and the skin was subcutaneously detached, then luciferase-expressing A549 cells 2 × 10⁷ cells/mL (50 µL, 5% Matrigel) was injected from the intercostal pleura to the left lung. After injection, the skin was sutured.
(2) After 1 week, the formation of cancer was confirmed by luciferin/luciferase chemiluminescence, and the intravenous administration of the drug was started. The drug was prepared by dissolving a 200 mM of DMSO stock in PBS, and administered in a total amount of 100 µL. 3 mg (200 µL) of luciferin was administered by intraperitoneal administration, and anesthetized with isoflurane. Thereafter, the chemiluminescent value at 20 minutes after administration was measured by an in vivo imaging device.
(3) On the day of evaluation, the tumor burden was evaluated by luciferin/luciferase chemiluminescence.
(4) After completion of the treatment, HMRef-βGlcNAc, 5 mg/kg (100 µL PBS) was administered by intravenous administration. 30 minutes after administration, the sternum was incised, and the lungs were taken out.
(5) The cancer lesion was fluorescently imaged by an in vivo fluorescence imaging device, and comparison between the Vehicle and the administration group was performed.

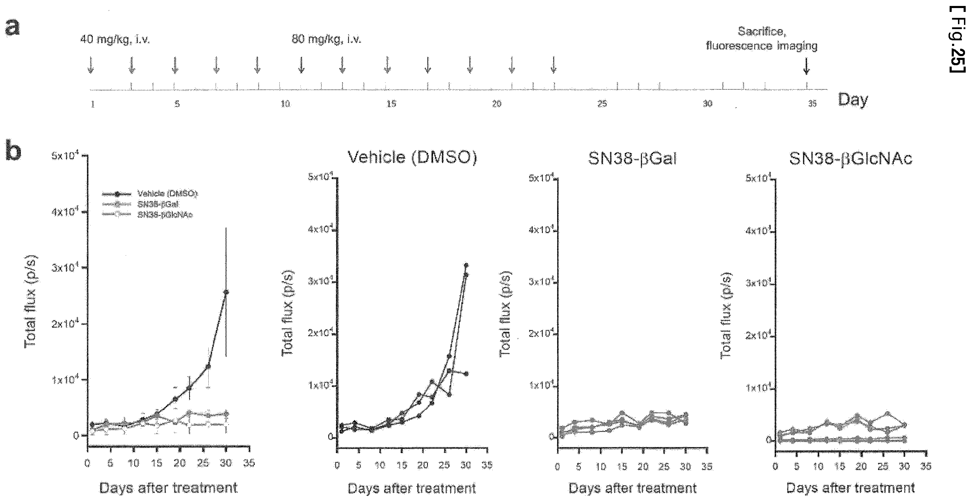

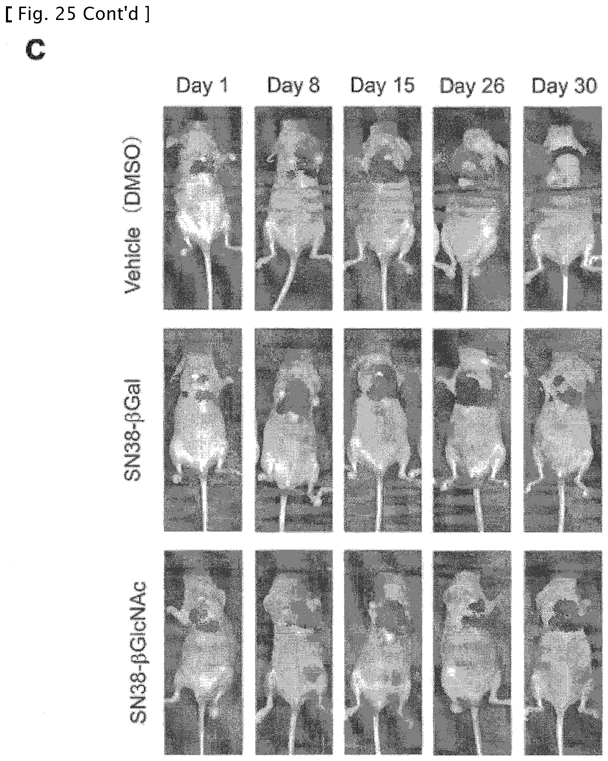

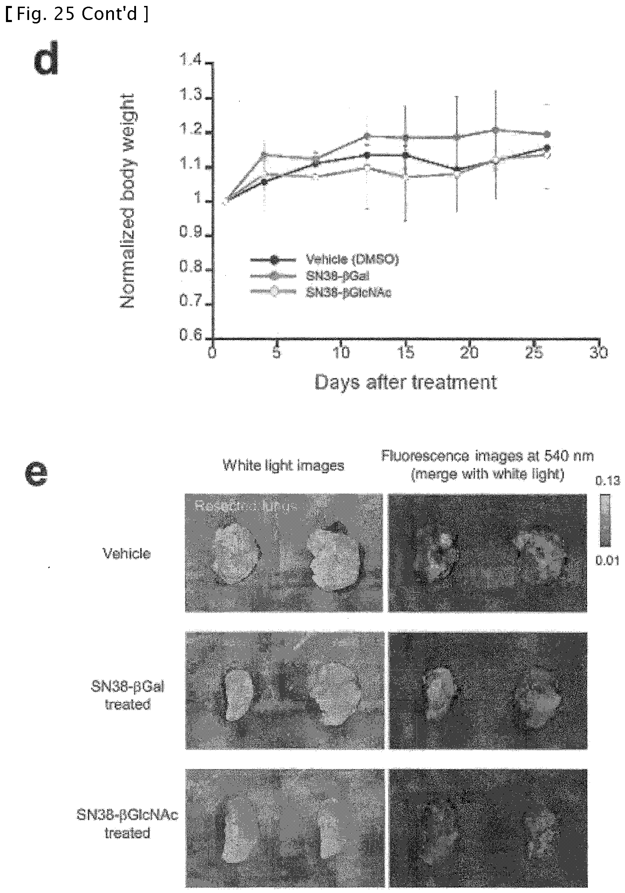

Fig. 25 illustrates the results of therapeutic evaluation of the A549 lung cancer orthotopic transplantation model by SN38-βGal and SN38-βGlcNAc. a of Fig. 25 illustrates the dosage schedule, and b illustrates the tumor suppression effect in each administration group. c of Fig. 25 illustrates an example of Luciferin/Luciferase chemiluminescent imaging in each group. d of Fig. 25 illustrates the transition of the body weight during the treatment period in each group, and e of Fig. 25 illustrates the results of fluorescence imaging of the lung cancer site after treatment in each group.

### [Example 14]

### Analysis of pharmacokinetic parameters in common marmoset

### Common marmoset (intravenous)

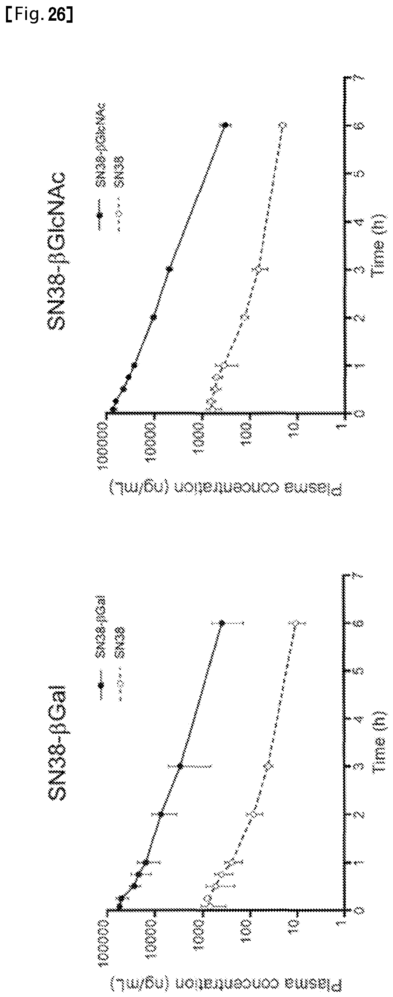

For the common marmoset, 10 mg/kg of SN38-βGal or SN38-βGlcNAc was administered intravenously at 11 minutes infusion, and each pharmacokinetic parameters were calculated (Fig. 26, Tables 9 and 10). The blood half-life of SN38-βGal was 58.3 minutes and the blood half-life of the released SN38 was 85.1 minutes. In addition, the blood half-life of SN38-βGlcNAc was 47.6 minutes, and the blood half-life of the released SN38 was 93.4 minutes. In addition, remarkable abnormalities, vomiting, and the like were not found in any individual after the drug administration.

A specific test procedure is shown below.
(1) Three marmosets (6 males in total) in each of the SN38-βGal and SN38-βGlcNAc administration groups were sequentially retained using a retention device, and a tail of the administration site was shaved.
(2) The base of the tail from the injection site was avascularized to cause blood vessels to become engorged. Veins running on both left and right sides of the tail were checked, the puncture site was disinfected with alcohol cotton, and then administration was performed with a 27 G injection needle using one of the left and right blood vessels. For administration, 1.0 mg/mL of prepared administration solution of SN38-βGal or SN38-βGlcNAc was intravenously administered at an injection rate of 350 µL/min (about 11 minutes: due to different body weight, the time τ actually required for the injection was accurately recorded for each individual) by an infusion pump at a dose of 10 mg/10 mL/kg.
(3) Immediately after the start of infusion administration, about 220 µL of blood was collected with a heparinized syringe at 5, 15, 30, 45 minutes, 1, 2, 3, and 6 hours, and centrifuged at 1200 G and 4°C for 15 minutes to collect plasma.
(4) After the plasma was dispensed into the microtube, 100 µL of the plasma was accurately taken, and a 200 µM of inhibitor N-(n-nonyl) deoxygalactonoijirimycin solution in the SN38-βGal administration group, a 200 µM of inhibitor PUGNAc solution in the SN38-βGlcNAc administration group, and 100 µL of each were added to the microtube dispensed in advance and mixed quickly. The obtained plasma was cryopreserved at -80°C or lower.
(5) 50 µL of plasma and 200 µL (MeOH: MeCN = 1:1+ internal standard camptothecin) solution were mixed, and the mixture was allowed to stand for about 10 minutes.
(6) The resultant was centrifuged at 1700 G at 4°C for 15 minutes to collect a supernatant, and further loaded on a cosmo spin filter and centrifuged at 12000 G at 4°C for 5 minutes to collect a deproteinized sample.
(7) 50 µL of MiliQ was added to 50 µL of the collected sample, and an MS spectrum was measured by UPLC-MS.
(8) The blood concentration was calculated from the calibration curve prepared using the plasma sample by the same method. The initial concentration was set to 0, and three points of 2, 3, and 6 hours were selected as the disappearance phase to calculate the blood half-life.

Fig. 26 illustrates changes in blood concentration after administration of SN38-βGal or SN38-βGlcNAc. 10 mg/kg of the drug was intravenously administered by infusion for 11 minutes. Error bars = S.D.

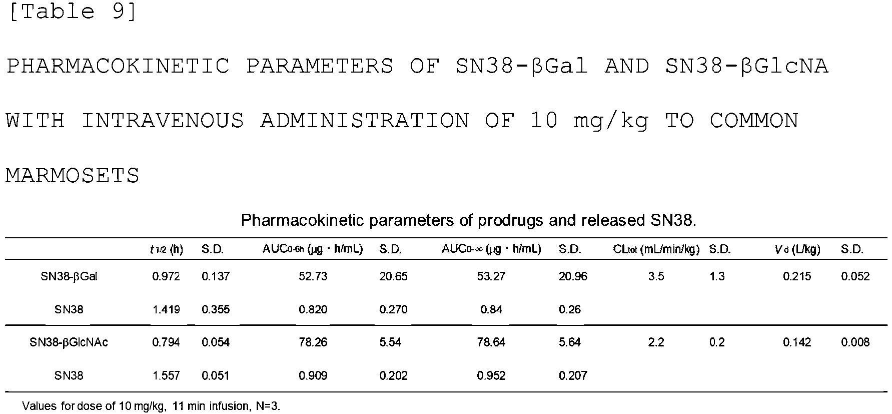

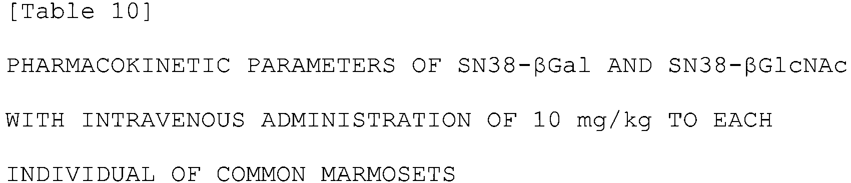

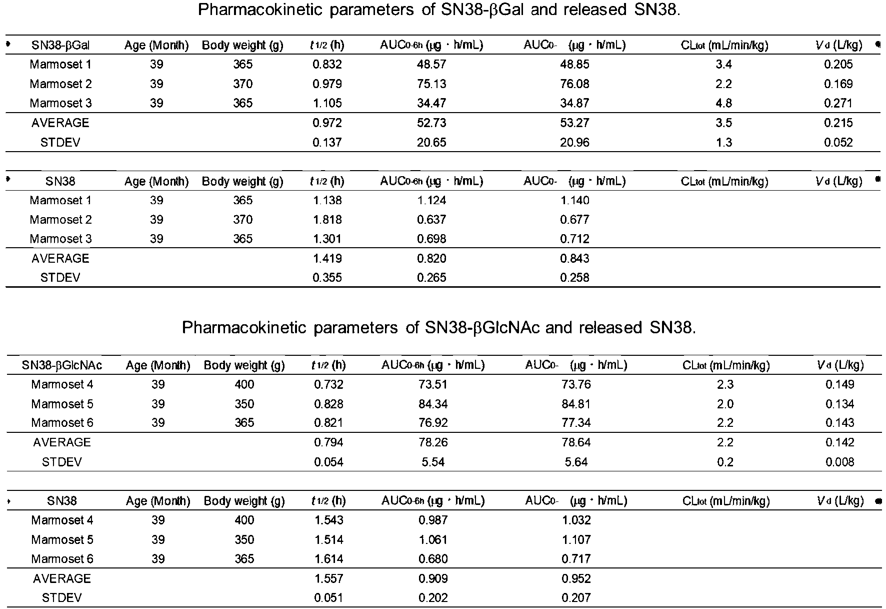

### [Example 15]

### (1) Cytotoxicity tests of developed prodrugs by MTT assay method (H226 cells)

A cytotoxicity test of SN38-βGal and SN38-βGlcNAc in cells of lung squamous carcinomas cell H226 was performed by an MTT assay method.

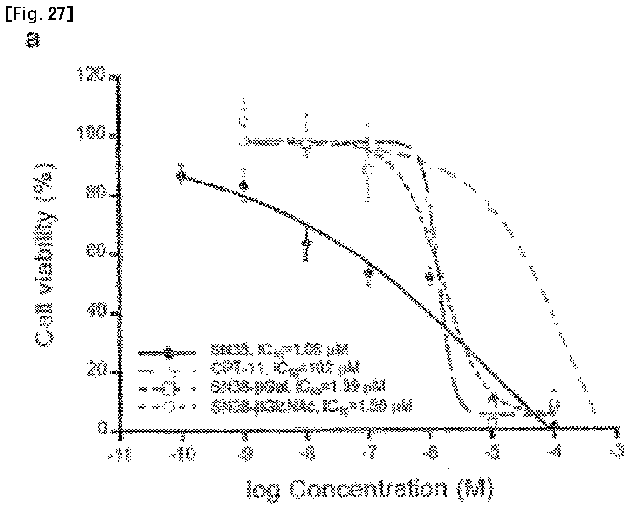

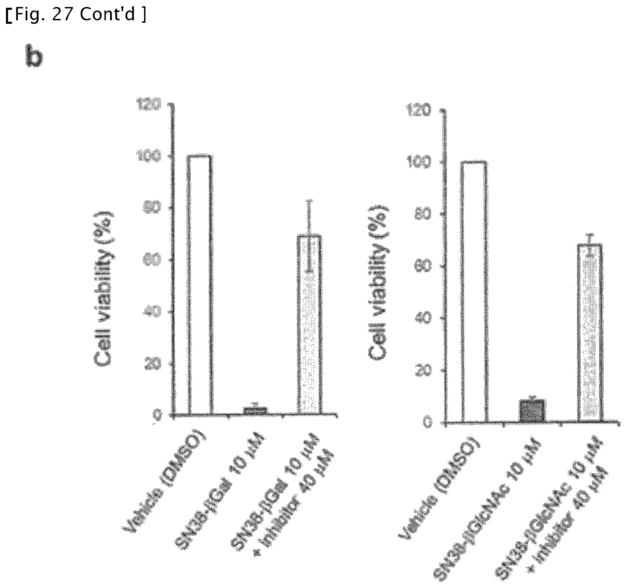

As a result, cell proliferation was suppressed as the concentrations of SN38-βGal and SN38-βGlcNAc increased (Fig. 27). Each IC50 value under the following measurement conditions were calculated as SN38 (1.08 µM), SN38-βGal (1.39 µM), SN38-βGlcNAc (1.50 µM), and CPT-11 (102 µM). SN38-βGal and SN38-βGlcNAc showed lower IC50 values than CPT-11.

In addition, with regard to SN38-βGal, it was confirmed that the cytotoxicity was significantly reduced by the addition of an inhibitor of β-galactosidase, N-(n-nonyl) deoxygalactonoijirimycin, 40 µM as a target enzyme. Regarding SN38-βGlcNAc, it was confirmed that the cytotoxicity was significantly reduced by the addition of a 40 µM of inhibitor of β-hexosaminidase, PUGNAc, as a target enzyme. In this test, Cell Proliferation Kit I (MTT) and SIGMA-ALDRICH were used.

The procedure of the specific cytotoxicity test is shown below.
(1) 100 µL of RPMI-1640 containing 6.0 × 10³ cells of H226 cells, 10%FBS, and 1%PS was added to a 96 well plate, and the plate was incubated in an environment of 37°C and 5% CO₂ for 24 hours.
(2) 10 µL of a medium containing each concentration of the drug was added, and the mixture was incubated in a 37°C, 5% CO₂ environment for 72 hours.
(3) 10 µL of MTT labeling reagent was added and incubated in a 37°C, 5% CO₂ environment for 4 hours.
(4) 100 uL solubilization buffer was added and incubated in 5% CO₂ environment for 24 hours.
(5) The absorbance at 560 nm was measured, cell viability was evaluated, and the IC50 value was calculated.

Fig. 27 illustrates results of the cytotoxicity test by MTT assay. a of Fig. 27 illustrates suppression of cell proliferation at each drug concentration of lung squamous cell carcinoma H226 cells, and b illustrates a cell proliferation suppression curve and IC50 value of each drug. c of Fig. 27 illustrates suppression of drug efficacy in the presence of an inhibitor.

### (2) Tumor proliferation suppression test in H226 subcutaneous tumor model

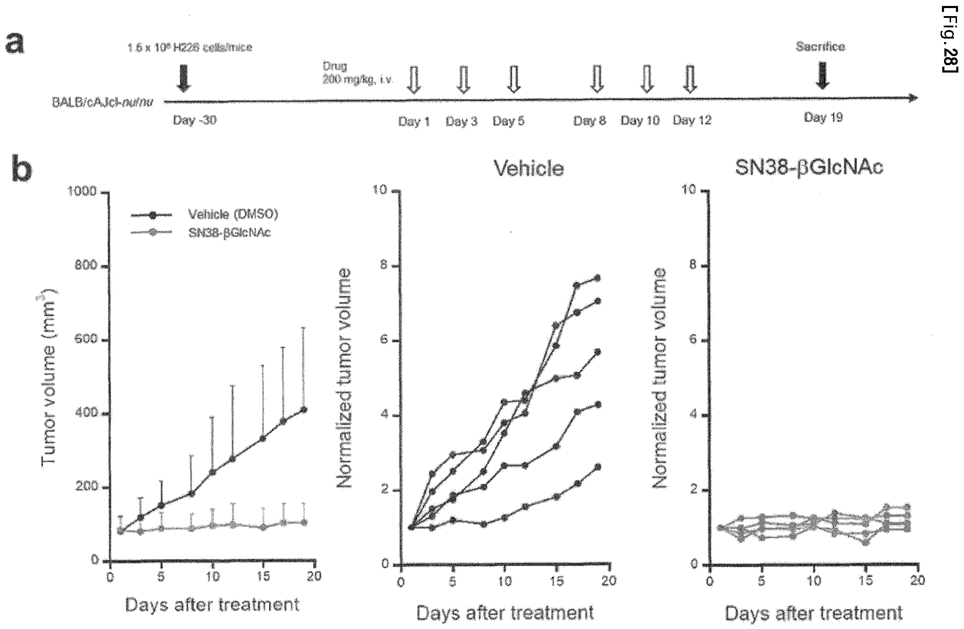

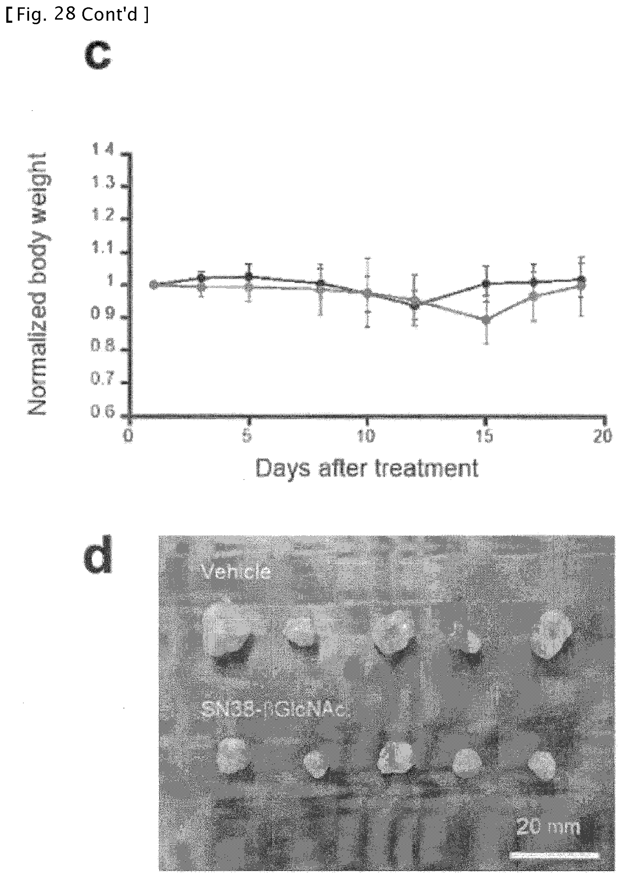

200 mg/kg of SN38-βGlcNAc was intravenously administered 3 times a week for 2 weeks to subcutaneous tumor model mice of lung squamous carcinomas cell H226, and the tumor proliferation suppression effect was evaluated. The Vehicle (DMSO administration) group and the SN38-βGlcNAc administration group were n = 5, respectively. Significant tumor growth was confirmed in the Vehicle group, whereas the tumor growth was almost completely suppressed in the SN38-βGlcNAc administration group (Fig. 28).

A specific procedure of the therapeutic experiment is shown below.
(1) H226 cells 1.5 × 10⁶ cells (50 µL) were subcutaneously injected into BALB/cAJcl-nu/nu (6 weeks old, female).
(2) The drug administration was started to the individual whose tumor volume reached about 50 to 150 mm³ after 31 days. The drug SN38-βGlcNAc was prepared by dissolving a 200 mM of DMSO stock in PBS, and was intravenously administered from the tail vein 3 times a week for 2 weeks at a dose of 200 mg/kg with a total amount of 100 µL.
(3) The body weight was measured on the day of evaluation, and the tumor volume was evaluated with a vernier caliper.
(4) Tumors were resected on day 19 from the start of dosing and their sizes were compared.

Fig. 28 illustrates a tumor proliferation suppression effect of SN38-βGlcNAc in H226 subcutaneous tumor model mice. a of Fig. 28 illustrates the administration schedule, and b illustrates the transition of the tumor volume. c of Fig. 28 illustrates the transition of the body weight change, and d illustrates a photograph of a resected tumor on Day 19. Error bars = S.D.

### [Example 16]

### Proliferation suppression test in organoids

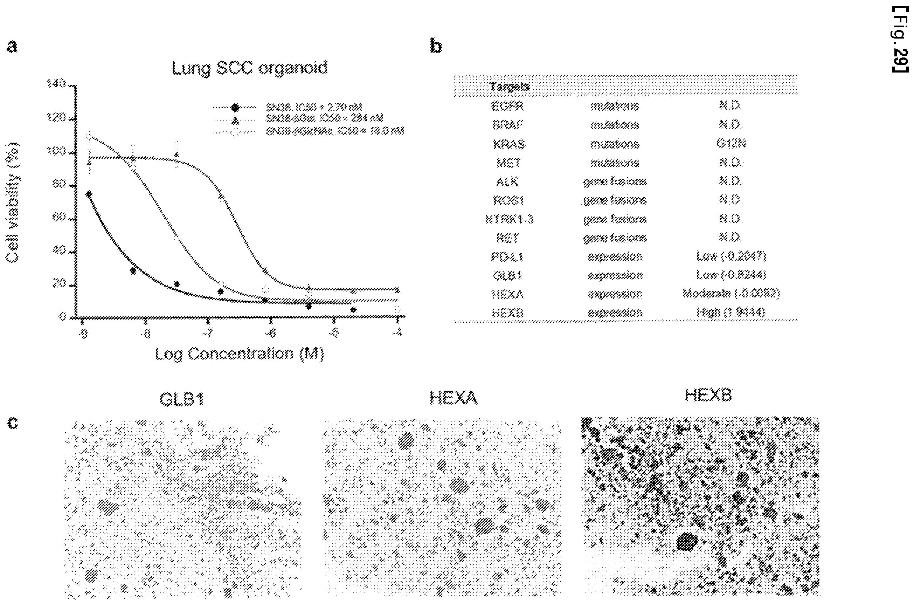

The proliferation suppression test of the developed prodrugs SN38-βGal and SN38-βGlcNAc in lung cancer organoids was performed. The organoid was an organoid of primary lung squamous cell carcinoma established at Fukushima Medical University Medical and Industrial TR Center, and RLUN016-2 (F-PDO_000124), which has low expression of gene mutation, fusion gene, and PD-L1 but high expression of HEXB in current lung cancer gene diagnosis, was used (b of Fig. 29). When the proliferation suppression test was performed for SN38, SN38-βGal, and SN38-βGlcNAc, the IC50 values were calculated to be 2.70 nM, 284 nM, and 18.0 nM, respectively, and all of them showed significant suppression of cell proliferation for the organoids (a of Fig. 29). In particular, SN38-βGlcNAc showed a lower IC50 value than SN38-βGal, suggesting that high expression of HEXB is involved in the proliferation suppression effect of the drug. When the PDX tumor established from the same cancer as these organoids was immunostained for the target enzymes GLB1, HEXA, and HEXB, expression of these enzymes was confirmed (c of Fig. 29).

A specific procedure of the therapeutic experiment is shown below.
(1) Frozen stocks of cells were thawed in a 37°C thermostat for 2 minutes. The cell suspension was collected and transferred to a 50 mL centrifuge tube containing 10 mL of medium. Centrifugation was performed at 200 × g for 3 minutes to remove the supernatant. 5 mL of the medium was added to resuspend the cells, and the cells were seeded in a 75 cm² flask and cultured in a CO2 incubator.
(2) Cells were collected in a 15 mL centrifuge tube. The centrifugation was performed at 200 × g for 2 minutes to remove 3 mL of the supernatant. After 2 mL of fresh medium was added and pipetted gently, 5 mL of the cell suspension was poured into a flask containing 10 mL of medium. After 4 mL of fresh medium was added and pipetted gently, 2 flasks containing 10 mL of the medium were dispensed with 5 mL of the cell suspension.
(3) All cells were collected in a 15 mL centrifuge tube and centrifuged at 200 x g for 2 minutes. After centrifugation, the pellet amount of the cells was measured, and it was confirmed that the pellet volume was 150 µL. The supernatant was removed by leaving 3 mL of the medium. A fresh medium was added so that the amount of the cell suspension was 15 mL, and the mixture was returned to the flask and cultured in a CO₂ incubator.
(4) 8 concentrations were prepared from DMSO stock solutions of SN38, SN38-βGal, SN38-βGlcNAc, and 100 mM. Each solution was dispensed into 96 well plates and was cryopreserved at -80°C until use.
(5) A required amount of cell culture medium was collected from the flask, and the cell mass was broken into small pieces using a CellPet FT (filter diameter 70 µm), and the mixture was diluted 30-fold to prepare a cell suspension. Each 150 µL of the prepared cell suspension was seeded on a 96 well spheroid plate by a Multi-drop Combi. The cells were cultured in a CO2 incubator until the addition of the drug.
(6) The plate into which the evaluation concentrations of SN38, SN38-βGal, and SN38-βGlcNAc had been dispensed and the cell plate prepared in the preparation of the cell suspension were set in a dispenser, and 0.15 µL of the drug was added to each well so as to have N = 3 and 8 concentrations. The final treatment concentrations of the drugs were 100, 20, 4, 0.8000, 0.1600, 0.0320, 0.0064, and 0.0013 µM, and the final concentration of DMSO was 0.1%. The cells were cultured in a CO2 incubator (5% CO2, 37°C) for 6 days until the measurement of the amount of ATP.
(7) The plate to be measured was taken out from a CO2 incubator and allowed to stand at room temperature for 30 minutes. 10 µL of CellTiter-Glo 3D reagent was added to each well. The mixture was stirred with a vortex mixer for 5 minutes, and then left to stand at room temperature for 10 minutes. The chemiluminescence value of each plate was measured, and an IC50 value was calculated from the ATP amount ratio in each group with the ATP amount of cells to which only DMSO was added as 100%.

Fig. 29 illustrates the results of a proliferation suppression test in lung squamous cell carcinoma organoids. a of Fig. 29 illustrates the proliferation suppression result in the organoid, and b illustrates lung cancer genetic test item information and expression information of GLB1, HEXA, and HEXB of the used organoid. c of Fig. 29 illustrates immunostaining of GLB1, HEXA, and HEXB in the PDX tumors of the organoids used. Error bars = S.D.

### [Example 17]

### Tumor proliferation suppression test in PDX model mice

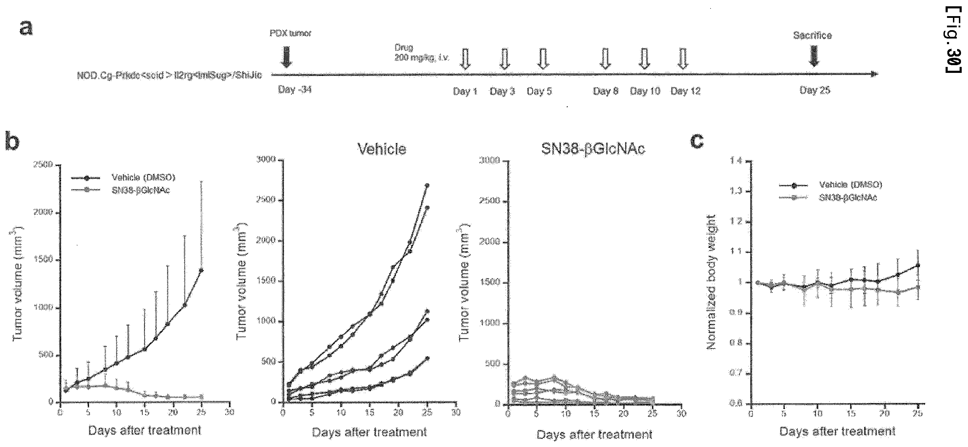

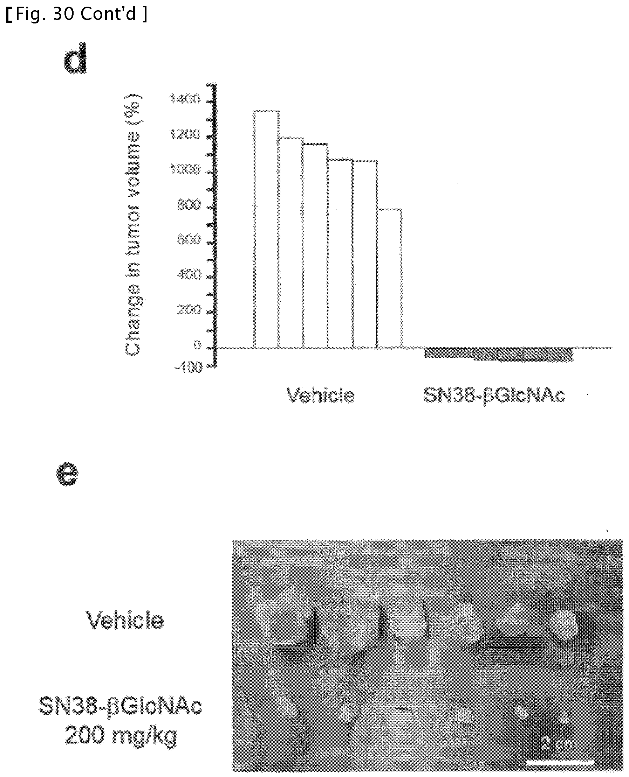

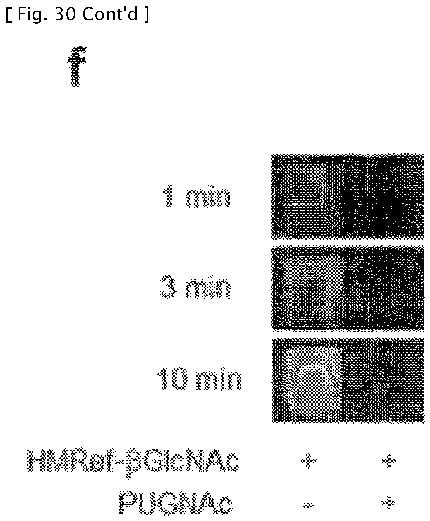

With respect to SN38-βGlcNAc showing high sensitivity by the organoid test, an in vivo tumor proliferation suppression test was performed using a primary lung squamous cell carcinoma PDX tumor (DLUN013/F_PDX_000162) established at Fukushima Medical University Medical and Industrial TR Center from the same patient. 200 mg/kg of SN38-βGlcNAc was intravenously administered to PDX subcutaneous tumor model mice a total of 6 times over 2 weeks as shown in the administration schedule (a of Fig. 30), and the tumor proliferation suppression effect was evaluated. The Vehicle (DMSO administration) group and the SN38-βGlcNAc administration group were n = 6, respectively.

Significant tumor growth was confirmed in the Vehicle group, whereas the tumor growth was significantly suppressed in the SN38-βGlcNAc administration group (b, d, and e of Fig. 30). No body weight change was observed in the administration of the drug (c of Fig. 30).

When the tumor of the Vehicle group was removed and then subjected to fluorescence imaging using HMRef-βGlcNAc (50 µM, PBS solution), significant activity of β-hexosaminidase, which is a target enzyme of SN38-βGlcNAc, was confirmed. The observed fluorescence was significantly suppressed in the presence of PUGNAc (500 µM), an inhibitor of β-hexosaminidase (f of Fig. 30).

A specific procedure of the therapeutic experiment is shown below.
(1) The tube containing the frozen tumor tissue was thawed in a thermostatic water bath at 37°C. The tissue pieces were transferred to a dish containing HBSS (+1% PS).
(2) NOG (NOD.Cg-Prkdc<scid>Il2rg<tmlSug>/ShiJic) (7 weeks old, female) anesthetized by intraperitoneal administration of an anesthetic (a mixture of three kinds of Domitor, Betorphal, and Mitazolam) was subjected to hair shaving with a hair clipper on the right back, and then the hair removal portion was disinfected with 70% ethanol, and applied with Isodine.
(3) The transplantation site in the right back was incised by about 3 mm, the tumor tissue was loaded in the distal opening of the trocar, and inserted into the transplantation site to push out the tumor piece (about 30 mg of tumor piece was transplanted per one). After transplantation, the incision was adhered with medical Aron Alpha. An antisedan (atipamezol) solution was administered intraperitoneally and the anesthesia was removed.
(4) The drug administration was started to the individual whose tumor volume reached about 40 to 250 mm³ after 34 days. The SN38-βGlcNAc was prepared by dissolving a 400 mM of DMSO stock in PBS, and was intravenously administered from the tail vein 3 times a week for 2 weeks at a dose of 200 mg/kg with a total amount of 100 µL.
(5) The tumor volume and body weight were measured on the day of evaluation.
(6) The tumor was removed, and then subjected to fluorescence imaging using HMRef-βGlcNAc (50 µM, PBS solution) in the presence and absence of PUGNAc (500 µM), which is an inhibitor of β-hexosaminidase.

Fig. 30 illustrates a tumor proliferation suppression effect of SN38-βGlcNAc in PDX model mice. a of Fig. 30 illustrates the administration schedule, and b illustrates the transition of the tumor volume. c of Fig. 30 illustrates the transition of the body weight change, and d illustrates the change in tumor volume on Day 25. e of Fig. 30 illustrates an image of the tumor removed on Day 25, and f illustrates the evaluation result of β-hexosaminidase activity by fluorescence imaging of the tumor. Error bars = S.D.

### [Example 18]

### Tumor proliferation suppression test in small cell lung cancer model

The tumor proliferation suppression effect of the developed prodrugs SN38-βGal and SN38-βGlcNAc in a small cell lung cancer model was evaluated. The model was created by orthotopically transplanting SHP77-luc in which luciferase was expressed in a small cell lung cancer cell line SHP77 relative to BALB/cAJcl-nu/nu into the lung. The Vehicle (DMSO administration) group, the SN38-βGal administration group, and the SN38-βGlcNAc administration group were n = 6, respectively. The administration form was intravenous administration (i.v.), and the dose was 40 mg/kg, and administration was performed twice a week for 4 weeks. The tumor burden was evaluated by the luciferin/luciferase luminescence value.

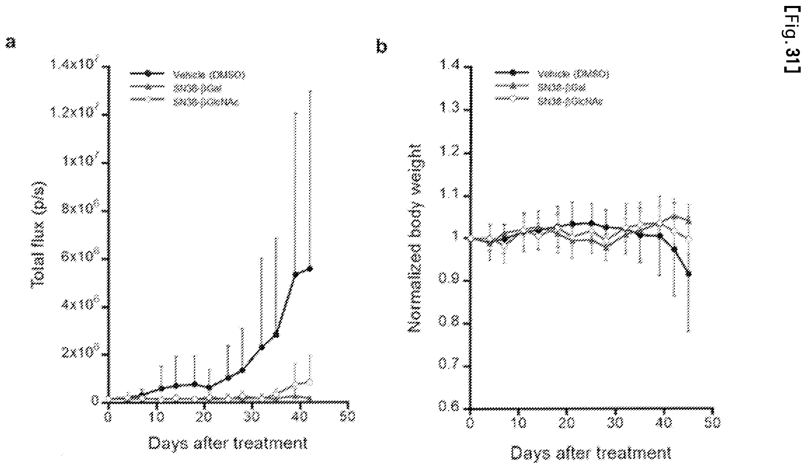

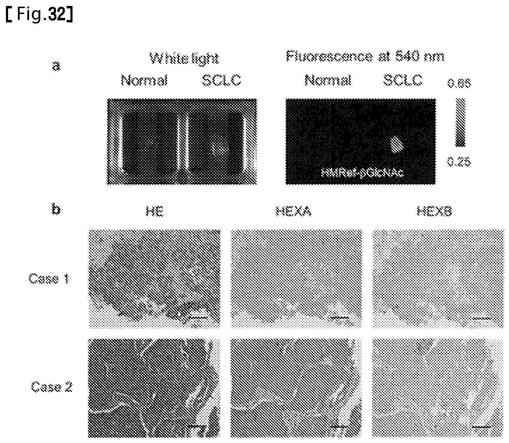

As a result, in the SN38-βGal and SN38-βGlcNAc administration groups, significant suppression of tumor growth was confirmed as compared with the Vehicle group (a of Fig. 31). The body weight loss in the Vehicle group is considered to be due to the progression of cancer, but no body weight loss was found during the treatment period in the SN38-βGal and SN38-βGlcNAc administration groups (b of Fig. 31). In addition, an example in which small cell lung cancer was fluorescently detected using HMRef-βGlcNAc was also confirmed (Fig. 32).

A specific procedure of the therapeutic experiment is shown below.
(1) Model mice were created by the following procedure using 20 BALB/cAJcl-nu/nu (7 weeks old/female) mice. 150 to 200 µL of an anesthetic (a mixture of 3 kinds of Domitor + Betorphal + Mitazolam) was intraperitoneally administered to perform anesthesia. The skin of the left chest was incised, the subcutaneous tissue was detached, and then SHP77 luc cell suspension 2 × 10⁷ cells/mL (50 µL, 5% Matrigel) was injected from the intercostal pleura to the left lung. The skin was sutured at 3 sites, and 150 to 200 µL of an anti-sedan (atipamezol) solution was intraperitoneally administered to check awakening of the mice.
(2) After 3 weeks, the formation of cancer was confirmed by luciferin/luciferase luminescence, and the administration of the drug was started. The drug was prepared by dissolving 200 mM of DMSO stock in PBS, and was administered from the tail vein in a total amount of 100 µL.3 mg (200 µL) of luciferin was intraperitoneally administered, and anesthetized with isoflurane. Thereafter, the luminescent value at 15 minutes after administration was measured by an in vivo imaging device.
(3) On the day of evaluation, the tumor burden was evaluated by the body weight and the luciferin/luciferase luminescence.

Fig. 31 illustrates the results of a therapeutic experiment in a small cell lung cancer orthotopic transplantation model. a of Fig. 31 illustrates the tumor suppression effect in each administration group, and b illustrates the transition of the body weight change. Error bars = S.D.

Fig. 32 illustrates an example of fluorescence detection of human small cell lung cancer tissue by HMRef-βGlcNAc. a of Fig. 32 illustrates fluorescence images of human normal lung tissue and small cell lung cancer tissue specimens 30 minutes after addition of HMRef-βGlcNAc (50 µM), and b illustrates immunostaining of HEXA and HEXB in small cell lung cancer tissue.

### [Example 19]

### Study on therapeutic effect of SN38-βGal in SKOV3 ovarian cancer peritoneal dissemination model

### (1) Results of therapeutic experiment (First)

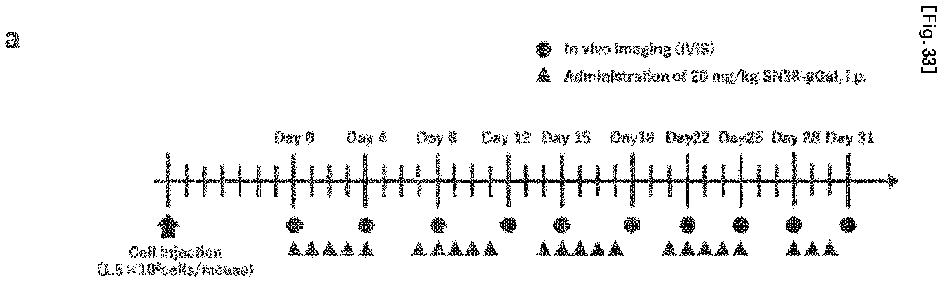

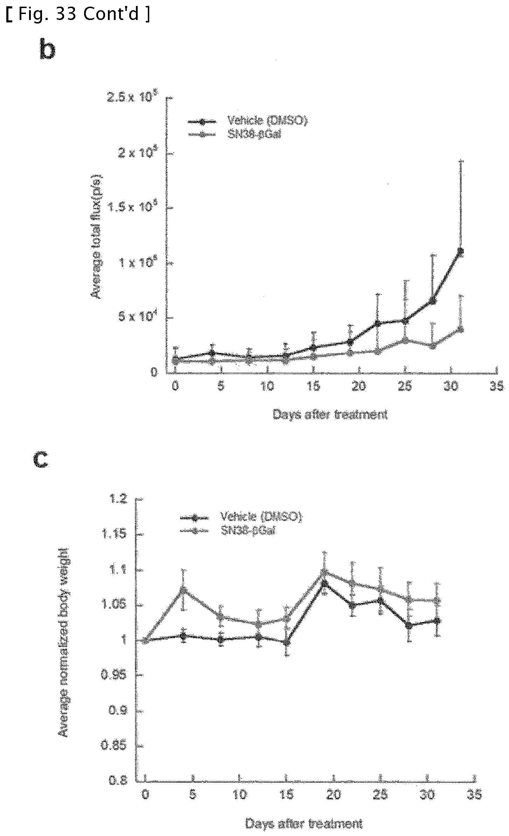

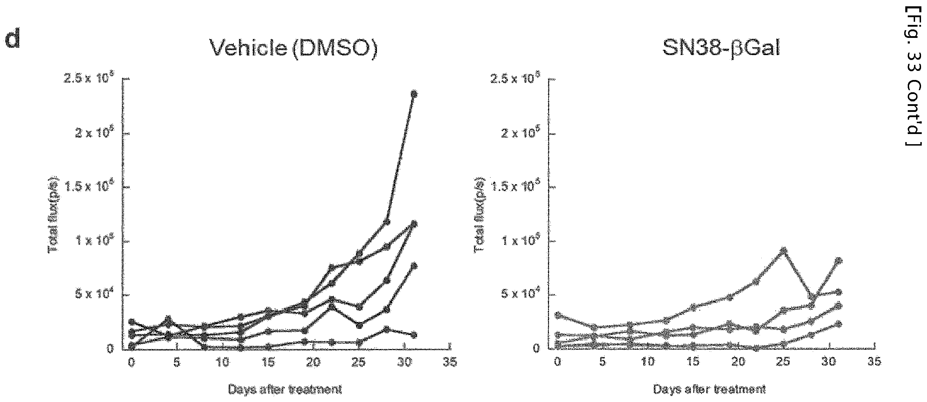

Next, the therapeutic effect of SN38-βGal in the ovarian cancer peritoneal dissemination model was verified. Vehicle (DMSO) group (n = 5), SN38-βGal, and 20 mg/kg (n = 4) were set, and 20 mg/kg was intraperitoneally administered 5 times a week (2 days off) over 4 weeks and 3 days. As a result, the tumor proliferation suppression effect was suggested again as compared with the Vehicle group. In addition, no significant body weight loss was found, suggesting that large side effects did not occur in this administration range (Fig. 33).

A specific procedure of the therapeutic experiment is shown below.
(1) 1.5 × 10⁶ luciferase-expressing SKOV3 cells were intraperitoneally administered to BALB/cAJcl-nu/nu (7 weeks old).
(2) After one week, the formation of cancer was confirmed by luciferin/luciferase chemiluminescence.
(3) Intraperitoneal administration of the drug was started in a cycle in which the drug was administered to the mice in which formation of cancer was confirmed for five consecutive days within 1 week and the drug was withdrawn for 2 days. The drug was prepared by dissolving a 100 mM of DMSO stock in PBS, and administered in a total amount of 100 µL.
(4) On the evaluation day, 3 mg of luciferin potassium dissolved in 200 µL of PBS was intraperitoneally administered, and anesthetized with isoflurane, and then the luminescence value 15 minutes after the administration was measured with an in vivo imaging device. At the same time, the body weight was measured.

Fig. 33 illustrates the results of a therapeutic experiment (first round) of SKOV3 ovarian cancer peritoneal dissemination model by SN38-βGal. a of Fig. 33 illustrates a dosage schedule, and b shows an evaluation of a tumor suppression effect. c of Fig. 33 illustrates the transition of the body weight change during the treatment period. d of Fig. 33 illustrates a comparison of chemiluminescent values from SKOV3 tumors in the Vehicle group and the SN38-βGal administration group. It is shown that SN38-βGal significantly suppressed tumor growth as compared with the Vehicle group.

### (2) Results of therapeutic experiment (Second)

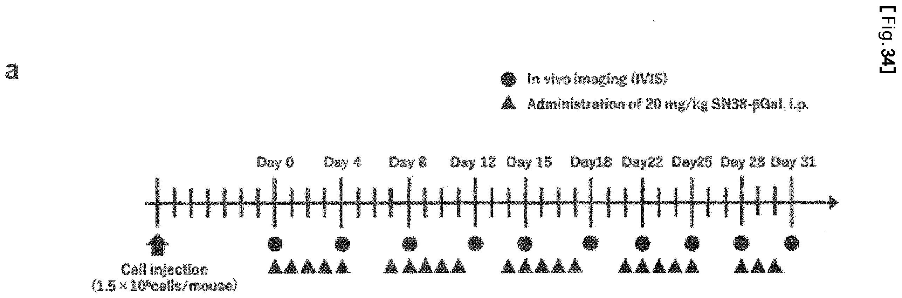

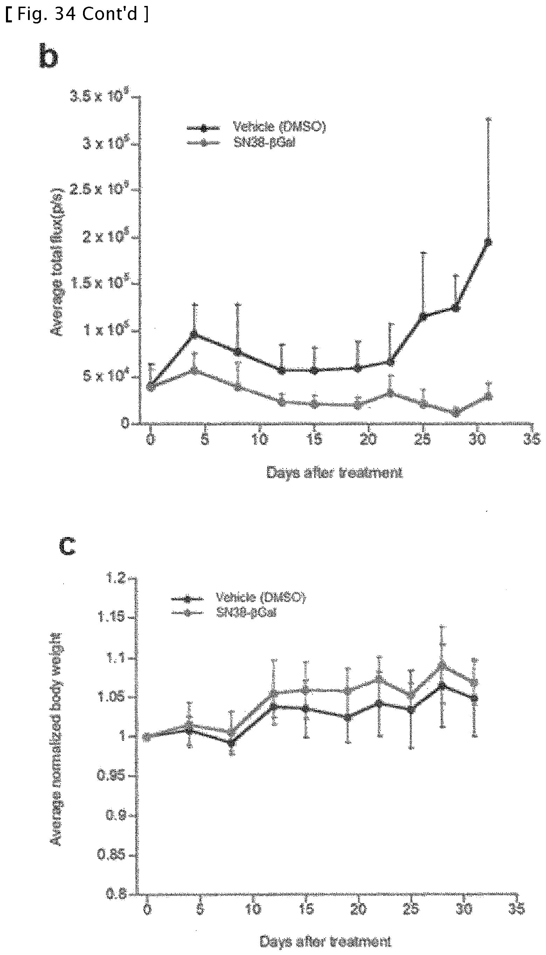

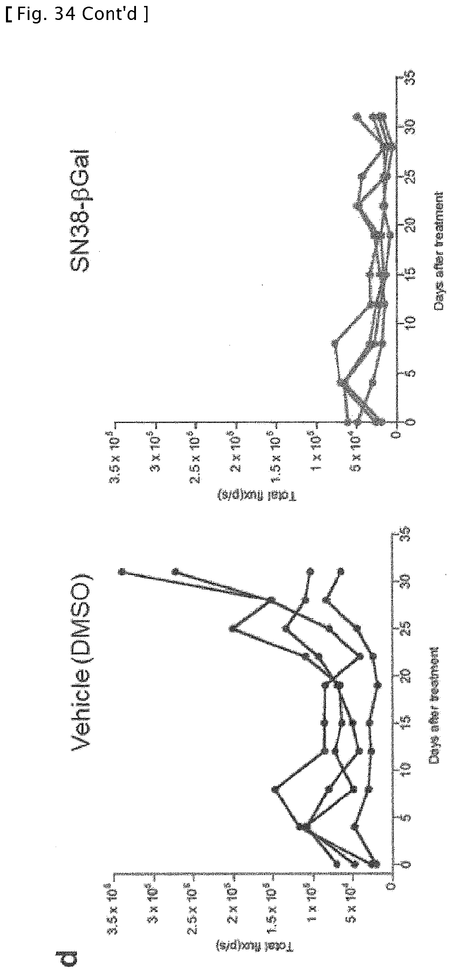

According to the same protocol as that of the first therapeutic experiment, Vehicle (DMSO) group (n = 5), SN38-βGal, and 20 mg/kg (n = 4) were set, and 20 mg/kg was intraperitoneally administered 5 times a week (2 days off) over 4 weeks and 3 days. As a result, the tumor proliferation suppression effect was confirmed again as compared with the Vehicle group. In addition, no significant body weight loss was found, suggesting that large side effects did not occur in this administration range (Fig. 34).

Fig. 34 illustrates the results of a therapeutic experiment (second round) of SKOV3 ovarian cancer peritoneal dissemination model by SN38-βGal. a of Fig. 34 illustrates a dosage schedule, and b shows an evaluation of a tumor suppression effect. c of Fig. 34 illustrates the transition of the body weight change during the treatment period. d of Fig. 34 illustrates a comparison of chemiluminescent values from SKOV3 tumors in the Vehicle group and the SN38-βGal administration group. It is shown that SN38-βGal significantly suppressed tumor growth as compared with the Vehicle group.

### [Example 20]

### Combination experiment with anti-PD-1 antibody

The effect of combination use of an anti-PD-1 antibody and SN38-βGlcNAc used as a main cancer immunotherapy in current lung cancer treatment was examined. Subcutaneous tumor model mice of a mouse Lewis lung cancer-derived cell line LLC (3LL) reported to have sensitivity to the anti-PD-1 antibody were evaluated, and the Vehicle (DMSO) group was n = 8, the anti-PD-1 antibody administration group was n = 5, the SN38-βGlcNAc administration group was n = 6, and the combination use group of the anti-PD-1 antibody and the SN38-βGlcNAc was n = 5. As an antibody, anti-mouse PD-1 antibody Clone RMP1-14 (Leinco Technologies, Inc.) was used. The anti-PD-1 antibody was administered intraperitoneally at 100 µg twice a week, and SN38-βGlcNAc was administered intravenously at 150 mg/kg three times a week.

As a result, as compared with the Vehicle group, the anti-PD-1 antibody administration group, the SN38-βGlcNAc administration group, and the combination use group of the anti-PD-1 antibody and SN38-βGlcNAc showed tumor proliferation suppression, and in particular, the highest tumor proliferation suppression was observed in the combination use group of the anti-PD-1 antibody and SN38-βGlcNAc. In addition, no body weight loss was found in any group.

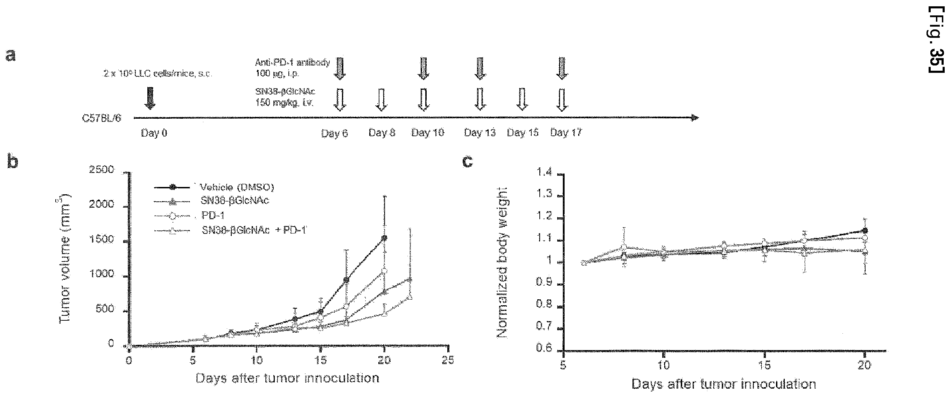

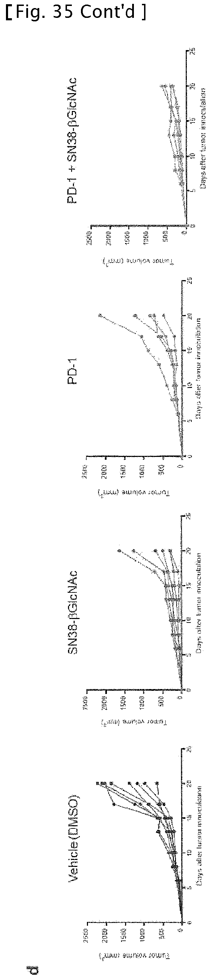

These results suggested that the combination of SN38-βGlcNAc with PD-1-based immunotherapy provides enhanced anticancer efficacy (Fig. 35).

A specific procedure of the therapeutic experiment is shown below.
(1) Hair on the dorsal side from the upper part of the right foot of C57BL/6JJcl (7 weeks old, female) was shaved with a hair clipper, and then LLC cells 2.0 ×10⁶ cells (50 µL) were subcutaneously injected.
(2) The drug administration was started to the individual whose tumor volume reached about 50 to 170 mm³ after 6 days. The SN38-βGlcNAc was prepared by dissolving a 400 mM of DMSO stock in PBS, and was administered 3 times a week for 2 weeks at a dose of 150 mg/kg with a total amount of 100 µL. After anesthetized by aspiration of isoflurane, the mixture was intravenously administered from the orbital vein. The anti-PD-1 antibody (Clone RMP1-14) was administered by intraperitoneal administration of 100 µg dissolved in 100 µL of PBS.
(3) The body weight was measured on the day of evaluation, and the tumor volume was evaluated with a vernier caliper.
(4) The time point when the tumor volume reached 2000 to 3000 mm³ was taken as the endpoint.

Fig. 35 illustrates the results of a combined experiment of an anti-PD-1 antibody and SN38-βGlcNAc. a of Fig. 35 illustrates the administration schedule, and b illustrates the transition of the tumor volume. c of Fig. 35 illustrates the transition of the body weight change, and d illustrates the transition of the tumor volume for each individual. Error bars = S.D.

### (B) Development of novel prodrug-type anticancer agent targeting protease activity

Next, as a novel prodrug-type anticancer agent targeting protease activity, prodrug KK-SN38 targeting promycin-sensitive aminopeptidase activity was synthesized, and the function as a prodrug-type anticancer agent was evaluated.

### 1. Synthesis of prodrug compounds

### [Synthesis Example 4]

### Synthesis of KK-SN38

Compound 5 (KK-SN38) of the present invention was synthesized according to the following Scheme 4.

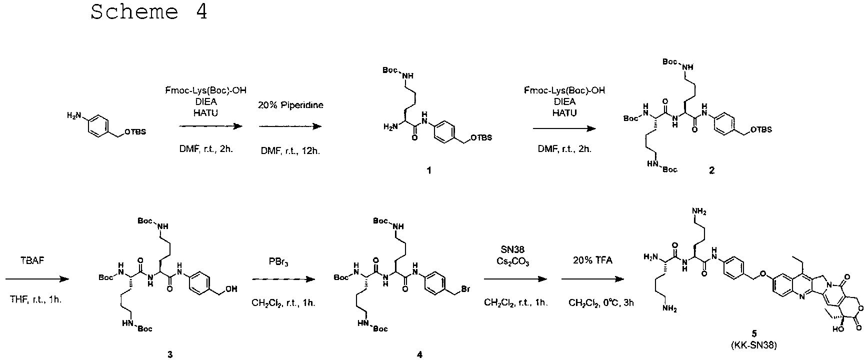

### (1) Synthesis of Compound 1

1.00 g (4.22 mmol) of 4-((tert-butyldimethylsilyloxy)methyl) aniline, 2.00 g (4.26 mmol) of Fmoc-Lys (Boc)-OH, 1.63 g (12.6 mmol) of DIEA, and 2.40 g (6.32 mmol) of HATU were dissolved in 30 mL of DMF (super dehydrated), and the mixture was stirred under argon substitution at normal temperature for 2 hours. 10 mL of AcOEt was added to a reaction solution, the mixture was washed with 30 mL (×2) of MilliQ and 30 mL (×2) of saturated saline, and dried over anhydrous MgSO₄, and the solvent was distilled off under reduced pressure. The obtained compound was dissolved in 15 mL of DMF (super dehydrated) in which 20% of piperidine was dissolved, and the solution was stirred for 1 hour at normal temperature under argon substitution. The reaction solution was washed with 30 mL (×2) of MilliQ, washed with 30 mL (×2) of saturated saline, and then dried over anhydrous MgSO₄. The solvent was distilled off under reduced pressure, and the residue was purified by silica gel column chromatography (CH₂Cl₂: MeOH = 96:4 → 90:10). The solvent was distilled off under reduced pressure to obtain yellow oily compound 1 (1.30 g, 2.79 mmol) at a yield of 66.1% obtained by combining two stages. The obtained target product was identified by ¹HNMR, ESI-HRMS.

¹H-NMR (400 MHz, METHANOL-D4) δ7.52 (d, J = 8.2 Hz, 2H), 7.25 (d, J = 8.7 Hz, 2H), 4.68 (s, 2H), 3.39 (t, J=6.4 Hz, 2H), 3.02 (t, J=6.2 Hz, 2H), 1.48-1.38 (m, 15H), 0.91 (s, 9H), 0.07 (s, 6H). HRMS(ESI+): calcd for [M+H]+466.31011; found 466.31273 (2.62 mDa).

### (2) Synthesis of Compound 2

1.30 g (2.79 mmol) of the compound 1, 1.00 g (2.89 mmol) of Boc-Lys (Boc)-OH, 1.10 g (8.52 mmol) of DIEA, and 1.70 mg (4.47 mmol) of HATU were dissolved in 20 mL of DMF (super dehydrated), and the mixture was stirred under argon substitution at normal temperature for 2 hours. 20 mL of AcOEt was added to the reaction solution, and the reaction solution was washed with 20 mL (×2) of MilliQ and washed with 20 mL (×2) of saturated saline, then dried over anhydrous MgSO₄. The solvent was distilled off under reduced pressure, and the residue was purified by silica gel column chromatography (hexane: AcOEt = 60:40 → 30:70). The solvent was distilled off under reduced pressure to obtain colorless solid compound 2 (2.08 g, 2.62 mmol) at a yield of 93.7%. The obtained target product was identified by ¹HNMR, ESI-HRMS.

¹H-NMR (400 MHz, METHANOL-D4) δ7.51 (d, J = 8.7 Hz, 2H), 7.24 (d, J = 8.7 Hz, 2H), 6.56 (s, 1H), 4.68 (s, 2H), 4.42 (dd, J = 8.5, 5.3 Hz, 1H), 4.00 (dd, J = 8.5, 5.3 Hz, 1H), 3.01 (m, 4H), 1.48-1.40 (m, 39H), 0.91 (s, 9H), 0.07 (s, 6H). HRMS(ESI+): calcd for [M+H]+794.51095; found 794.50993 (1.02 mDa).

### (3) Synthesis of Compound 3

In 30 mL of THF (super dehydrated), 2.10 g (2.64 mmol) of the compound 2, and 1.40 g (5.36 mmol) of TBAF were dissolved, and the mixture was stirred at normal temperature for 1 hour under argon substitution. 30 mL of AcOEt was added to the reaction solution, and the reaction solution was washed with 20 mL (×2) of MilliQ and then dried over anhydrous MgSO₄. The solvent was distilled off under reduced pressure, and the residue was purified by silica gel column chromatography (hexane: AcOEt = 20:80 → 0:100). The solvent was distilled off under reduced pressure to obtain colorless solid compound 3 (1.20 g, 1.76 mmol) at a yield of 66.7%. The obtained target product was identified by ¹HNMR, ESI-HRMS.

¹H-NMR (400 MHz, METHANOL-D4) δ7.53 (d, J = 8.7 Hz, 2H), 7.27 (d, J = 8.7 Hz, 2H), 4.53 (s, 2H), 4.42 (dd, J = 8.7, 5.5 Hz, 1H), 3.99 (dd, J = 8.7, 5.5 Hz, 1H), 3.00 (td, J=6.4, 6.4 Hz, 4H), 1.64-1.32 (m, 39H). HRMS(ESI+): calcd for [M+Na]+702.40547; found 702.40540 (0.08 mDa).

### (4) Synthesis of Compound 4

1, 20 g (1.76 mmol) of the compound 3, and 240 mg (0.886 mmol) of PBr₃ were dissolved in 20 mL of CH₂Cl₂ (super dehydrated), and the mixture was stirred at normal temperature for 1 hour under argon substitution, and then quenched with 10 mL of an aqueous NaHCO₃ solution. The reaction solution was washed with 30 mL (×2) of MilliQ, washed with 30 mL of saturated saline, and then dried over anhydrous MgSO₄. The solvent was distilled off under reduced pressure, and the residue was purified by silica gel column chromatography (hexane: AcOEt = 50:50 → 20:80) to obtain a compound 4 at a yield of 46%. The obtained target product was identified by ¹HNMR, ESI-HRMS.

¹H-NMR (400 MHz, METHANOL-D4) δ7.57 (d, J = 8.2 Hz, 2H), 7.36 (d, J = 8.7 Hz, 2H), 4.55 (s, 2H), 4.44 (dd, J = 8.5, 5.3 Hz, 1H), 4.10 (q, J = 7.2 Hz, 1H), 4.01 (dd, J = 8.5, 5.3 Hz, 1H), 3.03 (td, J = 5.9, 5.9 Hz, 4H), 1.27-1.68 (m, 39H). HRMS(ESI+): calcd for [M+Na]+766.31926; found 766.31895 (0.31 mDa).

### (5) Synthesis of Compound 5 (KK-SN38)

150 mg (0.202 mmol) of the compound 4, 55 mg (0.140 mmol) of SN38, and 70 mg (0.215 mmol) of Cs₂CO₃ were dissolved in 5 mL of DMF (super dehydrated), and the mixture was stirred at normal temperature for 1 hour under argon substitution. 5 mL of AcOE was added to the reaction solution, was washed with 5 mL (× 2)of MilliQ and 5 mL of saturated saline, and then dried over anhydrous MgSO₄. The solvent was distilled off under reduced pressure, and the residue was purified by silica gel column chromatography (CH₂Cl₂:MeOH = 95:5 → 90:10). The solvent was distilled off under reduced pressure to obtain a crude purified SN38 adduct. This was dissolved in 5 mL of CH₂Cl₂ (super dehydrated) containing 25%TFA, and the solution was stirred for 3 hours under ice cooling under argon substitution. The solvent was distilled off under reduced pressure, and purified by HPLC and then freeze-dried to obtain a target product compound 5 as a yellow solid and KK-SN38 (83 mg, 0.110 mmol) at a yield of 54.5% obtained by combining two steps. The obtained target product was identified by ¹HNMR, ¹³C-NMR, and ESI-HRMS.

¹H-NMR (400 MHz, DMSO-D6) δ8.00 (d, J = 8.7 Hz, 1H), 7.63 (s, 2H), 7.48 (m, 4H), 7.25 (s, 1H), 5.38 (dd, J = 25.2, 16.0 Hz, 2H), 5.24 (s, 2H), 5.16 (s, 2H), 4.40 (dd, J = 13.3, 7.8 Hz, 1H), 3.85 (m, 1H), 3.08 (m, 2H), 2.75 (m, 4H), 1.84-1.37 (m, 14H), 1.22 (t, J = 7.5 Hz, 3H), 0.85 (t, J = 7.3 Hz, 3H) .¹³C-NMR (101 MHz, DMSO-D6) δ173.1, 170.7, 169.1, 157.7, 157.4, 150.6, 150.2, 146.8, 145.0, 144.4, 139.2, 132.0, 129.4, 129.0, 128.3, 123.2, 119.7, 118.8, 116.0, 104.2, 96.6, 72.9, 70.1, 65.8, 54.1, 52.3, 50.1, 46.1, 39.0, 32.0, 31.0, 30.7, 27.3, 27.0, 23.0, 22.8, 21.5, 14.0, 8.3. HRMS(ESI+): calcd for [M+H]+754.39667; found 754.39282 (3.85 mDa).

### [Example 21]

### Therapeutic experiment of KK-SN38 in peritoneal dissemination model

### (1) Results of therapeutic experiment (First)

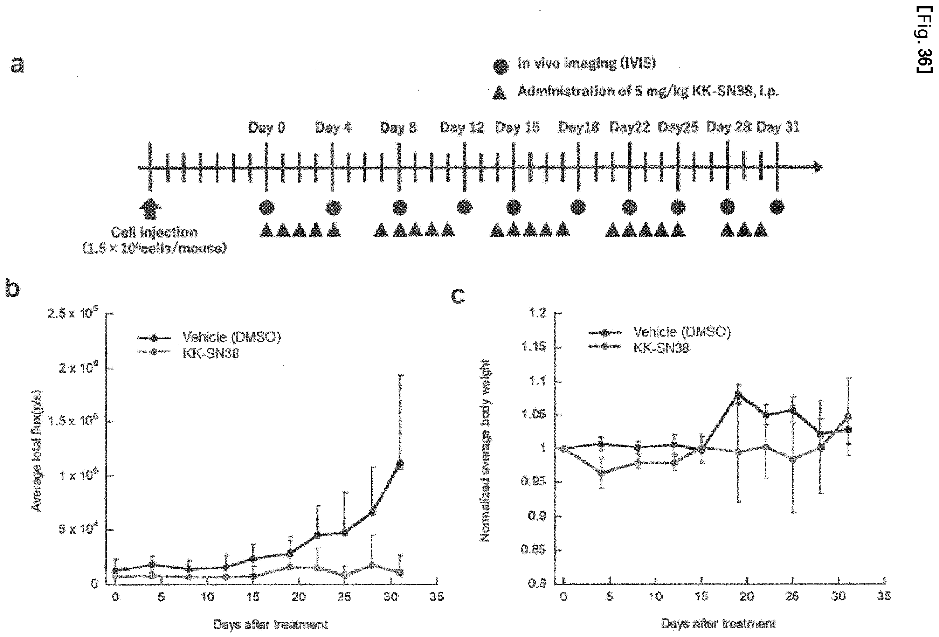

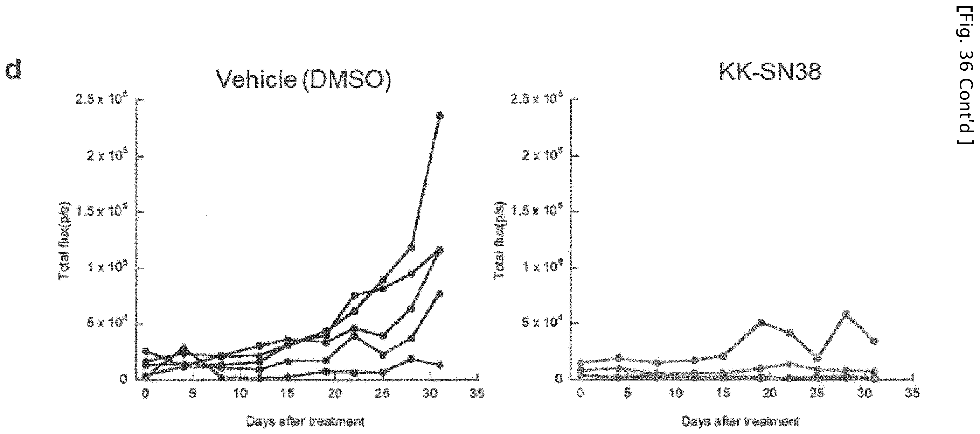

The therapeutic effect of a prodrug-type anticancer agent KK-SN38 having KK as a PSA target sequence as a substrate in an ovarian cancer SKOV3 peritoneal dissemination model was verified. Vehicle (DMSO) group (n = 5) and KK-SN38 group (n = 4) were set, KK-SN38 was intraperitoneally administered 5 times a week (2 days off) at 10 mg/kg for the first week, and intraperitoneally administered 5 times a week (2 days off) at 5 mg/kg thereafter over 3 weeks and 3 days. As a result, the tumor proliferation suppression effect was suggested as compared with the Vehicle group. In addition, no significant body weight loss was found, suggesting that large side effects did not occur (Fig. 36).

A specific procedure of the therapeutic experiment is shown below.
(1) 1.5 × 10⁶ luciferase-expressing SKOV3 cells were intraperitoneally administered to BALB/cAJcl-nu/nu (8 weeks old).
(2) After one week, the formation of cancer was confirmed by luciferin/luciferase chemiluminescence.
(3) Intraperitoneal administration of the drug was started in a cycle in which the drug was administered to the mice in which formation of cancer was confirmed for five consecutive days within 1 week and the drug was withdrawn for 2 days. The drug was prepared by dissolving a 100 mM of DMSO stock in PBS, and intraperitoneally administered in a total amount of 100 µL.
(4) On the evaluation day, 3 mg of luciferin potassium dissolved in 200 µL of PBS was intraperitoneally administered, and anesthetized with isoflurane, and then the luminescence value 15 minutes after the administration was measured with an in vivo imaging device. At the same time, the body weight was measured.

Fig. 36 illustrates the results of a therapeutic experiment (first round) of SKOV3 ovarian cancer peritoneal dissemination model by KK-SN38. a of Fig. 36 illustrates a dosage schedule, and b shows an evaluation of a tumor suppression effect. c of Fig. 36 illustrates the transition of the body weight change during the treatment period. d of Fig. 36 illustrates a comparison of chemiluminescent values from SKOV3 tumors in the Vehicle group and the KK-SN38 administration group. It is shown that KK-SN38 significantly suppressed tumor growth as compared with the Vehicle group.

### (2) Results of therapeutic experiment (Second)

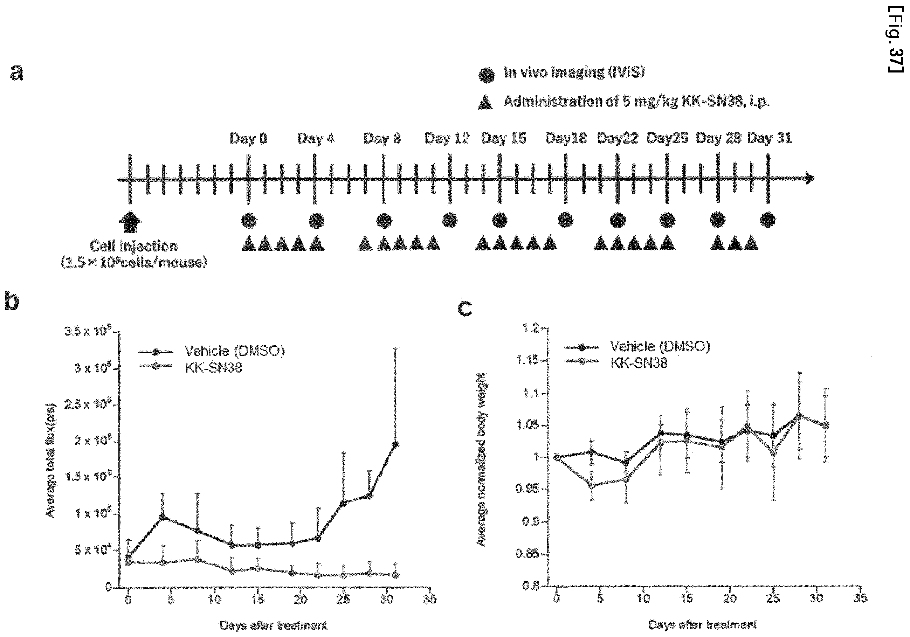

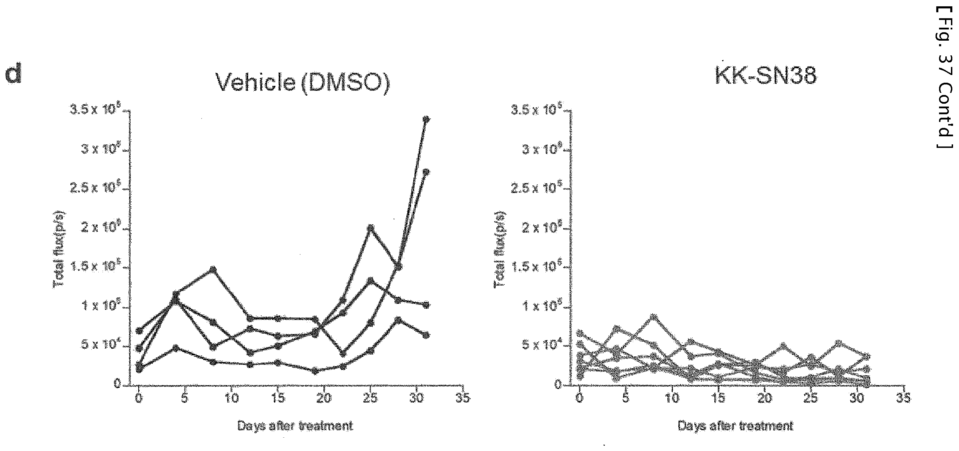

The therapeutic effect of KK-SN38 in the ovarian cancer peritoneal dissemination model was verified. Vehicle (DMSO) group (n = 4) and KK-SN38 (n = 7) were set, and KK-SN38 was intraperitoneally administered 5 times a week (2 days off) at 5 mg/kg over 4 weeks and 3 days. As a result, the tumor proliferation suppression effect was suggested again as compared with the Vehicle group. In addition, no significant body weight loss was found, suggesting that large side effects did not occur in this administration range (Fig. 37).

A specific procedure of the therapeutic experiment is shown below.
(1) 1.5 × 10⁶ luciferase-expressing SKOV3 cells were intraperitoneally administered to BALB/cAJcl-nu/nu (7 weeks old).
(2) After one week, the formation of cancer was confirmed by luciferin/luciferase chemiluminescence.
(3) Intraperitoneal administration of the drug was started in a cycle in which the drug was administered to the mice in which formation of cancer was confirmed for five consecutive days within 1 week and the drug was withdrawn for 2 days. The drug was prepared by dissolving a 100 mM of DMSO stock in PBS, and administered in a total amount of 100 µL.
(4) On the evaluation day, 3 mg of luciferin potassium dissolved in 200 µL of PBS was intraperitoneally administered, and anesthetized with isoflurane, and then the luminescence value 15 minutes after the administration was measured with an in vivo imaging device. At the same time, the body weight was measured.

Fig. 37 illustrates the results of a therapeutic experiment (second round) of SKOV3 ovarian cancer peritoneal dissemination model by KK-SN38. a of Fig. 37 illustrates a dosage schedule, and b shows an evaluation of a tumor suppression effect. c of Fig. 37 illustrates the transition of the body weight change during the treatment period. d of Fig. 37 illustrates a comparison of chemiluminescent values from SKOV3 tumors in the Vehicle group and the KK-SN38 administration group. It is shown that KK-SN38 significantly suppressed tumor growth as compared with the Vehicle group.

### (C) Prodrug-type anticancer agent including various anticancer agents

Next, a novel prodrug-type anticancer agent of the present invention in which a substrate site targeting cancer-specific enzyme activity was introduced into a molecule of an anticancer agent other than SN38 was synthesized, and the function as a prodrug-type anticancer agent was evaluated.

### 1. Synthesis of prodrug compounds

### (1) Synthesis of Compound 6

A compound 6 was synthesized according to the following scheme.

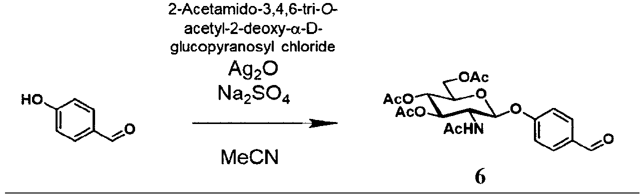

p-hydroxybenzaldehyde (3.00 g, 24.6 mmol), 2-acetamide-3,4,6-tri-O-acetyl-2-deoxy-α-D-glucopyranosyl chloride (9.00 g, 24.6 mmol), and Ag₂O (17.0 g, 73.4 mmol) were dissolved in 80.0 mL of acetonitrile, and the mixture was stirred at normal temperature for 20 hours. After Celite filtration, the solvent was distilled off under reduced pressure, and purified by silica gel column chromatography (gradient of CH₂Cl₂: MeOH = 100:0 to 70:30) to obtain a white solid compound 6 (10.53 g, 23.3 mmol) at a yield of 95.0%. The obtained target product was identified by ¹HNMR, ¹³CNMR, and ESI-HRMS.

¹HNMR (400 MHz, MeOD): δ 9.90 (s, 1H), 7.91 (d, J = 8.8 Hz, 2H), 7.21 (d, J = 8.8 Hz, 2H), 5.49 (d, J = 8.4 Hz, 1H), 5.39 (t, J = 9.2 Hz, 1H), 5.11 (t, J = 10 Hz, 1H), 4.34 (dd, J = 5.2 Hz, 12.4 Hz, 1H), 4.20-4.10 (m, 3H), 2.07 (s, 3H), 2.06 (s, 3H), 2.04 (s, 3H), 1.93 (s, 3H).¹³CNMR (101 MHz, MeOD): δ192.8, 173.7, 172.3, 171.8, 171.3, 163.2, 133.1, 132.9, 117.8, 98.9, 73.7, 73.3, 69.9, 63.1, 55.4, 22.7, 20.6, 20.6, 20.5. ESI-HRMS (ESI +) m/z calcd. for [M+Na]⁺, 474.13707; found, 474.13640.

### (2) Synthesis of Compound 7

Compound 7 was synthesized according to the following scheme.

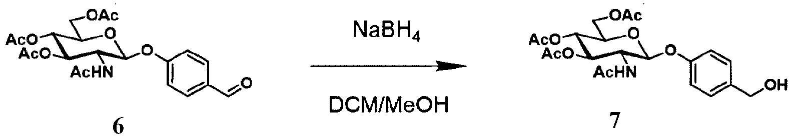

Compound 6 (750 mg, 1.66 mmol) and NaBH₄ (50.0 mg, 1.32 mmol) were dissolved in a mixed solvent of 5.0 mL of dichloromethane and 5.0 mL of methanol, and the mixture was stirred at normal temperature for 1.5 hours. Purified water was added, and the mixture was washed with dichloromethane to extract a target product. The organic phase was washed with saturated saline and then dried over sodium sulfate, and the solvent was distilled off under reduced pressure after filtration to obtain a white solid compound 7 (665 mg, 1.46 mmol) at a yield of 88.3%. The obtained target product was identified by ¹HNMR.

¹HNMR (400 MHz, CD₂Cl₂): δ7.33 (d, J = 8.4 Hz, 2H), 7.02 (d, J = 8.4 Hz, 2H), 5.70 (d, J = 8.4 Hz, 1H), 5.43 (t, J = 10 Hz, 1H), 5.31 (d, J = 8.0 Hz, 1H), 5.13 (t, J = 9.6 Hz, 1H), 4.30 (dd, J = 5.6 Hz, 12.0 Hz, 1H), 4.18-4.00 (m, 2H), 3.95-3.85 (m, 1H), 2.09-2.07 (m, 9H), 1.95 (s, 3H). ESI-HRMS (ESI +) m/z calcd. for [M+Na]⁺, 476.15272; found, 476.15320.

### (3) Synthesis of Compound 8

Compound 8 was synthesized according to the following scheme.

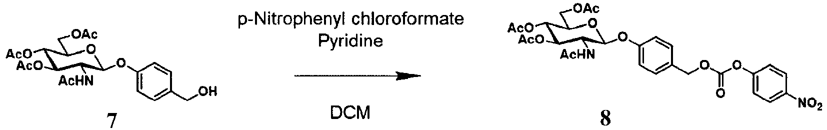

Compound 7 (278 mg, 0.614 mmol), p-nitrophenyl chloroformate (368 mg, 1.84 mmol), and pyridine (148 µL) were dissolved in 30.0 mL of ethyl acetate, and the mixture was stirred at room temperature under an argon atmosphere for 3 hours. A saturated sodium bicarbonate solution was added, and the mixture was washed with purified water to extract an ethyl acetate phase. The organic phase was washed with saturated saline and then dried over sodium sulfate, and the solvent was distilled off under reduced pressure after filtration. Purification was performed by silica gel column chromatography (gradient of AcOEt:hexane = 0:100 to 100:0) to obtain a white solid compound 8 (267 mg, 0.424 mmol) at a yield of 69.1%. The obtained target product was identified by ¹HNMR, ESI-HRMS.

¹HNMR (400 MHz, CD₂Cl₂): δ8.30 (d, J = 9.2 Hz, 2H), 7.44-7.41 (m, 4H), 7.07 (d, J = 8.8 Hz, 2H), 5.78 (d, J = 8.8 Hz, 1H), 5.48 (t, J = 9.6 Hz, 1H), 5.39 (d, J = 8.4 Hz, 1H), 5.27 (s, 2H), 5.14 (t, J = 9.6 Hz, 1H), 4.31 (dd, J = 5.6 Hz, 12.4 Hz, 1H), 4.19-4.07 (m, 2H), 3.97-3.92 (m, 1H), 2.08-2.07 (m, 9H), 1.95 (s, 3H). ESI-HRMS (ESI +) m/z calcd. for [M+Na]⁺, 641.15892; found, 641.16136.

### (4) Synthesis of Compound 9

Compound 9 was synthesized according to the following scheme.

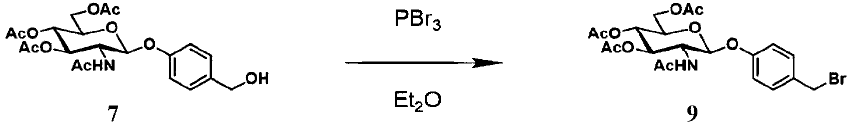

Compound 7 (565 mg, 1.25 mmol) and PBr₃ (337 mg, 1.84 mmol) were dissolved in 20.0 mL of diethyl ether, and the solution was stirred at normal temperature for 1 hour. A saturated sodium bicarbonate solution and ethyl acetate were added, and the mixture was washed with purified water to extract an ethyl acetate phase. The organic phase was washed with saturated saline and then dried over sodium sulfate, and the solvent was distilled off under reduced pressure after filtration to obtain a white solid compound 9 (440 mg, 0.854 mmol) at a yield of 68.5%. The obtained target product was identified by ESI-HRMS.

ESI-HRMS (ESI +) m/z calcd. for [M+Na]⁺, 538.06904; found, 538.06832.

### [Synthesis Example 5]

### Synthesis of DOX-βGlcNAc

DOX-βGlcNAc which is a compound of the present invention was synthesized according to the following scheme.

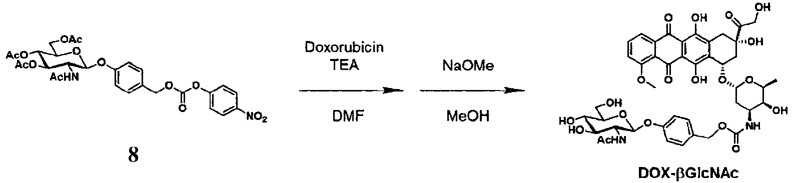

Compound 8 (170 mg, 0.275 mmol), doxorubicin (150 mg, 0.276 mmol), and triethylamine (600 µL) were dissolved in 8.0 mL of N,N-dimethylformamide, and the mixture was stirred at normal temperature for 1 hour. The solvent was distilled off under reduced pressure, diethyl ether was added thereto, and the red precipitate was collected by filtration. The red precipitate was dissolved in 20.0 mL methanol, NaOMe (149 mg, 2.76 mmol) was added thereto, and the mixture was stirred at normal temperature for 1 hour. Amberlite IR120 (300 mg) was added, and the mixture was stirred for 10 minutes, then removed by suction filtration, and the solvent was distilled off under reduced pressure. Purification was performed by silica gel column chromatography (gradient of CH₂Cl₂: MeOH = 100:0 to 75:25) to obtain a red solid DOX-βGlcNAc (69.0 mg, 0.0770 mmol) at a yield of 30.0%. The obtained target product was identified by ¹HNMR, ¹³CNMR, and ESI-HRMS.

¹HNMR (400 MHz, MeOD): δ7.83 (d, J = 7.2 Hz, 1H), 7.75 (t, J = 8.0 Hz, 1H), 7.47 (d, J = 8.0 Hz, 1H), 7.22 (d, J = 8.4 Hz, 2H), 6.93 (d, J = 8.4 Hz, 2H), 5.38 (s, 1H), 5.20-4.94 (m, overlaps with HOD), 4.74 (s, 1H), 4.31-4.24 (m, 1H), 3.98 (s, 3H), 3.90-3.81 (m, 4H), 3.74-3.51 (m, 9H), 4.77 (d, J = 4.4 Hz, 3H), 3.08-3.03 (m, 1H), 2.93-2.87 (m, 1H), 2.37-2.33 (m, 1H), 2.15-2.13 (m, 1H), 2.05-1.94 (m, 4H), 1.76-1.73 (m, 1H), 1.26 (d, J = 6.4 Hz, 3H).¹³CNMR (101 MHz, MeOD): 5214.0, 187.8, 187.5, 173.9, 162.3, 158.9, 158.0, 157.3, 156.1, 137.1, 136.2, 135.6, 135.0, 132.3, 130.6, 129.5, 121.3, 120.5, 120.2, 117.6, 112.3, 112.1, 102.2, 100.8, 78.2, 77.4, 75.8, 71.9, 71.8, 70.2, 68.7, 67.1, 65.8, 62.6, 57.4, 57.1, 37.3, 34.0, 30.9, 23.0, 17.3. ESI-HRMS (ESI +) m/z calcd. for [M+Na]⁺, 919.27435; found, 919.27385.

### [Synthesis Example 6]

### Synthesis of EPI-βGlcNAc

EPI-βGlcNAc which is a compound of the present invention was synthesized according to the following scheme.

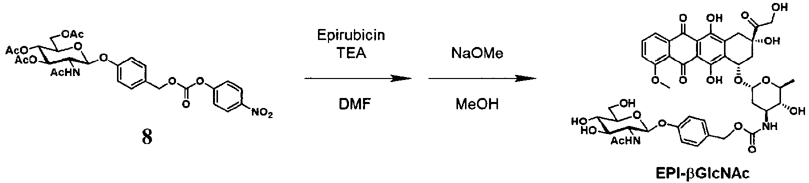

Compound 8 (85 mg, 0.138 mmol), epirubicin (75 mg, 0.138 mmol), and triethylamine (300 µL) were dissolved in 4.0 mL of N,N-dimethylformamide, and the mixture was stirred at normal temperature for 1 hour. The solvent was distilled off under reduced pressure, diethyl ether was added thereto, and the red precipitate was collected by filtration. The red precipitate was dissolved in 20.0 mL methanol, NaOMe (74.6 mg, 2.76 mmol) was added thereto, and the mixture was stirred at normal temperature for 1 hour. Amberlite IR120 (300 mg) was added, and the mixture was stirred for 10 minutes, then removed by suction filtration, and the solvent was distilled off under reduced pressure. Purification was performed by silica gel column chromatography (gradient of CH₂Cl₂:MeOH = 100:0 to 75:25) to obtain a red solid EPI-βGlcNAc (11.5 mg, 0.0128 mmol) at a yield of 9.3%. The obtained target product was identified by ¹HNMR, ESI-HRMS.

¹HNMR (400 MHz, MeOD): δ 7.87 (d, J = 7.2 Hz, 1H), 7.77 (t, J = 8.4 Hz, 1H), 7.49 (d, J = 8.4 Hz, 1H), 7.22 (d, J = 7.6 Hz, 2H), 6.94 (d, J = 8.0 Hz, 2H), 5.35 (s, 1H), 5.20-4.89 (m, overlaps with HOD), 3.99 (s, 3H), 3.93-3.85 (m, 4H), 3.74-3.51 (m, 9H), 3.39 (d, J = 5.2 Hz, 3H), 3.11-3.02 (m, 4H), 2.44-2.42 (m, 1H), 2.08-2.06 (m, 1H), 1.98-1.95 (m, 4H), 1.71-1.65 (m, 1H), 1.30 (d, J = 6.0 Hz, 3H). ESI-HRMS (ESI +) m/z calcd. for [M+Na]⁺, 919.27435; found, 919.27700.

### [Synthesis Example 7]

### Synthesis of Mel-βGlcNAc

Mel-βGlcNAc which is a compound of the present invention was synthesized according to the following scheme.

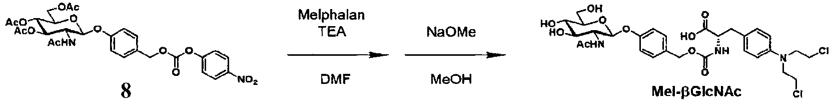

Compound 8 (203 mg, 0.328 mmol), melphalan (100 mg, 0.328 mmol), and triethylamine (400 µL) were dissolved in 15.0 mL of N,N-dimethylformamide, and the mixture was stirred at normal temperature for 1.5 hours. The solvent was distilled off under reduced pressure and dissolved in 20.0 mL methanol, and NaOMe (177 mg, 3.28 mmol) was added and stirred at normal temperature for 1 hour. Amberlite IR120 (300 mg) was added, and the mixture was stirred for 10 minutes, then removed by suction filtration, and the solvent was distilled off under reduced pressure. Purification was performed by HPLC to obtain a white solid Mel-βGlcNAc (92.6 mg, 0.141 mmol) at a yield of 43.0%. The obtained target product was identified by ¹HNMR, ¹³CNMR, and ESI-HRMS.

¹HNMR (400 MHz, MeOD): δ7.24 (d, J = 8.8 Hz, 2H), 7.08 (d, J = 8.4 Hz, 2H), 7.00 (d, J = 8.4 Hz, 2H), 6.65 (d, J = 8.4 Hz, 2H), 5.06 (d, J = 8.4 Hz, 1H), 4.33-4.30 (m, 1H), 3.98-3.91 (m, 2H), 3.76-3.57 (m, 10H), 3.47 (s, 2H), 3.33 (t, J = 1.2 Hz, 1H), 3.08 (dd, J = 4.8 Hz, J = 14 Hz, 1H), 3.08 (dd, J = 4.8 Hz, J = 14 Hz, 1H), 2.85 (dd, J = 8.4 Hz, J = 14 Hz, 1H), 2.66 (s, 3H), 2.00 (s, 2H).¹³CNMR (101 MHz, MeOD): δ176.5, 174.0, 158.8, 158.3, 146.5, 132.3, 131.6, 130.5, 127.3, 118.0, 117.6, 113.3, 100.8, 78.2, 75.8, 71.8, 67.2, 62.5, 57.7, 57.3, 54.5, 41.8, 40.5, 37.9, 23.1. ESI-HRMS (ESI +) m/z calcd. for [M+Na]⁺, 680.17482; found, 680.17122.

### [Synthesis Example 8]

### Synthesis of EXT-βGlcNAc

EXT-βGlcNAc which is a compound of the present invention was synthesized according to the following scheme.

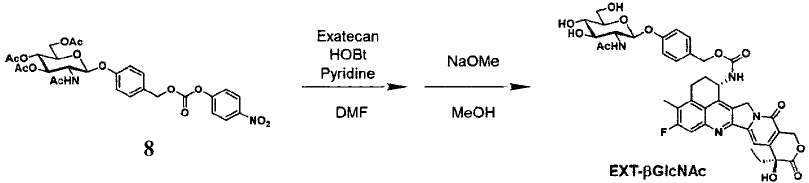

Compound 8 (170 mg, 0.275 mmol), exatecan (119 mg, 0.274 mmol), 1-hydroxybenzotriazole (HOBt) (37.0 mg, 0.274 mmol), and pyridine (1.0 mL) were dissolved in 4.0 mL of N,N-dimethylformamide, and the mixture was stirred at room temperature under an argon atmosphere for 20 hours. The solvent was distilled off under reduced pressure, and purified by silica gel column chromatography (gradient of CH₂Cl₂:MeOH = 100:0 to 65: 35) to obtain an acetylated sugar adduct of Exatecan. This was dissolved in 30.0 mL methanol, NaOMe (148 mg, 2.74 mmol) was added thereto, and the mixture was stirred at normal temperature for 1 hour. Amberlite IR120 (300 mg) was added, and the mixture was stirred for 10 minutes, then removed by suction filtration, and the solvent was distilled off under reduced pressure. Purification was performed by HPLC to obtain a brown solid EXT-βGlcNAc (32.0 mg, 0.0406 mmol) at a yield of 14.8%. The obtained target product was identified by ¹HNMR, ¹³CNMR, and ESI-HRMS.

¹HNMR (400 MHz, MeOD): δ7.40-7.33 (m, 3H), 7.23 (d, J = 10.4 Hz, 1H), 7.00 (d, J = 8.0 Hz, 2H), 5.45 (d, J = 16 Hz, 1H), 5.30-5.13 (m, 3H), 5.05 (d, J = 8.4 Hz, 1H, overlaps with HOD), 4.69 (d, J = 18.8 Hz, 1H), 3.95-3.90 (m, 2H), 3.75 (dd, J = 4.4 Hz, J = 11.6 Hz, 1H), 3.62-3.58 (m, 1H), 3.46-3.44 (m, 2H), 3.12-3.08 (m, 1H), 2.99-2.94 (m, 1H), 2.22 (s, 3H), 2.15 (d, J = 16 Hz, 2H), 2.00 (s, 3H), 1.93 (d, J = 6.8 Hz, 2H), 1.00 (t, J = 6.8 Hz, 3H) .¹³CNMR (101 MHz, MeOD): δ174.6, 173.9, 162.2, 159.0, 158.5, 158.3, 152.8, 152.3, 149.0, 148.8, 146.2, 142.4, 137.3, 132.1, 130.8, 127.0, 125.9, 125.8, 122.6, 120.8, 117.8, 116.5, 110.5, 100.9, 99.2, 78.3, 75.8, 74.1, 71.9, 67.9, 66.8, 62.6, 57.4, 50.9, 32.3, 29.7, 25.4, 23.1, 11.2, 8.3. ESI-HRMS (ESI +) m/z calcd. for [M+Na]⁺, 811.25972; found, 811.26103.

### [Synthesis Example 9]

### Synthesis of MMAE-βGlcNAc

MMAE-βGlcNAc which is a compound of the present invention was synthesized according to the following scheme.

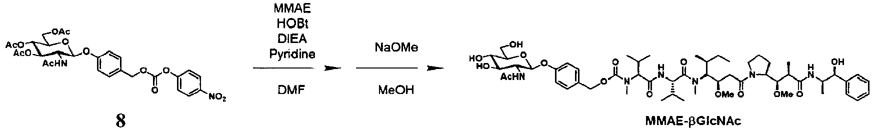

Compound 8 (70 mg, 0.113 mmol), monomethyl auristatin E (MMAE) (79.7 mg, 0.113 mmol), 1-hydroxybenzotriazole (HOBt) (15.3 mg, 0.113 mmol), N,N-diisopropylethylamine (DIEA) (30 µL), and pyridine (1.5 mL) were dissolved in 6.0 mL of N,N-dimethylformamide, and the mixture was stirred at room temperature under an argon atmosphere for 20 hours. The solvent was distilled off under reduced pressure and purified by HPLC to obtain an acetylated sugar adduct. This was dissolved in 10.0 mL methanol, NaOMe (61.0 mg, 1.13 mmol) was added thereto, and the mixture was stirred at normal temperature for 1 hour. Amberlite IR120 (300 mg) was added, and the mixture was stirred for 10 minutes, then removed by suction filtration, and the solvent was distilled off under reduced pressure. Purification was performed by HPLC to obtain a colorless solid MMAE-βGlcNAc (115 mg, 0.107 mmol) at a yield of 94.9%. The obtained target product was identified by ¹HNMR, ESI-HRMS.

¹HNMR (400 MHz, MeOD): δ7.43-7.30 (m, 6H), 7.25-7.21 (m, 1H), 7.04 (d, J = 8.0 Hz, 2H), 5.60-5.40 (m, overlaps with HOD), 5.19-5.07 (m, overlaps with HOD), 4.72-4.66 (m, 1H), 4.65-4.58 (m, 1H), 4.28-4.21 (m, 3H), 4.150-4.09 (m, 1H), 3.98-3.89 (m, 3H), 3.80-3.74 (m, 2H), 3.67-3.62 (m, 1H), 3.60-3.50 (m, 1H), 3.48 (d, J = 5.2 Hz, 3H), 3.43-3.29 (m, 10H), 3.25-3.15 (m, 1H), 3.12 (s, 1H), 2.99-2.92 (m, 3H), 2.53-2.48 (m, 2H), 2.30-2.10 (m, 3H), 2.00 (d, J = 4.0 Hz, 4H), 1.91-1.84 (m, 2H), 1.75-1.71 (m, 1H), 1.64-1.59 (m, 1H), 1.50-1.35 (m, 2H), 1.21-1.14 (m, 6H), 1.03-0.75 (m, 19H). ESI-HRMS (ESI +) m/z calcd. for [M+Na]⁺, 1093.60434; found, 1093.60145.

### [Synthesis Example 10]

### Synthesis of MMAF-βGlcNAc

MMAF-βGlcNAc which is a compound of the present invention was synthesized according to the following scheme.

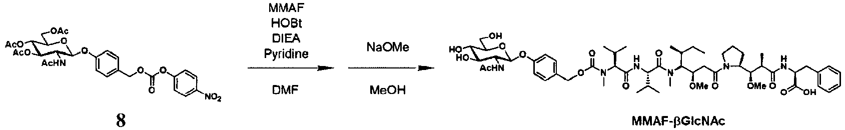

Compound 8 (127 mg, 0.205 mmol), monomethyl auristatin F (MMAF) (150 mg, 0.205 mmol), 1-hydroxybenzotriazole (HOBt) (27.8 mg, 0.206 mmol), N,N-diisopropylethylamine (DIEA) (53 µL), and pyridine (1.5 mL) were dissolved in 6.0 mL of N,N-dimethylformamide, and the mixture was stirred at room temperature under an argon atmosphere for 20 hours. The solvent was distilled off under reduced pressure and purified by HPLC to obtain an acetylated sugar adduct. This was dissolved in 10.0 mL methanol, NaOMe (110 mg, 2.04 mmol) was added thereto, and the mixture was stirred at normal temperature for 1 hour. Amberlite IR120 (300 mg) was added, and the mixture was stirred for 10 minutes, then removed by suction filtration, and the solvent was distilled off under reduced pressure. Purification was performed by HPLC to obtain a colorless solid MMAF-βGlcNAc (34.0 mg, 0.0313 mmol) at a yield of 15.3%. The obtained target product was identified by ¹HNMR, ¹³CNMR, and ESI-HRMS.

¹HNMR (400 MHz, MeOD): δ7.40-7.26 (m, 6H), 7.24-7.21 (m, 1H), 7.05-7.02 (m, 2H), 5.35-5.28 (m, overlaps with HOD), 5.20-5.06 (m, overlaps with HOD), 4.77-4.69 (m, 2H), 4.26-4.21 (m, 1H), 4.15-4.00 (m, 3H), 4.00-3.91 (m, 3H), 3.76-3.72 (m, 3H), 3.63-3.58 (m, 2H), 3.47-3.45 (m, 4H), 3.40-3.30 (m, 10H), 3.30 (s, 2H), 3.11 (s, 1H), 2.97-2.90 (m, 3H), 2.60-2.56 (m, 1H), 2.51-2.49 (m, 1H), 2.38-2.33 (m, 2H), 2.22-2.20 (m, 2H), 2.00 (s, 4H), 1.91-1.88 (m, 1H), 1.80-1.75 (m, 1H), 1.45-1.35 (m, 2H), 1.08-0.88 (m, 25H).¹³CNMR (101 MHz, MeOD): δ176.4, 176.2, 174.7, 174.6, 174.0, 172.1, 159.1, 138.7, 138.6, 132.2, 131.1, 130.6, 130.3, 130.3, 129.4, 129.4, 127.8, 117.8, 100.8, 87.5, 83.5, 78.2, 75.8, 71.9, 68.3, 65.9, 62.6, 62.2, 61.5, 60.9, 58.5, 58.4, 57.4, 56.1, 54.4, 54.3, 45.5, 45.4, 38.4, 33.0, 31.9, 30.3, 27.8, 27.0, 26.9, 25.9, 25.8, 24.5, 23.0. ESI-HRMS (ESI +) m/z calcd. for [M+Na]⁺, 1107.58221; found, 1107.58360.

### [Synthesis Example 11]

### Synthesis of NGT-βGlcNAc

NGT-βGlcNAc which is a compound of the present invention was synthesized according to the following scheme.

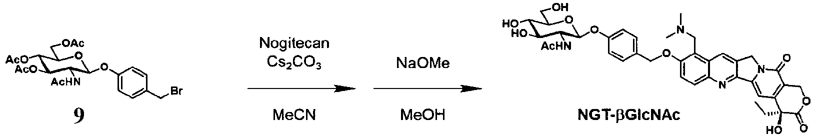

Compound 9 (168 mg, 0.326 mmol), nogitecan (164 mg, 0.340 mmol), and Cs₂CO₃ (1.0 g, 3.07 mmol) were dissolved in 30.0 mL of acetonitrile, and the mixture was stirred at normal temperature for 3 hours. The solvent was distilled off under reduced pressure, and purified by silica gel column chromatography (gradient of CH₂Cl₂:MeOH = 100:0 to 70: 30) to obtain an acetylated sugar adduct of Nogitecan. This was dissolved in 10.0 mL of methanol, NaOMe (88.1 mg, 1.63 mmol) was added thereto, and the mixture was stirred at normal temperature for 1 hour. Amberlite IR120 (300 mg) was added, and the mixture was stirred for 10 minutes, then removed by suction filtration, and the solvent was distilled off under reduced pressure. Purification was performed by HPLC to obtain a yellow solid NGT-βGlcNAc (3.6 mg, 0.00493 mmol) at a yield of 1.5%. The obtained target product was identified by ¹HNMR, ESI-HRMS.

¹HNMR (400 MHz, MeOD): δ8.88 (s, 1H), 8.37 (d, J = 9.6 Hz, 1H), 7.98 (d, J = 9.6 Hz, 1H), 7.50 (s, 1H), 7.52 (d, J = 8.4 Hz, 2H), 7.09 (d, J = 8.8 Hz, 2H), 5.58 (d, J = 16.4 Hz, 1H), 5.44-5.36 (m, overlaps with HOD), 5.10 (d, J = 8.8 Hz, overlaps with HOD), 4.90-3.87 (m, 3H), 3.71-3.57 (m, 5H), 3.48-3.92 (m, overlaps with MeOD), 2.95 (s, 6H), 2.05-1.90 (m, 5H), 1.01 (t, J = 7.2 Hz, 3H). ESI-HRMS (ESI +) m/z calcd. for [M+Na]⁺, 753.27423; found, 743.27355.

### · Synthesis of Compound 10

Compound 10 was synthesized according to the following scheme.

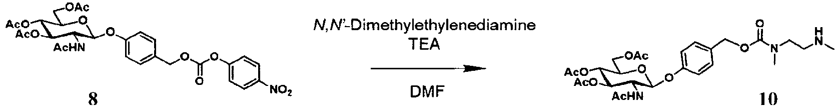

Compound 8 (200 mg, 0.324 mmol), N,N'-dimethylethylenediamine (114 mg, 1.29 mmol), and triethylamine (2.0 mL) were dissolved in 5.0 mL of N,N-dimethylformamide, and the mixture was stirred at normal temperature for 2 hours. The solvent was distilled off under reduced pressure, and purification was performed by silica gel column chromatography (gradient of CH₂Cl₂: MeOH = 100:0 to 0: 100) to obtain a white solid compound 10 (161 mg, 0.284 mmol) at a yield of 87.7%. The obtained target product was identified by ¹HNMR, ¹³CNMR, and ESI-HRMS.

¹HNMR (400 MHz, MeOD): δ7. 35(d, J = 8.4 Hz, 2H), 7.04 (d, J = 8.8 Hz, 2H), 5.40-5.33 (m, 2H), 5.11-5.07 (m, 3H), 4.34 (dd, J = 5.2 Hz, J = 12.4 Hz, 1H), 4.18-4.11 (m, 2H), 4.05-4.02 (m, 1H), 3.43 (s, 2H), 2.94 (s, 3H), 2.75-2.70 (m, 2H), 2.41 (s, 2H), 2.35 (s, 2H), 2.06-2.03 (m, 9H), 1.93 (s, 3H).¹³CNMR (101 MHz, MeOD): δ172.1, 170.8, 170.4, 169.8, 157.0, 131.3, 129.5, 129.2, 116.4, 98.4, 72.4, 71.6, 68.7, 66.5, 61.8, 54.1, 34.5, 33.9, 33.4, 21.3, 19.2, 19.2, 19.2. ESI-HRMS (ESI +) m/z calcd. for [M+Na]⁺, 590.23203; found, 590.23179.

### · Synthesis of Compound 11

Compound 11 was synthesized according to the following scheme.

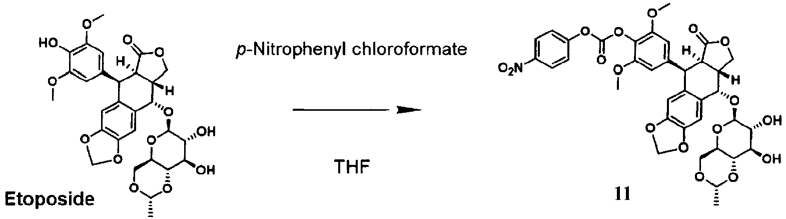

Etoposide (590 mg, 1.00 mmol) and p-nitrophenyl chloroformate (200 mg, 1.00 mmol) were dissolved in 30.0 mL of tetrahydrofuran and stirred at normal temperature for 1 hour. The precipitate was removed by suction filtration, and the solvent was distilled off under reduced pressure. Purification was performed by silica gel column chromatography (gradient of AcOEt:hexane = 0:100 to 80:20) to obtain a colorless solid compound 11 (410 mg, 0.544 mmol) at a yield of 54.3%. The obtained target product was identified by ¹HNMR, ¹³CNMR, and ESI-HRMS.

¹HNMR (400 MHz, CD₂Cl₂): δ8.18 (dd, J = 2.0 Hz, J = 6.8 Hz, 2H), 7.08 (dd, J = 2.4 Hz, J = 7.2 Hz, 2H), 6.78 (d, J = 10 Hz, 1H), 6.45 (d, J = 2.5 Hz, 1H), 6.27 (s, 2H), 5.91 (d, J = 8.4 Hz, 2H), 5.24 (s, 1H), 4.84 (d, J = 3.2 Hz, 1H), 4.64 (d, J = 5.2 Hz, 1H), 4.57-4.55 (m, 1H), 4.32-4.30 (m, 1H), 4.14 (t, J = 8.4 Hz, 1H), 4.08-4.05 (m, 1H), 3.66 (s, 6H), 3.54-3.44 (m, 3H), 3.28-3.15 (m, 4H), 3.04 (s, 1H), 2.84 (m, 1H), 1.24 (d, J = 5.2 Hz, 1H).¹³CNMR (101 MHz, CD₂Cl₂): δ175.1, 156.0, 151.6, 150.7, 149.3, 147.8, 146.9, 145.9, 139.5, 132.4, 129.3, 128.3, 125.7, 123.1, 110.8, 109.6, 108.0, 102.6, 102.3, 100.1, 80.3, 74.9, 74.0, 73.5, 68.5, 68.4, 66.8, 56.6, 44.4, 41.3, 38.1, 21.2, 20.5, 14.1. ESI-HRMS (ESI +) m/z calcd. for [M+Na]⁺, 776.17972; found, 776.17989.

### [Synthesis Example 12]

### Synthesis of ETP-βGlcNAc

ETP-βGlcNAc which is a compound of the present invention was synthesized according to the following scheme.

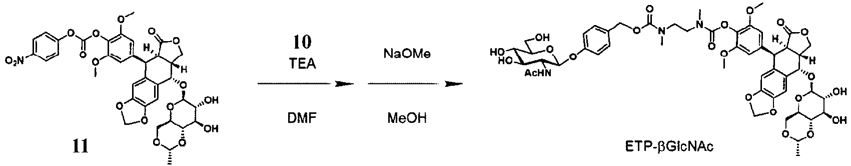

Compound 11 (214 mg, 0.284 mmol), the compound 10 (161 mg, 0.284 mmol), and triethylamine (2.0 mL) were dissolved in 5.0 mL of N,N-dimethylformamide, and the mixture was stirred at room temperature under an argon atmosphere for 12 hours. The solvent was distilled off under reduced pressure, and purified by silica gel column chromatography (gradient of CH₂Cl₂:MeOH = 100:0 to 0:100) to obtain an acetylated sugar adduct of Etoposide. This was dissolved in 10.0 mL methanol, NaOMe (107 mg, 1.98 mmol) was added thereto, and the mixture was stirred at normal temperature for 1 hour. Amberlite IR120 (300 mg) was added, and the mixture was stirred for 10 minutes, then removed by suction filtration, and the solvent was distilled off under reduced pressure. Purification was performed by HPLC to obtain a white solid ETP-βGlcNAc (192 mg, 0.182 mmol) at a yield of 64.1%. The obtained target product was identified by ¹HNMR, ¹³CNMR, and ESI-HRMS.

¹HNMR (400 MHz, MeOD): δ7.40-7.24 (m, 2H), 7.15 (s, 1H), 7.01-6.91 (m, 2H), 6.61-6.57 (m, 3H), 5.92 (d, J = 6.8 Hz, 2H), 5.372 (m, 1H, overlaps with HOD), 4.84 (s, 1H), 4.73 (d, J = 4.8 Hz, 1H), 4.50-4.47 (m, 1H), 4.39 (s, 1H), 4.35-4.25 (m, 1H), 4.15-4.05 (m, 1H), 3.99-3.89 (m, 4H), 3.76-3.71 (m, 9H), 3.66-3.45 (m, 10H), 3.34-3.20 (m, 5H), 3.11-2.97 (m, 5H), 2.88 (s, 1H), 2.03 (s, 3H), 1.99 (s, 2H), 1.30 (d, J = 5.2 Hz, 1H).¹³CNMR (101 MHz, MeOD): δ180.8, 173.1, 159.1, 158.7, 158.0, 157.1, 155.4, 155.2, 153.3, 148.6, 147.2, 140.7, 132.4, 131.3, 131.2, 130.2, 129.9, 129.6, 129.1, 117.4, 116.8, 114.2, 109.4, 107.9, 105.4, 102.1, 101.7, 99.8, 80.6, 77.2, 75.5, 75.0, 73.8, 70.9, 69.2, 68.2, 67.1, 66.5, 61.6, 56.4, 55.9, 55.8, 45.6, 44.1, 39.7, 34.7, 34.5, 22.1, 19.8, 0.0. ESI-HRMS (ESI +) m/z calcd. for [M+Na]⁺, 1078.36389; found, 1078.36515.

### · Synthesis of Compound 12

Compound 12 was synthesized according to the following scheme.

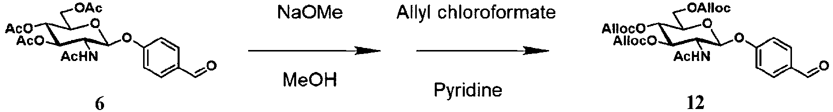

Compound 6 (5.05 g, 11.2 mmol) was dissolved in 10.0 mL of methanol, NaOMe (2.00 g, 37.0 mmol) was added, and then the mixture was stirred at normal temperature for 1 hour. Amberlite IR120 (2.00 g) was added, and the mixture was stirred for 10 minutes, then removed by suction filtration, and the solvent was distilled off under reduced pressure. Purification was performed by silica gel column chromatography (gradient of CH₂Cl₂: MeOH = 100:0 to 0: 100) to obtain a deprotected product of the compound 1. This was dissolved in 15.0 mL of pyridine, allyl chloroformate (13.0 g, 108 mmol) was added while cooling in an ice bath, and the mixture was stirred at normal temperature for 20 hours. Dichloromethane was added, and the mixture was washed with a saturated aqueous sodium bicarbonate solution, purified water, and saturated saline, and an organic phase was extracted. The organic phase was dried over sodium sulfate, and after filtration, the solvent was distilled off under reduced pressure, and purified by silica gel column chromatography (gradient of AcOEt:hexane = 0:100 to 100:0) to obtain a colorless solid compound 12 (144 mg, 0.249 mmol) at a yield of 2.2%. The obtained target product was identified by ¹HNMR, ¹³CNMR, and ESI-HRMS.

¹HNMR (400 MHz, CD₂Cl₂): δ8.29 (d, J = 9.2 Hz, 2H), 7.41 (d, J = 9.2 Hz, 4H), 7.10 (d, J = 8.8 Hz, 2H), 6.33 (d, J = 8.4 Hz, 1H), 6.01-5.91 (m, 3H), 5.59 (d, J = 8.4 Hz, 1H), 5.52 (t, J = 9.2 Hz, 1H), 5.40-5.26 (m, 8H), 5.01 (t, J = 10 Hz, 1H), 4.67-4.46 (m, 4H), 4.62 (d, J = 5.6 Hz, 1H), 4.43-4.39 (m, 1H), 4.35-4.31 (m, 1H), 4.15-4.00 (m, 2H),1.94 (s, 3H).¹³CNMR (101 MHz, CD₂Cl₂): δ171.1, 157.8, 156.0, 155.1, 154.9, 154.3, 152.8, 145.8, 132.0, 131.7, 131.7, 130.8, 129.5, 125.6, 122.3, 119.4, 119.2, 119.0, 117.4, 98.3, 75.9, 73.0, 71.8, 70.9, 69.6, 69.4, 69.1, 65.9, 55.5, 23.5. ESI-HRMS (ESI +) m/z calcd. for [M+Na]⁺, 767.19062; found, 767.19413.

### · Synthesis of Compound 13

Compound 13 was synthesized according to the following scheme.

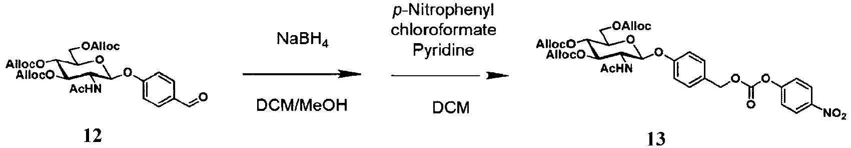

Compound 12 (144 mg, 0.249 mmol) and NaBH₄ (7.50 mg, 0.198 mmol) were dissolved in a mixed solvent of 3.0 mL of dichloromethane and 3.0 mL of methanol, and the mixture was stirred at normal temperature for 30 minutes. Purified water was added, and the mixture was washed with dichloromethane to extract a target product. The organic phase was washed with saturated saline and then dried over sodium sulfate, and the solvent was distilled off under reduced pressure after filtration to obtain a white solid intermediate. This was dissolved in 20.0 mL of dichloromethane, and p-nitrophenyl chloroformate (150 mg, 0.750 mmol), pyridine (60.3 µL) were added, and then the mixture was stirred for 1 hour. The solvent was distilled off under reduced pressure and was purified by silica gel column chromatography (gradient of AcOEt:hexane = 0:100 to 100:0) to obtain a compound 13 (89.3 mg, 0.120 mmol) as a white solid at a yield of 47.9%. The obtained target product was identified by ¹HNMR, ¹³CNMR, and ESI-HRMS.

¹HNMR (400 MHz, CD₂Cl₂): δ9.874 (s, 1H), 7. 88 (d, J = 8.8 Hz, 2H), 7.22 (d, J = 8.8 Hz, 2H), 5.99-5.92 (m, 3H), 5.66 (d, J = 8.4 Hz, 2H), 5.41-5.24 (m, 9H), 5.04-4.99 (m, 1H), 4.64-4.63 (m, 4H), 4.59 (d, J = 5.6 Hz, 1H), 4.40-4.30 (m, 1H), 4.30-4.20 (m, 2H), 1.92 (s, 3H).¹³CNMR (101 MHz, CD₂Cl₂): δ192.9, 173.8, 163.2, 156.1, 156.1, 155.5, 133.1, 133.1, 133.0, 133.0, 132.9, 119.4, 119.1, 119.0, 117.9, 98.6, 77.4, 74.0, 72.8, 70.3, 70.0, 69.8, 66.8, 55.7, 23.0. ESI-HRMS (ESI +) m/z calcd. for [M+Na]⁺, 600.16876; found, 600.16612.

### [Synthesis Example 13]

### Synthesis of PTX-βGlcNAc

PTX-βGlcNAc which is a compound of the present invention was synthesized according to the following scheme.

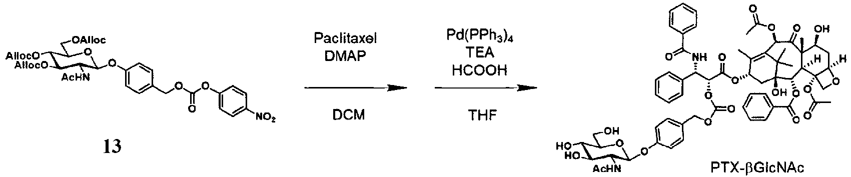

Compound 13 (67.2 mg, 0.0900 mmol), paclitaxel (100 mg, 0.117 mmol), and 4-dimethylaminopyridine (DMAP) (14.3 mg, 0.117 mmol) were dissolved in 5.0 mL of dichloromethane, and the mixture was stirred under an argon atmosphere at normal temperature for 15 hours. The solvent was distilled off under reduced pressure and was purified by silica gel column chromatography (gradient of AcOEt:hexane = 0:100 to 100:0) to obtain an Alloc protected form of PTX-βGlcNA. This was dissolved in 8.0 mL of tetrahydrofuran, HCOOH (8.1 µL) and triethylamine (45 µL) were added thereto, and the mixture was stirred at room temperature under an argon atmosphere for 10 minutes. Tetrakis (triphenylphosphine) palladium (0) (Pd (PPh₃)₄) (24.9 mg, 0.0216 mmol) was added while cooling in an ice bath, and the mixture was stirred under an argon atmosphere at normal temperature for 2 hours. The solvent was distilled off under reduced pressure, and purification was performed by silica gel column chromatography (gradient of CH₂Cl₂: MeOH = 100:0 to 85:15) to obtain a white solid PTX-βGlcNA (82.0 mg, 0.249 mmol) at a yield of 75.3%. The obtained target product was identified by ¹HNMR, ¹³CNMR, and ESI-HRMS.

¹HNMR (400 MHz, MeOD): δ8.14 (d, J = 8.4 Hz, 2H), 7.79 (d, J = 8.4 Hz, 2H), 7.68 (t, J = 7.6 Hz, 2H), 7.61-7.57 (m, 3H), 7.55-7.50 (m, 3H), 7.47-7.41 (m, 4H), 7.32-7.26 (m, 3H), 7.02 (d, J = 8.4 Hz, 2H), 6.48 (s, 1H), 6.10 (t, J = 8.8 Hz, 1H), 5.86 (d, J = 6.4 Hz, 1H), 5.66 (d, J = 7.2 Hz, 1H), 5.50 (d, J = 6.4 Hz, 1H), 5.16 (d, J = 4.0 Hz, 2H), 5.01 (d, J = 9.2 Hz, 2H), 4.39-4.36 (m, 1H), 4.20 (s, 2H), 3.97-3.92 (m, 2H), 3.84 (d, J = 6.8 Hz, 1H), 3.75 (dd, J = 4.8 Hz, J = 12 Hz, 1H), 3.61 (t, J = 7.6 Hz, 1H), 3.47-3.46 (m, 2H), 3.37-3.33 (m, 1H), 2.43 (s, 3H), 2.19 (s, 3H), 2.00 (s, 3H), 1.95 (s, 2H), 1.89-1.80 (m, 3H), 1.68 (s, 3H), 1.17 (s, 3H), 1.15 (s, 3H).¹³CNMR (101 MHz, MeOD): δ205.2, 174.0, 171.7, 171.4, 170.5, 170.3, 167.7, 159.4, 155.8, 142.3, 138.2, 135.4, 135.0, 134.7, 133.0, 131.4, 131.2, 130.5, 130.2, 129.8, 129.6, 128.6, 117.8, 100.7, 85.9, 82.3, 79.1, 78.6, 78.3, 77.5, 76.8, 76.3, 75.8, 73.2, 72.4, 71.9, 71.3, 62.6, 59.3, 57.4, 55.4, 47.9, 44.6, 37.5, 36.5, 27.0, 23.3, 23.1, 22.5, 20.9, 15.0, 10.6. ESI-HRMS (ESI +) m/z calcd. for [M+Na]⁺, 1229.43124; found, 1229.43200.

### [Synthesis Example 14]

### Synthesis of DTX-βGlcNAc

DTX-βGlcNAc which is a compound of the present invention was synthesized according to the following scheme.

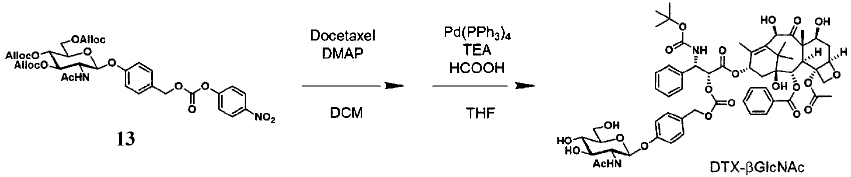

Compound 13 (10.0 mg, 0.0134 mmol), docetaxel (31.0 mg, 0.0348 mmol), and 4-dimethylaminopyridine (DMAP) (15.6 mg, 0.128 mmol) were dissolved in 5.0 mL of dichloromethane, and the mixture was stirred under an argon atmosphere at normal temperature for 15 hours. The solvent was distilled off under reduced pressure and was purified by silica gel column chromatography (gradient of AcOEt:hexane = 0:100 to 90:10) to obtain an Alloc protected form of DTX-βGlcNA. This was dissolved in 5.0 mL of tetrahydrofuran, HCOOH (2.0 mL) and triethylamine (20 µL) were added thereto, and the mixture was stirred at room temperature under an argon atmosphere for 10 minutes. Tetrakis (triphenylphosphine) palladium (0) (Pd (PPh₃) ₄) (5.00 mg, 0.00448 mmol) was added while cooling in an ice bath, and the mixture was stirred under an argon atmosphere at normal temperature for 2 hours. The solvent was distilled off under reduced pressure, and purification was performed by silica gel column chromatography (gradient of CH₂Cl₂: MeOH = 100:0 to 85:15) to obtain a white solid DTX-βGlcNA (14.1 mg, 0.0122 mmol) at a yield of 89.8%. The obtained target product was identified by ¹HNMR, ¹³CNMR, and ESI-HRMS.

¹HNMR (400 MHz, MeOD): δ8.11 (d, J = 7.2 Hz, 2H), 7.68 (d, J = 7.2 Hz, 2H), 7.59-7.55 (m, 3H), 7.40-7.39 (m, 4H), 7.35 (d, J = 8.4 Hz, 2H), 7.02 (d, J = 8.4 Hz, 2H), 6.16-6.09 (m, 1H), 5.63 (d, J = 7.2 Hz, 1H), 5.35-5.27 (m, 2H), 5.14-4.99 (m, overlaps with HOD), 4.30-4.19 (m, 3H), 3.93-3.85 (m, 3H), 3.72 (dd, J = 5.2 Hz, J = 12 Hz, 2H), 3.70-3.50 (m, 2H), 3.45-3.42 (m, 2H), 2.50-2.40 (m, 1H), 2.39 (s, 2H), 2.24-2.19 (m, 1H), 1.98 (s, 3H), 1.87 (s, 3H), 1.69 (s, 3H), 1.39 (s, 9H), 1.20-1.11 (m, 6H).¹³CNMR (101 MHz, MeOD): δ211.1, 174.0, 171.6, 170.3, 167.7, 159.4, 155.8, 139.5, 139.5, 137.9, 134.6, 133.1, 133.0, 131.5, 131.2, 130.6, 130.1, 129.9, 129.7, 129.4, 128.3, 117.7, 100.8, 86.0, 82.3, 80.9, 79.2, 78.3, 77.6, 76.5, 75.8, 75.6, 73.5, 72.6, 71.9, 71.1, 62.6, 58.9, 57.4, 56.4, 47.9, 44.5, 37.5, 36.6, 28.7, 27.0, 23.3, 23.0, 21.7, 14.6, 10.5. ESI-HRMS (ESI +) m/z calcd. for [M+Na]⁺, 1183.44689; found,. 1183.44764.

### [Synthesis Example 15]

### Synthesis of CBZ-βGlcNAc

CB2-βGlcNAc which is a compound of the present invention was synthesized according to the following scheme.

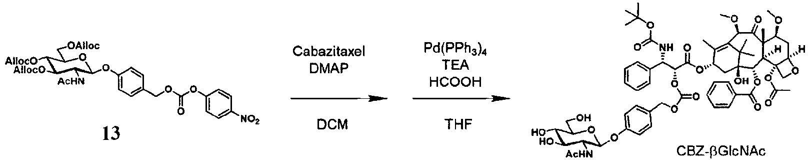

Compound 13 (12.0 mg, 0.0161 mmol), cabazitaxel (40 mg, 0.0479 mmol), and 4-dimethylaminopyridine (DMAP) (19.6 mg, 0.160 mmol) were dissolved in 5.0 mL of dichloromethane, and the mixture was stirred under an argon atmosphere at normal temperature for 15 hours. The solvent was distilled off under reduced pressure and was purified by silica gel column chromatography (gradient of AcOEt:hexane = 0:100 to 70:30) to obtain an Alloc protected form of CBZ-βGlcNA. This was dissolved in 5.0 mL of tetrahydrofuran, HCOOH (2.0 µL) and triethylamine (20 µL) were added thereto, and the mixture was stirred at room temperature under an argon atmosphere for 10 minutes. Tetrakis (triphenylphosphine) palladium (0) (Pd (PPh₃)₄) (5.0 mg, 0.00448 mmol) was added while cooling in an ice bath, and the mixture was stirred under an argon atmosphere at normal temperature for 2 hours. The solvent was distilled off under reduced pressure, and purification was performed by silica gel column chromatography (gradient of CH₂Cl₂: MeOH = 100:0 to 85:15) to obtain a white solid CBZ-βGlcNA (17.7 mg, 0.0149 mmol) at a yield of 92.4%. The obtained target product was identified by ¹HNMR, ¹³CNMR, and ESI-HRMS.

¹HNMR (400 MHz, MeOD): δ8.11 (d, J = 7.6 Hz, 2H), 7.67-7.65 (m, 1H), 7.56 (t, J = 7.6 Hz, 3H), 7.50-7.41 (m, 4H), 7.03 (d, J = 8.4 Hz, 2H), 6.13-6.11 (m, 1H), 5.60 (d, J = 6.8 Hz, 1H), 5.35-5.30 (m, 1H), 5.30-5.23 (m, 3H), 5.13 (s, 2H), 5.08-5.00 (m, overlaps with HOD), 4.18 (q, J = 8.0 Hz, 2H), 3.93-3.88 (m, 3H), 3.81 (d, J = 6.8 Hz, 1H), 3.72 (dd, J = 5.2 Hz, J = 12 Hz, 2H), 3.60-3.55 (m, 2H), 3.45-3.40 (m, 5H), 2.78-2.72 (m, 1H), 2.40 (s, 3H), 2.19-2.18 (m, 1H), 1.98-1.96 (m, 6H), 1.67 (s, 3H), 1.40 (s, 9H), 1.15-1.12 (m, 6H).¹³CNMR (101 MHz, MeOD): δ207.6, 173.9, 171.6, 170.3, 167.6, 159.5, 155.8, 141.4, 138.7, 136.1, 134.6, 131.4, 131.2, 130.5, 129.9, 129.7, 129.5, 128.3, 117.7, 100.7, 85.6, 83.8, 82.5, 82.0, 80.9, 79.1, 79.0, 78.3, 77.4, 76.1, 75.8, 73.4, 71.9, 71.1, 62.6, 58.2, 57.5, 57.4, 57.1, 56.3, 50.1, 44.7, 36.4, 33.1, 28.7, 27.2, 23.3, 23.0, 21.9, 15.0, 10.9. ESI-HRMS (ESI +) m/z calcd. for [M+Na]⁺, 1211.47819; found,. 1211.47639.

### · Synthesis of Compound 14

Compound 14 was synthesized according to the following scheme.

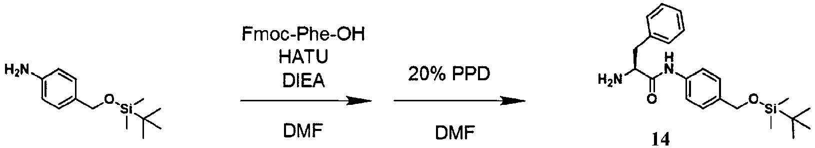

4-(((tert-butyldimethylsilyl)oxy)methyl)aniline (1.00 g, 4.22 mmol), (((9H-fluoren-9-yl)methoxy)carbonyl)-L-phenylalanine (1.63 g, 4.21 mmol), HATU (2.4 g, 6.31 mmol), and N,N-diisopropylethylamine (DIEA) (2.93 mL) were dissolved in 10.0 mL of N,N-dimethylformamide, and the mixture was stirred at room temperature for 3 hours. Dichloromethane was added, and the mixture was washed with purified water to extract the organic phase. The organic phase was washed with saturated saline and then dried over sodium sulfate, and the solvent was distilled off under reduced pressure after filtration. 10.0 mL of N,N-dimethylformamide in which 20% piperidine (PPD) had been dissolved was added, and the mixture was stirred at room temperature for 20 minutes. Dichloromethane was added, and the mixture was washed with purified water to extract the organic phase. The organic phase was washed with saturated saline and then dried over sodium sulfate, and the solvent was distilled off under reduced pressure after filtration. Purification was performed by silica gel column chromatography (gradient of CH₂Cl₂: MeOH = 100:0 to 90: 10) to obtain a compound 9 (500 mg, 1.30 mmol) at a yield of 30.9%. The obtained target product was identified by ESI-HRMS.

ESI-HRMS (ESI +) m/z calcd. for [M+Na]⁺, 407.21307; found, 407.21233.

### · Synthesis of Compound 15

Compound 15 was synthesized according to the following scheme.

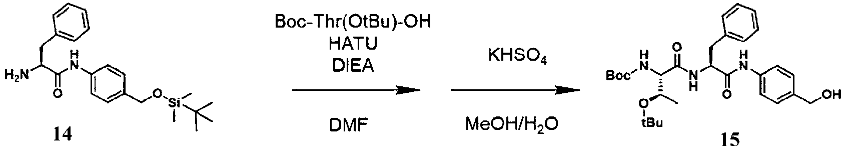

Compound 14 (500 mg, 1.30 mmol), N-(tert-butoxycarbonyl)-O-(tert-butyl)-L-threonine (537 mg, 1.95 mmol), HATU (742 mg, 1.95 mmol), and N,N-diisopropylethylamine (DIEA) (906 µL) were dissolved in 15.0 mL of N,N-dimethylformamide, and the mixture was stirred at normal temperature for 2 hours. Dichloromethane was added, and the mixture was washed with purified water to extract the organic phase. The organic phase was washed with saturated saline and then dried over sodium sulfate, and the solvent was distilled off under reduced pressure after filtration. Purification was performed by silica gel column chromatography (gradient of CH₂Cl₂: MeOH = 100:0 to 95: 5) to obtain an intermediate compound. This was dissolved in a mixed solvent of 8.0 mL of methanol and 2.0 mL of water, KHSO₄ (931 mg, 6.83 mmol) was added thereto, and the mixture was stirred at normal temperature for 30 minutes. Ethyl acetate was added, and the mixture was washed with purified water to extract the organic phase. The organic phase was washed with saturated saline and then dried over sodium sulfate, and the solvent was distilled off under reduced pressure after filtration. Purification was performed by silica gel column chromatography (gradient of AcOEt:hexane = 0:100 to 50:50) to obtain a white solid compound 10 (406 mg, 0.770 mmol) at a yield of 56.2%. The obtained target product was identified by ¹HNMR, ESI-HRMS.

¹HNMR (400 MHz, CD₂Cl₂): δ8.33 (s, 1H), 7.45 (d, J = 8.0 Hz, 2H), 7.26-7.15 (m, 7H), 6.50 (d, J = 9.2 Hz, 1H), 5.52 (d, J = 5.6 Hz, 1H), 4.83-4.75 (m, 1H), 4.53 (s, 2H), 4.05-3.96 (m, 3H), 3.62 (dd, J = 4.8 Hz, J = 13.6 Hz, 1H), 2.95 (dd, J = 5.6 Hz, J = 13.6 Hz, 1H), 1.31 (s, 9H), 1.02-0.99 (m, 12H). ESI-HRMS (ESI +) m/z calcd. for [M+Na]⁺, 550.28876; found, 550.28779.

### · Synthesis of Compound 16

Compound 16 was synthesized according to the following scheme.

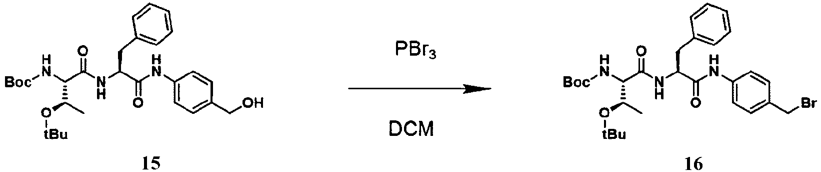

Compound 15 (406 mg, 0.770 mmol) and PBr₃ (72 µL) were dissolved in 10.0 mL of dichloromethane, and the mixture was stirred at normal temperature for 1 hour. A saturated aqueous sodium bicarbonate solution was added to extract an organic acetate phase. The organic phase was washed with purified water and saturated saline and then dried over sodium sulfate, and the solvent was distilled off under reduced pressure after filtration. Purification was performed by silica gel column chromatography (gradient of AcOEt:hexane = 0:100 to 70:30) to obtain a white solid compound 11 (180 mg, 0.307 mmol) at a yield of 39.9%. The obtained target product was identified by ¹HNMR, ESI-HRMS.

¹HNMR (400 MHz, CD₂Cl₂): δ8.54 (s, 1H), 7.43 (d, J = 8.0 Hz, 2H), 7.21-7.10 (m, 7H), 6.70 (d, J = 8.8 Hz, 1H), 5.53 (d, J = 5.6 Hz, 1H), 4.80-4.75 (m, 1H), 4.38 (s, 1H), 4.02-3.97 (m, 2H), 3.27 (dd, J = 5.2 Hz, J = 13.6 Hz, 1H), 2.95 (dd, J = 6.0 Hz, J = 13.6 Hz, 1H), 3.24-3.15 (m, 3H), 3.09-3.04 (m, 1H), 2.10-1.90 (m, 2H), 1.33 (t, J = 7.6 Hz, 3H), 1.18 (d, J = 6.4 Hz, 3H), 1.30 (s, 9H), 1.00 (s, 12H). ESI-HRMS (ESI +) m/z calcd. for [M+Na]⁺, 612.20436; found, 612.20521.

### [Synthesis Example 16]

### Synthesis of TF-SN38

TF-SN38 which is a compound of the present invention, was synthesized according to the following Scheme.

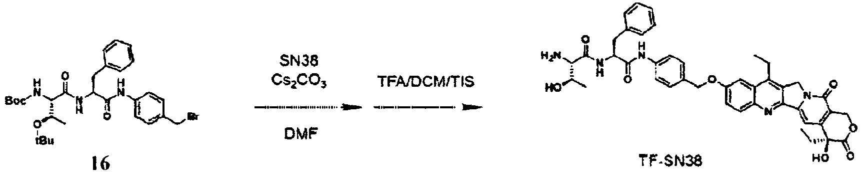

Compound 16 (180 mg, 0.307 mmol), 7-ethyl-10-hydroxycamptothecin (SN38) (359 mg, 0.916 mmol), and Cs₂CO₃ (299 mg, 0.918 mmol) were dissolved in 15.0 mL of N,N-dimethylformamide, and the mixture was stirred at normal temperature for 1 hour. Dichloromethane was added, washed with purified water and saturated saline, and then dried over sodium sulfate, and the solvent was distilled off under reduced pressure after filtration. Purification was performed by silica gel column chromatography (gradient of CH₂Cl₂: MeOH = 100:0 to 95: 5) to obtain an intermediate compound. A solvent obtained by mixing 7.5 mL of trifluoroacetic acid, 3.5 mL of dichloromethane, and triisopropylsilane (TIS) (125 µL) was added, and the mixture was stirred at normal temperature for 5 hours. The solvent was distilled off under reduced pressure, and then purified by silica gel column chromatography (gradient of CH₂Cl₂: MeOH = 100:0 to 75: 25) to obtain a target product for crude purification. This was purified by HPLC to obtain pale yellow solid TF-SN38 (31.0 mg, 0.0416 mmol) at a yield of 13.6%. The obtained target product was identified by ¹HNMR, ¹³CNMR, and ESI-HRMS.

¹HNMR (400 MHz, MeOD): δ8.03 (d, J = 9.2 Hz, 1H), 7.60-7.46 (m, 6H), 7.43 (d, J = 2.8 Hz, 1H), 7.31-7.20 (m, 5H), 5.58 (d, J = 16 Hz, 1H), 5.38 (d, J = 16 Hz, 1H), 5.23 (d, J = 8.4 Hz, 4H), 4.80-4.77 (m, 1H), 3.98-3.94 (m, 1H), 3.27 (d, J = 4.8 Hz, 1H), 3.24-3.15 (m, 3H), 3.09-3.04 (m, 1H), 2.10-1.90 (m, 2H), 1.33 (t, J = 7.6 Hz, 3H), 1.18 (d, J = 6.4 Hz, 3H), 1.03 (t, J = 7.6 Hz, 3H).¹³CNMR (101 MHz, MeOD): δ172.65, 172.54, 169.70, 157.12, 156.80, 150.03, 149.58, 146.24, 144.39, 143.82, 138.43, 137.13, 131.50, 131.41, 129.22, 129.70, 128.35, 128.06, 127.73, 126.40, 122.62, 119.34, 118.21, 103.65, 95.99, 72.37, 69.54, 67.60, 65.20, 60.08, 54.12, 49.50, 37.94, 30.16, 22.20, 19.83, 13.42, 7.73. ESI-HRMS (ESI +) m/z calcd. for [M+Na]⁺, 768.30038; found, 768.30030.

### · Synthesis of Compound 17

Compound 17 was synthesized according to the following scheme.

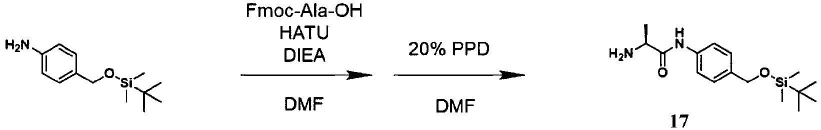

4-(((tert-butyldimethylsilyl)oxy)methyl)aniline (2.00 g, 8.44 mmol), (((9H-fluoren-9-yl)methoxy)carbonyl)-L-phenylalanine (2.63 g, 8.45 mmol), HATU (4.81 g, 12.6 mmol), and N,N-diisopropylethylamine (DIEA) (5.87 mL) were dissolved in 10.0 mL of N,N-dimethylformamide, and the mixture was stirred at room temperature for 3 hours. Ethyl acetate was added, and the mixture was washed with purified water to extract the organic phase. The organic phase was washed with saturated saline and then dried over sodium sulfate, and the solvent was distilled off under reduced pressure after filtration. 20.0 mL of N,N-dimethylformamide in which 20% piperidine (PPD) had been dissolved was added, and the mixture was stirred at room temperature for 20 minutes. Ethyl acetate was added, and the mixture was washed with purified water to extract the organic phase. The organic phase was washed with saturated saline and then dried over sodium sulfate, and the solvent was distilled off under reduced pressure after filtration. Purification was performed by silica gel column chromatography (gradient of CH₂Cl₂: MeOH = 100:0 to 90: 10) to obtain a compound 12 (2.16 g, 5.56 mmol) at a yield of 65.9%. The obtained target product was identified by ESI-HRMS.

ESI-HRMS (ESI +) m/z calcd. for [M+Na]+, 331.18123; found, 331.18179.

### · Synthesis of Compound 18

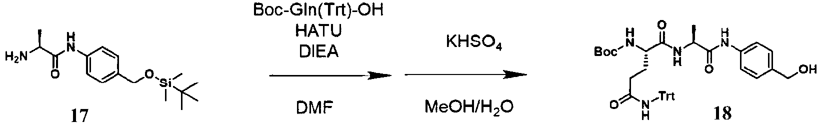

Compound 17 (2.00 **g,** 6.49 mmol), N²-(tert-butoxycarbonyl)-N⁵-trityl-L-glutamine (4.00 **g,** 8.19 mmol), HATU (3.70 mg, 9.74 mmol), and N,N-diisopropylethylamine (DIEA) (4.52 mL) were dissolved in 15.0 mL of N,N-dimethylformamide, and the mixture was stirred at normal temperature for 2.5 hours. Ethyl acetate was added, and the mixture was washed with purified water to extract the organic phase. The organic phase was washed with saturated saline and then dried over sodium sulfate, and the solvent was distilled off under reduced pressure after filtration. Purification was performed by silica gel column chromatography (gradient of AcOEt:hexane = 0:100 to 100:0) to obtain an intermediate compound. This was dissolved in a mixed solvent of 20.0 mL of methanol and 5.0 mL of water, KHSO₄ (4.41 g, 32.4 mmol) was added thereto, and the mixture was stirred at normal temperature for 1 hour. The mixture was distilled off under reduced pressure to remove half of methanol, then dichloromethane was added, and the mixture was washed with purified water to extract an organic phase. The organic phase was washed with saturated saline and then dried over sodium sulfate, and the solvent was distilled off under reduced pressure after filtration. Purification was performed by silica gel column chromatography (gradient of CH₂Cl₂: MeOH = 100:0 to 95:5) to obtain a compound 18 (1.36 g, 2.05 mmol) at a yield of 31.5%. The obtained target product was identified by ESI-HRMS.

ESI-HRMS (ESI +) m/z calcd. for [M+Na]+, 687.31531; found, 687.31599.

### · Synthesis of Compound 19

Compound 19 was synthesized according to the following scheme.

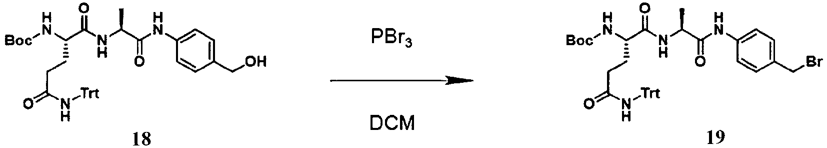

Compound 18 (340 mg, 0.512 mmol) and PBr₃ (48 µL) were dissolved in 10.0 mL of dichloromethane, and the mixture was stirred at normal temperature for 1 hour. A saturated aqueous sodium bicarbonate solution was added to extract an organic acetate phase. The organic phase was washed with purified water, saturated saline, and then dried over sodium sulfate, and the solvent was distilled off under reduced pressure after filtration to obtain a white solid compound 19 (276 mg, 0.380 mmol) at a yield of 86.1%.

### [Synthesis Example 17]

### Synthesis of QA-SN38

QA-SN38 which is a compound of the present invention, was synthesized according to the following Scheme.

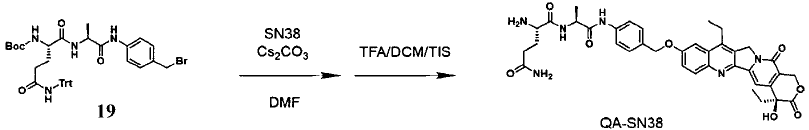

Compound 19 (200 mg, 0.319 mmol), 7-ethyl-10-hydroxycamptothecin (SN38) (100 mg, 0.255 mmol), and Cs₂CO₃ (312 mg, 0.958 mmol) were dissolved in 15.0 mL of N,N-dimethylformamide, and the mixture was stirred at normal temperature for 2 hours. Dichloromethane was added, washed with purified water and saturated saline, and then dried over sodium sulfate, and the solvent was distilled off under reduced pressure after filtration. Purification was performed by silica gel column chromatography (gradient of CH₂Cl₂: MeOH = 100:0 to 80: 20) to obtain an intermediate compound. A solvent obtained by mixing 1.5 mL of trifluoroacetic acid, 1.0 mL of dichloromethane, and triisopropylsilane (TIS) (72 µL) was added, and the mixture was stirred at normal temperature for 5 hours. The solvent was distilled off under reduced pressure, and purification was performed by HPLC to obtain pale yellow solid QA-SN38 (5.2 mg, 0.00747 mmol) at a yield of 2.3%. The obtained target product was identified by ¹HNMR, ¹³CNMR, and ESI-HRMS.

¹HNMR (400 MHz, MeOD): δ8.04 (d, J = 9.2 Hz, 1H), 7.64-7.60 (m, 3H), 7.54-7.50 (m, 3H), 7.46 (d, J = 9.2 Hz, 1H), 5.58 (d, J = 14 Hz, 1H), 5.38 (d, J = 16.4 Hz, 1H), 5.25 (d, J = 10.8 Hz, 1H), 4.57-4.52 (m, 1H), 4.00 (t, J = 6.4 Hz, 1H), 3.21-3.17 (m, 2H), 2.54 (t, J = 7.2 Hz, 2H), 2.19-2.14 (m, 2H), 2.00-1.94 (m, 2H), 1.51 (d, J = 7.2 Hz, 3H), 1.35 (t, J = 7.6 Hz, 3H), 1.03 (t, J = 7.6 Hz, 3H).¹³CNMR (101 MHz, MeOD): δ177.3, 175.0, 173.0, 169.6, 159.2, 152.8, 150.6, 148.0, 146.1, 146.0, 139.5, 134.0, 132.1, 129.5, 129.4, 129.3, 124.5, 121.3, 119.8, 104.6, 99.1, 74.3, 71.1, 66.7, 53.7, 51.3, 50.8, 32.0, 31.4, 28.2, 23.9, 18.2, 13.9, 8.2. ESI-HRMS (ESI +) m/z calcd. for [M+Na]⁺, 719.27998; found, 719.27903.

### [Example 21]

### Cytotoxicity test results using JFCR39 cell panel

With respect to total of 39 cell lines of JFCR39 cell panel (lung cancer type 7 (including adenocarcinoma, squamous cell carcinoma, small cell carcinoma, large cell carcinoma), gastric cancer type 6, large bowel cancer type 5, ovarian cancer type 5, brain tumor type 6, breast cancer type 5, renal cancer type 2, prostate cancer type 2, and melanoma type 1), effects of suppression of cell proliferation on existing drugs SN38, CPT-11, nogitecan, exatecan, etoposide, doxolibicin, epirubicin, melphalan, MMAE, MMAF, paclitaxel, docetaxel, cabazitaxel, and SN38-βGal, SN38-βGlcNAc, SN38-αMan, KK-SN38, QA-SN38, TF-SN38, NGT-βGlcNAc, EXT-βGlcNAc, ETP-βGlcNAc, DOX-βGlcNAc, EPI-βGlcNAc, Melphalan (Mel)-βGlcNAc, MMAE-βGlcNAc, MMAF-βGlcNAc, PTX-βGlcNAc, DTX-βGlcNAc, and CBZ-βGlcNAc, which are novel prodrugs of the present invention, were evaluated.

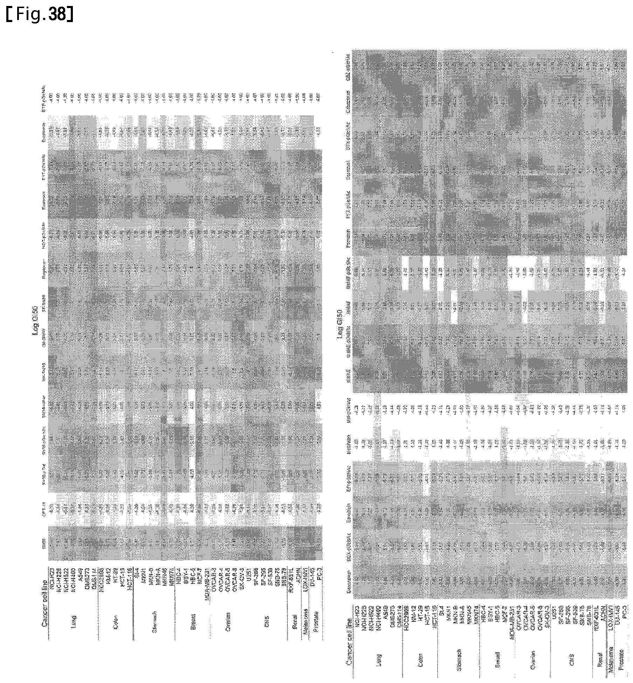

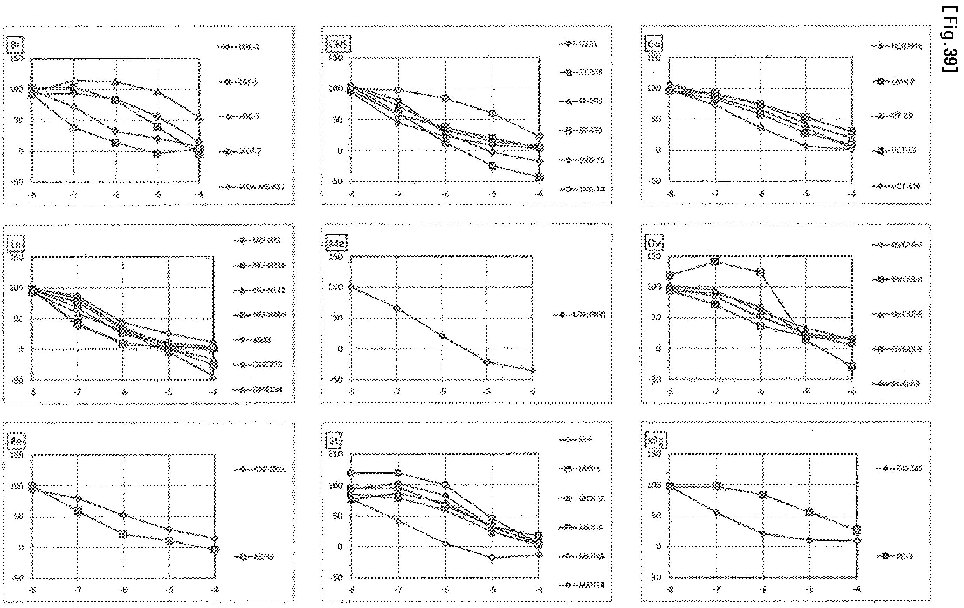

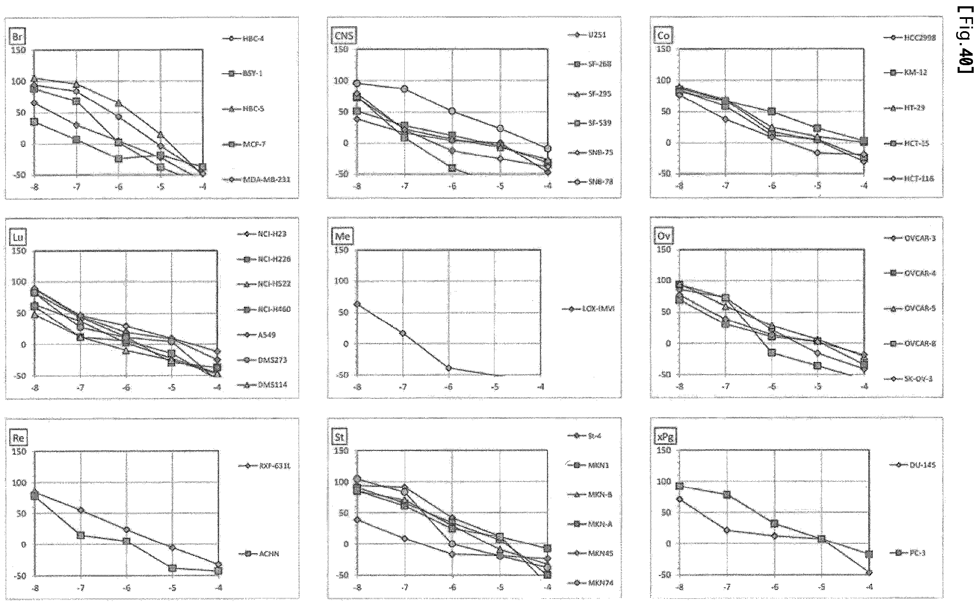

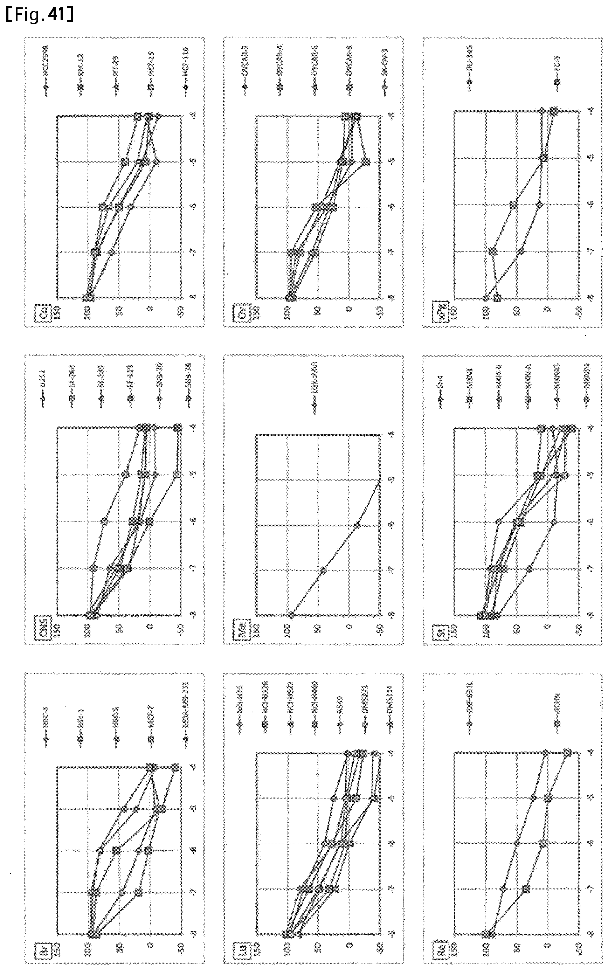

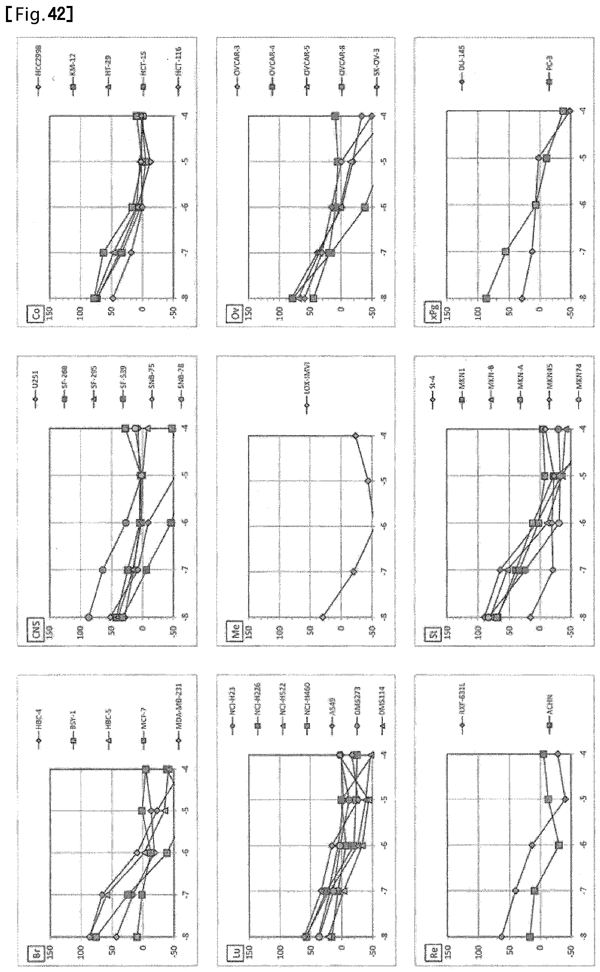

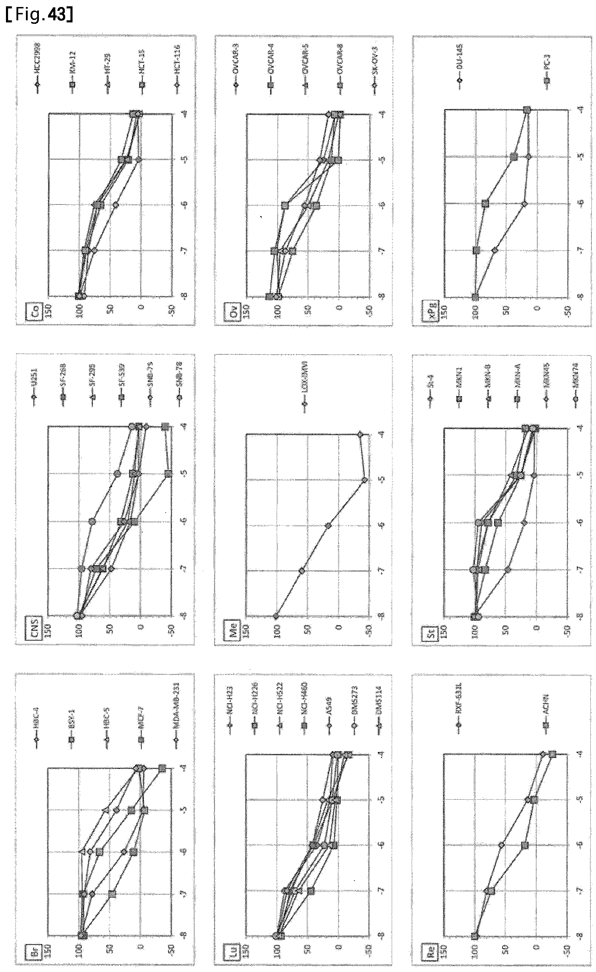

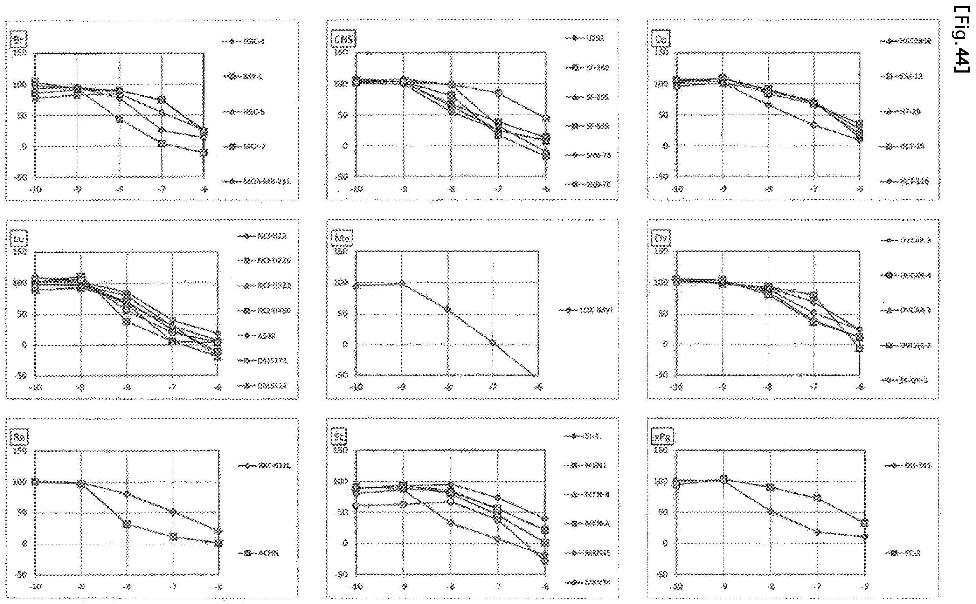

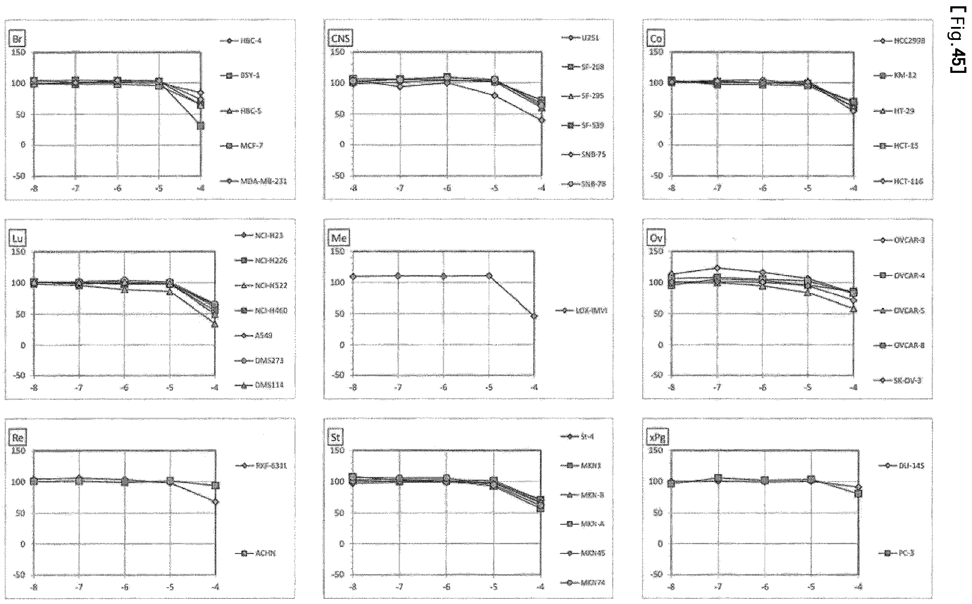

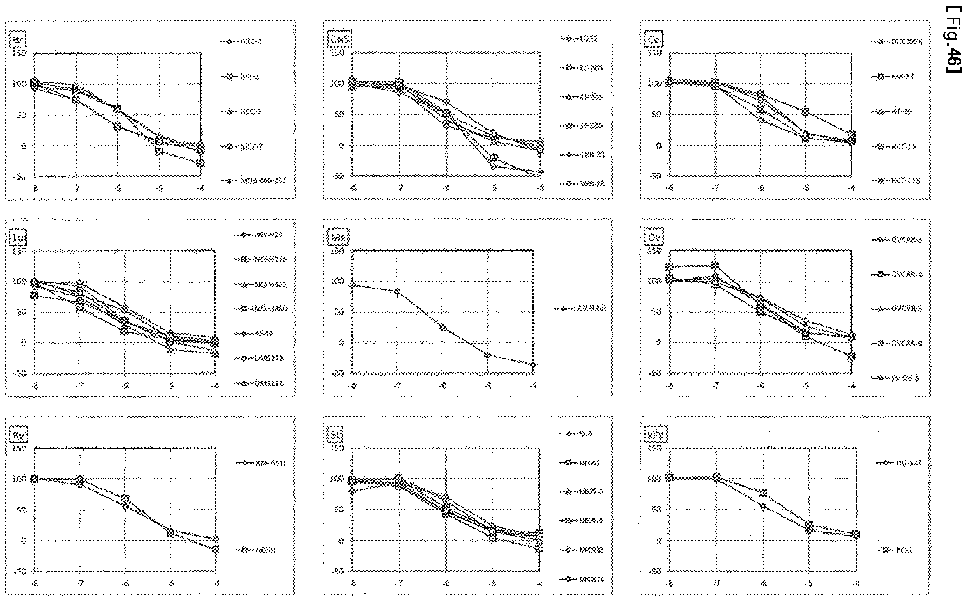

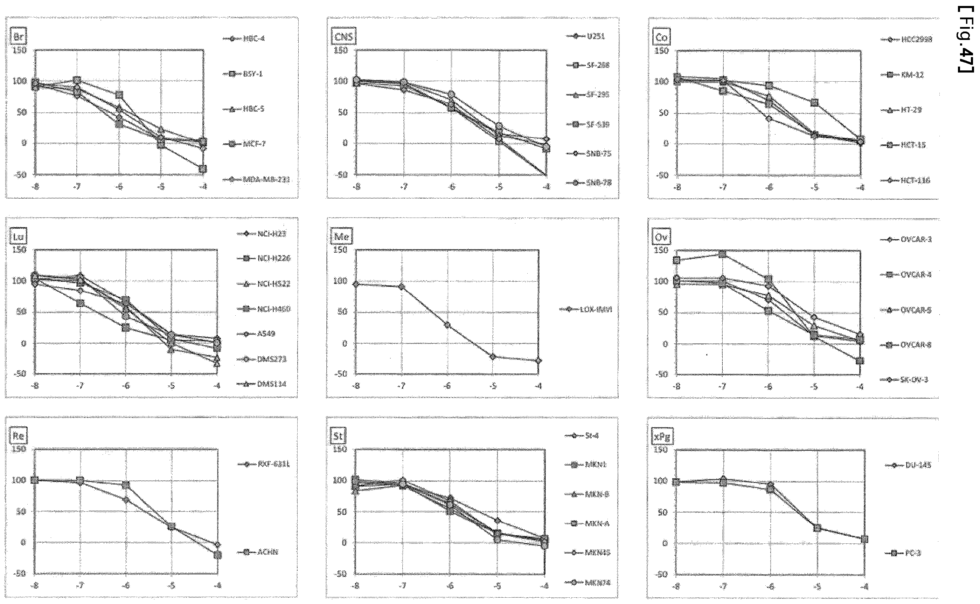

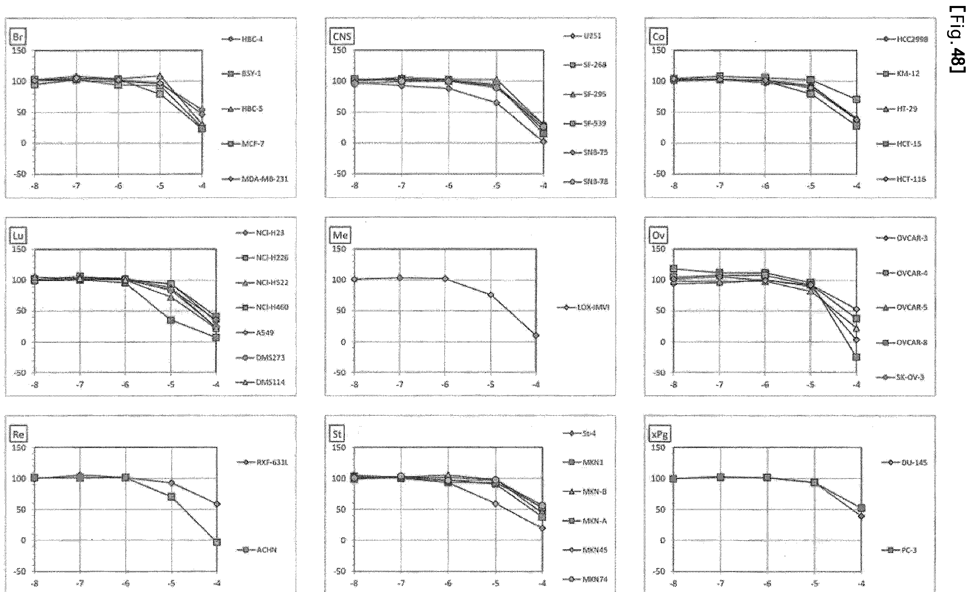

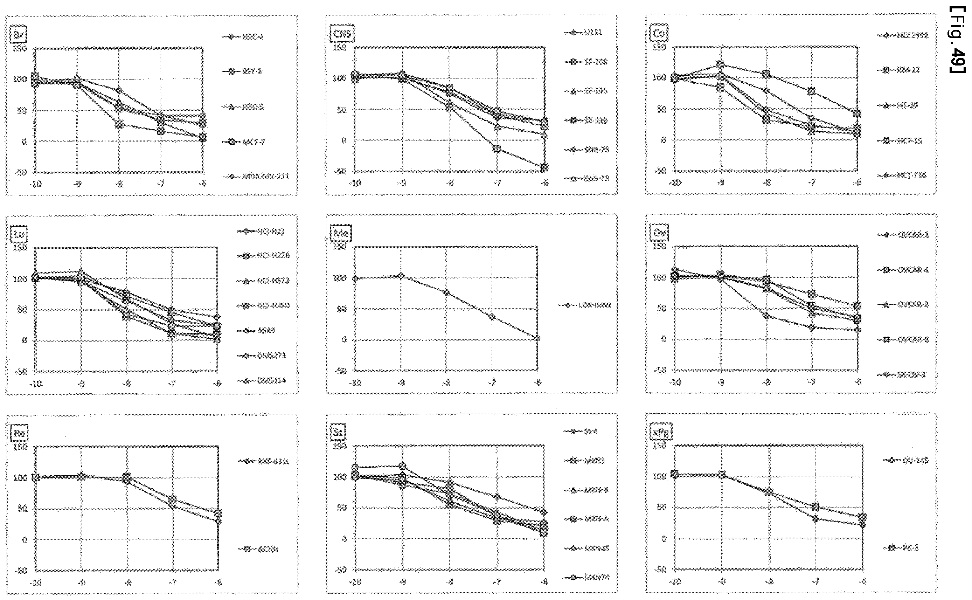

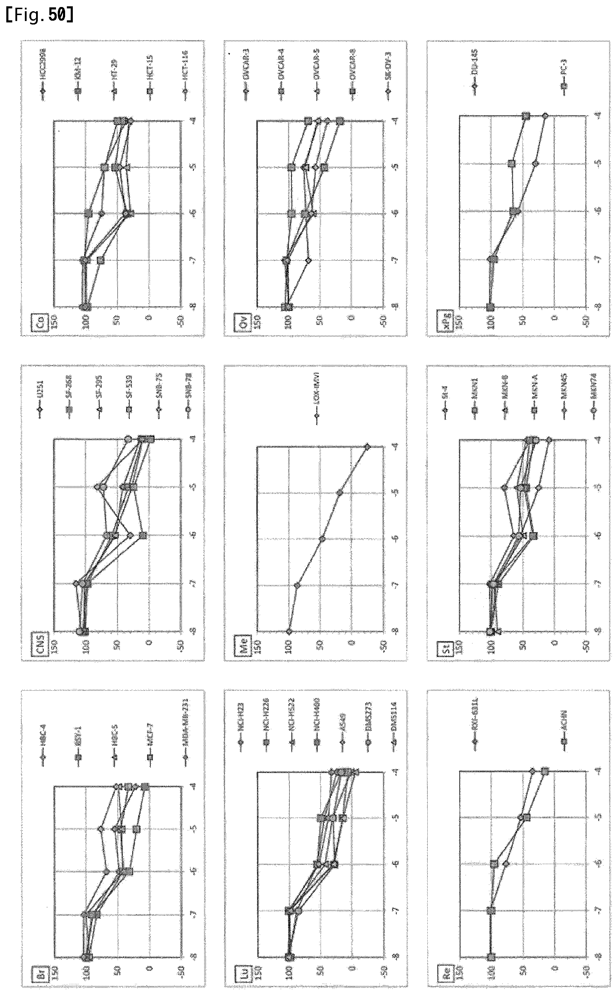

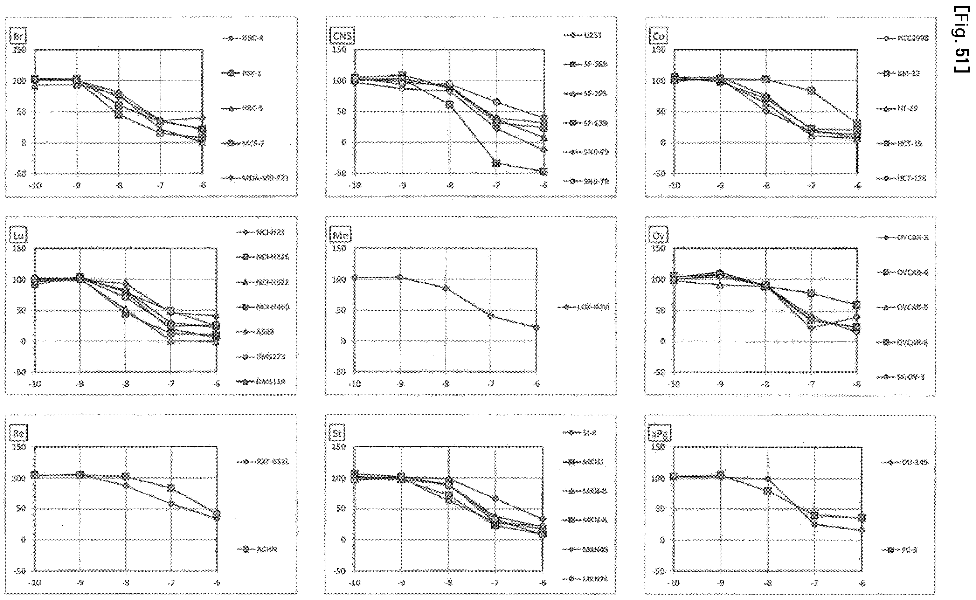

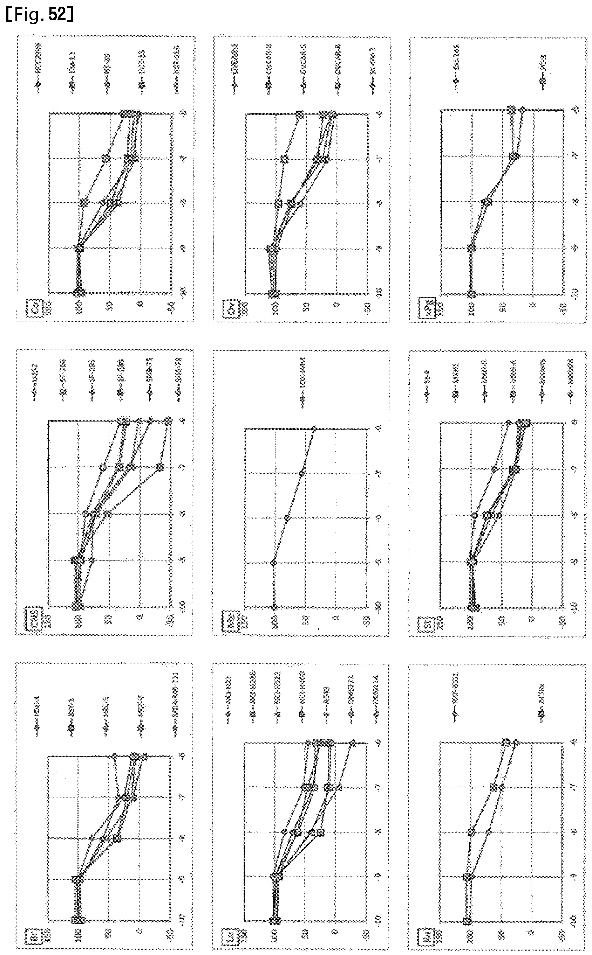

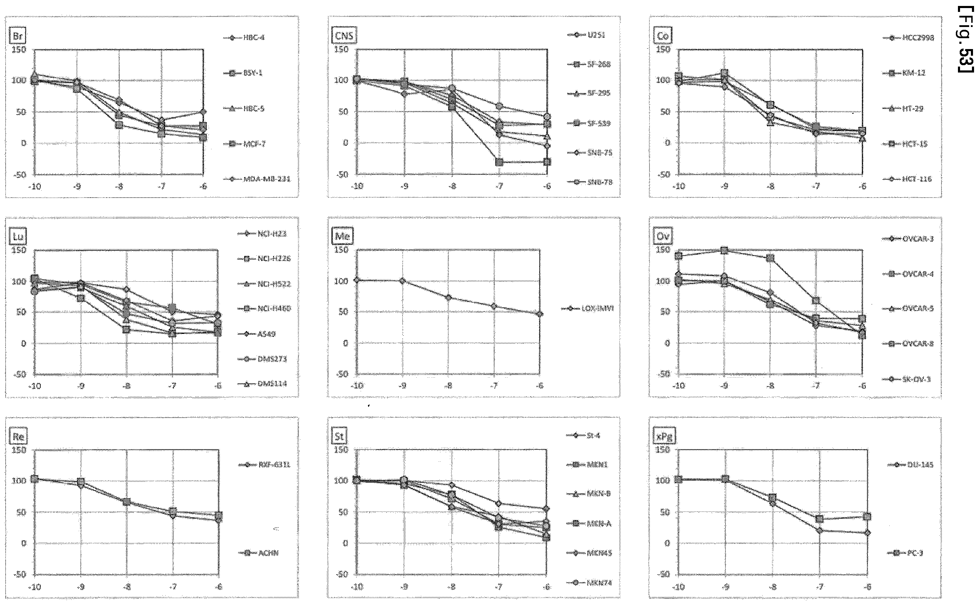

As shown in the table of Fig. 38, it was shown that these prodrugs can suppress proliferation of each cancer cell (Figs. 39 to 53). In the lung cancer, the drug efficacy was confirmed in all of adenocarcinoma, squamous cell carcinoma, small cell carcinoma, and large cell carcinoma.

A specific test procedure is shown below.

Each cancer cell was seeded in a 96 well plate, and on the next day, the drug solution was added according to the dilution series, and after culturing for 2 days (48 hours), cell proliferation was measured by colorimetric quantification with sulforhodamine B.

The table in Fig. 38 illustrates GI₅₀ values in each cancer cell. Here, -4.00 indicates that the GI₅₀ value is 10⁻⁴ or more.

Figs. 39 to 53 illustrate suppression of cell proliferation of each cancer cell in SN38-αMan, KK-SN38, QA-SN38, TF-SN38, NGT-βGlcNAc, EXT-βGlcNAc, ETP-βGlcNAc, DOX-βGlcNAc, EPI-βGlcNAc, Melphalan-βGlcNAc, MMAE-βGlcNAc, MMAF-βGlcNAc, PTX-βGlcNAc, DTX-βGlcNAc, and CBZ-βGlcNAc, respectively.

### [Example 22]

### Therapeutic experiment of each prodrug in peritoneal dissemination model

Using the developed prodrug-type anticancer agents (DOX-βGlcNAc, Mel-βGlcNAc, MMAE-βGlcNAc, PTX-βGlcNAc, ETP-βGlcNAc, and SN38-βGlcNAc), the therapeutic effect was verified using a peritoneal dissemination model. Whether the tumor suppression effect was confirmed was evaluated as compared with the Vehicle group (DMSO administration). n = 5 for each group, and evaluation was performed on luciferase-expressing A549 cell peritoneal dissemination model mice using BALB/cAJcl-nu/nu. The administration form was intraperitoneal administration (i.p.), and administration was performed according to the administration schedule shown in each drawing of Fig. 54-a to Fig. 54-f. The tumor burden was evaluated by the luciferin/luciferase luminescence.

As a result, the tumor proliferation suppression effect was confirmed in each administration condition in the prodrug administration group as compared with the Vehicle group. From the above results, the therapeutic effect of the developed prodrug in the cancer model mice was confirmed.

A specific procedure of the therapeutic experiment is shown below.
(1) Luciferase expressing A549 cells 1 x 10⁶ cells (200 µL) was intraperitoneally administered to BALB/cAJcl-nu/nu (7 weeks old).
(2) After 1 week, formation of peritoneal dissemination was confirmed by luciferin/luciferase chemiluminescence, and drug administration was started. 3 mg (200 µL) of luciferin was administered by intraperitoneal administration, and anesthetized with isoflurane. Thereafter, the luminescence value at 15 minutes after administration was measured by an in vivo imaging device. The drug was prepared by dissolving a 100 mM of DMSO stock in PBS, and administered in a total amount of 100 µL.

## Claims

1. A compound represented by the following General Formula (I) or a pharmaceutically acceptable salt thereof:
Y-T-(X₁)ₙ (I)
(wherein,
Y is selected from
-OL₁ (1)
or
-NH-L₂ (2),
L₁ is a partial structure of saccharides,
the partial structure of the saccharides is a structure obtained by removing one hydroxyl group from the saccharides, and
L₂ is a monovalent substituent containing an amino acid residue or an oligopeptide residue;
T is a linker or a bond;
X₁ is at least one selected from the group consisting of
(1) a binding residue of bioactive compound having one or more functional groups selected from the group consisting of an aliphatic hydroxyl group, an aromatic hydroxyl group, an amino group, and a carboxy group,
(2) an antibody, and
(3) a ligand selected from a small molecule, a peptide, an aptamer, or a hormone-drug conjugate,
here, at least one of X₁ is a binding residue of the bioactive compound, and
the bioactive compound is an anticancer agent; and
n is an integer of 1 or more.)

2. The compound or a pharmaceutically acceptable salt thereof according to claim 1, wherein Y is selected from any of the following groups.

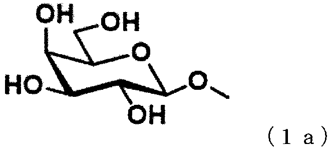

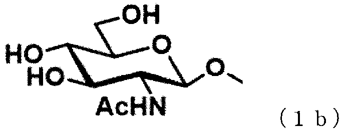

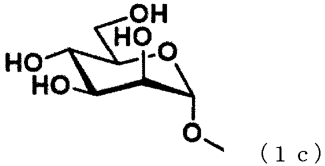

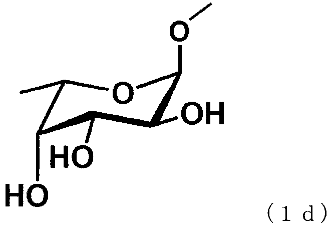

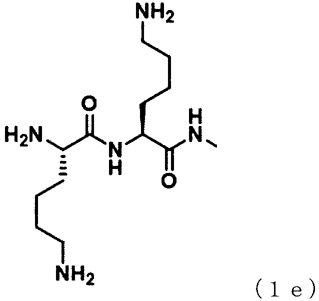

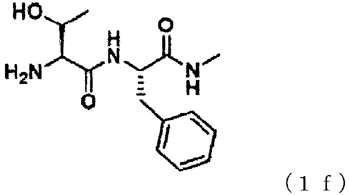

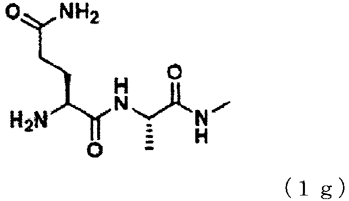

3. The compound or a pharmaceutically acceptable salt thereof according to claim 1, wherein the linker is selected from the group consisting of the following structures (a) to (f), and a combination of two or more thereof. 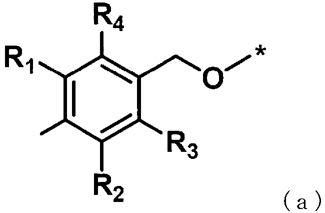 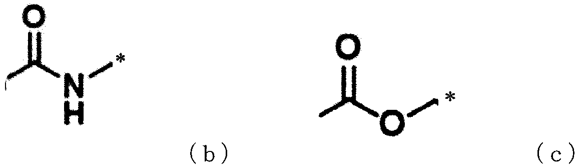 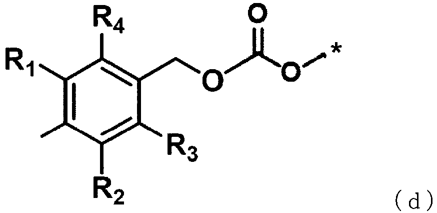 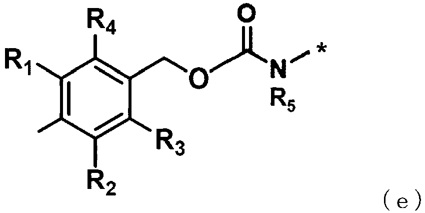 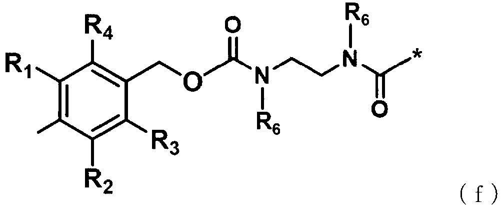
(wherein, R₁ to R₄ may be the same or different and are each independently selected from the group consisting of hydrogen, a methyl group, a methoxy group, -COOMe, - NHMe, F, Cl, Br, and NO₂ (Me represents a methyl group);
R₅ is hydrogen or an alkyl group having 1 to 3 carbon atoms;
R₆, in each occurrence, is independently hydrogen or an alkyl group having 1 to 3 carbon atoms,
(i) at least one of R₁ to R₄ may be a linking group selected from the following: 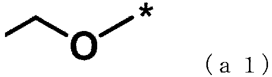 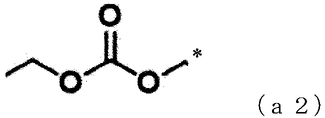 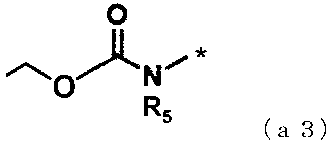 where, when at least one of R₁ to R₄ is a linking group, a linker is also bound to X₁ via the linking group, and
(ii) at least one of R₁ to R₄ may be a linker having a binding site that is usable as a point of attachment to an antibody or ligand;
* represents a direction of binding with X₁,
here, when T has a structure in which T is a combination of two or more of Formulae (a) to (f), * for one or more linkers other than the linker that binds to X₁ may represent the direction of binding to the linker; and
when at least one X₁ is an antibody or a ligand, the linker of Formulae (a) to (f), the linking group of a1 to a3 is capable of binding to a linker having a binding site that is usable as a binding point to the antibody or the ligand in the direction indicated by *.)

4. The compound or the pharmaceutically acceptable salt thereof according to claim 1, wherein the bioactive compound is selected from any one of the following.

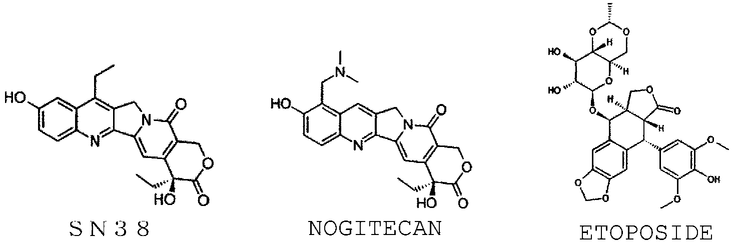

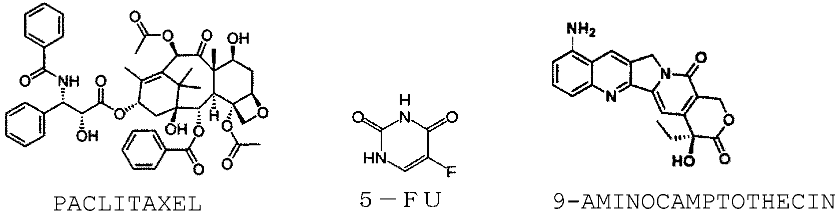

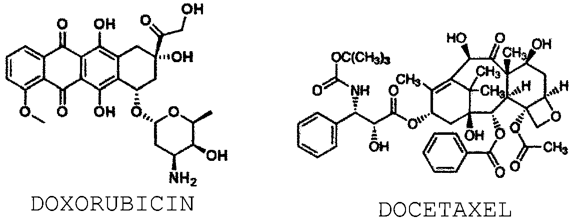

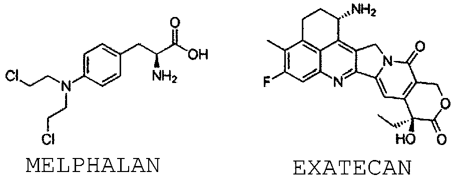

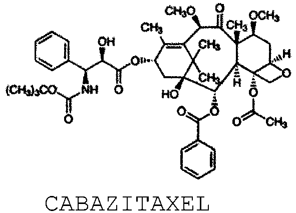

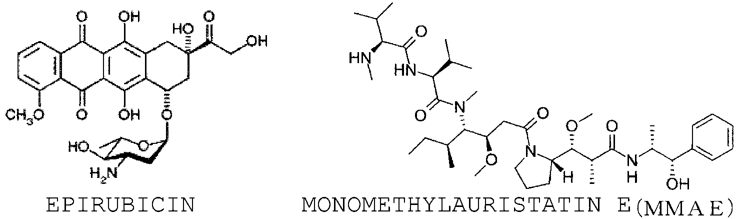

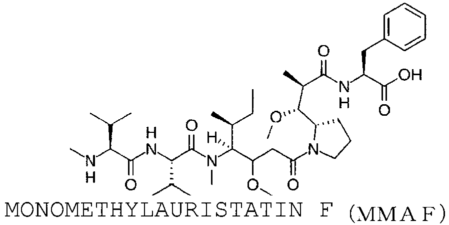

5. The compound or the pharmaceutically acceptable salt thereof according to claim 1, wherein the bioactive compound is SN38, nogitecan, exatecan, etoposide, doxolibicin, epirubicin, melphalan, MMAE, MMAF, paclitaxel, docetaxel, or cabazitaxel.

6. The compound or the pharmaceutically acceptable salt thereof according to claim 2, wherein the bioactive compound is SN38, nogitecan, exatecan, etoposide, doxolibicin, epirubicin, melphalan, MMAE, MMAF, paclitaxel, docetaxel, or cabazitaxel.

7. A prodrug-type anticancer agent containing the compound or the pharmaceutically acceptable salt thereof according to any one of claims 1 to 6.

8. A prodrug-type anticancer agent comprising the compound or the pharmaceutically acceptable salt thereof according to any one of claims 1 to 6, wherein the prodrug-type anticancer agent selectively acts on cells by cancer cell-specific enzyme activity.

9. The prodrug-type anticancer agent according to claim 7 or 8, wherein the enzyme is peptidase or glycosidase.

10. A fluorescent probe for detecting lung cancer, comprising a compound represented by the following General Formula (II) or a salt thereof: 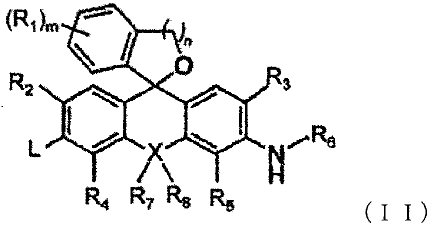 (wherein,
R₁, if present, represents the same or different monovalent substituents present on a benzene ring;
R₂ and R₃ each independently represent a hydrogen atom, an alkyl group having 1 to 6 carbon atoms, or a halogen atom;
R₄ and R₅ each independently represent a hydrogen atom, an alkyl group having 1 to 6 carbon atoms, or a halogen atom;
R₆ represents a hydrogen atom, an alkyl group having 1 to 5 carbon atoms, or a fluoroalkyl group having 1 to 5 carbon atoms;
R₇ and R₈, if present, each independently represent an alkyl group or an aryl group having 1 to 6 carbon atoms,
here, when X is an oxygen atom, R₇ and R₈ are not present;
X represents an oxygen atom, a silicon atom, or a carbon atom;
n is an integer of 1 to 3; and
L is a group of the following Formula (1b).)

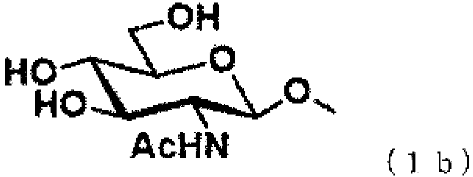

11. The fluorescent probe according to claim 10, wherein X is an oxygen atom.

12. The fluorescent probe according to claim 10 or 11, wherein R₆ is a fluoroalkyl group having 1 to 5 carbon atoms.

13. A companion diagnostic agent for diagnosing a need for cancer treatment with a prodrug-type anticancer agent, comprising a compound represented by the following General Formula (I) or a pharmaceutically acceptable salt thereof, said companion diagnostic agent comprising: a compound represented by the following General Formula (II) or a salt thereof.
Y-T-(X₁)ₙ (I)
(wherein,
Y is selected from any of the following groups; 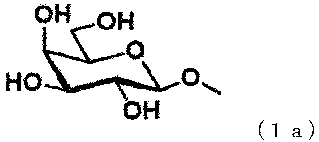 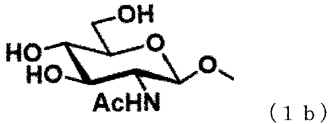 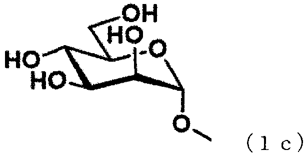 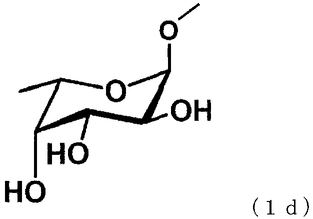 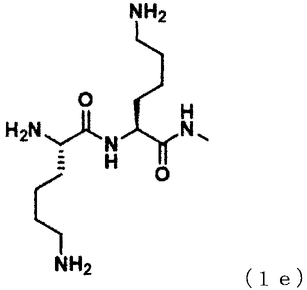 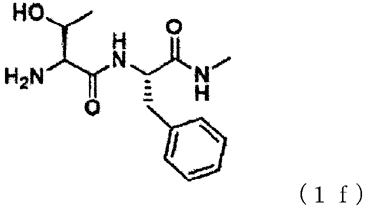 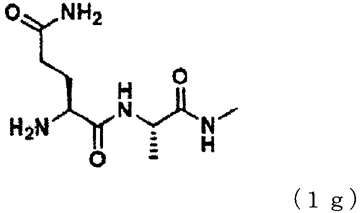
T is a linker or a bond;
X₁ is at least one selected from the group consisting of
(1) a binding residue of bioactive compound having one or more functional groups selected from the group consisting of an aliphatic hydroxyl group, an aromatic hydroxyl group, an amino group, and a carboxy group,
(2) an antibody, and
(3) a ligand selected from a small molecule, a peptide, an aptamer, or a hormone-drug conjugate,
here, at least one of X₁ is a binding residue of the bioactive compound, and
the bioactive compound is an anticancer agent; and
n is an integer of 1 or more.) 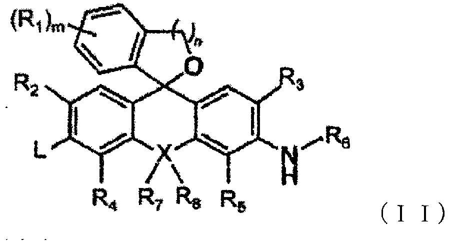
(wherein,
R₁, if present, represents the same or different monovalent substituents present on a benzene ring;
R₂ and R₃ each independently represent a hydrogen atom, an alkyl group having 1 to 6 carbon atoms, or a halogen atom;
R₄ and R₅ each independently represent a hydrogen atom, an alkyl group having 1 to 6 carbon atoms, or a halogen atom;
R₆ represents a hydrogen atom, an alkyl group having 1 to 5 carbon atoms, or a fluoroalkyl group having 1 to 5 carbon atoms;
R₇ and R₈, if present, each independently represent an alkyl group or an aryl group having 1 to 6 carbon atoms,
here, when X is an oxygen atom, R₇ and R₈ are not present;
X represents an oxygen atom, a silicon atom, or a carbon atom;
n is an integer of 1 to 3; and
L is selected from any of the following groups.)

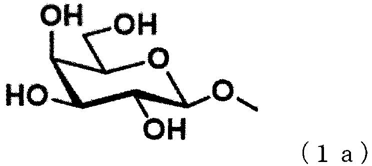

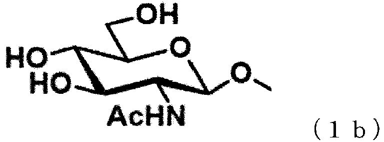

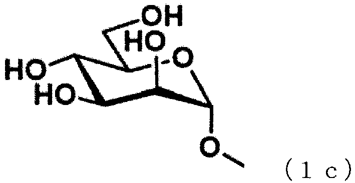

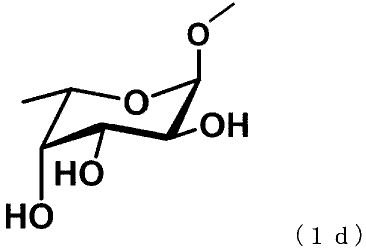

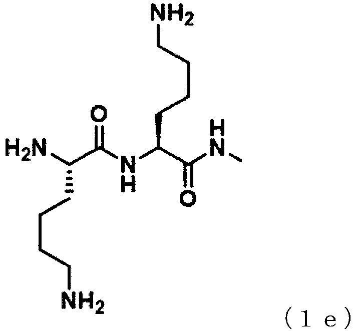

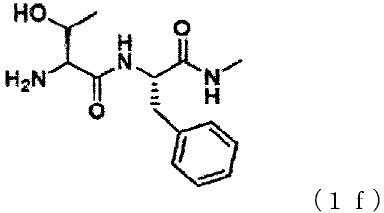

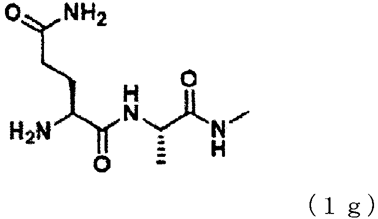

14. A pharmaceutical composition for treating or preventing cancer, comprising: the compound or the pharmaceutically acceptable salt thereof according to any one of claims 1 to 6.

15. A pharmaceutical composition for treating or preventing cancer, further comprising: another anticancer agent, in addition to the compound represented by General Formula (I) or the pharmaceutically acceptable salt thereof, a hydrate, or a solvate.

16. The pharmaceutical composition according to claim 15, wherein the another anticancer agent is selected from methotrexate, doxorubicin, cisplatin, or an anti-PD-1 antibody.

17. A method for treating a patient with cancer, the method comprising:
a step of administering a therapeutically effective amount of the prodrug-type anticancer agent according to any one of claims 7 to 9 to the patient.

18. The treatment method according to claim 17, wherein the prodrug-type anticancer agent acts cell selectively with an enzyme activity specific to cancer cells, and the cancer is **characterized by** a higher expression level of the enzyme.

19. The treatment method according to claim 17, wherein the prodrug-type anticancer agent acts cell selectively with an enzyme activity specific to cancer tissue, and the cancer is **characterized by** a higher expression level of the enzyme.

20. The treatment method according to claim 18, wherein the prodrug-type anticancer agent releases a molecule of the anticancer agent by cleaving an enzyme recognition site and/or a linker site due to an enzyme activity specific to cancer cells.

21. The treatment method according to claim 19, wherein the prodrug-type anticancer agent releases a molecule of the anticancer agent by cleaving an enzyme recognition site and/or a linker site due to an enzyme activity specific to cancer tissue.

22. The treatment method according to claim 20, further comprising: identifying the level of enzyme activity of the enzyme in a patient's biological sample using a visualization agent, or one or more enzyme diagnostic agents selected from an enzyme activity reporter, and defining groups that may respond to treatment as a function of the enzyme activity.

23. The treatment method of claim 20 or 22, further comprising: stratifying a set of patients and defining a subset of patients that may respond to the prodrug-type anticancer agent by using the enzyme diagnostic agent.

24. Use of an enzyme recognition site-linker binding site represented by General Formula (III),
wherein the enzyme recognition site has an enzyme activity specific to a cancer cell, and
the enzyme recognition site-linker binding site is used for activating an antibody or a ligand (a small molecule, a peptide, an aptamer, a hormone-drug conjugate, or the like) having a payload released by cleavage of the binding site by the enzyme activity specific to the enzyme recognition site-linker binding site.
Y-T1 (III)
(wherein,
Y is selected from
-OL₁ (1)
or
-NH-L₂ (2),
L₁ is a partial structure of saccharides,
here, the partial structure of the saccharides is a structure obtained by removing one hydroxyl group from the saccharide, and
L₂ is a monovalent substituent containing an amino acid residue or an oligopeptide residue; and
T1 is a linker.)

25. Use of an enzyme recognition site-linker binding site represented by General Formula (III),
wherein the enzyme recognition site has an enzyme activity specific to a cancer cell, and
the enzyme recognition site-linker binding site is used for preventing prodrug activation or antibody or ligand binding until the enzyme recognition site-linker binding site is cleaved by enzyme activity specific to the binding site.

26. Use of an enzyme recognition site-linker binding site represented by General Formula (III),
wherein the enzyme recognition site has an enzyme activity specific to a cancer cell, and
the enzyme recognition site-linker binding site is used for activating an antibody or a ligand having a payload that is released or activated by cleaving the enzyme recognition site-linker binding site by the enzyme activity specific to the binding site in a target cell or tissue overexpressing an oncogene.

27. Use of an enzyme recognition site-linker binding site represented by General Formula (III):
wherein the enzyme recognition site has an enzyme activity specific to a cancer cell, and
the enzyme recognition site-linker binding site is used for targeting cancer.
